(19) Europäisches Patentamt
European Patent Office
Office européen des brevets

(11) EP 4 186 905 A1

(12) **EUROPEAN PATENT APPLICATION**
published in accordance with Art. 153(4) EPC

(43) Date of publication:
**31.05.2023 Bulletin 2023/22**

(21) Application number: **21842091.7**

(22) Date of filing: **13.07.2021**

(51) International Patent Classification (IPC):
***C07D 487/04*** (2006.01) ***C07D 519/00*** (2006.01)
***C07D 471/04*** (2006.01) ***A61K 31/4985*** (2006.01)
***A61K 31/437*** (2006.01) ***A61K 31/519*** (2006.01)
***A61P 19/02*** (2006.01) ***A61P 37/08*** (2006.01)
***A61P 37/06*** (2006.01) ***A61P 1/00*** (2006.01)
***A61P 1/04*** (2006.01) ***A61P 11/06*** (2006.01)
***A61P 17/00*** (2006.01) ***A61P 7/04*** (2006.01)
***A61P 25/28*** (2006.01) ***A61P 9/00*** (2006.01)
***A61P 11/00*** (2006.01) ***A61P 17/06*** (2006.01)
***A61P 35/00*** (2006.01) ***A61P 35/02*** (2006.01)

(52) Cooperative Patent Classification (CPC):
**A61K 31/437; A61K 31/4985; A61K 31/519; A61P 1/00; A61P 1/04; A61P 7/04; A61P 9/00; A61P 11/00; A61P 11/06; A61P 17/00; A61P 17/06; A61P 19/02; A61P 25/28; A61P 35/00; A61P 35/02;** (Cont.)

(86) International application number:
**PCT/CN2021/105961**

(87) International publication number:
**WO 2022/012510 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2020 CN 202010679776**
**25.11.2020 CN 202011337022**

(71) Applicants:
- **Chengdu Hyperway Pharmaceuticals Co., Ltd.**
  **Chengdu, Sichuan 610041 (CN)**
- **Shenzhen Hyperway Pharmaceuticals Co., Ltd.**
  **Shenzhen, Guangdong 518116 (CN)**

(72) Inventors:
- **YUE, Chunchao**
  **Chengdu, Sichuan 610041 (CN)**
- **LIU, Guanfeng**
  **Chengdu, Sichuan 610041 (CN)**
- **LI, Shai**
  **Chengdu, Sichuan 610041 (CN)**
- **LI, Jing**
  **Chengdu, Sichuan 610041 (CN)**
- **CHEN, Gang**
  **Chengdu, Sichuan 610041 (CN)**
- **HE, Yangtong**
  **Chengdu, Sichuan 610041 (CN)**
- **ZHANG, Rui**
  **Chengdu, Sichuan 610041 (CN)**
- **YUAN, Chenguang**
  **Chengdu, Sichuan 610041 (CN)**
- **LI, Yingfu**
  **Chengdu, Sichuan 610041 (CN)**

(74) Representative: **Müller, Christian Stefan Gerd**
**ZSP Patentanwälte PartG mbB**
**Hansastraße 32**
**80686 München (DE)**

(54) **COMPOUND SERVING AS BTK INHIBITOR, PREPARATION METHOD THEREFOR, AND USE THEREOF**

(57) A compound serving as a BTK inhibitor, a preparation method therefor, and a use thereof. The compound has a structure represented by formula I, or a tautomer, a mesomer, a racemate, an enantiomer, a diastereomer or a mixture thereof, or a pharmaceutically acceptable hydrate, solvent, or salt thereof, wherein $A_1$, $A_2$,

$A_3$, $A_4$, $A_5$ and $A_6$ are each independently selected from C-$R_5$ and N, and at least one of those is N; and M is selected from substituted or unsubstituted saturated or heterosaturated hydrocarbyl, substituted or unsubstituted unsaturated cyclic group or heterocyclyl, and substituted or unsubstituted monocyclic, bicyclic, or tricyclic aryl or heteroaryl. The provided BTK protein kinase inhibitor has strong inhibition for wild BTK and mutated BTK (C481S), has good pharmacokinetic properties, and has good application prospects.

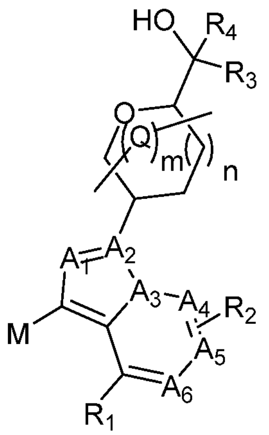

Formula I

(52) Cooperative Patent Classification (CPC): (Cont.)
**A61P 37/06; A61P 37/08; C07D 471/04; C07D 487/04; C07D 519/00;** Y02P 20/55

## Description

This application claims the priorities of Chinese Patent Application No. 202010679776.6, filed with the China National Intellectual Property Administration on July 15, 2020, and titled with "COMPOUND SERVING AS BTK INHIBITOR, PREPARATION METHOD THEREFOR, AND USE THEREOF", and Chinese Patent Application No. 202011337022.9, filed with the China National Intellectual Property Administration on November 25, 2020, and titled with "COMPOUND SERVING AS BTK INHIBITOR, PREPARATION METHOD THEREFOR, AND USE THEREOF", which are hereby incorporated by reference in entirety.

## FIELD

The present disclosure relates to the technical field of medicine, and specifically relates to a compound as a BTK protein kinase inhibitor, a preparation method and application thereof.

## BACKGROUND

Bruton's tyrosine kinase (BTK), a member of the Tec family of non-receptor protein tyrosine kinases, is mainly expressed in hematopoietic stem cells. The Tec family is the second largest family after the Src family among human non-receptor kinases, including BTK, BMX (etk), ITK, TEC and TXK (RLK) as main members. In 1993, BTK was identified as a deficient protein in human X-linked agammaglobulinemia (XLA). BTK is a key regulator of the B cell receptor (BCR) signal transduction pathway, plays an important role in the activation, proliferation, differentiation and survival of B cells, and is closely related to B cell tumors and autoimmune diseases.

The structure of BTK contains five main domains, namely the PH (Pleckstrin homology) domain, TH (Tec homology) domain, SH3 (Src homology 3) domain, SH2 (Src homology 2) domain and SH1 (Src homology 1) domain. BTK is activated (phosphorylated) initially in the activation loop in the SH1 domain, and further in the SH2 and SH3 domains containing the major autophosphorylation sites. These SH domains also contain the nuclear localization signal (NLS) and nuclear export sequence (NES) required for nucleocytoplasmic shuttling of BTK.

BTK plays an irreplaceable role in the generation of B lymphocytes, as it can control the development and differentiation of B cells by activating positive regulatory factors and differentiation factors of the cell cycle, and can also control the survival and proliferation of B cells by regulating the expression of pro-apoptotic and anti-apoptotic proteins. Sustained activation of BTK is a prerequisite for the development of chronic lymphocytic leukemia (CLL), and abnormal BCR-BTK signaling will promote the survival of the activated B-cell subset of diffuse large B-cell lymphoma (DLBCL). BTK's gain-of-function mutations have also been confirmed in colorectal cancer, acute lymphoblastic leukemia (ALL), and chronic myeloid leukemia (CML). It can be seen that the abnormal activation of BTK-dependent pathways has been proved to be closely related to the occurrence and development of various tumors.

The currently approved irreversible BTK inhibitors such as Ibrutinib, acalabrutinib, and Zanubrutinib, achieve the purpose of treating related diseases by selectively binding to the cysteine residue (Cys-481) of BTK and forming an irreversible covalent bond to inhibit the activity of BTK. However, some cancer patients would develop drug resistance to the first-generation BTK inhibitors, thus emerging new unmet clinical needs. The BTK-C481S mutation, as demonstrated by studies, is dominant mechanism related to such drug resistance. Therefore, drugs capable of targeting and inhibiting the BTK-C481S mutation could provide new treatment options, for example, ARQ-531, which is an orally bioavailable, potent, and reversible dual inhibitor of wild-type and C481S-mutated BTK, and has demonstrated effectiveness for patients with C481 S-mutated BTK as indicated by the initial clinical results of ARQ-531.

## SUMMARY

In view of this, the present application provides a compound as a BTK inhibitor and a preparation method and use thereof. The compound provided by the present disclosure can be used as a BTK protein kinase inhibitor with the characteristics of high inhibitory activity and the like.

The present disclosure provides a compound, having a structure represented by formula I, or a tautomer, a mesomer, a racemate, an enantiomer, a diastereomer or a mixture thereof, or a pharmaceutically acceptable hydrate, solvate or salt thereof,

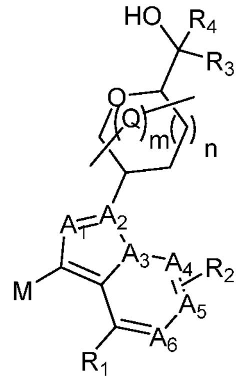

Formula I

wherein $A_1$, $A_2$, $A_3$, $A_4$, $A_5$ and $A_6$ are each independently selected from the group consisting of $C-R_5$ and nitrogen (N), and at least one of $A_1$, $A_2$, $A_3$, $A_4$, $A_5$ and $A_6$ is N;

M is selected from the group consisting of substituted or unsubstituted saturated hydrocarbyl or heterosaturated hydrocarbyl, substituted or unsubstituted unsaturated cyclyl or heterocyclyl, and substituted or unsubstituted monocyclic, bicyclic or tricyclic aryl or heteroaryl; wherein the substituent is each independently selected from the group consisting of aryl or heteroaryl, alkyl or heteroalkyl, cycloalkyl or heterocycloalkyl, unsaturated cyclyl or heterocyclyl, phenoxy, halogen, hydroxyl, cyano, amino, an ester group, nitro, mercapto, amido, sulfonyl, phosphoryl, alkyl phosphoryl, alkyl sulfone, and alkyl sulfoxide that are substituted by any group; further, the substituent is aryl or heteroaryl substituted by any group, more preferably phenyl substituted by any group;

Q is selected from the group consisting of $C-R_{10}R_{11}$, $N-R_{12}$, oxygen (O), sulfur (S), S(O), and $S(O)_2$;

$R_1$, $R_2$, $R_3$, $R_4$, $R_5$, $R_{10}$, $R_{11}$ and $R_{12}$ are each independently selected from the group consisting of hydrogen, deuterium, halogen, substituted or unsubstituted alkyl or heteroalkyl, substituted or unsubstituted cycloalkyl or heterocycloalkyl, substituted or unsubstituted unsaturated cyclyl or heterocyclyl, substituted or unsubstituted aryl or heteroaryl, hydroxyl, cyano, amino, an ester group, nitro, mercapto, amido, sulfonyl, phosphoryl, alkyl phosphoryl, alkyl sulfone and alkyl sulfoxide; or $R_3$, $R_4$ and the carbon atom connecting therewith together form substituted or unsubstituted C3-C10 cycloalkyl or heterocycloalkyl; wherein the substituent is selected from the group consisting of halogen, hydroxyl, cyano, amino, mercapto, nitro, carboxyl, hydroxylamino, alkyl, cycloalkyl, heteroalkyl, heterocycloalkyl, aryl, heteroaryl, an ester group, acyl, amido, sulfonyl and phosphoryl;

m is an integer selected from 0 to 6; n is an integer selected from 0 to 3.

The compound described in the present disclosure is in any form with the structure of formula I, including tautomers, mesomers, racemates, enantiomers, diastereomers or mixtures thereof, pharmaceutical acceptable hydrates, solvates or salts etc.

In this application, regarding "selected from the group consisting of", the numbers in the group are generally a parallel relationship of "or". In the structure represented by formula I, three or four of $A_1$, $A_2$, $A_3$, $A_4$, $A_5$ and $A_6$ are preferably N; the position of $R_2$ is not limited and is preferably at the para position of $R_1$. In this application, the substitution may be monosubstitution or polysubstitution (such as disubstitution and trisubstitution), and its specific substitution position is not limited. The unsubstituted saturated hydrocarbyl includes unsubstituted alkyl and unsubstituted cycloalkyl. The heterocyclyl or heteroaryl may have one or more carbon atoms therein replaced by heteroatom that is an atom other than carbon (C) such as oxygen, sulfur, nitrogen and phosphorus (P). In addition, the halogen includes fluorine (F), chlorine (Cl), bromine (Br) and the like, and preferably is fluorine or chlorine. The "C3-C10" indicates the number of carbon atoms is an integer from 3 to 10. Below, similar expressions will not be repeated.

In this application, the bridging atom is connected to the ring with a chemical bond to form a ring system (as shown in the following formula), which means that the bridging atom may be connected with any connectable C atom on the ring to form any spiro or bridged ring structure compound. For example, the following formula shows that a bridging atom Q may be connected to any C atom capable of connecting to the bridging atom (s) on the six-membered ring, to form a spiro compound when connected to a common C atom, e.g., when the bridging atoms are all connected to the #2 C atom or #3 C atom; or to form a bridged ring compound when connected to different C atoms, e.g., when the bridging atom(s) is connected to the #1 and #4 C atoms or the #2 and #4 C atoms;

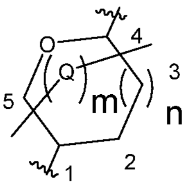

.

Preferably, provided is the compound having a structure represented by formula II, or a tautomer, a mesomer, a racemate, an enantiomer, a diastereomer or a mixture thereof, or a pharmaceutically acceptable hydrate, solvate or salt thereof:

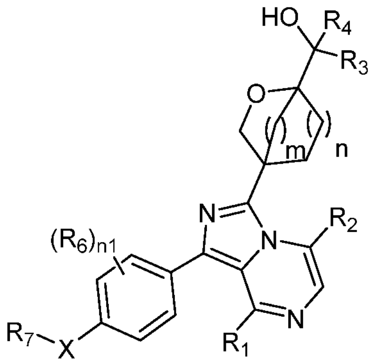

Formula II

wherein $R_1$ is selected from the group consisting of hydrogen, halogen, hydroxyl, cyano, amino, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; further, $R_1$ is selected from the group consisting of hydrogen, amino, methyl, ethyl, methoxy, cyano, trifluoromethyl, isopropyl and cyclopropyl; further, $R_1$ is selected from the group consisting of hydrogen (H), amino ($NH_2$) and methyl ($CH_3$).

$R_2$ is selected from the group consisting of hydrogen, halogen, hydroxyl, cyano, amino, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; further, $R_2$ is selected from the group consisting of hydrogen, fluorine, chlorine, bromine, methyl, ethyl, methoxy, cyano, trifluoromethyl, isopropyl and cyclopropyl; further, $R_2$ is selected from the group consisting of hydrogen, chlorine and methyl;

$R_3$ and $R_4$ are selected from the group consisting of hydrogen, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; or $R_3$, $R_4$ and the carbon atom connecting therewith together form substituted or unsubstituted C3-C6 cycloalkyl or heterocycloalkyl containing N or O atom;

further, $R_3$ and $R_4$ are selected from the group consisting of hydrogen, methyl, ethyl, isopropyl and cyclopropyl, or $R_3$, $R_4$ and the carbon atom connecting therewith together form cyclopropyl, azetidinyl, azacyclopentyl, azacyclohexyl, oxetanyl, oxacyclopentyl, or oxacyclohexyl;

$R_6$ is selected from the group consisting of hydrogen, halogen, hydroxyl, cyano, amino, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; further, $R_6$ is selected from the group consisting of hydrogen, halogen, cyano, substituted or unsubstituted C1-C3 alkyl, and substituted or unsubstituted C1-C3 alkoxy; further, $R_6$ is selected from the group consisting of hydrogen, fluorine, chlorine, bromine, trifluoromethyl, methyl, methoxy, trifluoromethoxy and difluoromethoxy; further, $R_6$ is hydrogen or fluorine.

m is selected from 0, 1, 2 or 3; n is selected from 0, 1, or 2; n1 is selected from 0, 1, 2, 3 or 4;

$R_7$ is selected from the group consisting of substituted or unsubstituted aryl, or substituted or unsubstituted pyridyl, wherein the substituent is independently selected from halogen, hydroxyl, amino, cyano, alkyl, heteroalkyl, cycloalkyl and heterocycloalkyl; further, the substituent is independently selected from the group consisting of fluorine, chlorine,

bromine, cyano, amino, C1-C3 alkyl, C1-C3 alkoxy, C3-C6 cycloalkyl and C3-C6 heterocycloalkyl; further, the substituent is independently selected from the group consisting of fluorine, chlorine, bromine, cyano, trifluoromethyl, trifluoromethoxy, difluoromethoxy, methoxy, deuterated methoxy, cyclopropyl, cyclopropylmethoxy, ethyl, isopropyl and isobutyl; wherein the number of the substituent is an integer between 0 and 5;

X is selected from the group consisting of

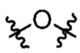, 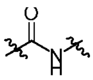, 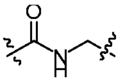, 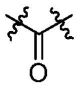, 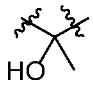,

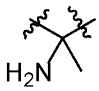, 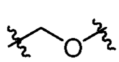, 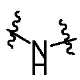, 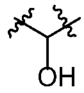, 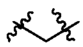

and other acceptable linking groups. In some embodiments, X is

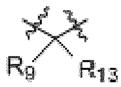,

wherein $R_9$ and $R_{13}$ are independently selected from the group consisting of hydrogen, halogen, hydroxyl, amino, cyano, C1-C3 alkyl, C1-C3 alkoxyl, C3-C6 cycloalkyl and C3-C6 heterocycloalkyl, or $R_9$, $R_{13}$ and the carbon atom connecting therewith together form substituted or unsubstituted C3-C6 cycloalkyl, or substituted or unsubstituted C3-C6 heterocycloalkyl containing N or O; further, $R_9$ and $R_{13}$ are independently selected from the group consisting of hydrogen, fluorine, chlorine, cyano, methyl, ethyl, isopropyl, cyclopropyl, trifluoromethyl and isobutyl, or $R_9$, $R_{13}$ and the carbon atom connecting therewith together form cyclopropyl; further, $R_9$ and $R_{13}$ are selected from the group consisting of hydrogen, fluorine, deuterium, chlorine, methyl, hydroxyl and amino. Specifically, X may be

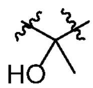, 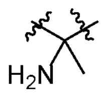,

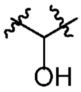, 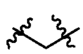, 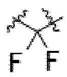, 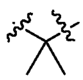, 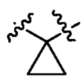, 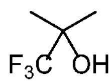, 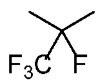, or 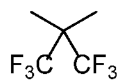.

The compounds containing

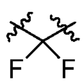, 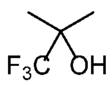, 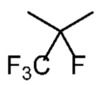, or 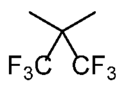

as X are preferably used as the brain-permeable BTK inhibitor or HER2 inhibitor. More preferably, $R_9$ and $R_{13}$ both are fluorine.

In some embodiments of the present application, provided is a compound having a structure represented by formula III or formula IV, or a tautomer, a mesomer, a racemate, an enantiomer, a diastereomer or a mixture thereof, or a pharmaceutically acceptable hydrate, solvate or salt thereof,

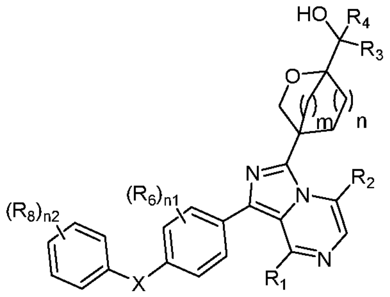 Formula III 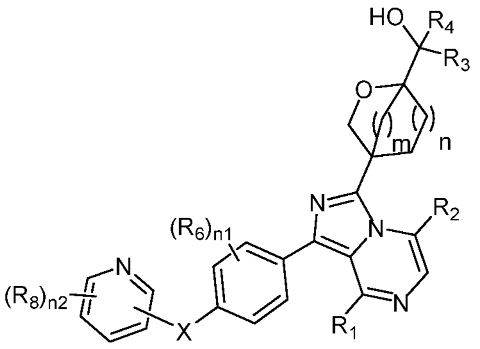 Formula IV

wherein, $R_1$, $R_2$, $R_3$, $R_4$, $R_6$ and X have a structure as described above; m, n, and n1 are also as described above; for example, X is

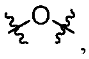, 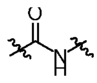, 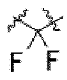,

and the like.

In formulas III-formula IV, n2 is selected from 0, 1, 2, 3 or 4;
$R_8$ is independently selected from the group consisting of hydrogen, halogen, hydroxyl, amino, cyano, alkyl, heteroalkyl, cycloalkyl and heterocycloalkyl; further, $R_8$ is independently selected from the group consisting of hydrogen, fluorine, chlorine, bromine, cyano, amino, C1-C3 alkyl, C1-C3 alkoxy, C3-C6 cycloalkyl and C3-C6 heterocycloalkyl; further, $R_8$ is independently selected from the group consisting of hydrogen, fluorine, chlorine, bromine, cyano, trifluoromethyl, trifluoromethoxy, difluoromethoxy, methoxy, deuterated methoxy, cyclopropyl, cyclopropylmethoxy, ethyl, isopropyl and isobutyl; wherein the number of the substituent is an integer between 0 and 5 (including endpoints); multiple substituents may be the same or different; in formula IV, the substituted or unsubstituted pyridyl is connected to a non-limiting position, such as the adjacent position of N.

In some embodiments of the present application, the N-containing fused rings in formulas II-IV may be replaced by

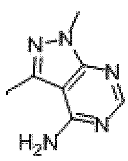 or 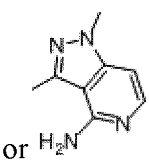,

wherein the single bonds at both ends are connecting bonds. In addition, in the X structure of formulas II-IV, a single bond with a curved line represents a connecting bond. The position of $R_6$ is not limited; n1 is preferably 0, 1 or 2. When n = 0, it is a five-membered ring; when n is 1, it is a six-membered ring, and so on.

Preferably, in formulas II-IV, $R_1$ is amino, $R_2$ is hydrogen or chlorine, $R_6$ is hydrogen or monosubstituted fluorine; $R_7$ in formula II is substituted or unsubstituted phenyl or pyridyl; X is primarily an ether or amide structure and the nitrogen of the amide is connected to $R_7$. Preferably, n is 0 or 1, m is 0 or 2, and both $R_3$ and $R_4$ are hydrogen, methyl or form a cyclopropyl with the carbon atoms connecting them.

Specifically, the structure of the compound described in this application is selected from one of the following (wherein there is a methyl group in the form of a single bond at one end, as shown in formula 5 of compound 5). The compounds having a structure represented by formula 2, 5, 34, 42. 89, 100, 101, 103, 106, 109, 111, 114, 116, 118, 121, 125, 130, 145, 146, 152 or 155 are preferred, as they have better performances.

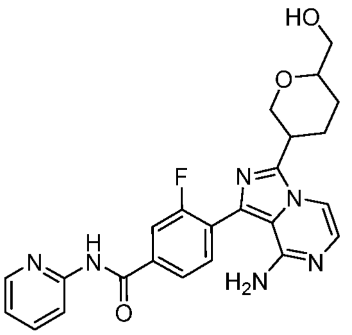 1,

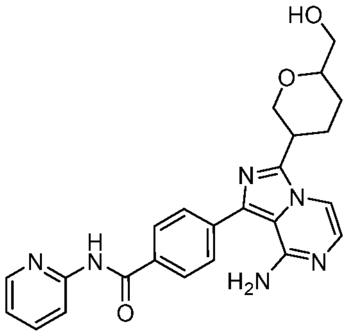 2,

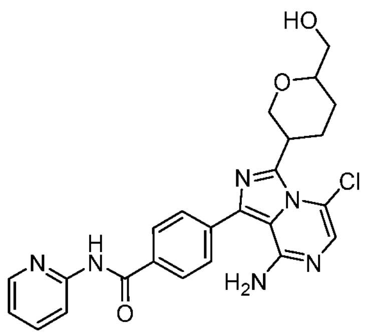 3,

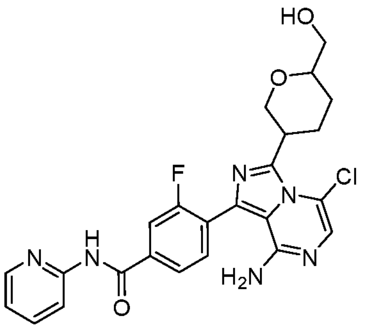 4,

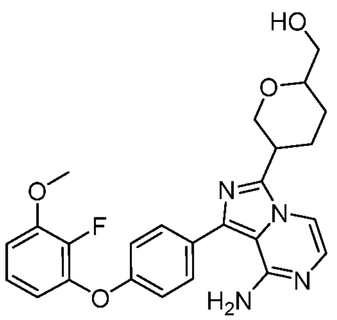 5,

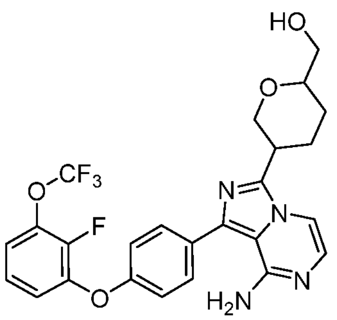 6,

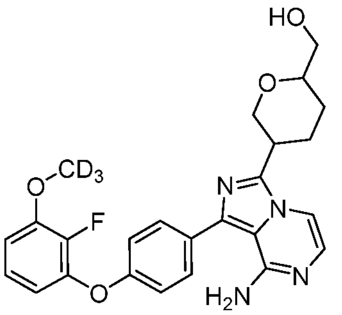 7,

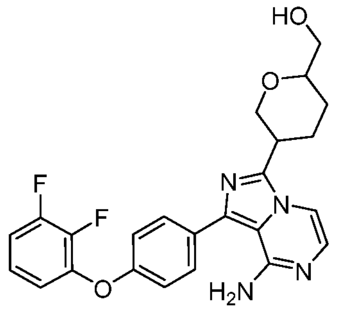 8,

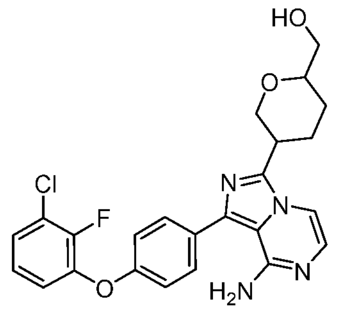 9,

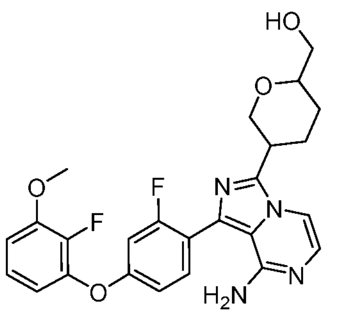 10,

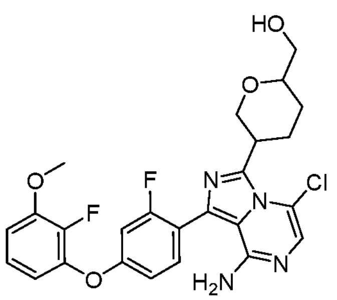 11,

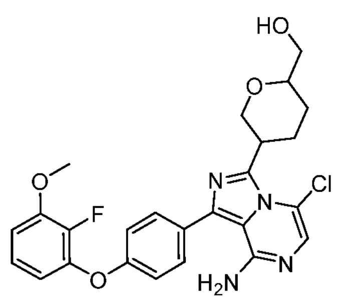 12,

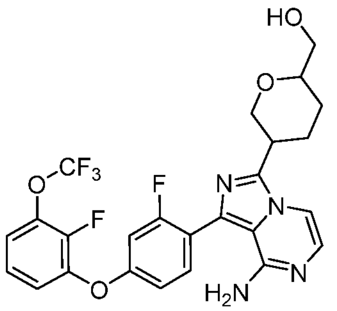 13,

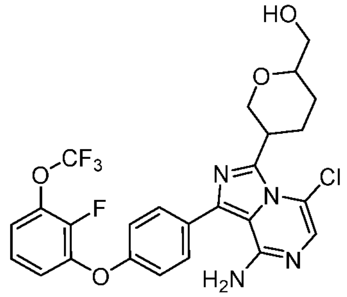 14,

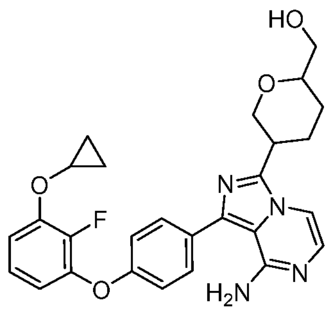 15,

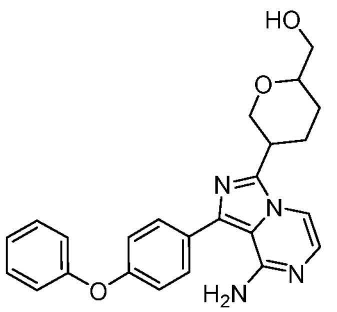 16,

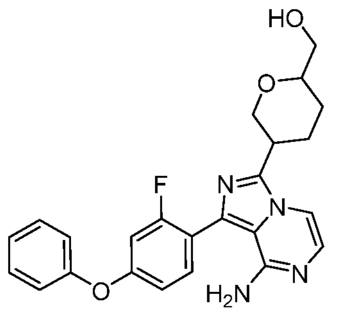 17,

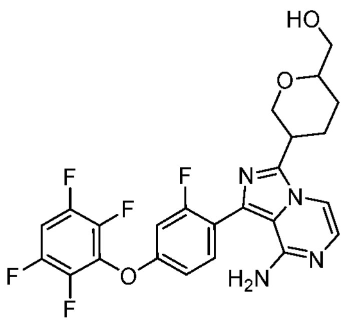 18,

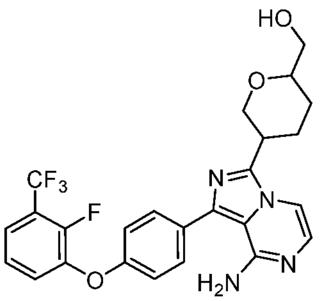 19,

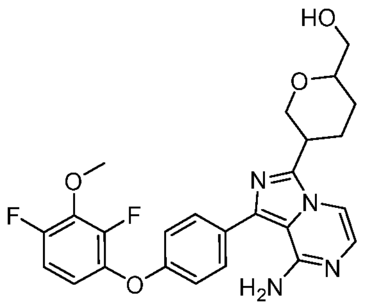 20,

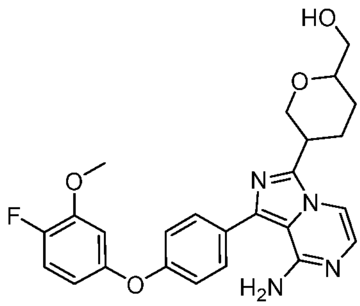 21,

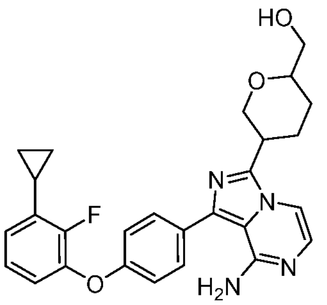 22,

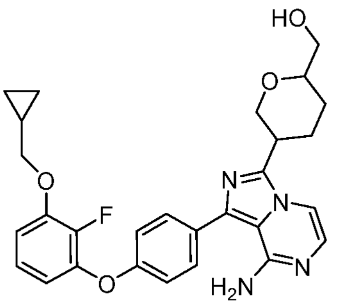 23,

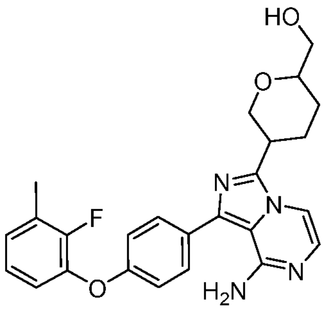 24,

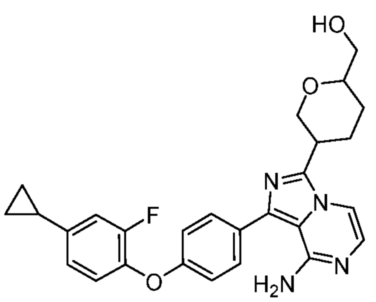 25,

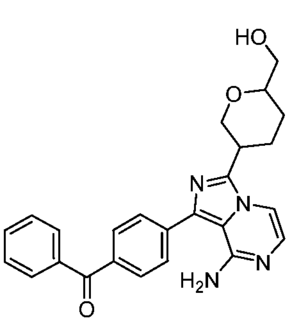 26,

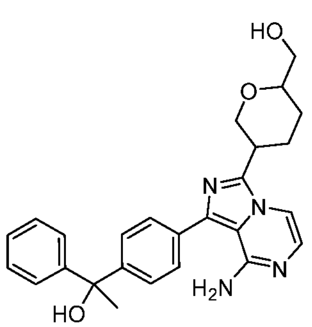 27,

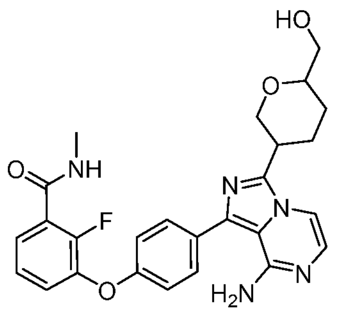 28, 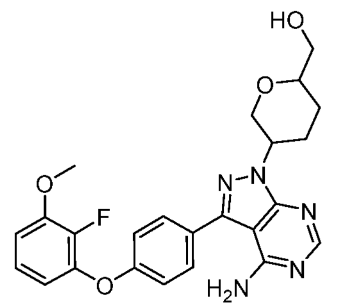 29, 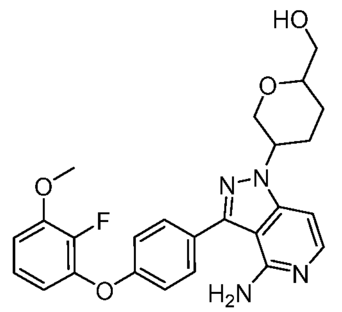 30,

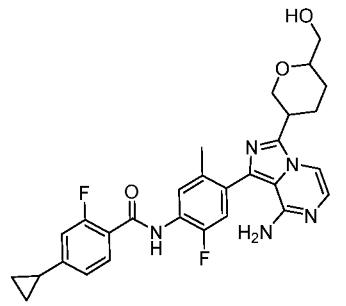 31, 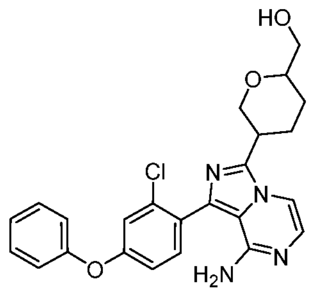 32, 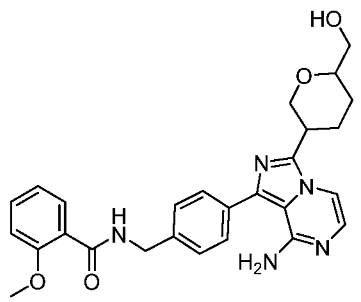 33,

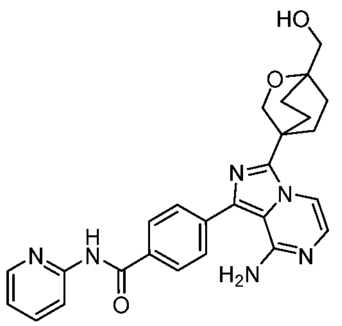 34, 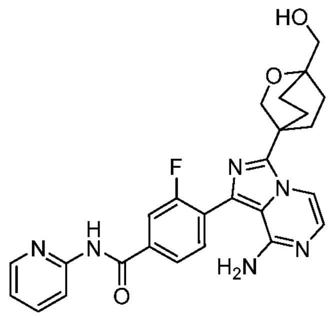 35, 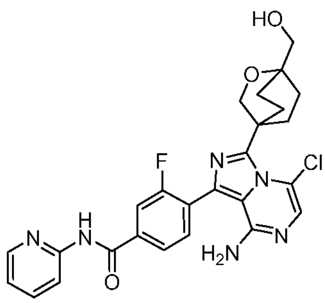 36,

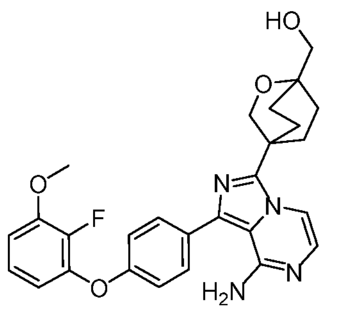 37, 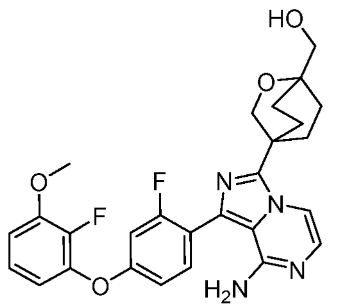 38, 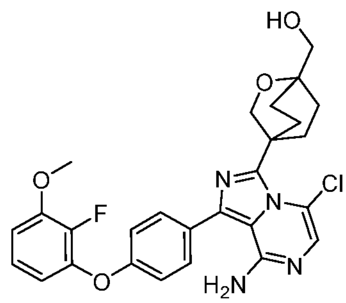 39,

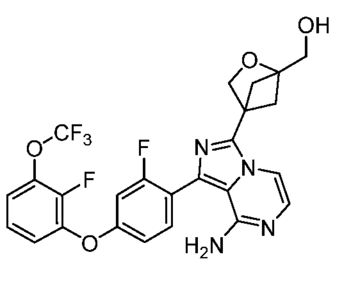 40, 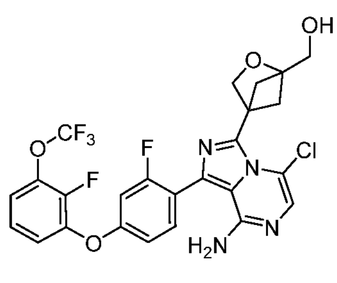 41, 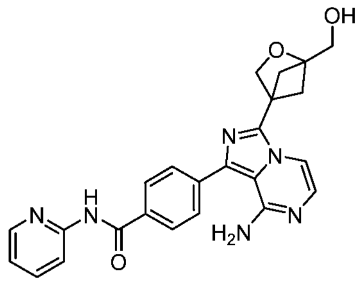 42,

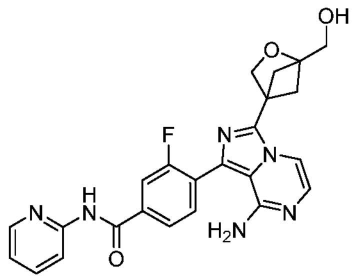 43, 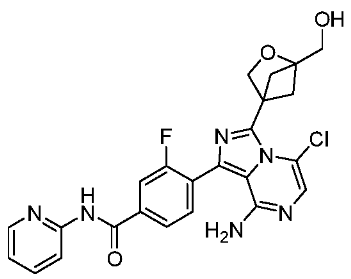 44, 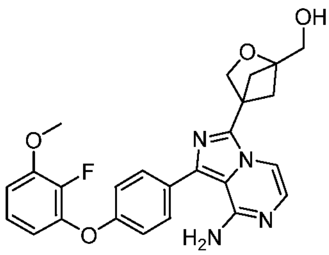 45,

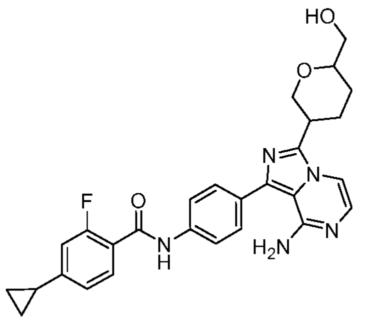 46, 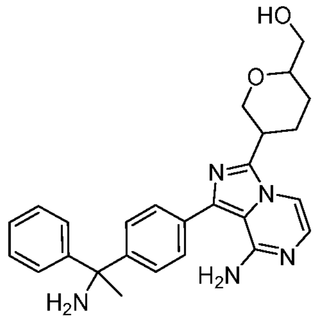 47, 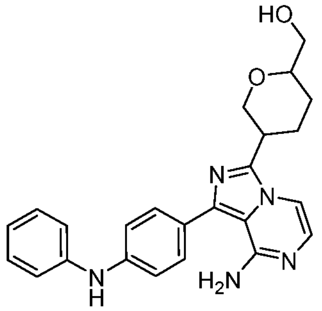 48,

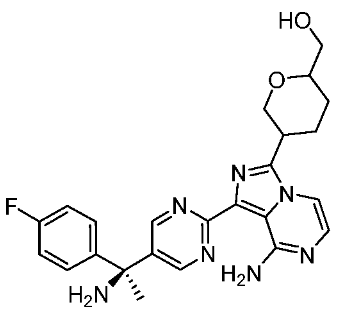 49, 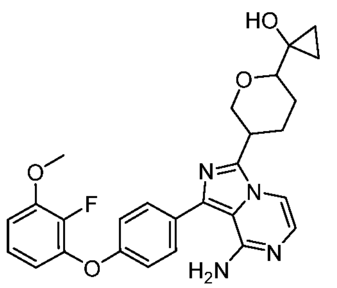 50, 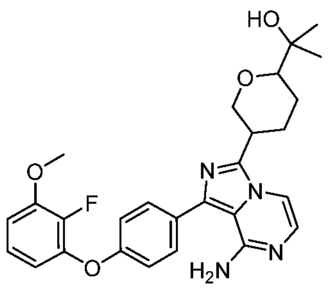 51,

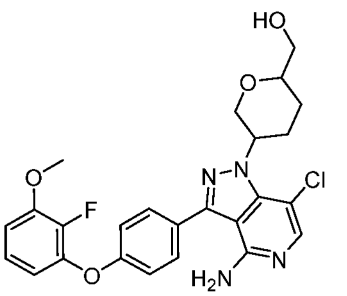 52, 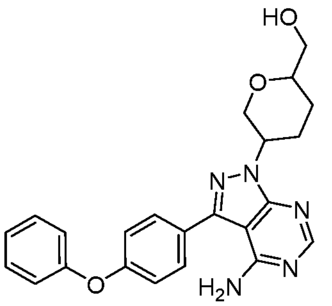 53, 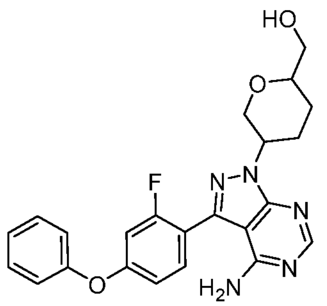 54,

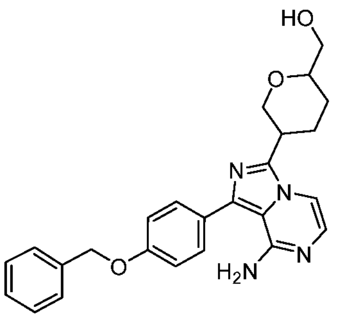 55, 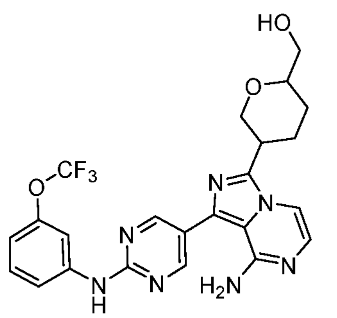 56, 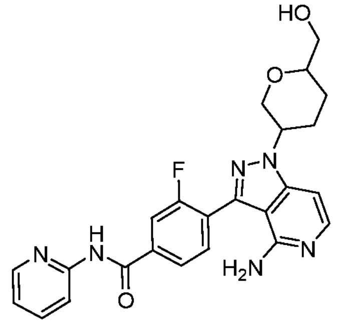 57,

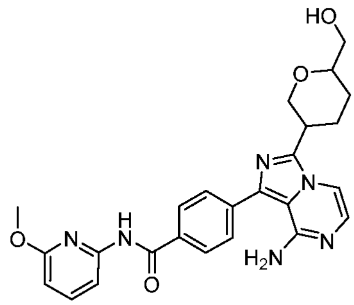 58, 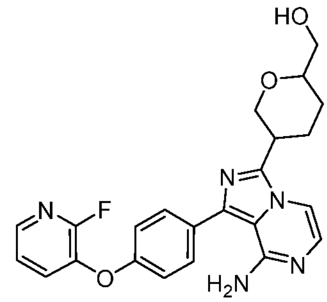 59, 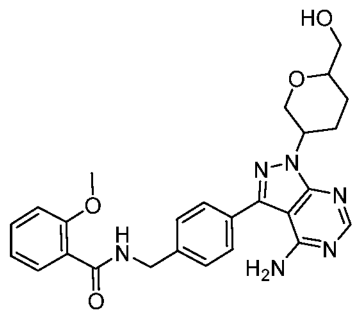 60,

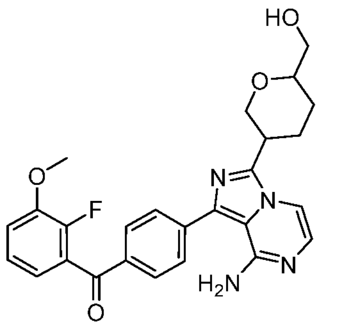 61, 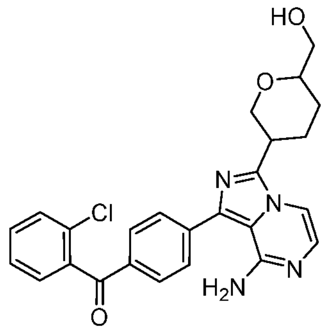 62, 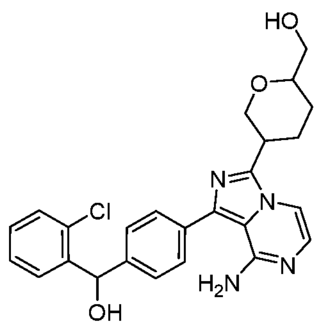 63,

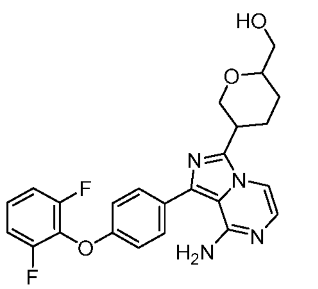 64, 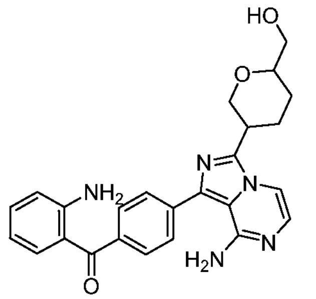 65, 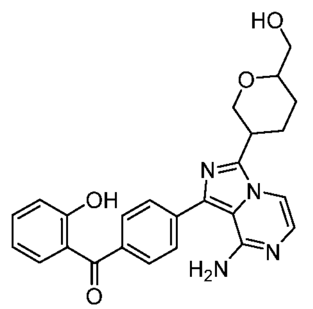 66,

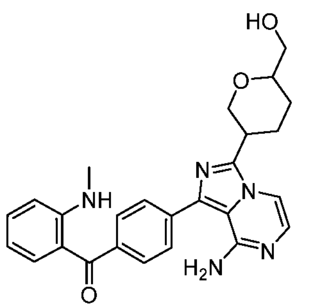 67, 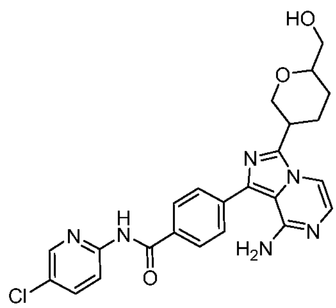 68, 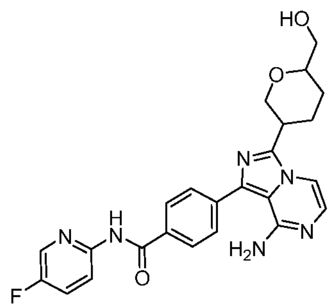 69,

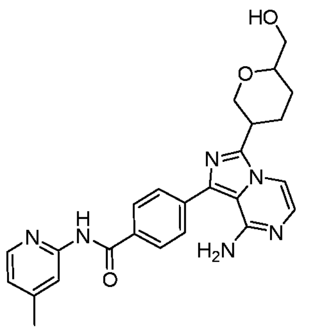 70, 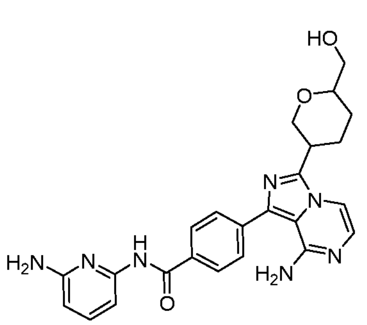 71, 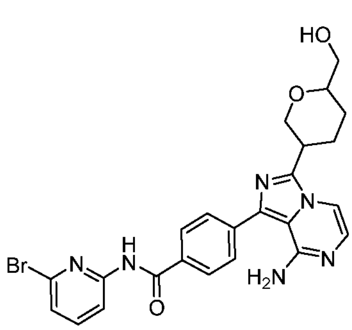 72,

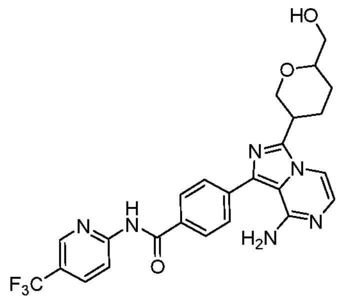 73, 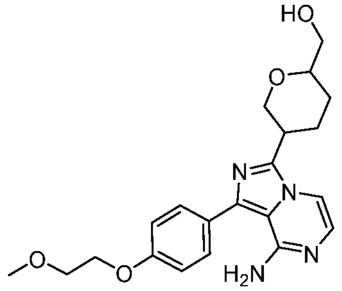 74, 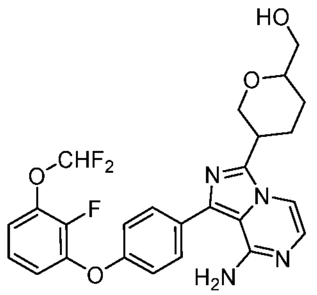 75,

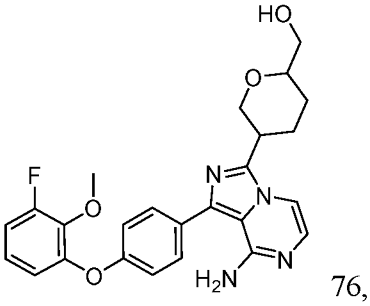 76, 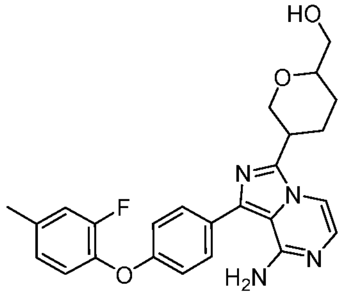 77, 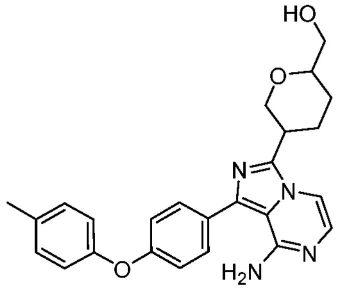 78,

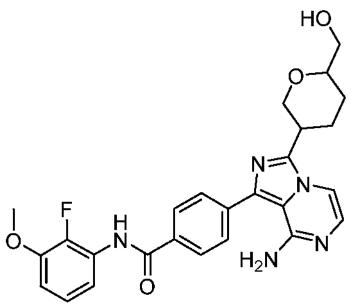 79, 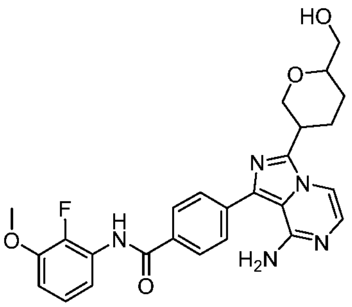 80, 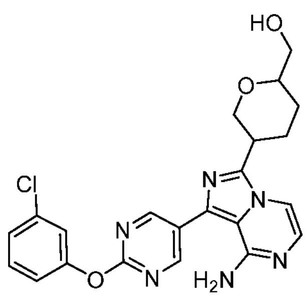 81,

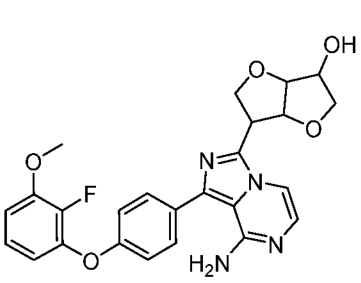 82, 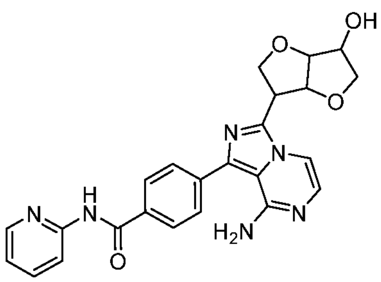 83, 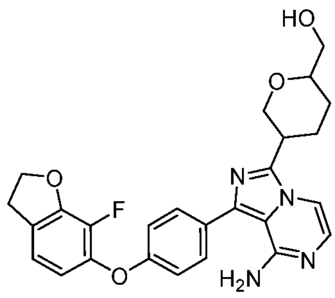 84,

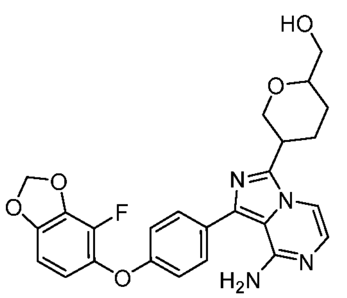 85, 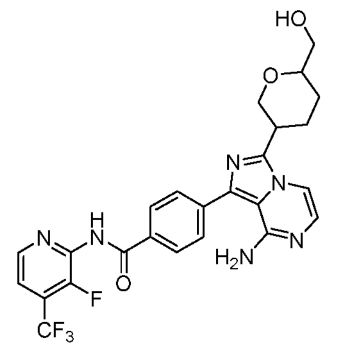 86, 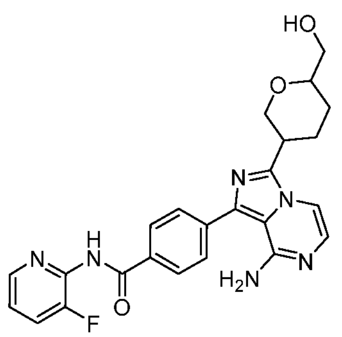 87,

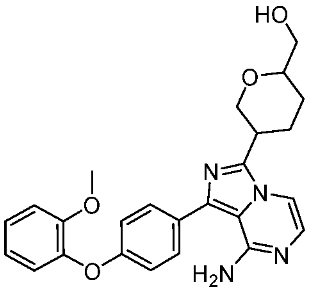 88, 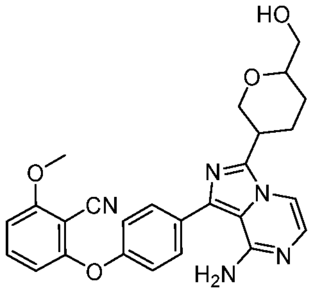 89, 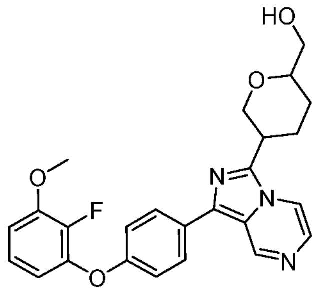 90,

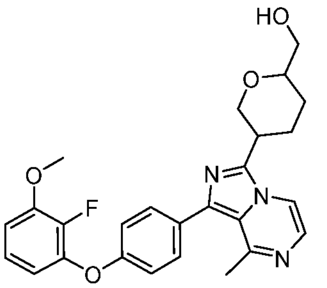 91, 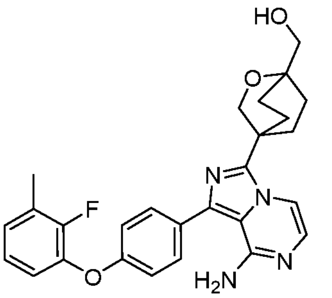 92, 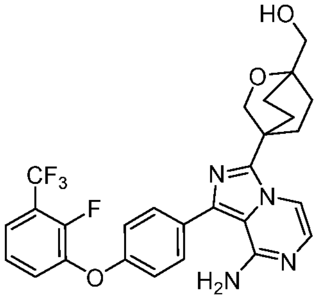 93,

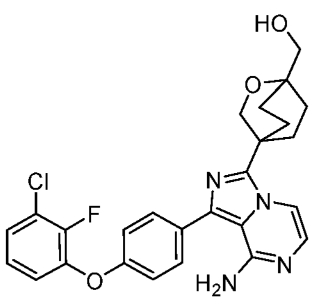 94, 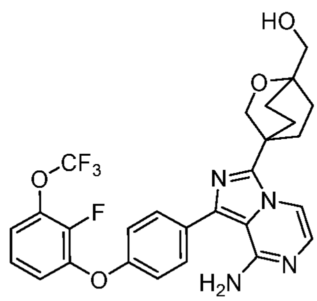 95, 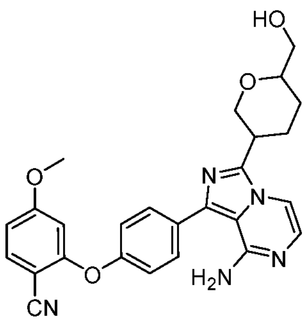 96,

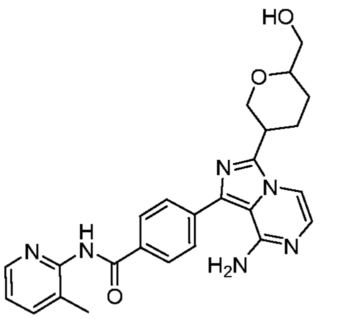 97, 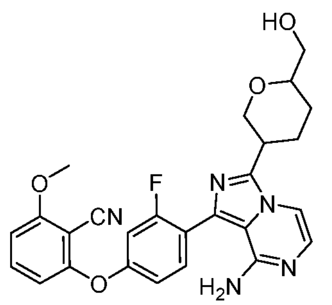 98, 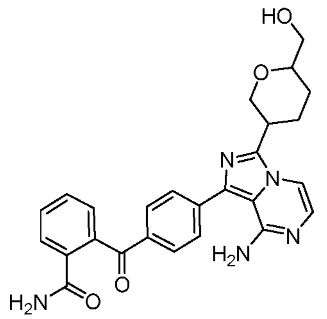 99,

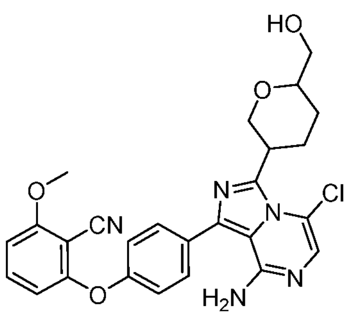 100, 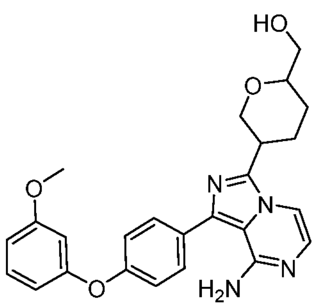 101, 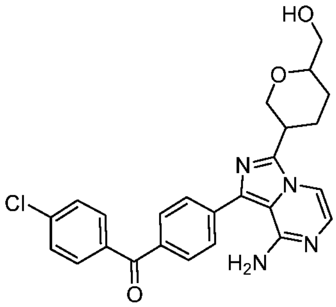 102,

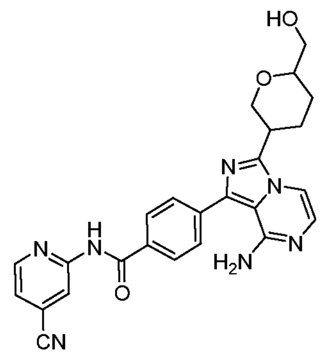
103,
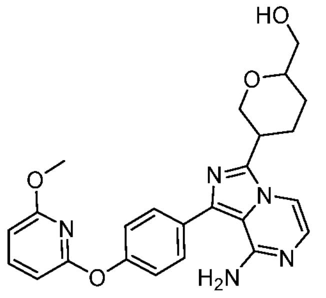
104,
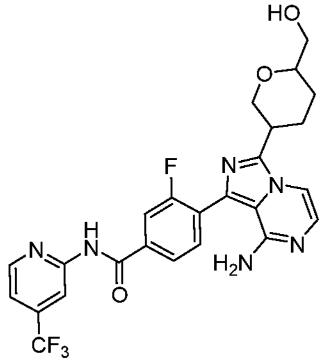
105,

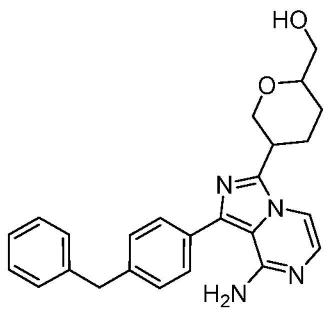
106,
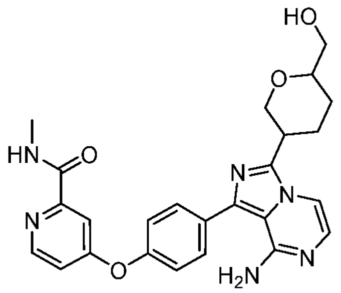
107,
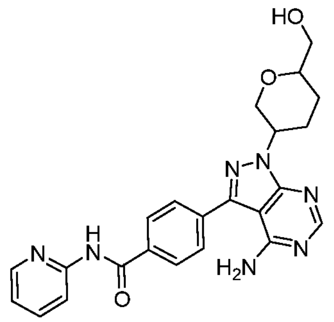
108,

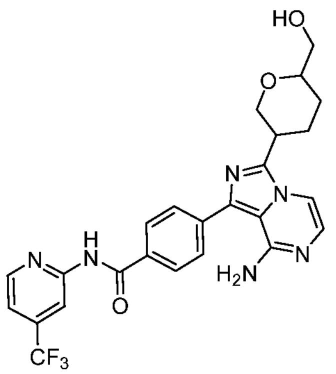
109,
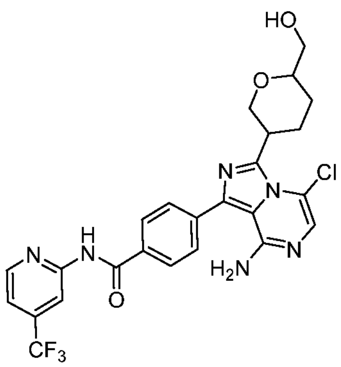
110,
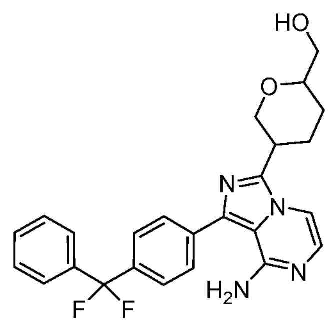
111,

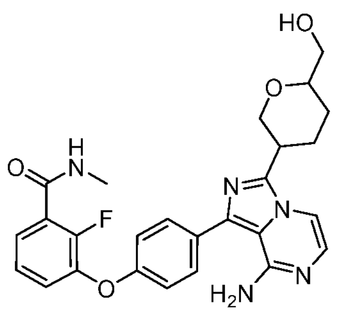
112,
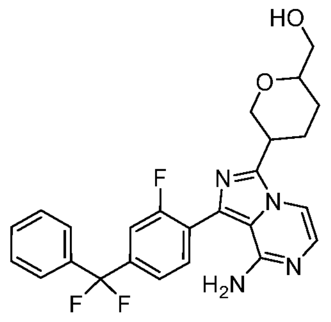
113,
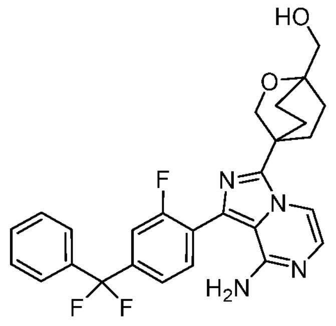
114,

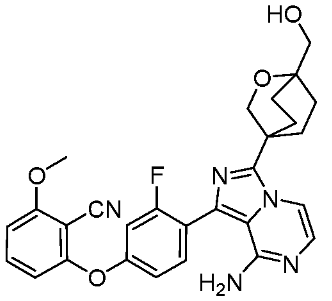 115, 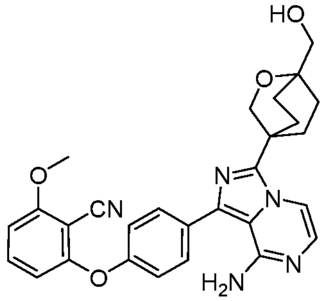 116, 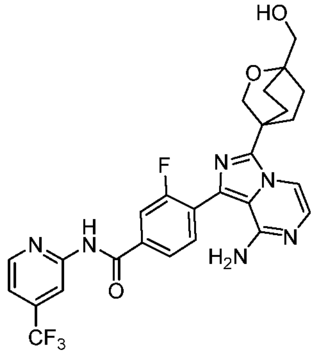 117,

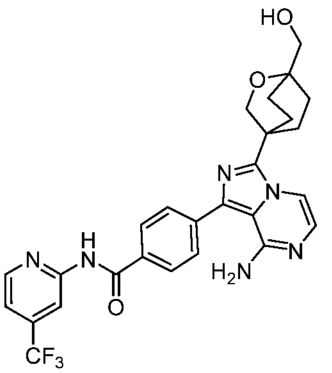 118, 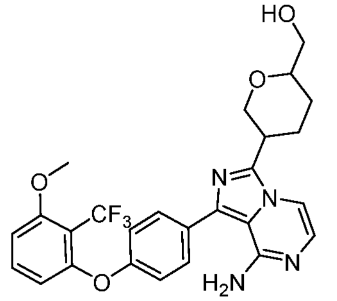 119, 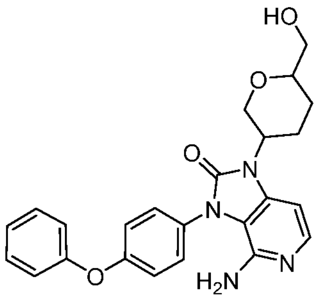 120,

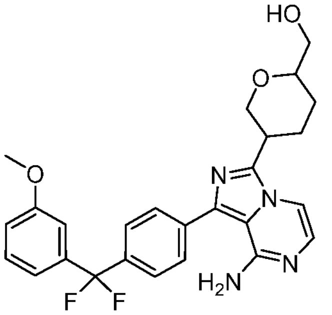 121, 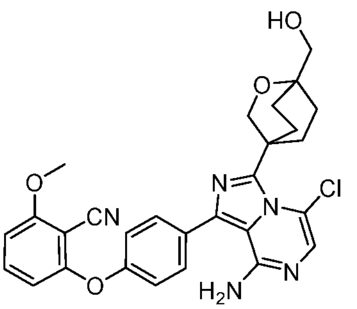 122, 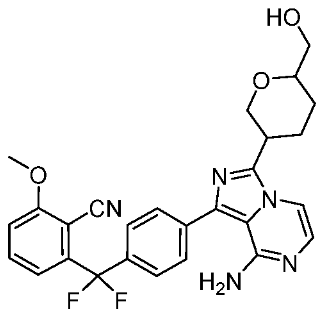 123,

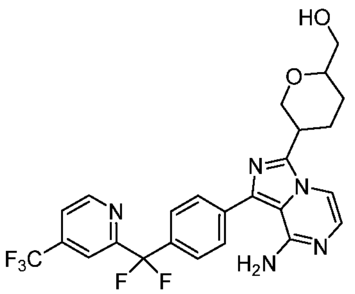 124, 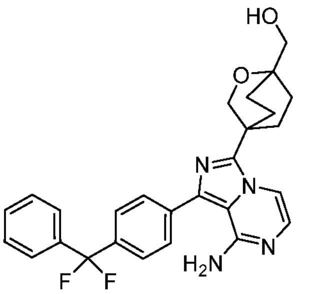 125, 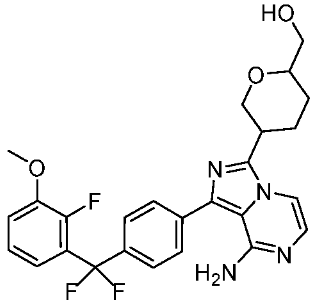 126,

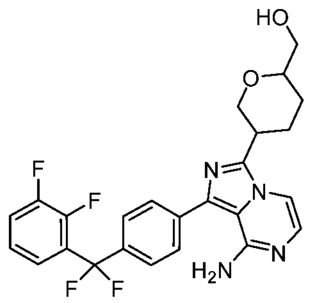 127, 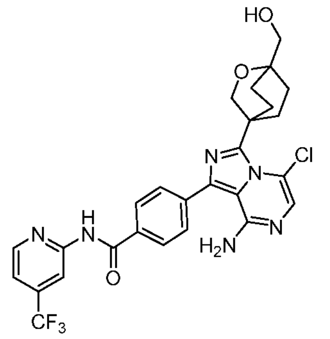 128, 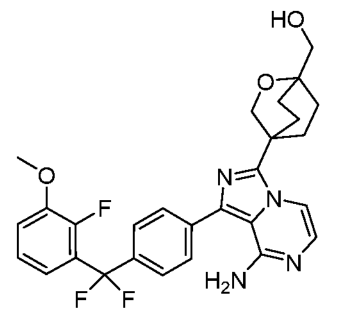 129,

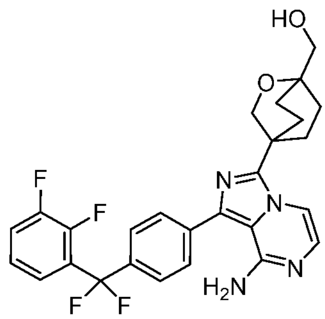 130, 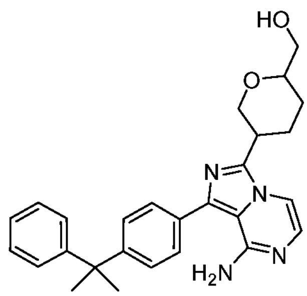 131, 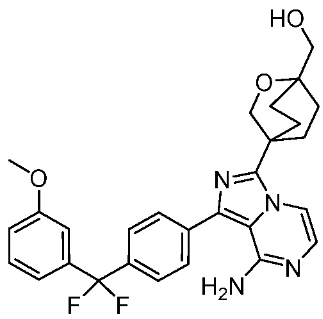 132,

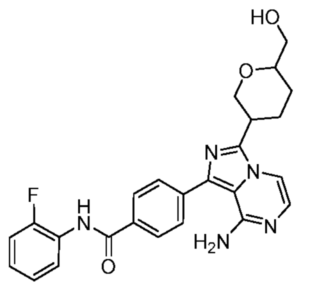 133, 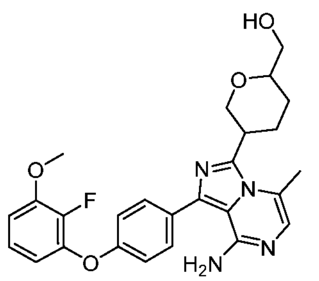 134, 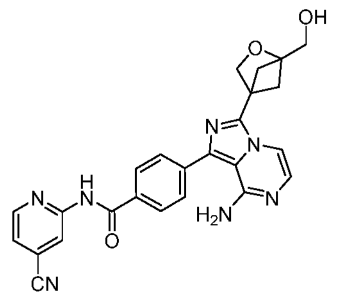 135,

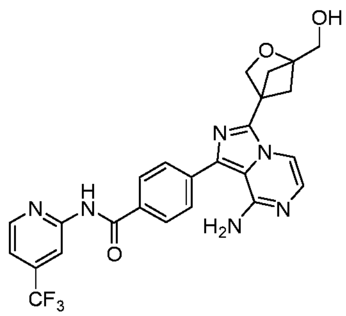 136, 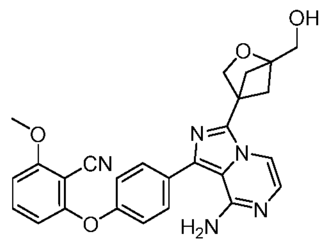 137, 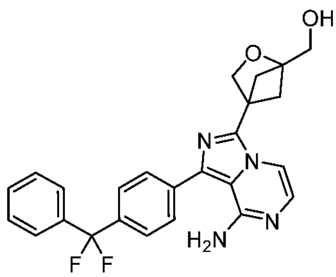 138,

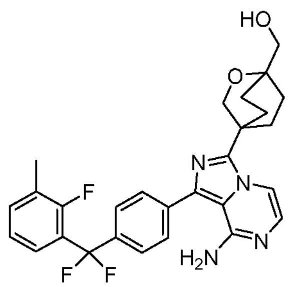 139, 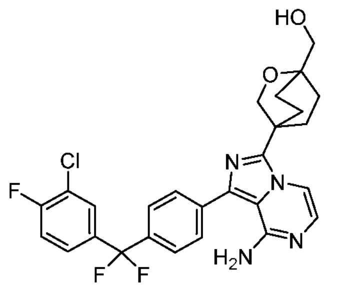 140, 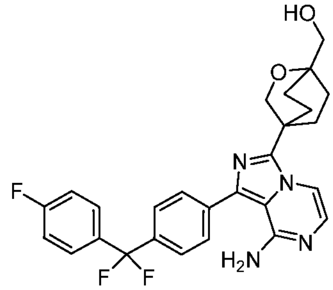 141,

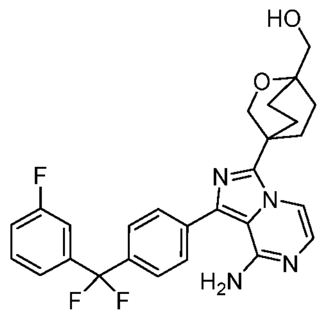 142, 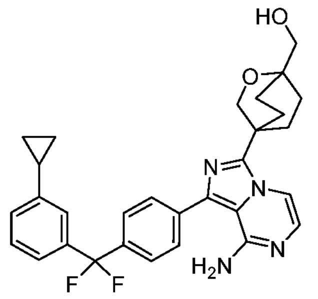 143, 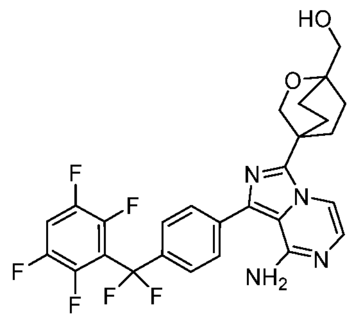 144,

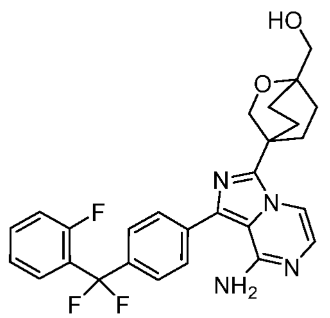 145, 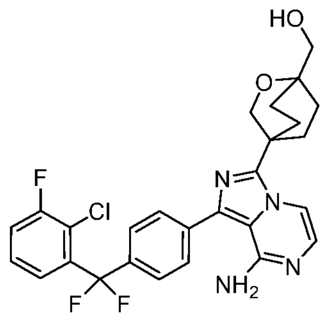 146, 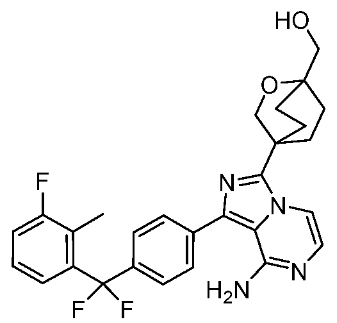 147,

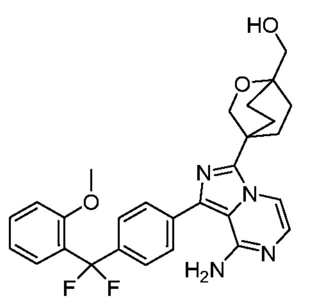 148, 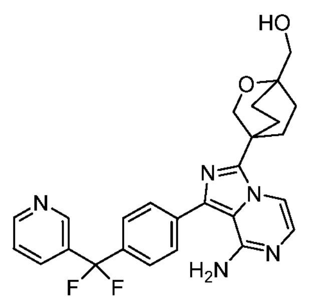 149, 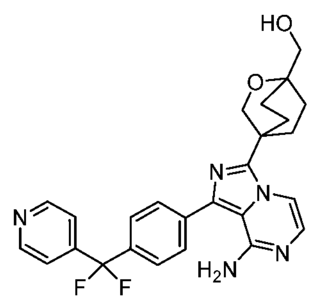 150,

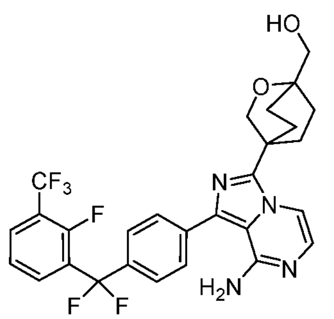 151, 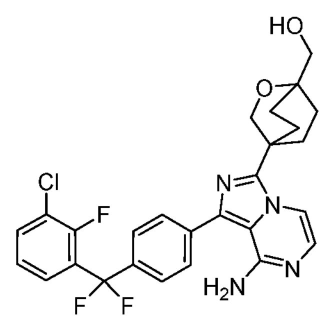 152, 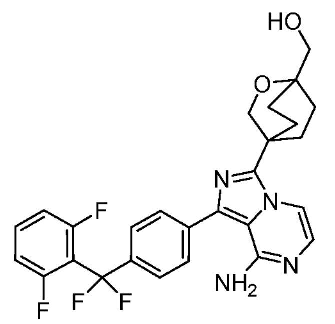 153,

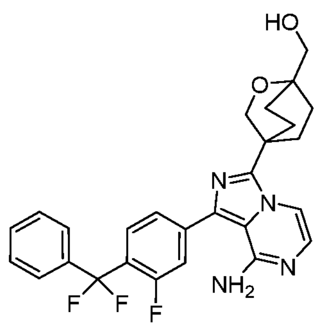 154, 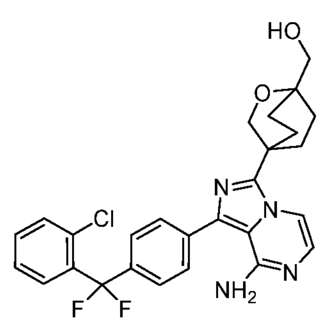 155, 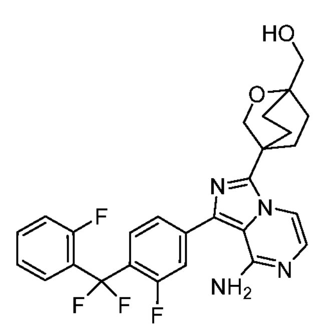 156,

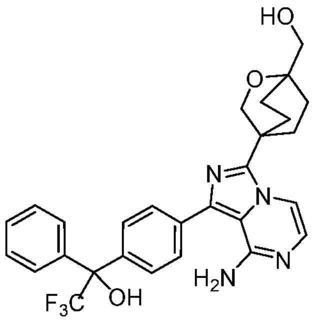 157, 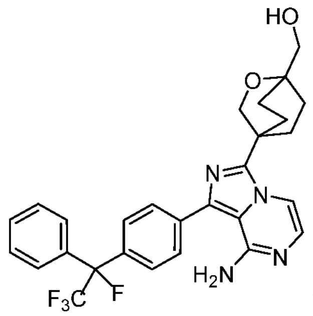 158, 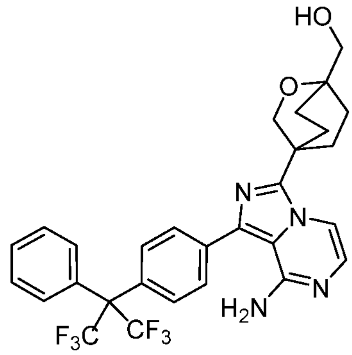 159.

The present disclosure provides a pharmaceutical composition containing an active ingredient selected from the group consisting of the aforementioned compounds or the stereoisomer, solvate, hydrate, pharmaceutically acceptable salt or cocrystal thereof, and a combination thereof. In addition, in the present disclosure, the pharmaceutical composition is not limited in respect of its formulation type.

The present disclosure provides use of the aforementioned compound or the stereoisomer, solvate, hydrate, pharmaceutically acceptable salt or cocrystal thereof in the manufacture of a protein kinase inhibitor; further, the kinase inhibitor is a BTK inhibitor or HER2 inhibitor. Alternatively, the present disclosure provides use of the aforementioned compound or the stereoisomer, solvate, hydrate, pharmaceutically acceptable salt or cocrystal thereof in the manufacture of a medicament for the treatment of a diseases caused by overexpression of BTK kinase or HER2 kinase.

The present disclosure provides use of the aforementioned compound or the stereoisomer, solvate, hydrate, pharmaceutically acceptable salt or cocrystal thereof in the manufacture of a medicament for the treatment of a disease selected from the group consisting of an autoimmune disease, inflammatory disease, thromboembolic disease, hypersensitivity, infectious disease, proliferative disorder, a cancer, and a combination thereof.

Further, the disease may be selected from the group consisting of arthritis, rheumatoid arthritis, urticaria, vitiligo, organ transplant rejection, ulcerative colitis, Crohn's disease, dermatitis, asthma, Sjögren's syndrome, systemic lupus erythematosus, multiple sclerosis, idiopathic thrombocytopenic purpura, rash, antineutrophil cytoplasmic antibody-associated vasculitis, pemphigus, pemphigus vulgaris, chronic obstructive pulmonary disease, psoriasis, breast cancer, mantle cell lymphoma, ovarian cancer, esophageal cancer, laryngeal cancer, glioblastoma, neuroblastoma, gastric cancer, hepatocellular carcinoma, gastric cancer, glioma, endometrial carcinoma, melanoma, kidney cancer, bladder cancer, melanoma, bladder cancer, biliary tract cancer, renal carcinoma, pancreatic cancer, lymphoma, hairy cell leukemia, nasopharyngeal cancer, pharyngeal cancer, colorectal cancer, rectal cancer, cancer of brain and central nervous system, cervical cancer, prostate cancer, testicular cancer, genitourinary tract cancer, lung cancer, non-small cell lung cancer, small cell cancer, lung adenocarcinoma, bone cancer, colon cancer, adenoma, pancreatic cancer, adenocarcinoma, thyroid cancer, follicular carcinoma, Hodgkin's leukemia, bronchial carcinoma, thyroid carcinoma, corpus carcinoma, cervical carcinoma, multiple myeloma, acute myeloid leukemia, chronic myeloid leukemia, lymphocytic leukemia, chronic lymphoblastic leukemia, myelogenous leukemia, non-Hodgkin's lymphoma and primary macroglobulinemia.

In the existing technology, ARQ-531 needs to be improved on its inhibitory activity, since its inhibitory activity against cells such as TMD8 and REC-1 is poor, resulting in excessive clinical doses and serious side effects. In addition, ARQ-531 has poor selectivity, since its inhibitory activity on TEC and EGFR is high, and thus easily causing side effects such as bleeding, diarrhea and eczema. Moreover, ARQ-531 failed to show an ideal pharmacokinetics, as preclinical studies have indicated that its bioavailability was only 38% in the dog PK experiments. Therefore, ARQ-531 has a large room for improvement in terms of inhibitory activity, selectivity, and pharmacokinetics.

In the tests on the activity of inhibiting BTK and HER2 kinase in vitro in the examples of the present disclosure, powder of the compound is dissolved in 100% DMSO to prepare a 10 mM stock solution and stored at-20°C in the dark. During the kinase reaction, the test compounds are tested at a concentration of 1 μM, diluted to a 100-fold final concentration of 100% DMSO solution in a 384 source plate, and 3-fold diluted to 10 concentrations. In addition, the compounds are subjected to experiments such as liver microsome metabolic stability, rat PK, rat brain penetration rate, and drug efficacy model in the examples of the present disclosure. Compared with the existing clinical drug (ARQ-531), the compound of the present disclosure as a BTK protein kinase inhibitor has advantages in terms of the inhibitory activity against BTK or BTK (C481S), liver microsome metabolic stability, rat pharmacokinetics and toxicity. Compared with the existing marketed drug Tirabrutinib, the compound of the present disclosure as a BTK protein kinase inhibitor has advantages in terms of the inhibitory activity against BTK and BTK (C481S), cell activity, liver microsome metabolic stability, rat pharmacokinetics, and rat blood-brain barrier permeability.

In the examples of the present disclosure, a plurality of target compounds are designed and synthesized. A specific preparation process is shown in the following: reacting intermediate A (also known as boric acid or a borate

compound represented by formula A) with intermediate B (bromide represented by formula B) in a manner of a Suzuki reaction to synthesize intermediate C (intermediate represented by formula C), and then performing deprotection to obtain the compound with the structure represented by formula II. In a specific example, intermediate C is prepared by coupling commercially available boronic acid A or homemade borate A with homemade bromide B under palladium catalysis, and the intermediate C is then deprotected to obtain the example compound. Compared with the second-stage clinical drug ARQ-531, the compound of the present disclosure has significantly improved inhibitory activity against BTK and BTK (C481S), liver microsome metabolic stability and rat pharmacokinetics.

In addition, the following synthetic method described in the examples of the present disclosure is simple and convenient, with a relatively high yield.

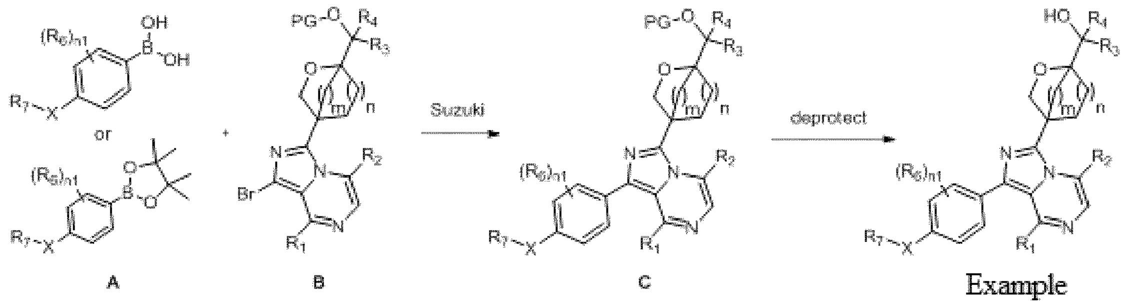


A B C Example

An embodiment of the present disclosure provides an intermediate compound for preparing the aforementioned BTK inhibitor. The intermediate compound has a structure of:

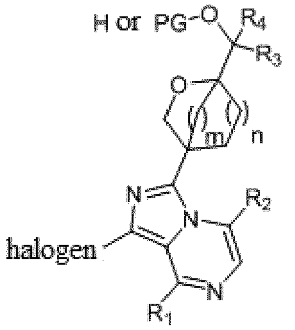

Formula B',

wherein $R_1$, $R_2$, $R_3$, $R_4$, m, and n are as described above; for example:

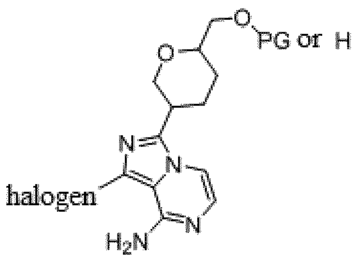

or

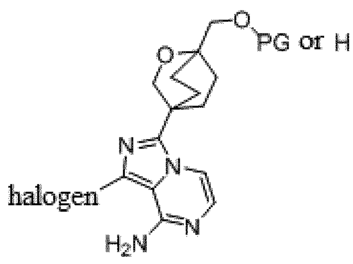

.

In addition, the intermediate compounds further include .

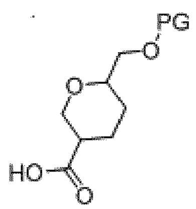

,

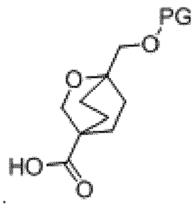

,

and the like.

## BRIEF DESCRIPTION OF DRAWINGS

FIG. 1 shows the test results of some compounds of the present disclosure based on a TMD8 pharmacodynamic model;
FIG. 2 shows the test results of some compounds of the present disclosure based on a TMD8 pharmacodynamic model;
FIG. 3 shows the test results of some compounds of the present disclosure based on a DOHH-2-Luc intracerebral tumor efficacy model; and
FIG. 4 shows fluorescence photos of the test results of some compounds of the present disclosure based on a DOHH-2-Luc intracerebral tumor efficacy model.

## DETAILED DESCRIPTION

Hereinafter the technical solutions in the embodiments of the present application will be described clearly and completely. Apparently, the embodiments to be described are only a part of the embodiments of the present application, rather than all of them. All the other embodiments, which are obtained on the basis of the embodiments in the present disclosure by those skilled in the art without any creative work, will fall within the scope of the present disclosure.

In order to aid in understanding of the present application, the compound that can be used as a BTK protein kinase inhibitor and a preparation method and use thereof provided in the present application are specifically described below in conjunction with the examples.

In the examples of the present disclosure, the structures of the compounds are determined by mass spectrometry (MS) or nuclear magnetic resonance ($^1H$ NMR) equipment. The term "room temperature" means a temperature of 10°C-25°C. Chemical abbreviations have the following meanings:

DMF: N,N-dimethylformamide; DIEA: N,N-diisopropylethylamine;
HATU: O-(7-azabenzotriazol-1-yl)-N,N,N';-tetramethyluronium hexafluorophosphate;
$PdCl_2$ (dppf): [1,1'-bis(diphenylphosphino)ferrocene]palladium (II) dichloride;
DCM: dichloromethane; TEA: triethylamine; TBDPSCl: lithium bistrimethylsilylamide;
9-BBN: 9-boronbicyclo[3.3.1]nonane; Dess-Martin: Dess-Martin periodinane;
DME: dimethoxyethane; TosMIC: p-toluenesulfonylmethyl isocyanide;
t-BuOK: potassium tert-butoxide; Dibal-H: diisobutylaluminum hydride;
THF: tetrahydrofuran; NBS: N-bromosuccinimide;
TBAF: tetrabutylammonium fluoride; DMSO: dimethylsulfoxide;
LDA: lithium diisopropylamide;

HBTU: O-Benzotriazole-N,N,N',N'-tetramethyl-uronium-hexafluorophosphate;
NMP: N-methylpyrrolidone; BAST: bis(2-methoxyethyl)aminosulfur trifluoride;
PMDTA: pentamethyldiethylenetriamine; DMA: N,N-dimethylacetamide;
dppf: 1,1'-bis(diphenylphosphino)ferrocene;
$Pd_2(dba)_3$: tris(dibenzylideneacetone)dipalladium;
TsCl: 4-toluenesulfonyl chloride; DMAP: 4-dimethylaminopyridine;
PDC: pyridinium dichromate;

(continued)

| | |
|---|---|
| DIAD: diisopropyl azodicarboxylate; | NCS: N-chlorosuccinimide. |

**Preparation of Intermediate A-1:**

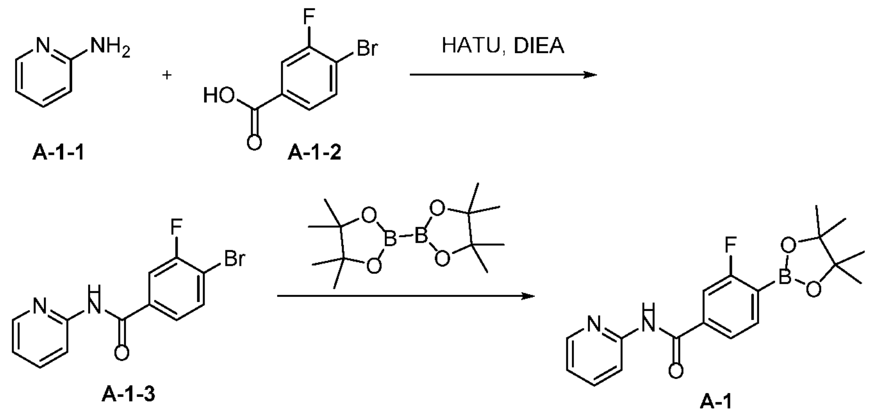


A-1-1 A-1-2 A-1-3 A-1

To a reaction flask, compound A-1-1 (5.0 g, 53.1 mmol), DMF (50 mL), A-1-2 (11.6 g, 53.1 mmol) and DIEA (20.6 g, 159.3 mmol) were added. The reaction solution was subjected to nitrogen replacement, cooled to 0°C, and added with HATU (24.2 g, 63.7 mmol) in portions. The reaction mixture was slowly warmed to room temperature and stirred overnight, as TLC showed that the raw materials reacted completely. The reaction system was added with water and extracted twice with ethyl acetate. The organic phases were combined, washed with water followed by saturated brine, dried over anhydrous $Na_2SO_4$, evaporated in vacuo, and purified by silica gel column chromatography to obtain 11.9 g of product A-1-3 with a yield of 76%.

To a reaction flask, compound A-1-3 (5.0 g, 16.9 mmol), dioxane (50 mL), bis(pinacolato)diboron (5.2 g, 20.3 mmol) and potassium acetate (2.5 g, 25.4 mmol) were added. The reaction solution was subjected to nitrogen replacement, added with $PdCl_2$ (dppf) (500 mg, 0.68 mmol), and subjected to nitrogen replacement again. The reaction mixture was heated to 90°C and stirred overnight, as TLC showed that the raw materials reacted completely. After being cooled, the reaction system was added with silica gel directly to mix the sample, and then purified by silica gel column chromatography to obtain a crude product. The crude product was pulped with petroleum ether to obtain 3.4 g of product A-1 with a yield of 62%.

**Preparation of Intermediate A-2:**

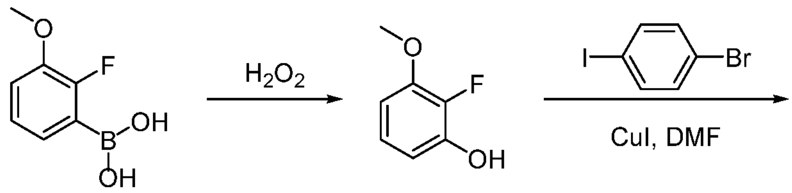


A-2-1 A-2-2

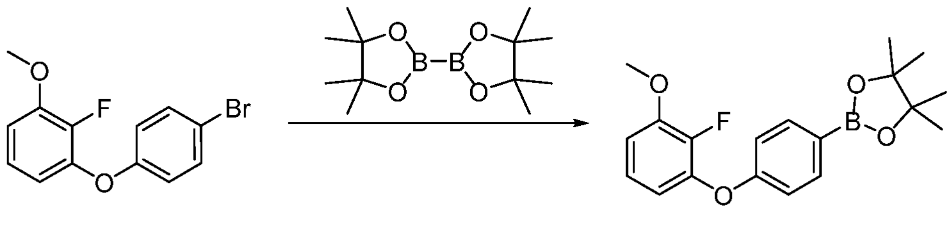

A-2-3 A-2

To a reaction flask, compound A-2-1 (2.0 g, 11.8 mmol) and dioxane (20 mL) were added, and cooled by an ice water bath. The reaction solution was added dropwise with hydrogen peroxide (20 mL, 30%), and then stirred at room temperature overnight until the reaction stopped. The reaction system was added with water, and extracted 4 times with ethyl acetate. The organic phases were combined, washed with water followed by saturated brine, evaporated in vacuo, and purified by silica gel column chromatography to obtain 1.6 g of product A-2-2 with a yield of 96%.

To a reaction flask, compound A-2-2 (1.00 g, 7.04 mmol), DMF (20 mL), p-bromoiodobenzene (1.99 g, 7.04 mmol), tetrabutylammonium bromide (230 mg, 0.704 mmol), potassium phosphate (2.99 g, 14.1 mmol) and cuprous iodide (140 mg, 0.704 mmol) were added. The reaction solution was subjected to nitrogen replacement, heated to 140°C and stirred overnight. The reaction system was cooled to room temperature, added with water, and extracted 3 times with ethyl acetate. The organic phases were combined, washed with water followed by saturated brine, evaporated in vacuo, and purified by silica gel column chromatography to obtain 800 mg of product A-2-3 with a yield of 38%.

To a reaction flask, compound A-2-3 (800 mg, 2.69 mmol), dioxane (16 mL), bis(pinacolato)diboron (821 mg, 3.23 mmol) and potassium acetate (528 mg, 5.38 mmol) were added. The reaction solution was subjected to nitrogen replacement, added with $PdCl_2$ (dppf) (80 mg, 0.109 mmol), and subjected to nitrogen replacement again. The reaction mixture was heated to 80°C and stirred for 16 h. After being cooled, the reaction system was added with silica gel directly to mix the sample, and then purified by silica gel column chromatography to obtain 520 mg of product A-2 with a yield of 56%.

**Preparation of Intermediate A-3:**

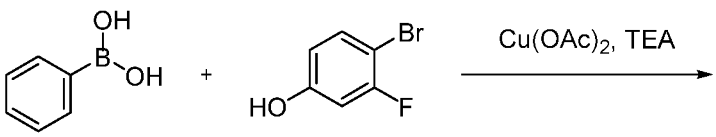


A-3-1 A-3-2

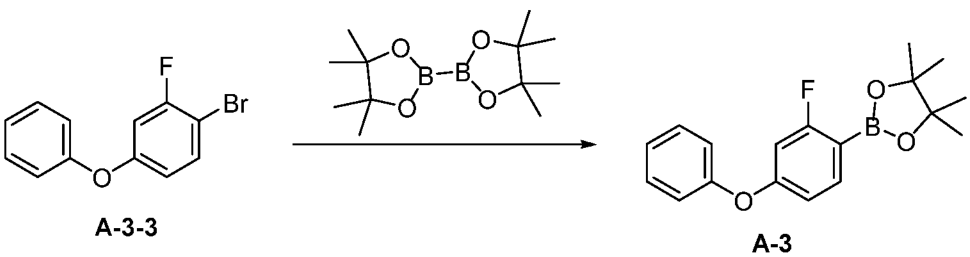

A-3-3 A-3

To a reaction flask, compound A-3-1 (1.28 g, 10.5 mmol), DCM (40 mL), A-3-2 (1.0 g, 5.2 mmol), $Cu(OAc)_2$ (945 mg, 5.2 mmol), TEA (1.58 g, 15.6 mmol) and 4A molecular sieve (1.66 g) were added. The reaction solution was

stirred at room temperature overnight, and then subjected to suction filtration. The filtrate was mixed with silica gel, and then purified by silica gel column chromatography to obtain 600 mg of product A-3-3 with a yield of 43%.

A-3 was synthesized with reference to the method of synthesizing A-2 from A-2-3.

**Preparation of Intermediate A-4:**

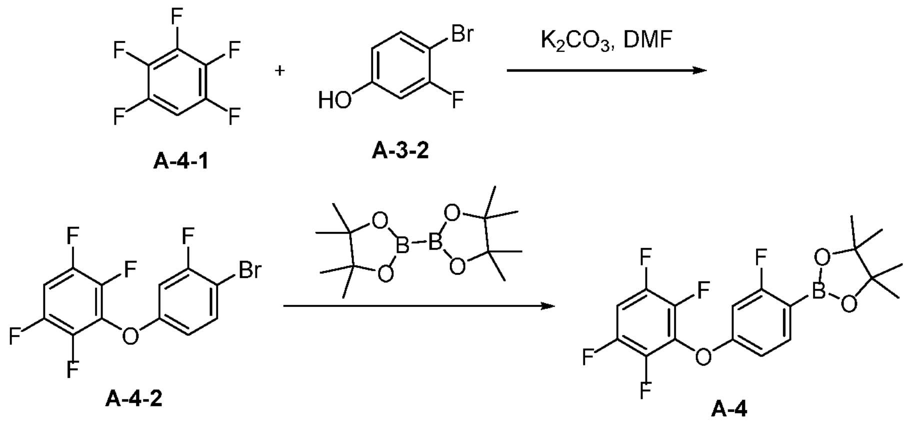


A-4-1 A-3-2

A-4-2 A-4

To a reaction flask, compound A-4-1 (1.06 g, 6.3 mmol), DMF (10 mL), A-3-2 (1.0 g, 5.2 mmol) and potassium carbonate (1.45 g, 10.5 mmol) were added. The reaction solution was heated to 100°C and stirred overnight, as TLC showed that the raw materials reacted completely. The reaction system was added with water and extracted twice with ethyl acetate. The organic phases were combined, washed with water followed by saturated brine, dried over anhydrous $Na_2SO_4$, and evaporated in vacuo to obtain 1.8 g of product A-4-2 with a yield of 100%. The product was used directly in the next step without purification.

The synthesis method of A-4 refers to the method of synthesizing A-2 from A-2-3.

The following compounds were prepared from commercially available corresponding raw materials according to the method for preparing the above intermediates.

Table 1: Structure and Synthesis of Intermediates A-5 to A-12

| Intermediate | Structural formula | Raw material | Reference synthetic method |
|---|---|---|---|
| A-5 | 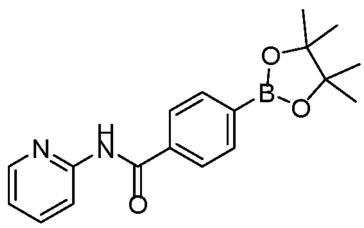 | 2-Aminopyridine 4-Carboxybenzeneboronic acid pinacol ester | A-1-3 |
| A-6 | 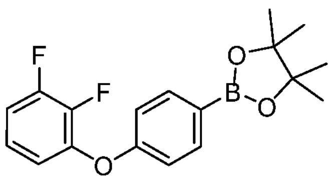 | 2,3-Difluorophenol p-Bromoiodobenzene | A-2 |

(continued)

| Intermediate | Structural formula | Raw material | Reference synthetic method |
|---|---|---|---|
| A-7 | 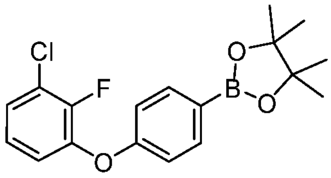 | 3-Chloro-2-fluorophenol p-Bromoiodobenzene | A-2 |
| A-8 | 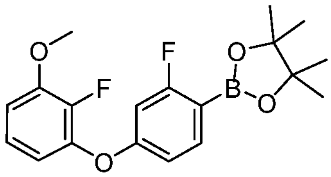 | A-2-2 1-Bromo-2-fluoro-4-iodobenzene | A-2 |
| A-9 | 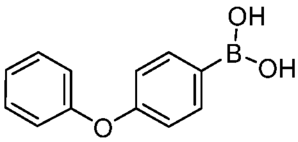 | \ | Commercially available |
| A-10 | 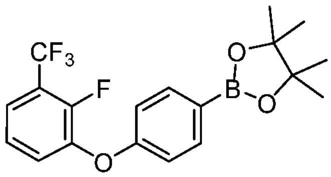 | 2-Fluoro-3-(trifluoromethyl)phenol p-Bromoiodobenzene | A-2 |
| A-11 | 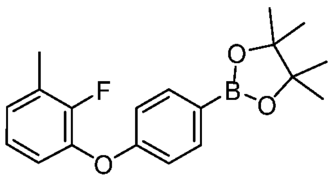 | 2-Fluoro-3-methylphenol p-Bromoiodobenzene | A-2 |
| A-12 | 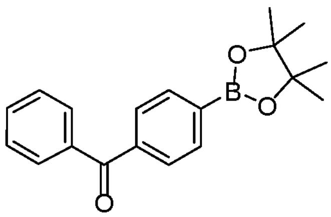 | 4-Bromobenzophenone | A-2 |

**Preparation of Intermediate A-13:**

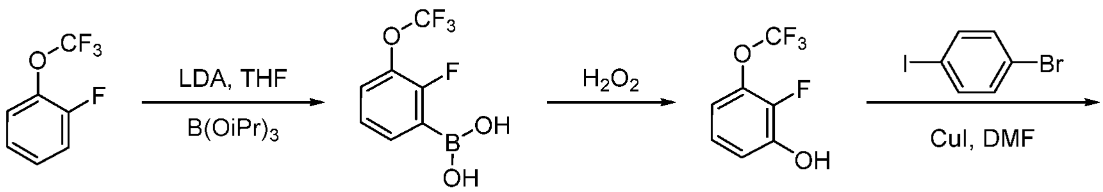


A-13-1 A-13-2 A-13-3

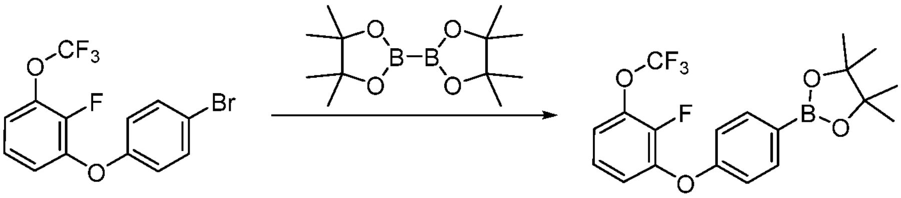

A-13-4 A-13

To a reaction flask, compound A-13-1 (1.00 g, 5.55 mmol), THF (1.5 mL) and triisopropyl borate (1.25 g, 6.66 mmol) were added. The reaction solution was cooled to -70°C, and added dropwise with LDA (2 M, 3.3 mL, 6.6 mmol). After the addition, the reaction solution was slowly warmed to room temperature, added with dilute hydrochloric acid to quench the reaction, and extracted twice with ethyl acetate. The organic phases were combined, washed with saturated brine, and spin-dried. The residue was purified by silica gel column chromatography to obtain 838 mg of product A-13-2 with a yield of 67%.

A-13 was synthesized with reference to the method of synthesizing A-2 from A-2-1.

**Preparation of Intermediate A-14:**

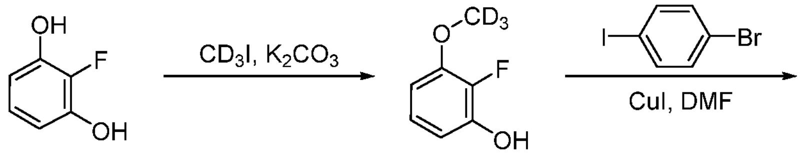


A-14-1 A-14-2

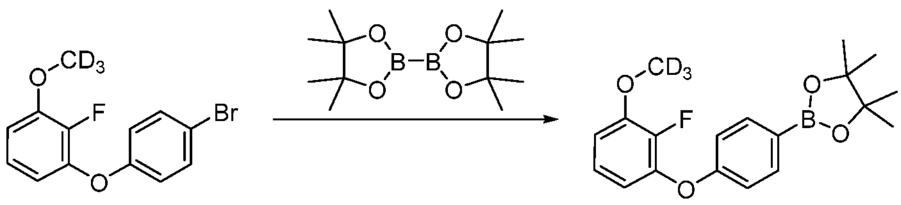

A-14-3 A-14

To a reaction flask, compound A-14-1 (500 mg, 3.90 mmol), acetonitrile (5 mL), potassium carbonate (647 mg, 4.68 mmol) and deuterated iodomethane (566 mg, 3.90 mmol) were added. The reaction solution was heated to 50°C and stirred overnight. The reaction solution was poured into water, adjusted with dilute hydrochloric acid, and extracted twice with ethyl acetate. The organic phases were combined, washed with saturated brine, and spin-dried. The residue was purified by silica gel column chromatography to obtain 432 mg of product A-14-2 with a yield of 76%.

The synthesis method of A-14 was synthesized with reference to the method of synthesizing A-2 from A-2-2.

**Preparation of Intermediate A-15:**

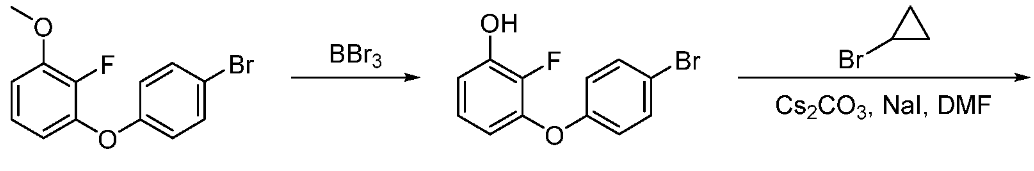


A-2-3 A-15-1

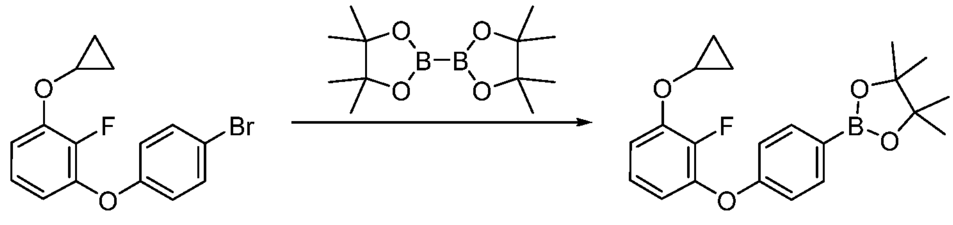

A-15-2 A-15

To a reaction flask, compound A-2-3 (500 mg, 1.68 mmol) and dichloromethane (8 mL) were added. The reaction solution was cooled to -70°C, and added dropwise with dichloromethane solution of boron tribromide (1 M, 5 mL, 5.0 mmol). After the addition, the reaction was kept at a constant temperature for 2 h. The reaction solution was quenched with water, and extracted twice with dichloromethane. The organic phases were combined and spin-dried, and the residue was purified by silica gel column chromatography to obtain 390 mg of product A-15-1 with a yield of 82%.

To a reaction flask, compound A-15-1 (200 mg, 0.706 mmol), DMF (2 mL), bromocyclopropane (171 mg, 1.41 mmol), cesium carbonate (276 mg, 0.847 mmol) and sodium iodide (53 mg, 0.353 mmol) were added. The reaction solution was heated to 150°C for 15 h of reaction. The reaction solution was poured into water, and extracted twice with ethyl acetate. The organic phases were combined, washed with water followed by saturated brine, and spin-dried. The residue was purified by silica gel column chromatography to obtain 121 mg of product A-15-2 with a yield of 53%.

A-15 was synthesized with reference to the method of synthesizing A-2 from A-2-3.

**Preparation of Intermediate A-16:**

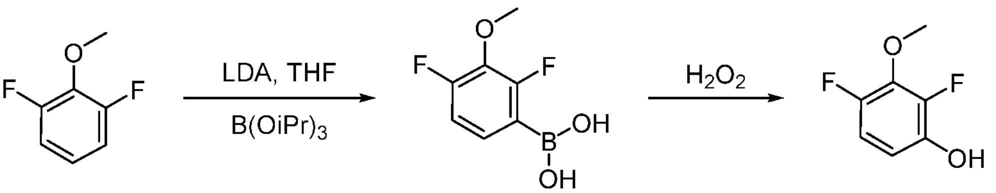


A-16-1 A-16-2 A-16-3

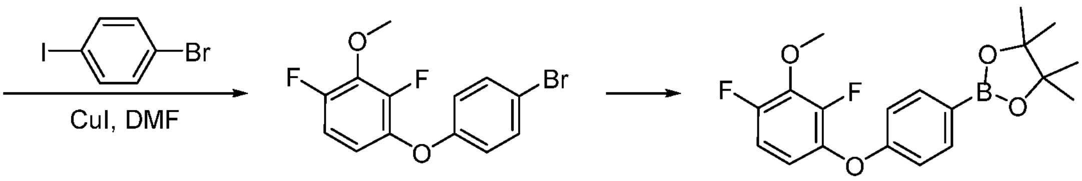


A-16-4 A-16

A-16 was synthesized with reference to the method of synthesizing A-13 from A-13-1.

**Preparation of Intermediate A-17:**

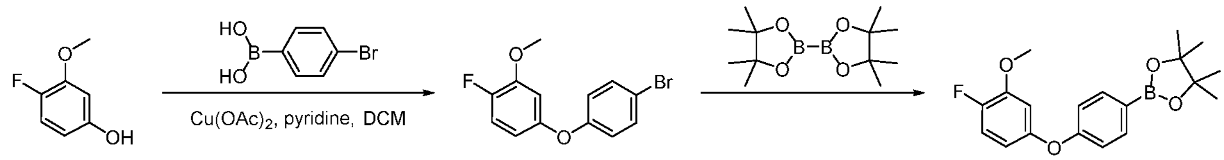


A-17-1 A-17-2 A-17

To a reaction flask, compound A-17-1 (250 mg, 1.76 mmol), dichloromethane (5 mL), p-bromophenylboronic acid (706 mg, 3.52 mmol), copper acetate (320 mg, 1.76 mmol), pyridine (418 mg, 5.28 mmol) and 4A molecular sieve (powder, 500 mg) were added. The reaction solution was stirred at room temperature for two days under ambient air, mixed with silica gel directly and spin-dried, and then purified by silica gel column chromatography to obtain 367 mg of product A-17-2 with a yield of 70%.

A-17 was synthesized with reference to the method of synthesizing A-2 from A-2-3.

**Preparation of Intermediate A-18:**

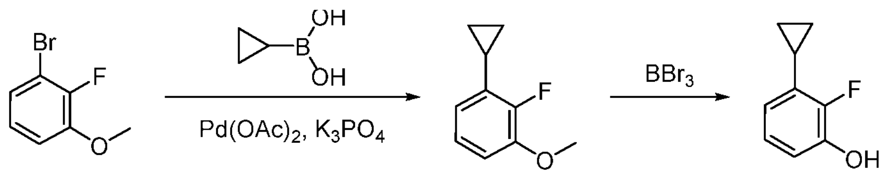


A-18-1 A-18-2 A-18-3

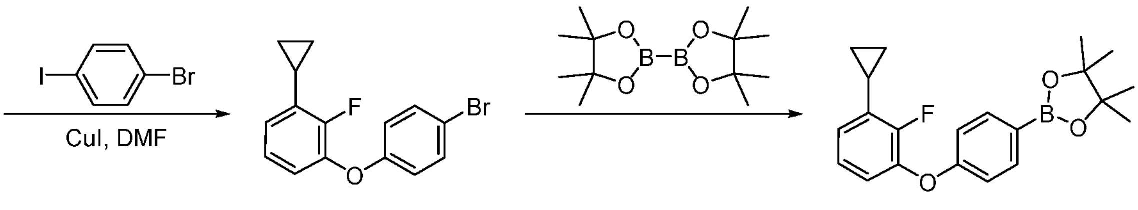


A-18-4 A-18

To a reaction flask, compound A-18-1 (500 mg, 2.44 mmol), toluene (10 mL), cyclopropylboronic acid (315 mg, 3.66 mmol), potassium phosphate (1036 mg, 4.88 mmol), tricyclohexylphosphine (68 mg, 0.244 mmol), palladium acetate (30 mg) and water (0.5 mL) were added. The reaction solution was subjected to nitrogen replacement, and heated to 100°C for reaction overnight. After being cooled, the reaction system was mixed with silica gel directly and spin-dried, and then purified by silica gel column chromatography to obtain 316 mg of product A-18-2 with a yield of 78%.

A-18-3 was synthesized with reference to the method of synthesizing A-15-1 from A-2-3.

A-18 was synthesized with reference to the method of synthesizing A-2 from A-2-2.

The following compounds were prepared from commercially available corresponding raw materials according to the method for preparing the above intermediates.

Table 2: Structure and Synthesis of Intermediates A-19 and A-20

| Intermediate | Structural formula | Raw material | Reference synthetic method |
|---|---|---|---|
| A-19 | 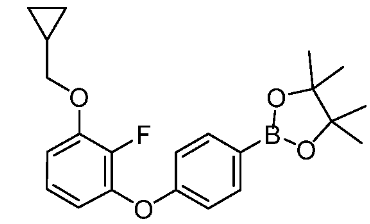 | A-5-1<br>Bromomethylcyclopropane | A-15 (The temperature of the substitution reaction was lowered to 60°C.) |
| A-20 | 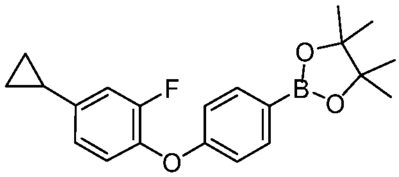 | 4-Bromo-2-fluoroanisole | A-18 |

**Preparation of Intermediate A-21:**

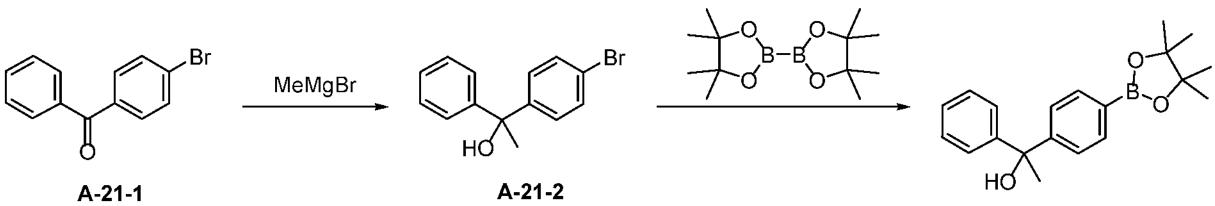


A-21-1

A-21-2

A-21

To a reaction flask, compound A-21-1 (500 mg, 1.91 mmol) and tetrahydrofuran (8 mL) were added. The reaction solution was subjected to nitrogen replacement, cooled by an ice-salt bath, added dropwise with methylmagnesium bromide (1 M tetrahydrofuran solution, 2.3 mL, 2.3 mmol), and warmed to room temperature for 1 h of reaction After the addition. The reaction solution was quenched with saturated aqueous ammonium chloride solution, and extracted twice with ethyl acetate. The organic phases were combined, washed with water followed by saturated brine, dried over anhydrous sodium sulfate, suction-filtered and spin-dried to obtain 535 mg of product A-21-1 with a yield of 100%. The product was used directly in the next step without further purification.

A-21 was synthesized with reference to the method of synthesizing A-2 from A-2-3.

**Preparation of Intermediate A-22:**

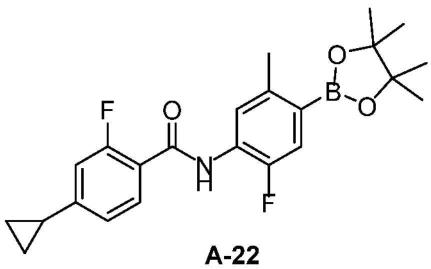

**A-22**

A-22 was synthesized with reference to the literature, Journal of Medicinal Chemistry, 2020, vol. 63, # 10, 5102-5118.

The following compounds were prepared from commercially available corresponding raw materials according to the method for preparing the above intermediates.

Table 3: Structure and Synthesis of Intermediates A-23 to A-25

| Intermediate | Structural formula | Raw material | Reference synthetic method |
|---|---|---|---|
| A-23 | 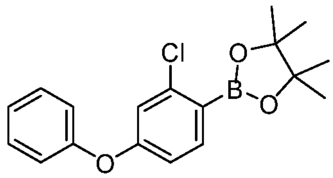 | 4-Bromo-3-chlorophenol Phenylboronic acid | A-3 |
| A-24 | 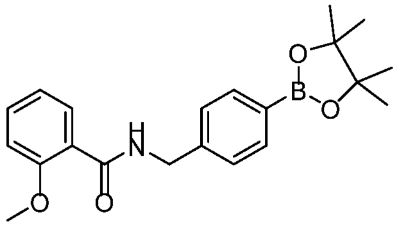 | 2-Methoxybenzoic acid p-Bromobenzylamine | A-1 |
| A-25 | 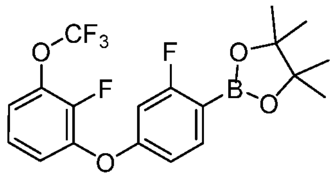 | A-13-3 1-Bromo-2-fluoro-4-iodobenzene | A-2 |

**Preparation of Intermediate A-26:**

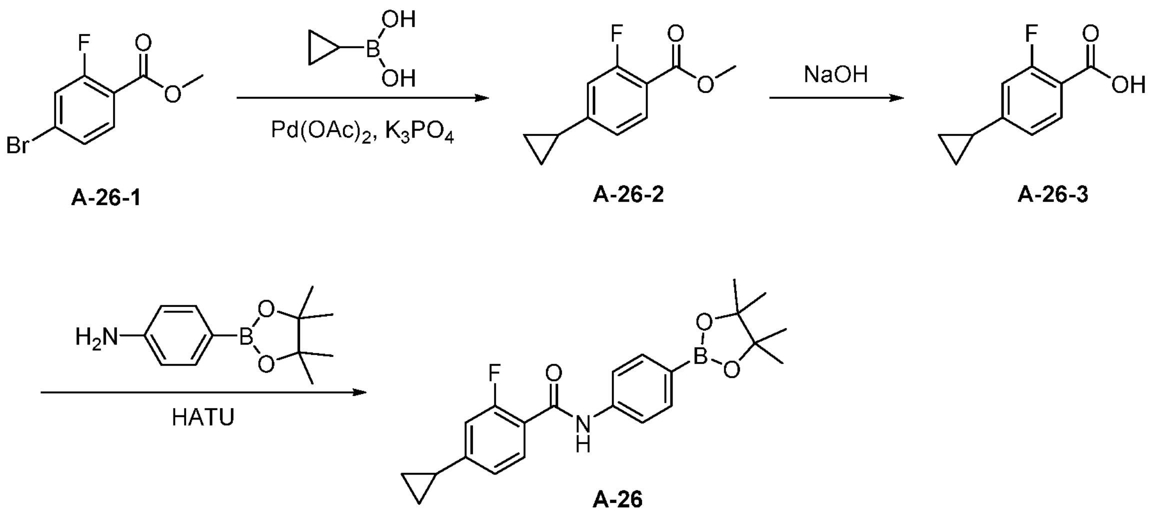


A-26-1 A-26-2 A-26-3 A-26

A-26-2 was synthesized with reference to the method of synthesizing A-18-2 from A-18-1.

To a reaction flask, compound A-26-2 (500 mg, 2.57 mmol), methanol (8 mL) and aqueous sodium hydroxide solution (1 M, 5.1 mL, 5.1 mmol) were added. The reaction solution was reacted overnight at room temperature. The reaction solution was diluted with water, adjusted with dilute hydrochloric acid, and extracted twice with ethyl acetate. The organic phases were combined, washed with water followed by saturated brine, dried over anhydrous sodium sulfate, suction-filtered and spin-dried to obtain 440 mg of product A-26-3 with a yield of 95%. The product was used directly in the next step without further purification.

To a reaction flask, compound A-26-3 (200 mg, 1.11 mmol), DMF (2 mL), 4-aminophenylboronic acid pinacol ester (268 mg, 1.22 mmol) and DIEA (430 mg, 3.33 mmol) were added. HATU (633 mg, 1.67 mmol) was added to the reaction solution in one portion. The mixture was reacted overnight at room temperature. The reaction solution was quenched with water, and extracted twice with ethyl acetate. The organic phases were combined, washed with water followed by saturated brine, dried over anhydrous $Na_2SO_4$, evaporated in vacuo, and purified by silica gel column chromatography to obtain 219 mg of product A-26 with a yield of 52%.

**Preparation of Intermediate A-27:**

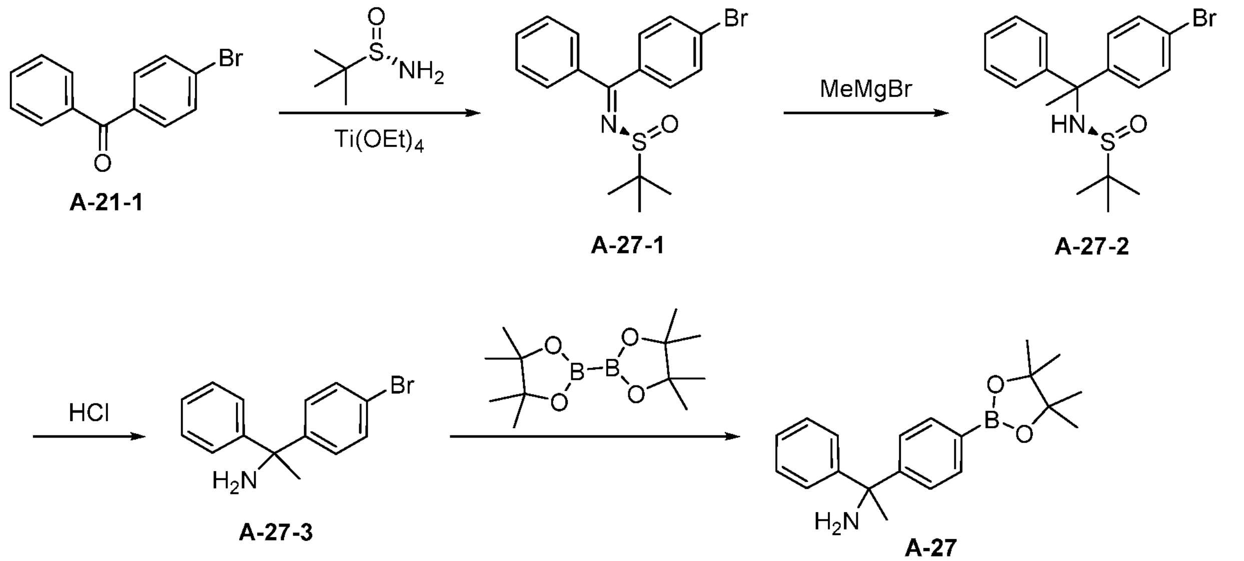


A-21-1 A-27-1 A-27-2 A-27-3 A-27

To a reaction flask, compound A-21-1 (1000 mg, 3.83 mmol), S-tert-butylsulfinamide (511 mg, 4.21 mmol) and 1,4-dioxane (10 mL) were added. The reaction solution was subjected to nitrogen replacement, and added with tetraethyl titanate (2184 mg, 9.58 mmol). The reaction solution was heated to 100°C and stirred for 5 h. The reaction solution was cooled, quenched with water, and extracted twice with ethyl acetate. The organic phases were combined, washed with water followed by saturated brine, evaporated in vacuo, and purified by silica gel column chromatography to obtain 882 mg of product A-27-1 with a yield of 63%.

To a reaction flask, compound A-27-1 (882 mg, 2.42 mmol) and tetrahydrofuran (14 mL) were added. The reaction solution was subjected to nitrogen replacement, cooled by an ice-salt bath, added dropwise with methylmagnesium bromide (1 M tetrahydrofuran solution, 2.9 mL, 2.9 mmol), and warmed to room temperature for 1 h of reaction After the addition. The reaction solution was quenched with aqueous saturated ammonium chloride solution, and extracted twice with ethyl acetate. The organic phases were combined, washed with water followed by saturated brine, evaporated in vacuo, and purified by silica gel column chromatography to obtain 630 mg of product A-27-2 with a yield of 68%.

To a reaction flask, compound A-27-2 (630 mg, 1.66 mmol) and methanol (10 mL) were added, and then a solution of hydrogen chloride in 1,4-dioxane (4 M, 6 mL) was added. The reaction solution was reacted at room temperature for 1 h and then concentrated to dryness under reduced pressure. The residue was added with water, adjusted with aqueous sodium hydroxide solution, and extract twice with ethyl acetate. The organic phases were combined, washed with water followed by saturated brine, evaporated in vacuo, and purified by silica gel column chromatography to obtain 371 mg of product A-27-3 with a yield of 81%.

A-27 was synthesized with reference to the method of synthesizing A-2 from A-2-3.

**Preparation of Intermediate A-28:**

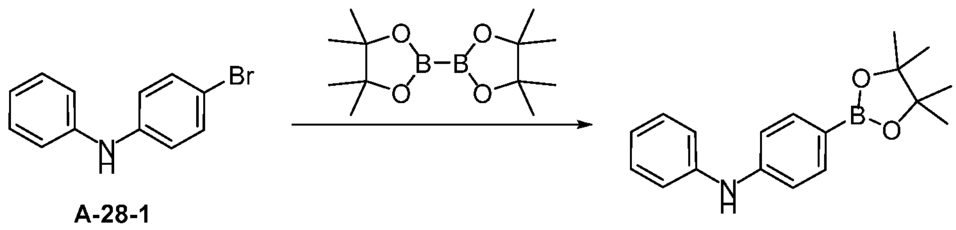

A-28-1

A-28

A-28 was synthesized with reference to the method of synthesizing A-2 from A-2-3.

**Preparation of Intermediate A-29:**

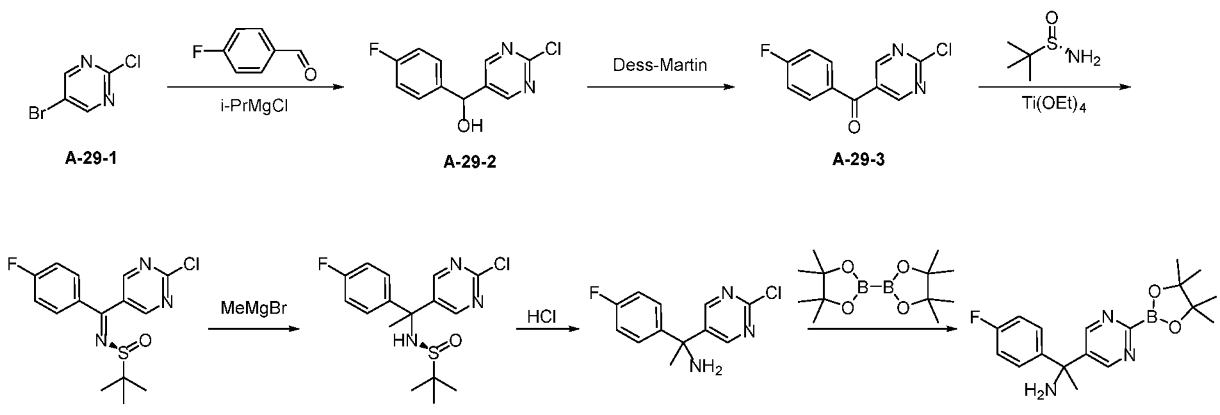


A-29-1 A-29-2 A-29-3

A-29-4 A-29-5 A-29-6 A-29

To a reaction flask, compound A-29-1 (1000 mg, 5.17 mmol) and tetrahydrofuran (10 mL) were added. The reaction solution was subjected to nitrogen replacement, cooled by an ice water bath. Isopropylmagnesium chloride (1 M tetrahydrofuran solution, 6.2 mL, 6.2 mmol) was added dropwise to the reaction solution. After the addition, the reaction solution was warmed to room temperature, stirred for 1 h, and added dropwise with p-fluorobenzaldehyde (770 mg, 6.20 mmol) in tetrahydrofuran (4 mL). After the addition, the reaction solution was stirred at room temperature for 1 h, quenched with saturated aqueous ammonium chloride solution, and extracted twice with ethyl acetate. The organic phases were combined, washed with water followed by saturated brine, evaporated in vacuo, and purified by silica gel column chromatography to obtain 612 mg of product A-29-2 with a yield of 50%.

To a reaction flask, compound A-29-2 (612 mg, 2.56 mmol), dichloromethane (12 mL) and Dess-Martin periodinane (1632 mg, 3.85 mmol) were added. The reaction solution was reacted at room temperature for 1 h, as TLC showed that the reaction was complete. The reaction solution was mixed with silica gel directly and spin-dried, and then purified by silica gel column chromatography to obtain 495 mg of product A-29-3 with a yield of 82%.

The synthesis method of A-29 was synthesized with reference to the method of synthesizing A-27 from A-21-1.

**Preparation of Intermediate A-30:**

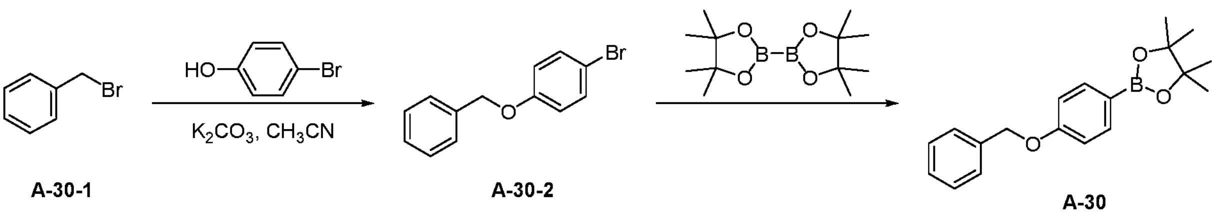


A-30-1 A-30-2 A-30

To a reaction flask, compound A-30-1 (500 mg, 2.92 mmol), acetonitrile (5 mL), p-bromophenol (607 mg, 3.51

mmol) and potassium carbonate (485 mg, 3.51 mmol) were added. The reaction solution was heated to 70°C and stirred overnight. The reaction solution was cooled, filtered under vacuum, and washed with ethyl acetate. After the filtrate was evaporated in vacuo, purification by silica gel column was performed to obtain 742 mg of product A-30-2 with a yield of 97%.

A-30 was synthesized with reference to the method of synthesizing A-2 from A-2-3.

**Preparation of Intermediate A-31:**

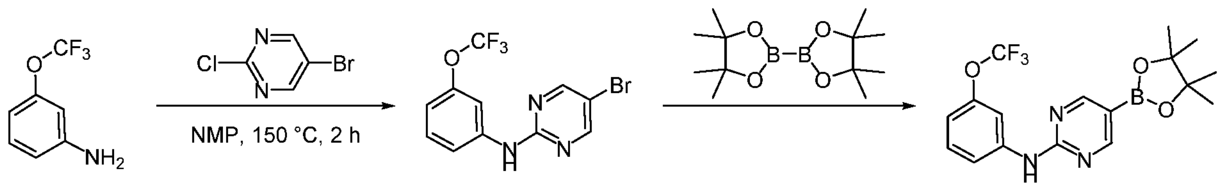


A-31-1 A-31-2 A-31

To a reaction flask, compound A-31-1 (500 mg, 2.82 mmol), 5-bromo-2-chloropyrimidine (546 mg, 2.82 mmol) and N-methylpyrrolidone (5 mL) were added. The reaction solution was heated to 150°C and stirred for 2 h. The reaction solution was cooled, diluted with water, and extracted twice with ethyl acetate. The organic phases were combined, washed with water followed by saturated brine, evaporated in vacuo, and purified by silica gel column chromatography to obtain 580 mg of product A-31-2 with a yield of 62%.

A-31 was synthesized with reference to the method of synthesizing A-2 from A-2-3.

The following compounds were prepared from commercially available corresponding raw materials according to the method for preparing the above intermediates.

Table 4: Structure and Synthesis of Intermediates A-32 to A-33

| Intermediate | Structural formula | Raw material | Reference synthetic method |
|---|---|---|---|
| A-32 | 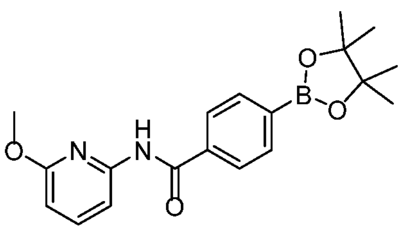 | 2-Amino-6-methoxypyridine 4-Carboxyphenylboronic acid pinacol ester | A-1-3 |
| A-33 | 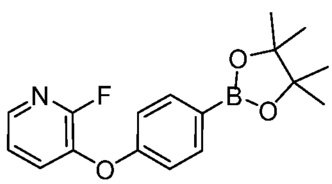 | 2-Fluoro-3-hydroxypyridine 4-Bromophenylboronic acid | A-17 |

**Preparation of Intermediate A-34:**

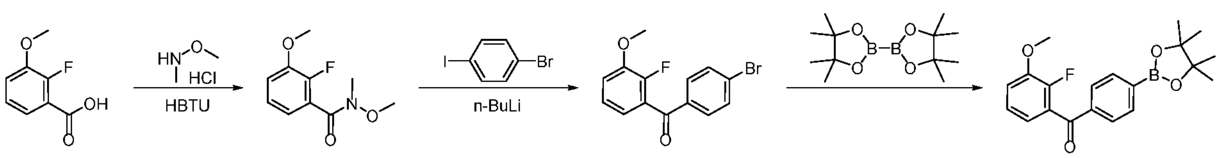


A-34-1 A-34-2 A-34-3 A-34

To a reaction flask, compound A-34-1 (500 mg, 2.94 mmol), DMF (5 mL), dimethylhydroxylamine hydrochloride (344 mg, 3.53 mmol) and DIEA (1520 mg, 11.8 mmol) were added. HBTU (1449 mg, 3.82 mmol) was added to the reaction solution in one portion with stirring. The reaction solution was stirred at room temperature overnight, poured into water, and extracted 4 times with ethyl acetate. The organic phases were combined, washed with saturated brine, evaporated in vacuo, and purified by silica gel column chromatography to obtain 600 mg of product A-34-2 with a yield of 96%.

To a reaction flask, compound p-bromoiodobenzene (876 mg, 3.10 mmol) and tetrahydrofuran (10 mL) were added. The reaction solution was subjected to nitrogen replacement, and cooled in dry ice/ethanol bath to -70°C. The reaction solution was added dropwise with n-butyllithium (2.5 M, 1.24 mL, 3.10 mmol), stirred for 30 min, and then added dropwise with a solution of A-34-2 (600 mg, 2.81 mmol) in tetrahydrofuran (3 mL). After the addition, the reaction solution was slowly warmed to room temperature for 1 h of reaction. The reaction solution was quenched with water and extracted twice with ethyl acetate. The organic phases were combined, washed with saturated brine, evaporated in vacuo, and purified by silica gel column chromatography to obtain 360 mg of product A-34-3 with a yield of 41%.

A-34 was synthesized with reference to the method of synthesizing A-2 from A-2-3.

**Preparation of Intermediate A-35:**

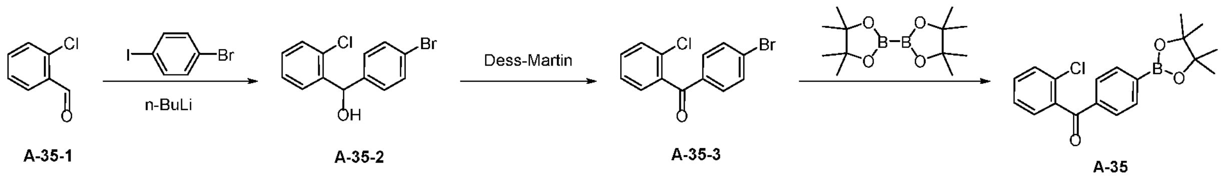


A-35-1 A-35-2 A-35-3 A-35

A-35-2 was synthesized with reference to the method of synthesizing A-34-3 from A-34-2.

A-35-3 was synthesized with reference to the method of synthesizing A-29-3 from A-29-2.

A-35 was synthesized with reference to the method of synthesizing A-2 from A-2-3.

The following compounds were prepared from commercially available corresponding raw materials according to the method for preparing the above intermediates.

Table 5: Structure and Synthesis of Intermediates A-36 to A-39

| Intermedi ate | Structural formula | Raw material | Reference synthetic method |
|---|---|---|---|
| A-36 | 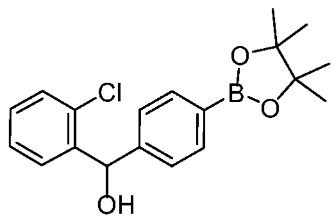 | A-35-2 | A-2 |
| A-37 | 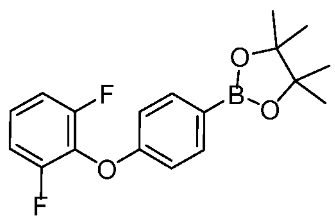 | 2,6-Difluorophenol 4-Bromophenylboronic acid | A-17 |

(continued)

| Intermediate | Structural formula | Raw material | Reference synthetic method |
|---|---|---|---|
| A-38 | 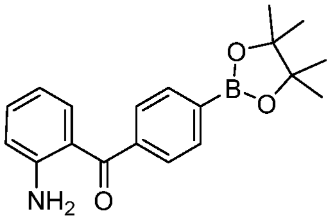 | 2-Amino-4'-bromobenzophenone | A-2 |
| A-39 | 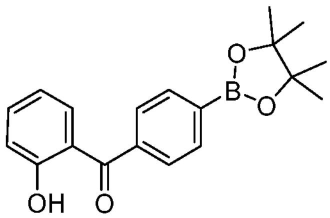 | Salicylaldehyde p-Bromoiodobenzene | A-35 |

**Preparation of Intermediate A-40:**

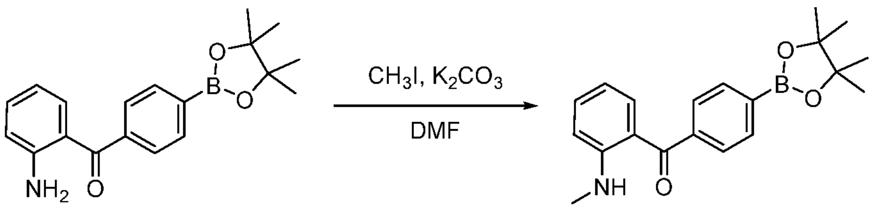


**A-38** **A-40**

To a reaction flask, compound A-38 (100 mg, 0.309 mmol), DMF (1 mL), methyl iodide (48 mg, 0.340 mmol) and potassium carbonate (51 mg, 0.371 mmol) were added. The reaction solution was heated to 80°C for 5 h of reaction, cooled, poured into water, and extracted twice with ethyl acetate. The organic phases were combined, washed with water followed by saturated brine, dried over anhydrous sodium sulfate, evaporated in vacuo, and purified by a preparative silica gel plate to obtain 62 mg of product A-40 with a yield of 60%.

The following compounds were prepared from commercially available corresponding raw materials according to the method for preparing the above intermediates.

Table 6: Structure and Synthesis of Intermediates A-41 to A-47

| Intermediate | Structural formula | Raw material | Reference synthetic method |
|---|---|---|---|
| A-41 | 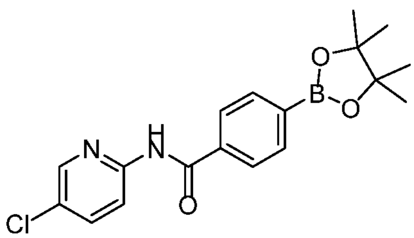 | 2-Amino-5-chloropyridine 4-Carboxyphenylboronic acid pinacol ester | A-1-3 |

(continued)

| Intermediate | Structural formula | Raw material | Reference synthetic method |
|---|---|---|---|
| A-42 | 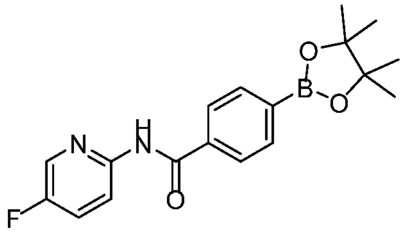 | 2-Amino-5 -fluoropyridine 4-Carboxyphenylboronic acid pinacol ester | A-1-3 |
| A-43 | 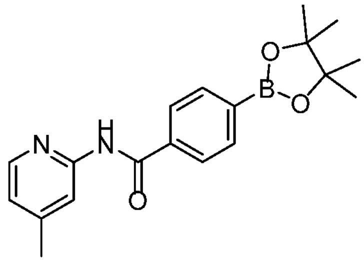 | 2-Amino-4-methylpyridine 4-Carboxyphenylboronic acid pinacol ester | A-1-3 |
| A-44 | 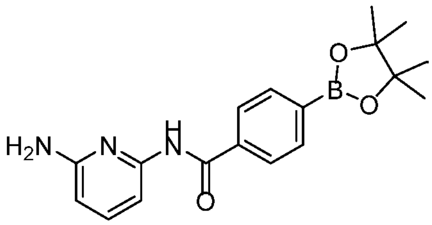 | 2,6-Diaminopyridine 4-Carboxyphenylboronic acid pinacol ester | A-1-3 |
| A-45 | 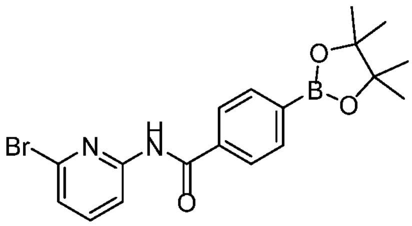 | 2-amino-6-bromopyridine 4-Carboxyphenylboronic acid pinacol ester | A-1-3 |
| A-46 | 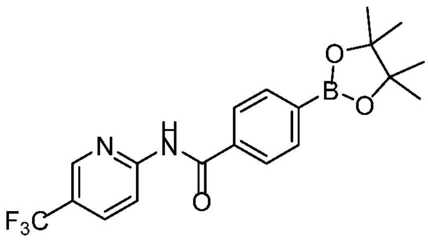 | 2-Amino-5-trifluoromethylpyridine 4-Carboxyphenylboronic acid pinacol ester | A-1-3 |
| A-47 | 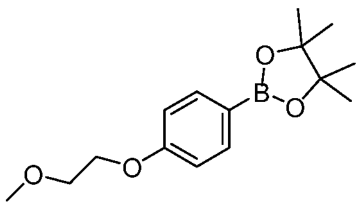 | 1-Bromo-4-(2-methoayethoxy) benzene | A-2 |

**Preparation of Intermediate A-48:**

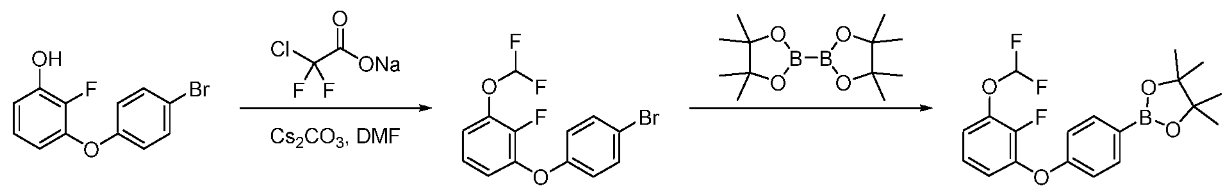


A-15-1 A-48-1 A-48

To a reaction flask, compound A-15-1 (200 mg, 0.706 mmol), DMF (4 mL) and sodium chlorodifluoroacetate (215 mg, 1.41 mmol) were added. The reaction solution was subjected to nitrogen replacement, and heated to 100°C for 6 h of reaction. The reaction solution was cooled, poured into water, and extracted twice with ethyl acetate. The organic phases were combined, washed with water followed by saturated brine, evaporated in vacuo, and purified by silica gel column chromatography to obtain 128 mg of product A-48-1 with a yield of 54%.

A-48 was synthesized with reference to the method of synthesizing A-2 from A-2-3.

The following compounds were prepared from commercially available corresponding raw materials according to the method for preparing the above intermediates.

Table 7: Structure and Synthesis of Intermediates A-49 to A-53

| Intermediate | Structural formula | Raw material | Reference synthetic method |
|---|---|---|---|
| A-49 | 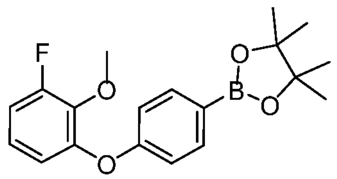 | 3-Fluoro-2-methoxyphenol p-Bromoiodobenzene | A-2 |
| A-50 | 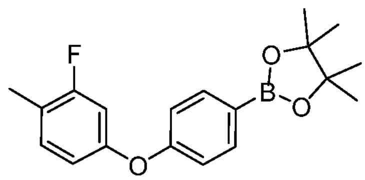 | 3-Fluoro-4-methylphenol 4-Bromophenylboronic acid | A-17 |
| A-51 | 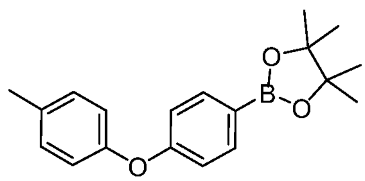 | 4-Tolylboronic acid 4-Bromophenol | A-3 |
| A-52 | 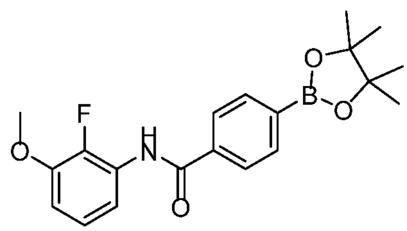 | 2-Fluoro-3 -methoxy aniline 4-Carboxyphenylboronic acid pinacol ester | A-1-3 |
| A-53 | 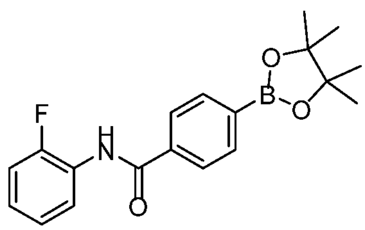 | 2-Fluoroaniline 4-Carboxyphenylboronic acid pinacol ester | A-1-3 |

**Preparation of Intermediate A-54:**

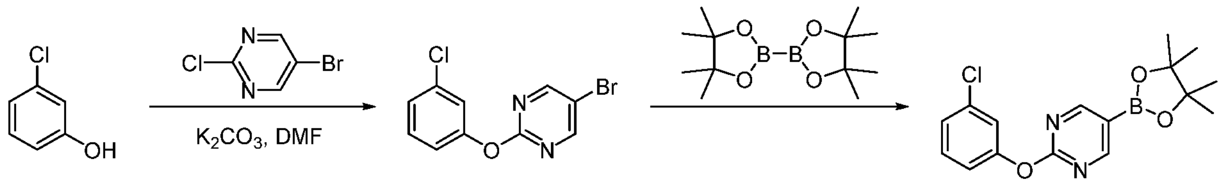


A-54-1 A-54-2 A-54

To a reaction flask, compound A-54-1 (500 mg, 3.89 mmol), 5-bromo-2-chloropyrimidine (752 mg, 3.89 mmol), DMF (5 mL) and potassium carbonate (645 mg, 4.67 mmol) were added. The reaction solution was heated to 100°C for 4 h of reaction. The reaction solution was cooled, poured into water, and extracted twice with ethyl acetate. The organic phases were combined, washed with water followed by saturated brine, evaporated in vacuo, and purified by silica gel column chromatography to obtain 511 mg of product A-54-2 with a yield of 46%.

A-54 was synthesized with reference to the method of synthesizing A-2 from A-2-3.

**Preparation of Intermediate A-55:**

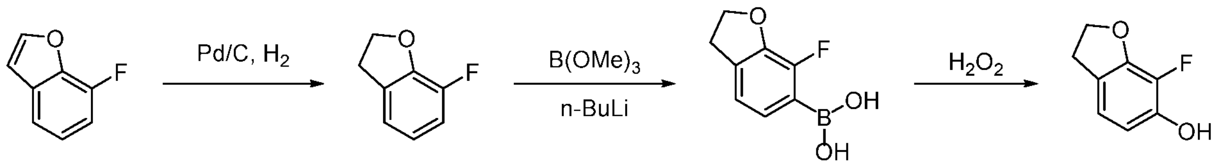


A-55-1 A-55-2 A-55-3 A-55-4

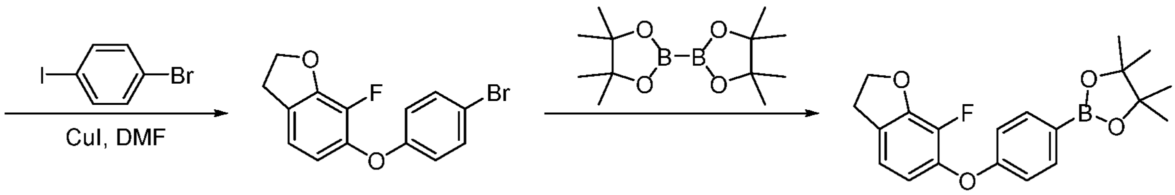


A-55-5 A-55

To a reaction flask, compound A-55-1 (1.00 g, 7.35 mmol), Pd/C (10%, 200 mg) and methanol (25 mL) were added. The reaction solution was subjected to nitrogen replacement, and then stirred overnight under hydrogen pressure (balloon). The reaction solution was filtered under vacuum, and the filtrate was directly spin-dried to obtain 1.00 g of product A-55-2 with a yield of 99%. The product was used directly in the next step without purification.

To a reaction flask, compound A-55-2 (800 mg, 5.79 mmol) and tetrahydrofuran (10 mL) were added. The reaction solution was subjected to nitrogen replacement, and cooled in a dry ice/ethanol bath to -70°C. The reaction solution was added dropwise with n-butyllithium (2.5 M, 2.8 mL, 6.95 mmol), stirred for 30 min, and then added dropwise with a solution of trimethyl borate (723 mg, 6.95 mmol) in tetrahydrofuran (3 mL). After the addition, the reaction solution was slowly warmed to room temperature for 30 min of reaction. The reaction solution was quenched with dilute hydrochloric acid, and extracted twice with ethyl acetate. The organic phases were combined, washed with saturated brine, evaporated in vacuo, and purified by silica gel column chromatography to obtain 430 mg of product A-55-3 with a yield of 41%.

A-55 was synthesized with reference to the method of synthesizing A-2 from A-2-1.

**Preparation of Intermediate A-56:**

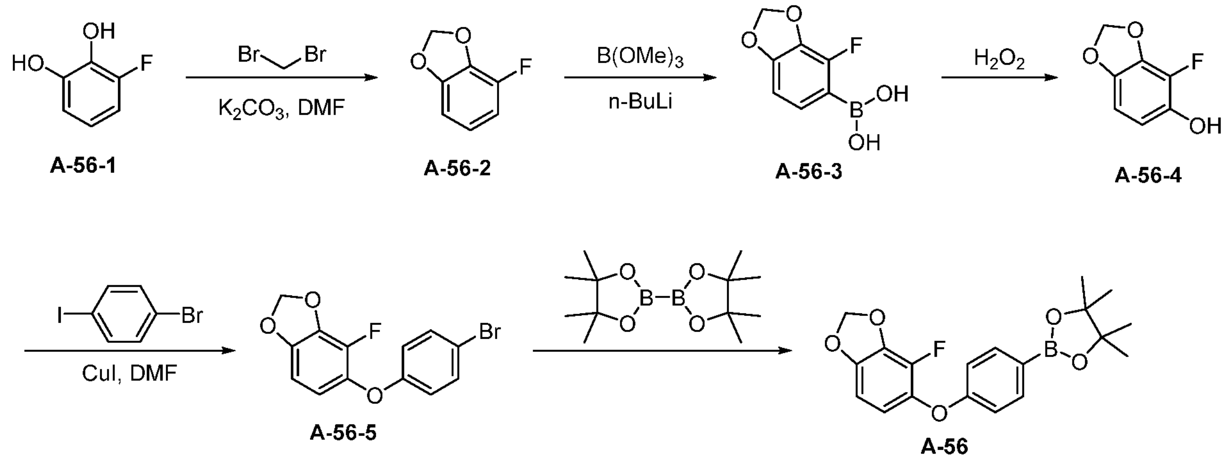


A-56-1 A-56-2 A-56-3 A-56-4 A-56-5 A-56

To a reaction flask, compound A-56-1 (500 mg, 3.90 mmol), dibromomethane (1018 mg, 5.85 mmol), DMF (8 mL) and potassium carbonate (1348 mg, 9.75 mmol) were added. The reaction solution was heated to 100°C for 4 h of reaction. The reaction solution was cooled, poured into water, and extracted twice with ethyl acetate. The organic phases were combined, washed with water followed by saturated brine, evaporated in vacuo, purified by silica gel column chromatography to obtain 320 mg of product A-56-2 with a yield of 59%.

A-56 was synthesized with reference to the method of synthesizing A-55 from A-55-2.

The following compounds were prepared from commercially available corresponding raw materials according to the method for preparing the above intermediates.

Table 8: Structure and Synthesis of Intermediates A-57 to A-59

| Intermediate | Structural formula | Raw material | Reference synthetic method |
|---|---|---|---|
| A-57 | 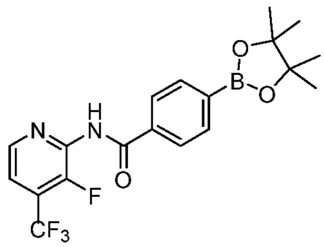 | 2-Amino-3-fluoro-4-trifluoromethylpyridine 4-Carboxyphenylboronic acid pinacol ester | A-1-3 |
| A-58 | 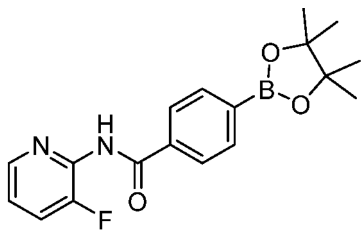 | 2-Amino-3 -fluoropyridine 4-Carboxyphenylboronic acid pinacol ester | A-1-3 |
| A-59 | 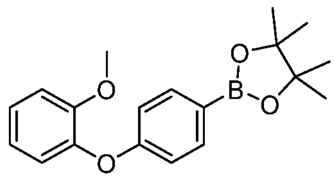 | o-Methoxyphenol p-Bromoiodobenzene | A-2 |

**Preparation of Intermediate A-60:**

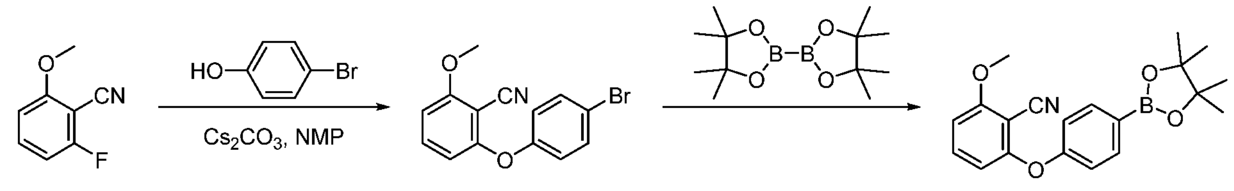


A-60-1 A-60-2 A-60

To a reaction flask, compound A-60-1 (500 mg, 3.31 mmol), p-bromophenol (685 mg, 3.96 mmol), NMP (10 mL) and cesium carbonate (3.20 g, 9.90 mmol) were added. The reaction solution was heated to 80°C for reaction overnight. The reaction solution was cooled, poured into water, and extracted twice with ethyl acetate. The organic phases were combined, washed with water followed by saturated brine, evaporated in vacuo, and purified by silica gel column chromatography to obtain 830 mg of product A-60-2 with a yield of 82%.

A-60 was synthesized with reference to the method of synthesizing A-2 from A-2-3.

The following compounds were prepared from commercially available corresponding raw materials according to the method for preparing the above intermediates.

Table 9: Structure and Synthesis of Intermediates A-61 to A-63

| Intermediate | Structural formula | Raw material | Reference synthetic method |
|---|---|---|---|
| A-61 | 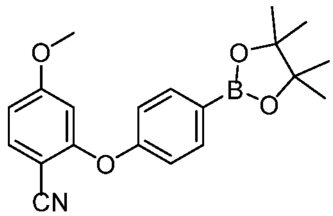 | 2-Fluoro-4-methoxybenzonitrile p-Bromophenol | A-60 |
| A-62 | 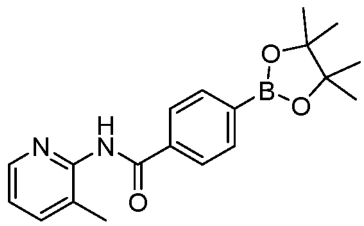 | 2-Amino-3-methylpyridine 4-Carboxyphenylboronic acid pinacol ester | A-1-3 |
| A-63 | 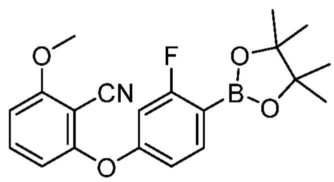 | 2-Fluoro-6-methoxybenzonitrile 4-Bromo-3-fluorophenol | A-60 |

**Preparation of Intermediate A-64:**

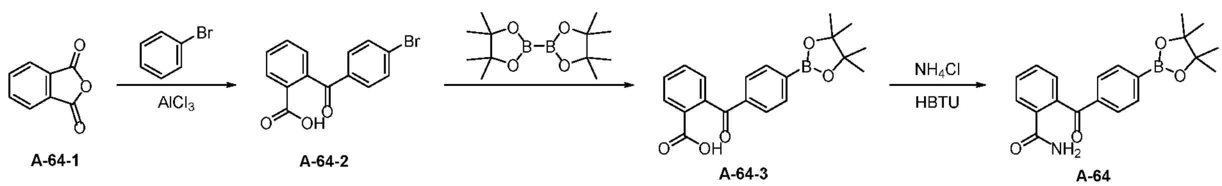


A-64-1 A-64-2 A-64-3 A-64

To a reaction flask, compound A-64-1 (1.192 g, 8.05 mmol), bromobenzene (10.1 g, 64.3 mmol) and aluminum

trichloride (2.15 g, 16.1 mmol) were added. The reaction solution was subjected to nitrogen replacement and heated to 90°C for 3 h of reaction. The reaction solution was cooled, poured into dilute hydrochloric acid, and extracted three times with dichloromethane. The organic phases were combined and extracted 3 times with aqueous sodium carbonate solution. The aqueous phase was adjusted to pH 3 with dilute hydrochloric acid. The precipitated solid was filtered under vacuum and washed with water. The filter cake was collected, and dried in vacuo to obtain 2.0 g of product A-64-2 with a yield of 81%. The product was used directly in the next step without further purification.

A-64-3 was synthesized with reference to the method of synthesizing A-2 from A-2-3.

To a reaction flask, compound A-64-3 (200 mg, 0.57 mmol), DMF (3 mL), ammonium chloride (152 mg, 2.85 mmol) and DIEA (220 mg, 1.71 mmol) were added. HBTU (324 mg, 0.85 mmol) was added to the reaction solution in one portion with stirring. The reaction solution was stirred at room temperature overnight, poured into water, and extracted three times with ethyl acetate. The organic phases were combined, washed with saturated brine, evaporated in vacuo, and purified by a preparative silica gel plate to obtain 70 mg of product A-64 with a yield of 35%.

The following compounds were prepared from commercially available corresponding raw materials according to the method for preparing the above intermediates.

Table 10: Structure and Synthesis of Intermediates A-65 to A-67

| Intermediate | Structural formula | Raw material | Reference synthetic method |
|---|---|---|---|
| A-65 | 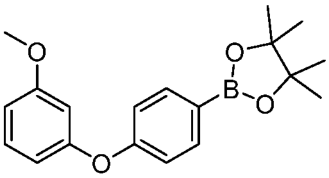 | 3-Methoxyphenol p-Bromoiodobenzene | A-2 |
| A-66 | 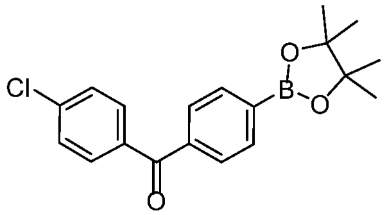 | p-Chlorobenzaldehyde p-Bromoiodobenzene | A-35 |
| A-67 | 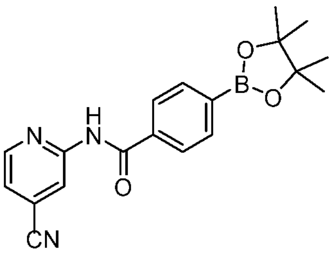 | 2-Amino-4-cyanopyridine 4-Carboxyphenylboronic acid pinacol ester | A-1-3 |

**Preparation of Intermediate A-68:**

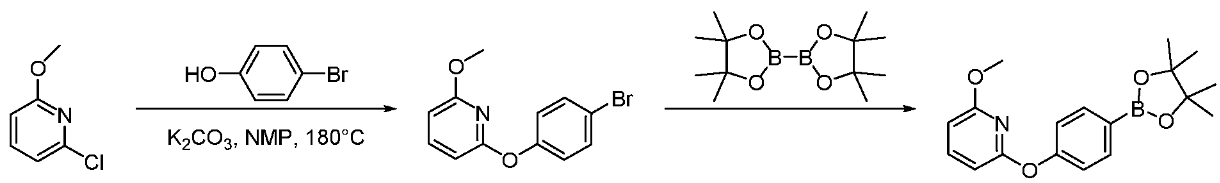


A-68-1 A-68-2 A-68

To a reaction flask, compound A-68-1 (200 mg, 1.39 mmol), p-bromophenol (361 mg, 2.09 mmol), NMP (2 mL)

and potassium carbonate (384 mg, 2.78 mmol) were added. The reaction solution was heated to 180°C for 8 h of reaction. The reaction solution was cooled, poured into water, and extracted twice with ethyl acetate. The organic phases were combined, washed with water followed by saturated brine, dried over anhydrous sodium sulfate, evaporated in vacuo, purified by a preparative silica gel plate to obtain 60 mg of product A-68-2 with a yield of 15%.

A-68 was synthesized with reference to the method of synthesizing A-2 from A-2-3.

**Preparation of Intermediate A-69:**

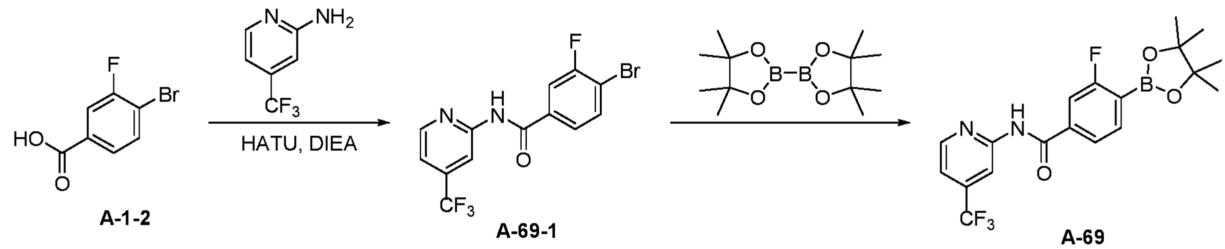


A-1-2 A-69-1 A-69

A-69 was synthesized with reference to the method of synthesizing A-1 from A-1-1 and A-1-2.

**Preparation of Intermediate A-70:**

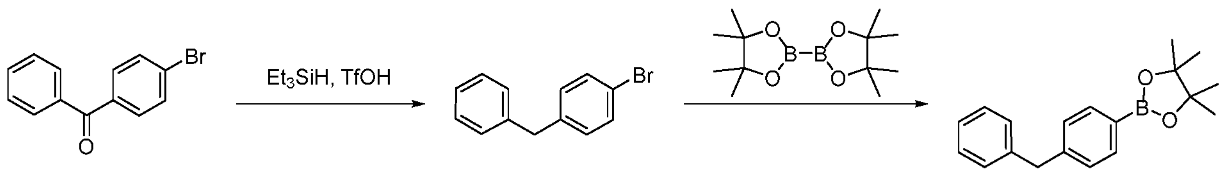


A-21-1 A-70-1 A-70

To a reaction flask, compound A-21-1 (200 mg, 0.766 mmol), triethylsilane (267 mg, 2.31 mmol), dichloromethane (4 mL) and trifluoromethanesulfonic acid (35 mg, 0.231 mmol) were added. The reaction solution was stirred overnight at room temperature, then poured into water, and extracted twice with ethyl acetate. The organic phases were combined, evaporated in vacuo, and purified by silica gel column chromatography to obtain 190 mg of product A-70-1 with a yield of 100%.

A-70 was synthesized with reference to the method of synthesizing A-2 from A-2-3.

**Preparation of Intermediate A-71:**

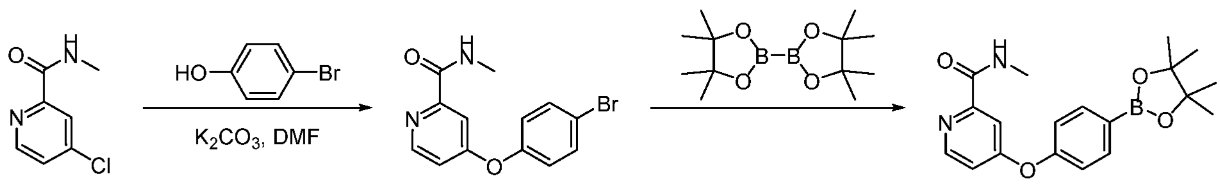


A-71-1 A-71-2 A-71

A-71 was synthesized with reference to the method of synthesizing A-54 from A-54-1.

**Preparation of Intermediate A-72:**

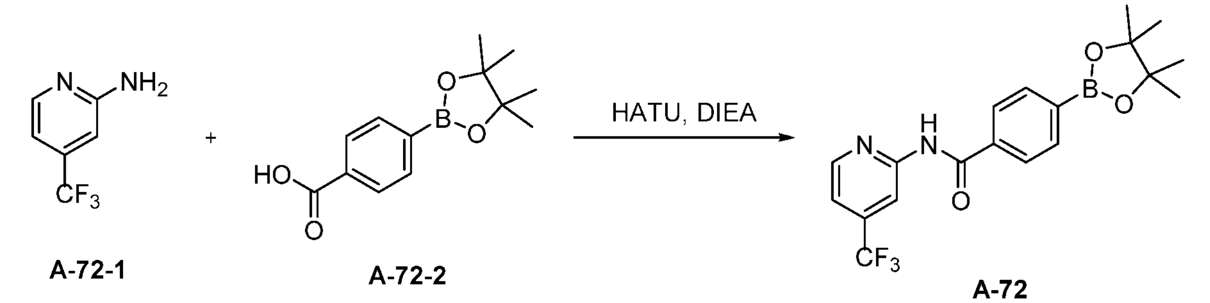


A-72-1 A-72-2 A-72

To a reaction flask, compound A-72-2 (2.00 g, 8.06 mmol), DMF (15 mL), A-72-1 (1.31 g, 8.06 mmol), DIEA (3.12 g, 24.2 mmol) and HATU (4.60 g, 12.1 mmol) were added. The reaction solution was heated to 60°C for reaction overnight. The reaction solution was cooled, poured into water, and extracted twice with ethyl acetate. The organic phases were combined, washed with water followed by saturated brine, evaporated in vacuo, and purified by silica gel column chromatography to obtain 1.56 g of product A-72 with a yield of 49%.

**Preparation of Intermediate A-73:**

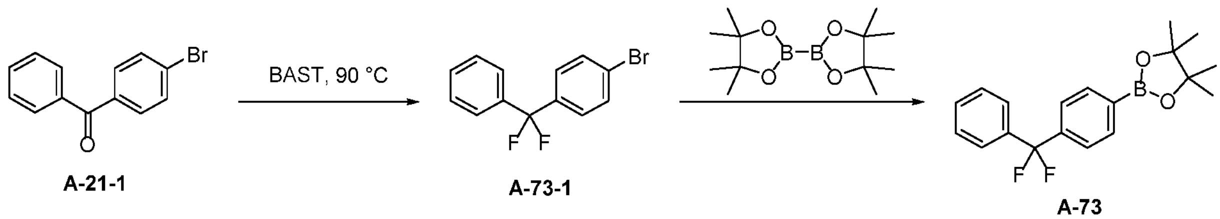


A-21-1 A-73-1 A-73

To a reaction tube, compound A-21-1 (300 mg, 1.15 mmol) and BAST (3 mL) were added. The reaction tube was sealed and heated to 90°C for reaction overnight. The reaction solution was cooled, poured into water, and extracted twice with ethyl acetate. The organic phases were combined, washed with saturated brine, dried over anhydrous sodium sulfate, evaporated in vacuo, and purified by a preparative silica gel plate to obtain 270 mg of product A-73-1 with a yield of 83%.

The synthesis method of A-73 was synthesized with reference to the method of synthesizing A-2 from A-2-3.

**Preparation of Intermediate A-74:**

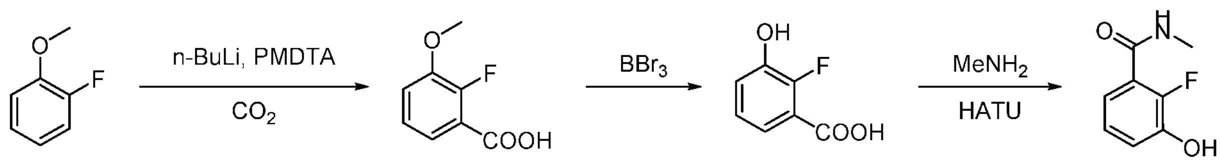


A-74-1 A-74-2 A-74-3 A-74-4

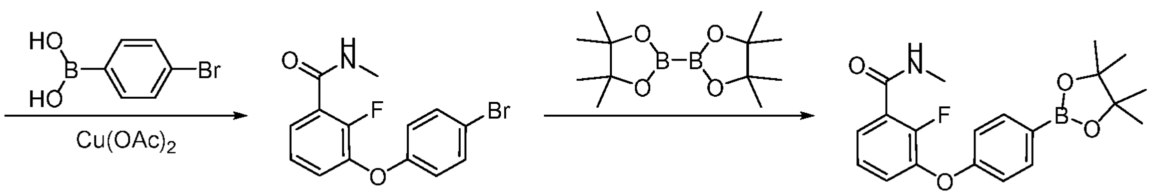


A-74-5 A-74

To a reaction flask, compound A-74-1 (1.00 g, 7.93 mmol), PMDTA(1.44 g, 8.32 mmol) and tetrahydrofuran

(10 mL) were added. The reaction solution was subjected to nitrogen replacement, and cooled in a dry ice/ethanol bath to -70°C. The reaction solution was added dropwise with n-butyllithium (2.5 M, 3.3 mL, 8.30 mmol), stirred for 2 h at this temperature, then added with dry ice carefully, and slowly warmed to room temperature. The reaction solution was quenched with dilute hydrochloric acid, and extracted three times with dichloromethane. The organic phases were combined, washed with saturated brine, dried over anhydrous sodium sulfate, and evaporated in vacuo to obtain 1.00 g of product A-74-2 with a yield of 74%. The product was used directly in the next step without further purification.

To a reaction flask, compound A-74-2 (800 mg, 4.70 mmol) and dichloromethane (8 mL) were added. The reaction solution was subjected to nitrogen replacement, and cooled by an ice water bath. A solution of boron tribromide in dichloromethane (17%, 27.7 g, 18.8 mmol) was added dropwise to the reaction solution. After the addition, the reaction solution was warmed to room temperature for 30 min of reaction, then cooled by an ice water bath again, and added dropwise with methanol slowly to quench the reaction. After the reaction solution was directly evaporated in vacuo, purification by silica gel column was performed to obtain 560 mg of product A-74-3 with a yield of 76%.

To a reaction flask, compound A-74-3 (560 mg, 3.59 mmol), DMF (5 mL), methylamine alcohol solution (30%, 557 mg, 5.38 mmol), DIEA (1392 mg, 10.8 mmol) and HATU (1775 mg, 4.67 mmol). The reaction solution was stirred overnight at room temperature, then poured into water, and extracted 6 times with ethyl acetate. The organic phases were combined, evaporated in vacuo, and purified by silica gel column chromatography to obtain 254 mg of product A-74-4 with a yield of 42%.

A-74 was synthesized with reference to the method of synthesizing A-17 from A-17-1.

**Preparation of Intermediate A-75:**

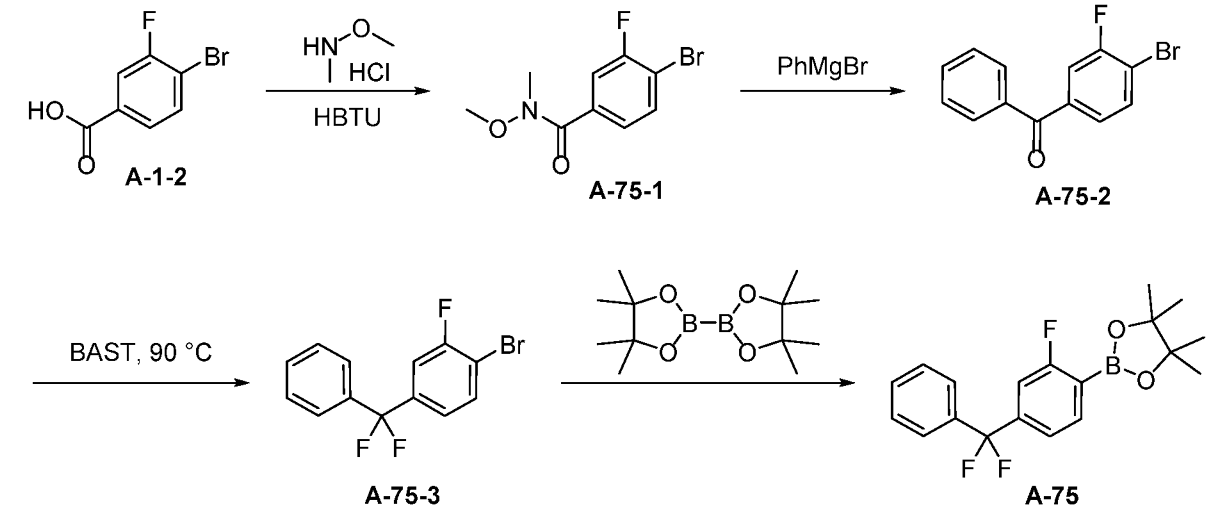


A-1-2 A-75-1 A-75-2 A-75-3 A-75

A-75-1 was synthesized with reference to the method of synthesizing A-34-2 from A-34-1.

To a reaction flask, compound A-75-1 (500 mg, 1.91 mmol) and tetrahydrofuran (8 mL) were added. The reaction solution was subjected to nitrogen replacement, and cooled by an ice-salt bath. The reaction solution was added dropwise with a solution of phenylmagnesium bromide in tetrahydrofuran (1 M, 2.3 mL, 2.3 mmol). After the addition, the reaction solution was slowly warmed to room temperature and stirred for 1 hour. The reaction solution was quenched with dilute hydrochloric acid, and extracted twice with ethyl acetate. The organic phases were combined, washed with water followed by saturated brine, dried over anhydrous sodium sulfate, and evaporated in vacuo to obtain 538 mg of product A-75-2 with a yield of 100%. The product was used directly in the next step without further purification.

A-75 was synthesized with reference to the method of synthesizing A-73 from A-21-1.

**Preparation of Intermediate A-76:**

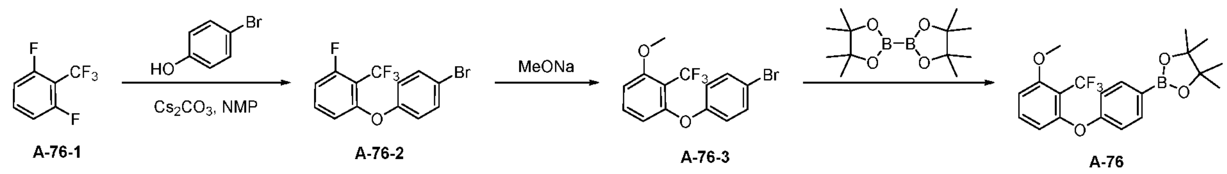


A-76-1 A-76-2 A-76-3 A-76

To a reaction flask, compound A-76-1 (200 mg, 1.10 mmol), p-bromophenol (228 mg, 1.32 mmol), NMP (2 mL) and cesium carbonate (538 mg, 1.65 mmol) were added. The reaction solution was heated to 80°C for reaction overnight. The reaction solution was cooled, poured into water, and extracted twice with ethyl acetate. The organic phases were combined, washed with water followed by saturated brine, evaporated in vacuo, and purified by silica gel column chromatography to obtain 280 mg of product A-76-2 with a yield of 76%.

To a reaction flask, compound A-76-2 (200 mg, 0.597 mmol), sodium methoxide (161 mg, 2.98 mmol) and NMP (2 mL) were added. The reaction solution was heated to 80°C for reaction overnight. The reaction solution was cooled, poured into water, and extracted twice with ethyl acetate. The organic phases were combined, washed with water followed by saturated brine, evaporated in vacuo, and purified by silica gel column chromatography to obtain 116 mg of product A-76-3 with a yield of 56%.

A-76 was synthesized with reference to the method of synthesizing A-2 from A-2-3.

**Preparation of Intermediate A-77:**

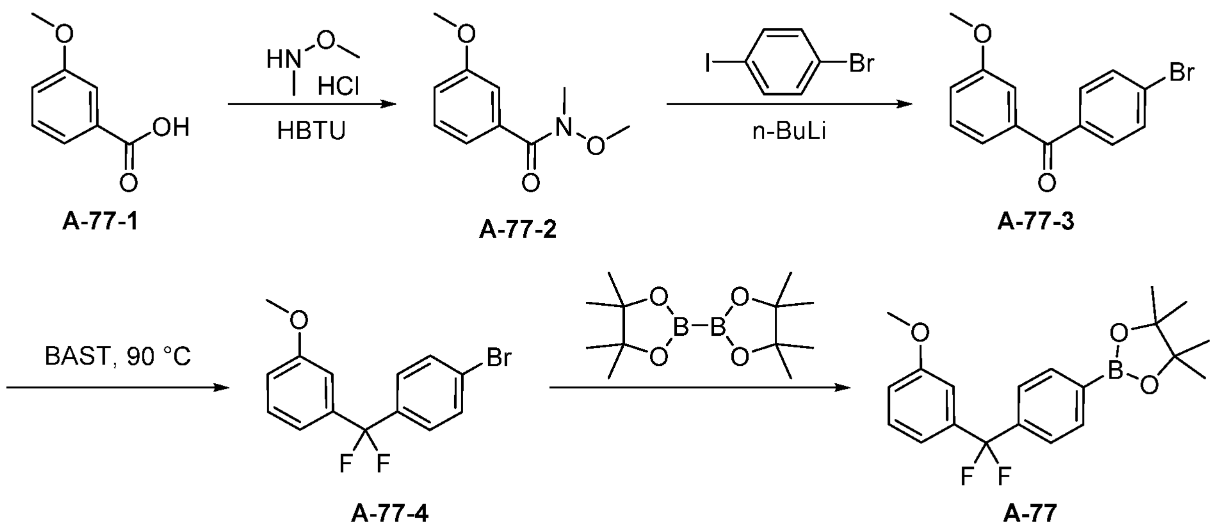


A-77-1 A-77-2 A-77-3 A-77-4 A-77

A-77-3 was synthesized with reference to the method of synthesizing A-34-3 from A-34-1.

The synthesis method of A-77 was synthesized with reference to the method of synthesizing A-73 from A-21-1.

**Preparation of Intermediate A-78:**

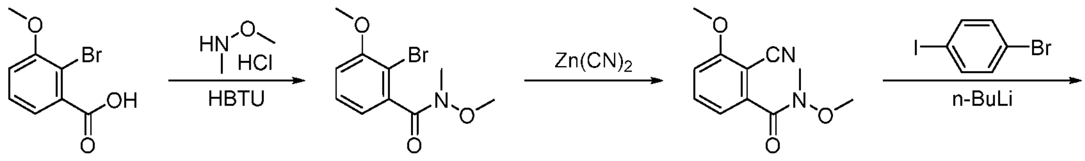


A-78-1 A-78-2 A-78-3

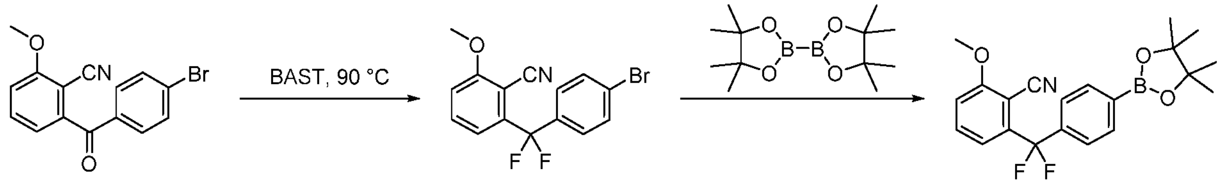


A-78-4 A-78-5 A-78

A-78-2 was synthesized with reference to the method of synthesizing A-34-2 from A-34-1.

To a reaction flask, compound A-78-2 (474 mg, 1.73 mmol), zinc cyanide (305 mg, 2.59 mmol), DMA (5 mL), zinc powder (47 mg), dppf (94 mg) and $Pd_2(dba)_3$ (94 mg), The reaction solution was subjected to nitrogen replacement, heated to 110 °C overnight. The reaction solution was cooled, poured into water, and extracted twice with ethyl acetate. The organic phases were combined, washed with saturated brine, evaporated in vacuo, and purified by silica gel column chromatography to obtain 473 mg of product A-78-3 with a yield of 100%.

A-78-4 was synthesized with reference to the method of synthesizing A-34-3 from A-34-2.

A-78 was synthesized with reference to the method of synthesizing A-73 from A-21-1.

The following compounds were prepared from commercially available corresponding raw materials according to the method for preparing the above intermediates.

Table 10: Structure and Synthesis of Intermediates A-79 to A-81

| Intermediate | Structural formula | Raw material | Reference synthetic method |
|---|---|---|---|
| A-79 | 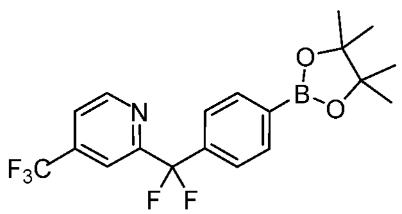 | 4-Trifluoromethylpyridine-2-carboxylic acid<br>p-Bromoiodobenzene | A-77 |
| A-80 | 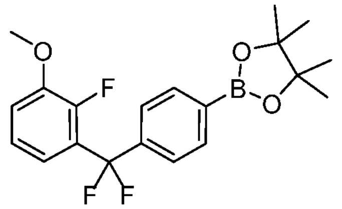 | A-34-3<br>p-Bromoiodobenzene | A-73 |
| A-81 | 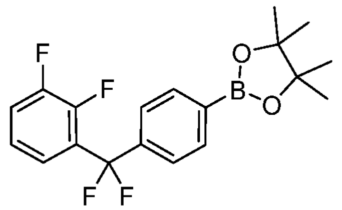 | 2,3-Difluorobenzoic acid<br>p-Bromoiodobenzene | A-77 |

**Preparation of Intermediate A-82:**

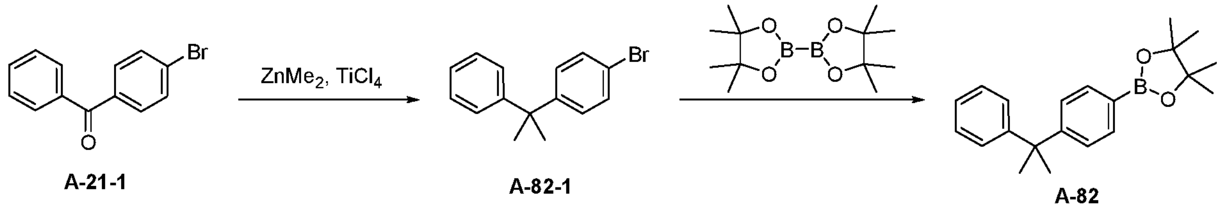

A-21-1 A-82-1 A-82

Dichloromethane (5 mL) was added to a reaction flask, subjected to nitrogen replacement, cooled in a dry ice/acetonitrile bath to -40°C, added with titanium tetrachloride (1453 mg, 7.66 mmol), and then slowly added dropwise with a solution of dimethyl zinc in toluene (1 M, 7.7 mL, 7.7 mmol). After the addition, the reaction was carried out for 30 min at a constant temperature. To the reaction solution, a solution of compound A-21-1 (500 mg, 1.91 mmol) in dichloromethane (3 mL) was added dropwise, kept at a constant temperature for 1 h of reaction after the addition, then slowly warmed to room temperature and stirred overnight. The reaction solution was quenched with water, and extracted twice with dichloromethane. The organic phases were combined, washed with water, evaporated in vacuo, and purified by silica gel column chromatography to obtain 326 mg of product A-82-1 with a yield of 62%.

A-82 was synthesized with reference to the method of synthesizing A-2 from A-2-3.

The following compounds were prepared from commercially available corresponding raw materials according to the method for preparing the above intermediates.

Table 12: Structure and Synthesis of Intermediates A-83 to A-100

| Intermedi ate | Structural formula | Raw material | Reference synthetic method |
|---|---|---|---|
| A-83 | 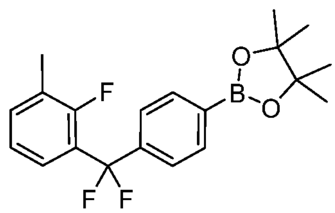 | 2-Fluoro-3-methylbenzoic acid<br>p-Bromoiodobenzene | A-77 |
| A-84 | 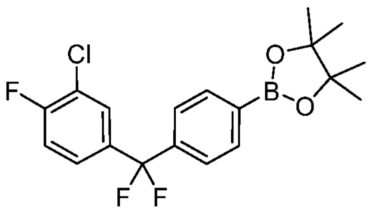 | 3-Chloro-4-fluorobenzoic acid<br>p-Bromoiodobenzene | A-77 |
| A-85 | 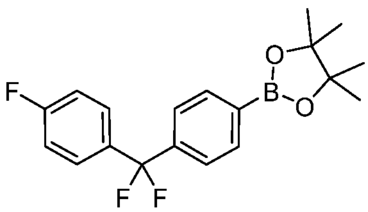 | p-Fluorobenzoic acid<br>p-Bromoiodobenzene | A-77 |
| A-86 | 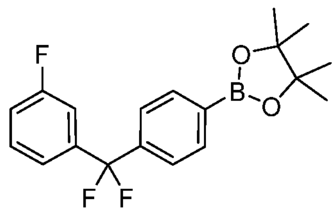 | m-Fluorobenzoic acid<br>p-Bromoiodobenzene | A-77 |

(continued)

| Intermedi ate | Structural formula | Raw material | Reference synthetic method |
|---|---|---|---|
| A-87 | 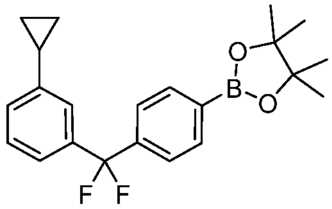 | m-Bromobenzoic acid<br>p-Bromoiodobenzene | A-18-2 A-77 |
| A-88 | 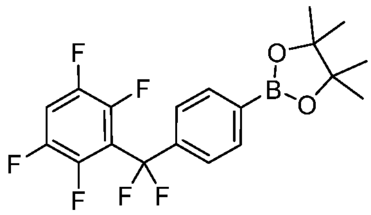 | 2,3,5,6-tetrafluorobenzoic acid<br>p-Bromoiodobenzene | A-77 |
| A-89 | 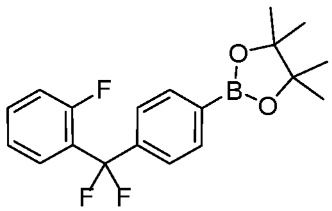 | o-Fluorobenzoic acid<br>p-Bromoiodobenzene | A-77 |
| A-90 | 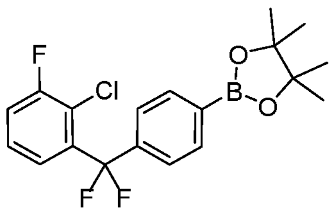 | 2-Chloro-3-fluorobenzoic acid<br>p-Bromoiodobenzene | A-77 |
| A-91 | 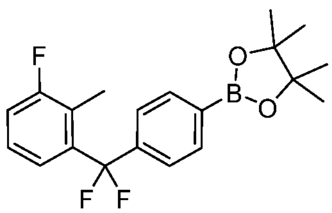 | 3-Fluoro-2-methylbenzoic acid<br>p-Bromoiodobenzene | A-77 |
| A-92 | 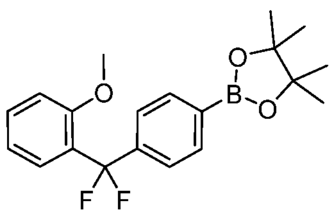 | o-Methoxybenzoic acid<br>p-Bromoiodobenzene | A-77 |
| A-93 | 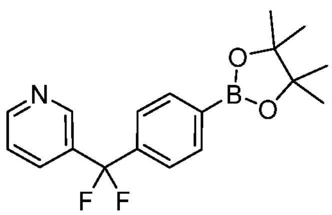 | Niacin<br>p-Bromoiodobenzene | A-77 |

(continued)

| Intermedi ate | Structural formula | Raw material | Reference synthetic method |
|---|---|---|---|
| A-94 | 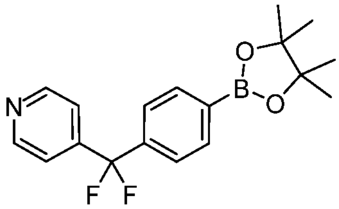 | Isonicotinic acid<br><br>p-Bromoiodobenzene | A-77 |
| A-95 | 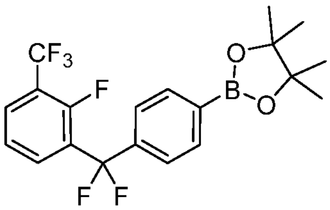 | 2-Fluoro-3-(trifluoromethyl) benzoi c acid<br><br>p-Bromoiodobenzene | A-77 |
| A-96 | 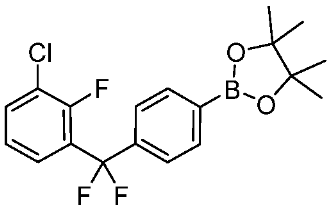 | 3-Chloro-2-fluorobenzoic acid<br><br>p-Bromoiodobenzene | A-77 |
| A-97 | 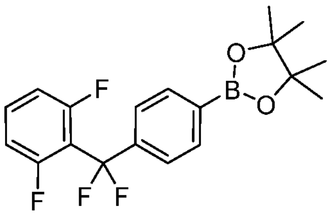 | 2,6-Difluorobenzoic acid<br><br>p-Bromoiodobenzene | A-77 |
| A-98 | 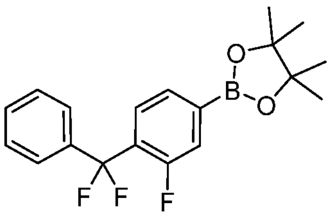 | 4-Bromo-2-fluorobenzoic acid<br><br>Phenyl magnesium bromide | A-75 |
| A-99 | 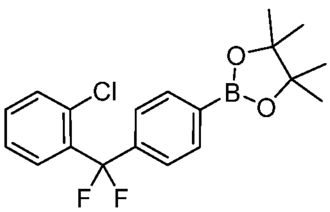 | 2-Chlorobenzoic acid<br><br>p-Bromoiodobenzene | A-77 |
| A-100 | 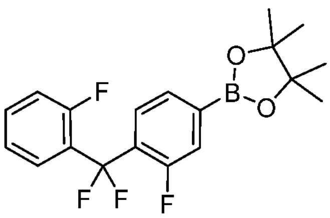 | o-Fluorobenzoic acid<br><br>1-Bromo-3-fluoro-4-iodobenzene | A-77 |

**Preparation of Intermediate A-101:**

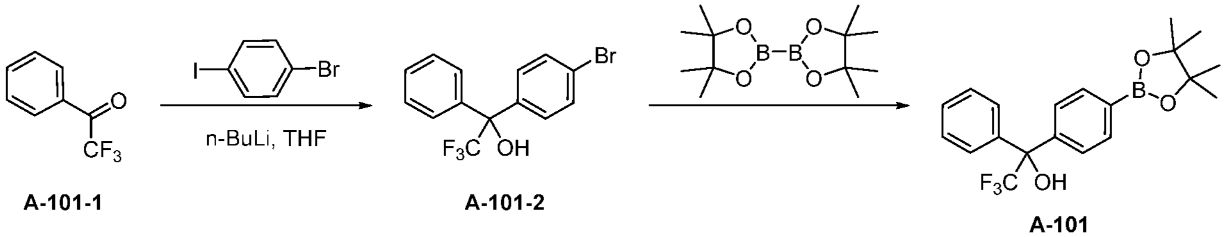


A-101-1 A-101-2 A-101

p-Bromoiodobenzene (1.71 g, 6.03 mmol) and tetrahydrofuran (15 mL) were added to a reaction flask, subjected to nitrogen replacement, cooled in a dry ice/ethanol bath to -70°C, then slowly added dropwise with n-butyllithium (2.5 M, 2.4 mL, 6.03 mmol). After the addition, the reaction was carried out for 30 min at a constant temperature. A-101-1 (1.00 g, 5.74 mmol) was dissolved in tetrahydrofuran (5 mL), and added dropwise to the reaction solution. After 10 min, the reaction solution was slowly warmed to room temperature. The reaction solution was quenched with water, and extracted twice with ethyl acetate. The organic phases were combined, washed with water, evaporated in vacuo, and purified by silica gel column chromatography to obtain 1.4 g of product A-101-2 with a yield of 73%.
A-101 was synthesized with reference to the method of synthesizing A-2 from A-2-3.

**Preparation of Intermediate A-102:**

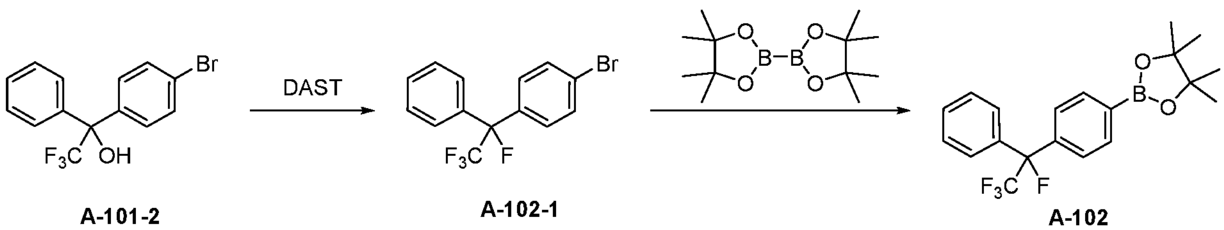


A-101-2 A-102-1 A-102

A-101-2 (550 mg, 1.66 mmol) and dichloromethane (6 mL) were added to a reaction flask, subjected to nitrogen replacement, added with DAST (402 mg, 2.49 mmol) in an ice bath, and kept at a constant temperature for 2 h of reaction. The reaction solution was quenched with aqueous sodium bicarbonate solution, and extracted twice with dichloromethane. The organic phases were combined, dried over anhydrous sodium sulfate, evaporated in vacuo, and purified by a preparative silica gel plate to obtain 410 mg of product A-102 with a yield of 74%.
A-102 was synthesized with reference to the method of synthesizing A-2 from A-2-3.

**Preparation of Intermediate A-103:**

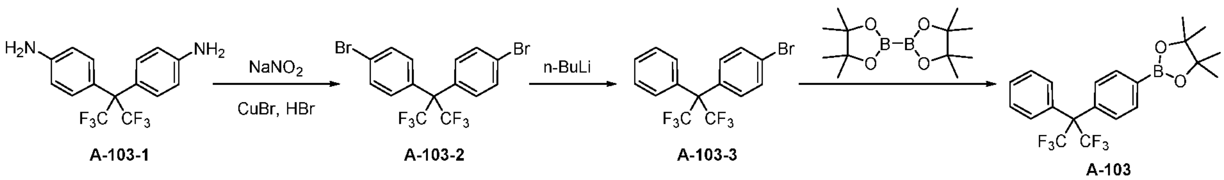


A-103-1 A-103-2 A-103-3 A-103

A-103-1 (500 mg, 1.50 mmol) and aqueous hydrobromic acid solution (5 mL) were added to a reaction flask, cooled by an ice bath, added with sodium nitrite (645 mg, 9.35 mmol), and then kept at a constant temperature for 20 min of reaction. The reaction solution was added with CuBr (2.69 g, 18.75 mmol) in aqueous hydrobromic acid solution (5 mL), and slowly warmed to room temperature for 3 h of reaction. The reaction solution was diluted with water, and extracted twice with ethyl acetate. The organic phases were combined, washed with water, evaporated in vacuo, and

purified by silica gel column chromatography to obtain 620 mg of product A-103-2 with a yield of 89%.

A-103-2 (200 mg, 0.43 mmol) and tetrahydrofuran (5 mL) were added to a reaction flask, subjected to nitrogen replacement, cooled in a dry ice/ethanol bath to -70°C, then slowly added dropwise with n-butyllithium (2.5 M, 0.17 mL, 0.43 mmol). After the addition, the reaction was kept at a constant temperature for 1 h. The reaction solution was quenched with dilute hydrochloric acid, and extracted twice with ethyl acetate. The organic phases were combined, dried over anhydrous sodium sulfate, and evaporated in vacuo to obtain 170 mg crude product A-103-3. The crude product was directly used in the next step without purification.

A-103 was synthesized with reference to the method of synthesizing A-2 from A-2-3.

**Preparation of Intermediate B-1:**

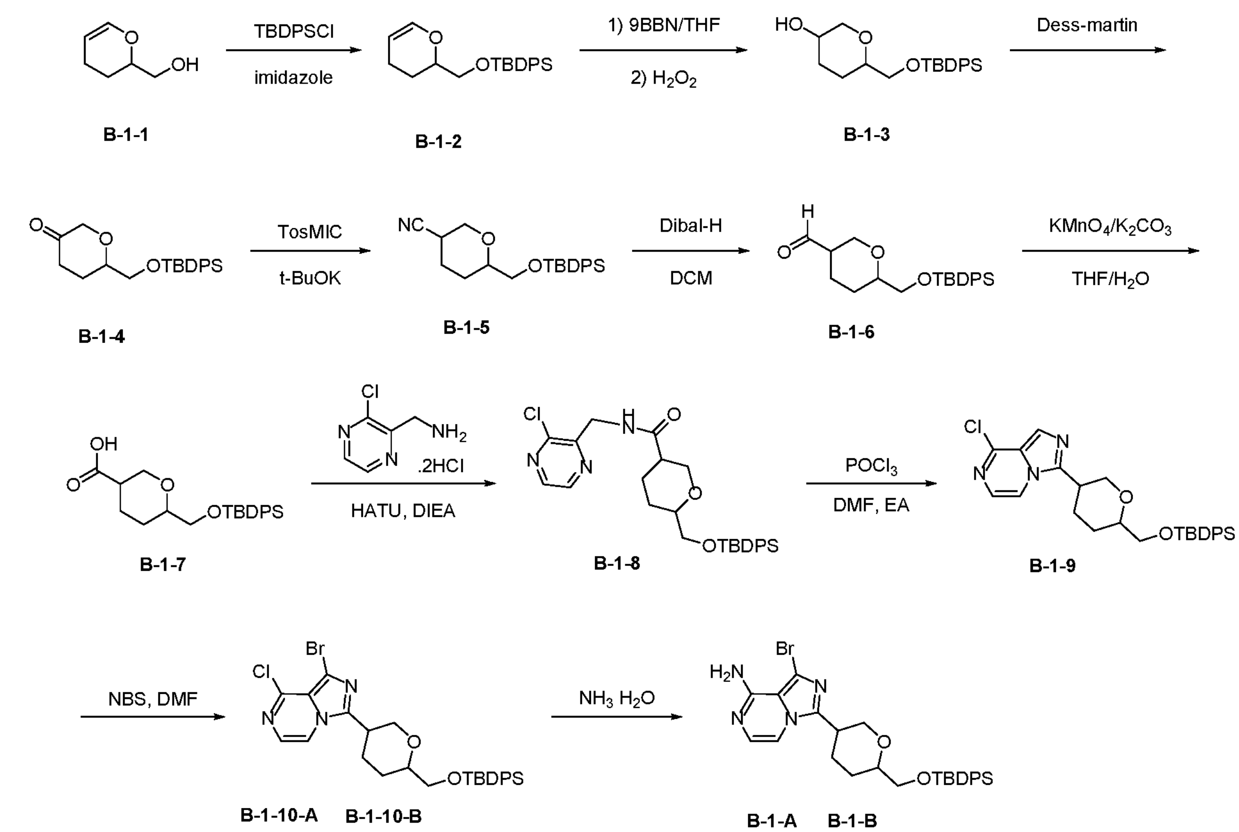


B-1-1 B-1-2 B-1-3 B-1-4 B-1-5 B-1-6 B-1-7 B-1-8 B-1-9 B-1-10-A B-1-10-B B-1-A B-1-B

To a reaction flask, compound B-1-1 (2.00 g, 17.5 mmol), imidazole (1.43 g, 21.0 mmol) and DMF solution (10 mL) were added. The reaction solution was subjected to nitrogen replacement, and added with TBDPSCl (5.30 g, 19.3 mmol) in an ice bath. After the addition, the ice was removed, the reaction mixture was stirred at room temperature for 16 h, as TLC showed that the reaction was complete. The reaction solution was poured into water, extracted twice with ethyl acetate. The organic phases were combined, washed twice with water, then washed with saturated NaCl solution, finally dried over anhydrous $Na_2SO_4$, and directly evaporated in vacuo to obtain 6.68 g product B-1-2. The product was directly used in the next step without purification.

B-1-2 (6.68 g, 18.9 mmol) in tetrahydrofuran (35 mL) was added to a reaction flask. The reaction solution was subjected to nitrogen replacement, and added dropwise with 9-BBN (0.5 M, 91 mL) under an ice water bath. After the addition, the reaction mixture was stirred at room temperature for 16 h, as TLC showed that the raw materials reacted completely. The reaction solution was cooled again with an ice water bath, slowly added with 10% NaOH solution (24 mL) and 30% $H_2O_2$ solution (12 mL), and stirred for 1 h, as TLC showed that the intermediate reacted completely. The reaction solution was poured into water, extracted twice with ethyl acetate, combined, washed twice with water, washed with saturated NaCl solution, directly mixed with silica gel, and then purified by silica gel column chromatography to obtain 6.49 g of product B-1-3, with a two-step yield of 100%.

To a reaction flask, a solution of compound B-1-3 (6.49 g, 17.5 mmol) in dichloromethane (50 mL) was added. The reaction solution was subjected to nitrogen replacement. Dess-Martin periodinane (11.14 g, 26.3 mmol) was added

to the reaction flask in an ice water bath and stirred for 1.5 h, as TLC showed that the reaction was complete. The reaction solution was directly mixed with silica gel, and then purified by silica gel column to obtain 6.45 g of product B-1-4 with a yield of 100%.

Compound B-1-4 (6.45 g, 17.5 mmol), ethanol (926 mg, 20.1 mmol), DME (60 mL) and TosMIC (3.92 g, 20.1 mmol) were added to a reaction flask, subjected to nitrogen replacement, and cooled by an ice water bath. The reaction solution was added with t-BuOK (3.83 g, 34.1 mmol), stirred for 30 min, then slowly warmed to room temperature, and stirred for 1.5 h. TLC showed the raw materials reacted completely. The reaction solution was poured into saturated $NH_4Cl$ solution (350 mL), and extracted twice with ethyl acetate. The organic phases were combined, mixed with silica gel, and then purified by silica gel column chromatography to obtain 2.27 g of product B-1-5 (TLC showed two spots) with a yield of 34%.

To a reaction flask, a solution (35 mL) of B-1-5 (2.27 g, 5.97 mmol) in dichloromethane was added. The reaction solution was subjected to nitrogen replacement, cooled in a dry ice-ethanol bath, slowly added dropwise with Dibal-H (1M, 9 mL), and stirred at this temperature for 1.5 h, as TLC showed that the reaction was complete. The reaction solution was added to cold dilute hydrochloric acid (1 M, 10 mL), stirred until no bubbles, and then added with DCM to extract twice. The organic phase was combined, washed with saturated NaCl solution, and finally dried over anhydrous $Na_2SO_4$. The reaction solution was directly evaporated in vacuo to obtain 2.22 g of product B-1-6 with a yield of 97%.

To a reaction flask, compound B-1-6 (2.22 g, 5.81 mmol), THF (60 mL), water (30 mL) and $K_2CO_3$ (4. 8 2 g, 34.8 mmol) were added, and then $KMnO_4$ (3.67 g, 23.2 mmol) was added in portions. The reaction solution was stirred at room temperature for 30 min, as TLC showed that the raw materials reacted completely. The reaction solution was added with dilute hydrochloric acid to adjust the pH, then added into $NaHSO_3$ solution until the reaction solution became colorless, and extracted twice with ethyl acetate. The organic phases were combined, washed with saturated NaCl solution, and finally dried over anhydrous $Na_2SO_4$. The reaction solution was directly evaporated in vacuo to obtain 1.89 g of product B-1-7 with a yield of 82%.

B-1-7 (1.89 g, 4.74 mmol), (3-chloropyrazin-2-yl)methanamine dihydrochloride (1.03 g, 4.74 mmol) and DMF (20 mL) were added to a reaction flask, subjected to nitrogen replacement, cooled by an ice water bath, and added with HATU (2.16 g, 5.69 mmol) and DIEA (3.06 g, 23.7 mmol). The reaction solution was stirred for 30 min of reaction in the ice bath and stirred for another 30 min at room temperature. TLC showed the raw materials reacted completely. The reaction solution was poured into water, extracted twice with ethyl acetate. The organic phases were combined, washed twice with water, then with saturated NaCl solution, mixed with silica gel, and then purified by silica gel column chromatography to obtain 1.94 g of product B-1-8 (TLC showed two spots) with a yield of 78%.

Compound B-1-8 (790 mg, 1.51 mmol), DMF (0.8 mL) and ethyl acetate (8 mL) were added to a reaction flask, and subjected to nitrogen replacement. The reaction solution was added dropwise with phosphorus oxychloride (1.39 g, 9.08 mmol) under an ice water bath, and stirred for 1 h. TLC showed the raw materials reacted completely. The reaction solution was poured into $NaHCO_3$ solution (4.5 g $NaHCO_3$/30 mL $H_2O$) to quench the reaction, extracted twice with ethyl acetate. The organic phases were combined, washed twice with water, then washed with saturated NaCl solution, finally dried over anhydrous $Na_2SO_4$, and directly evaporated in vacuo to obtain 680 mg of product B-1-9 (TLC showed two spots) with a yield of 89.0%.

A solution of B-1-9 (680 mg, 1.34 mmol) in DMF (7 mL) was added to a reaction flask. The reaction solution was subjected to nitrogen replacement, added into NBS (287 mg, 1.61 mmol) under an ice water bath, and stirred for 40 min, as TLC showed that the reaction was complete. The reaction solution was poured into water to quench the reaction, extracted twice with ethyl acetate. The organic phases were combined, washed twice with water, then washed with saturated NaCl solution, mixed with silica gel, and then purified by silica gel column chromatography to obtain 298 mg of product B-1-10-A (spot with low polarity on TLC, eluted first) and 339 mg of product B-1-10-B (spot with high polarity on TLC, eluted second) with an overall yield of 81%.

To a high pressure digestion tank, compound B-1-10-A (298 mg, 0.509 mmol), ammonia water (6 mL) and n-butanol solution (3 mL) were added. The reaction bottle was sealed, heated to 95°C and stirred for 16 h. The reaction solution was cooled, spin-dried in vacuo, and then purified by silica gel column chromatography to obtain 184 mg of product B-1-A with a yield of 64%.

B-1-B was prepared by reacting B-1-10-B with ammonia water according to the method for synthesizing B-1-A.

**Preparation of Intermediate B-2:**

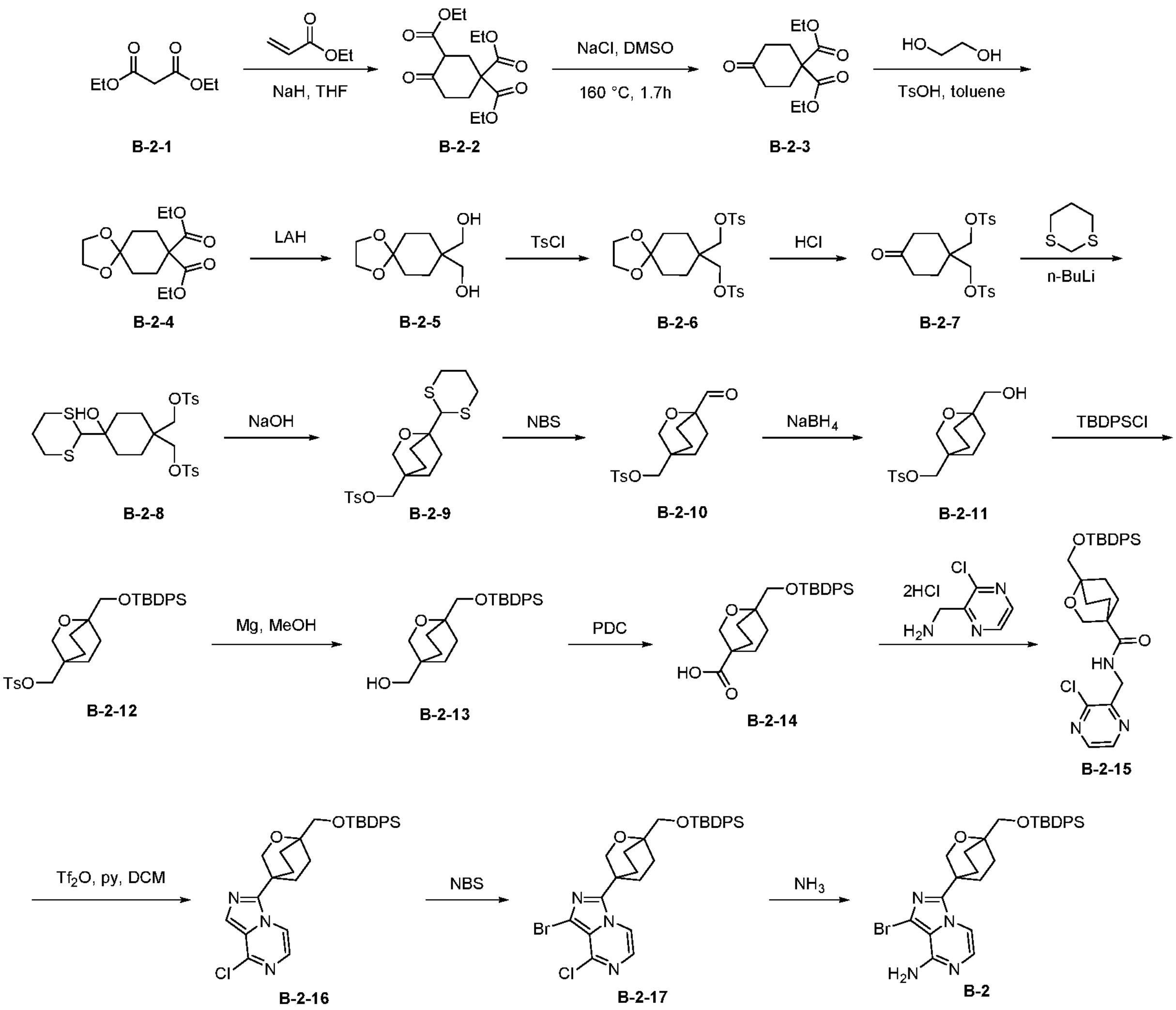


B-2-1 B-2-2 B-2-3 B-2-4 B-2-5 B-2-6 B-2-7 B-2-8 B-2-9 B-2-10 B-2-11 B-2-12 B-2-13 B-2-14 B-2-15 B-2-16 B-2-17 B-2

NaH (8.56 g, 357 mmol) was suspended in anhydrous THF (80 mL), heated to 40-45°C, and added dropwise with a solution of compound B-2-1 (22.85 g, 149 mmol) in THF. After the addition, the reaction was kept at this temperature and stirred for 15 min. After a solution of ethyl acrylate in THF was added dropwise, the reaction was continued for another 15 min. After being cooled to room temperature, the reaction solution was added to ice water, added with concentrated HCl to adjust the pH to 3, and extracted twice with ethyl acetate. The organic phases were combined, dried over anhydrous sodium sulfate, filtered, concentrated under reduced pressure, and purified by column chromatography to obtain compound B-2-2 as colorless oil (37.6 g, 83%).

Compound B-2-2 (37.6 g, 120 mmol) and sodium chloride (20.97 g, 359 mmol) were added to DMSO (170 mL) and $H_2O$ (5 mL) to react at 160°C for 1.7 h. The reaction solution was cooled, added to ice water, and extracted with ethyl acetate. The organic phase was dried over anhydrous sodium sulfate, filtered, and concentrated under reduced pressure. After purification by column chromatography, compound B-2-3 was obtained as colorless oil (21.6 g, 75%).

Compound B-2-3 (21.6 g, 89.2 mmol), ethylene glycol (6.64 g, 107 mmol) and p-toluenesulfonic acid-hydrate (169 mg, 0.89 mmol) were added to toluene (180 mL), and refluxed at 120°C for 4 h. The reaction solution was cooled, added to saturated $NaHCO_3$ aqueous solution, and extracted with ethyl acetate. The organic phase was dried over anhydrous sodium sulfate, filtered, concentrated under reduced pressure, and purified by column chromatography to obtain compound B-2-4 as a light yellow liquid (23.7 g, 93%).

To a three-necked flask where LAH (6.47 g, 166 mmol) was weighed out, anhydrous THF (150 mL) was added. After nitrogen replacement, a solution of compound B-2-4 (23.7 g, 82.8 mmol) in THF (100 mL) was added dropwise under an ice-salt bath. After the addition, the reaction solution was slowly warmed to room temperature for 5 h of reaction. To the reaction solution, $H_2O$/THF (1:1, 30 mL) was slowly added dropwise under an ice water bath, then 5 N aqueous sodium hydroxide solution (8 mL) was added, and stirred overnight at room temperature. The reaction solution was diluted by adding DCM/MeOH (5:1, 250 mL) in the reaction flask, filtered and rinsed with DCM/MeOH (5:1). The filtrate was added with 50 g of silica gel, stirred for 15 min, filtered, and rinsed. The filtrate was concentrated under reduced

pressure to obtain compound B-2-5 (16.7 g, 99%).

To a reaction flask where compound B-2-5 (16.7 g, 82.6 mmol) was weighed out, pyridine (100 mL) was added, then TsCl (34.6 g, 182 mmol) was added under an ice water bath, and stirred overnight at room temperature. The reaction solution was diluted with ethyl acetate, and washed with 10% citric acid solution and saturated sodium chloride. The organic phase was dried over anhydrous sodium sulfate, filtered, and concentrated under reduced pressure. The obtained crude product was pulped with ethanol to obtain compound B-2-6 as a white solid (35 g, 83%).

To a reaction flask, compound B-2-6 (35 g, 68.5 mmol) was weighed out, 1 N HCl solution (260 mL) and THF (300 mL) were added to react at 80°C for 5 h. The reaction solution was extracted with ethyl acetate. The organic phase was washed with water, dried over anhydrous sodium sulfate, filtered, and concentrated under reduced pressure. After purification by column chromatography, compound B-2-7 (26.2 g, 82%) was obtained.

To a reaction flask, 1,3-dithiane (2.1 g, 17.4 mmol) and anhydrous THF (40 mL) were added. After nitrogen replacement, n-butyl lithium (2.5 M, 8.5 mL) was added dropwise under the cooling bath of dry ice-ethanol, and then warmed to 0°C for 1 h of reaction after the addition. Under cooling bath of dry ice-ethanol, a solution of compound B-2-7 (6.5 g, 13.9 mmol) in THF was added dropwise, and warmed, after the addition, to room temperature for 1 h of reaction. The reaction solution was added to saturated $NH_4Cl$ solution to quench the reaction, and extracted with ethyl acetate. The organic phase was dried over anhydrous sodium sulfate, filtered, and concentrated under reduced pressure. After purification by column chromatography, compound B-2-8 (6.77 g, 83%) was obtained.

Compound B-2-8 (6.77 g, 11.5 mmol), NaOH (1.38 g, 34.6 mmol) and THF (170 mL) were added into a reaction flask and refluxed overnight at 70°C. The reaction liquid was cooled to room temperature, added with water, and extracted with ethyl acetate. The organic phase was dried over anhydrous sodium sulfate, filtered, and concentrated under reduced pressure. After purification by column chromatography, compound B-2-9 (3.7 g, 77%) was obtained.

To a reaction flask, compound B-2-9 (3.7 g, 8.92 mmol), acetonitrile (50 mL) and water (12.5 mL) were added, and then NBS (5.56 g, 31.2 mmol) was added under an ice water bath. After the addition, the mixture was allowed to react at room temperature for 3 h. The reaction solution was added to saturated $NaHCO_3$ solution, and extracted with ethyl acetate. The organic phases were combined, backwashed with saturated NaCl solution, dried over anhydrous sodium sulfate, filtered, and concentrated under reduced pressure. A crude product of compound B-2-10 was obtained, without further purification.

The crude product of compound B-2-10 obtained above was dissolved in ethanol, and added into NaBH4 (508 mg, 13.4 mmol) under an ice water bath. The mixture was allowed to react for 1 h at room temperature. The reaction solution was added into saturated $NH_4Cl$ solution to quench the reaction and extracted with ethyl acetate. The organic phase was dried over anhydrous sodium sulfate, filtered, and concentrated under reduced pressure. After purification by column chromatography, compound B-2-11 (2.66 g, two-step yield of 91%) was obtained.

To a reaction flask, compound B-2-11 (2.56 g, 7.84 mmol), DMAP (287 mg, 2.35 mmol), imidazole (1.06 g, 15.7 mmol) and DMF (15 mL), and then TBDPSCl (2.59 g, 9.41 mmol) was added. The mixture was allowed to react for 0.5 h at room temperature. The reaction solution was added to water and extracted with ethyl acetate. The organic phases were combined, backwashed with saturated NaCl solution, dried over anhydrous sodium sulfate, filtered, and concentrated under reduced pressure. After purification by column chromatography, compound B-2-12 (4.0 g, 90%) was obtained.

Compound B-2-12 (4.0 g, 7.08 mmol) and methanol (120 mL) were added to a reaction flask, and added with Mg (1.89 g, 77.9 mmol) under stirring at room temperature. After 30 min, the reaction was exothermic violently, and stirred overnight. The reaction solution was added to saturated $NH_4Cl$ solution, and extracted with ethyl acetate. The organic phase was dried over anhydrous sodium sulfate, filtered, and concentrated under reduced pressure. After purification by column chromatography, compound B-2-13 (2.4 g, 83%) was obtained.

To a reaction flask, compound B-2-13 (2.4 g, 5.85 mmol) and DMF (30 mL) were added, and then PDC (6.6 g, 17.6 mmol) was added under an ice water bath. After the addition, the mixture was allowed for 2 h of reaction at room temperature. The reaction solution was diluted by adding ethyl acetate in the reaction flask, and extracted with water. The organic phases were combined, backwashed with saturated NaCl solution, dried over anhydrous sodium sulfate, filtered, and concentrated under reduced pressure. After purification by column chromatography, compound B-2-14 (1.99 g, 80%) was obtained.

To a reaction flask, compound B-2-14 (1.99 g, 4.69 mmol), (3-chloropyrazin-2-yl)methanamine dihydrochloride (1.02 g, 4.69 mmol) and DMF (10 mL) were added. Then the reaction solution was added with HBTU (2.13 g, 5.62 mmol) and DIEA (2.42 g, 18.8 mmol) to react at room temperature for 1 h. The reaction solution was added to water, and extracted twice with ethyl acetate. The organic phases were combined, backwashed with saturated NaCl solution, dried over anhydrous sodium sulfate, filtered, and concentrated under reduced pressure. After purification by column chromatography, compound B-2-15 (1.99 g, 77%) was obtained.

To a reaction flask, compound B-2-15 (1.59 g, 2.89 mmol) was added and dissolved in DCM (30 mL) under nitrogen gas protection. Then, pyridine (1.83 g, 23.1 mmol) and trifluoromethanesulfonic anhydride (4.89 g, 17.3 mmol) were added under an ice water bath. After the addition, the mixture was allowed to react for 4 h at room temperature.

The reaction solution was added into saturated $NaHCO_3$ solution and extracted with ethyl acetate. The organic phases were combined, backwashed with saturated NaCl solution, dried over anhydrous sodium sulfate, filtered, and concentrated under reduced pressure to obtain a crude product of compound B-2-16 without further purification.

The crude product of compound B-2-16 obtained above was dissolved in DMF (8 mL), and added into NBS (566 mg, 3.18 mmol) to react at room temperature for 0.5 h. The reaction solution was added to $NaHCO_3$ solution, and extracted with ethyl acetate. The organic phases were combined, backwashed with saturated NaCl solution, dried over anhydrous sodium sulfate, filtered, and concentrated under reduced pressure. After purification by column chromatography, compound B-2-17 (1.43 g, two-step yield of 81%) was obtained.

To a high pressure digestion tank, compound B-2-17 (1.43 g, 2.34 mmol), ammonia water (20 mL) and n-butanol (8 mL) were added. The reaction system was heated to 95°C and stirred for 16 h. The reaction solution was spin-dried in vacuo and purified by column chromatography to obtain compound B-2 (1.2 g, 87%).

**Preparation of Intermediate B-3:**

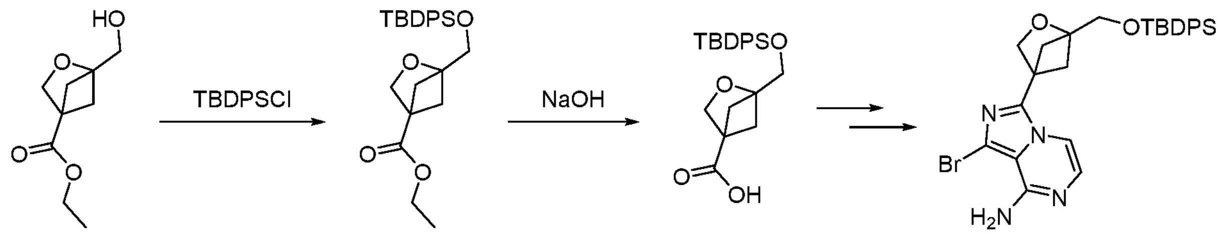


B-3-1 B-3-2 B-3-3 B-3

B-3-1 was prepared with reference to the method in literature (Angew. Chem. Int. Ed. 2020, 59, 7161-7167).

To a reaction flask, compound B-3-1 (1.25 g, 6.71 mmol), imidazole (548 mg, 8.06 mmol) and DMF (12 mL) were added, and then TBDPSCl (1.94 g, 7.05 mmol) was added. The mixture was allowed to react at room temperature overnight. The reaction solution was added to water and extracted twice with ethyl acetate. The organic phases were combined, washed with water followed by saturated NaCl solution, dried over anhydrous sodium sulfate, filtered, and concentrated under reduced pressure. Compound B-3-2 (2.85 g, 100%) was obtained, without further purification.

Compound B-3-2 (2.85 g, 6.71 mmol) was dissolved in ethanol (25 mL), and added with an aqueous solution (10 mL) of sodium hydroxide (403 mg, 10.1 mmol). The reaction solution was heated to 60°C for reaction overnight. The reaction solution was cooled, added to water, adjusted with dilute hydrochloric acid, and extracted twice with ethyl acetate. The organic phases were combined, washed with water followed by saturated NaCl solution, dried over anhydrous sodium sulfate, filtered, and concentrated under reduced pressure. Compound B-3-3 (2.53 g, 95%) was obtained, without further purification.

B-3 was prepared from B-3-3 with reference to the method for preparing B-2 from B-2-14.

**Preparation of Intermediate B-4:**

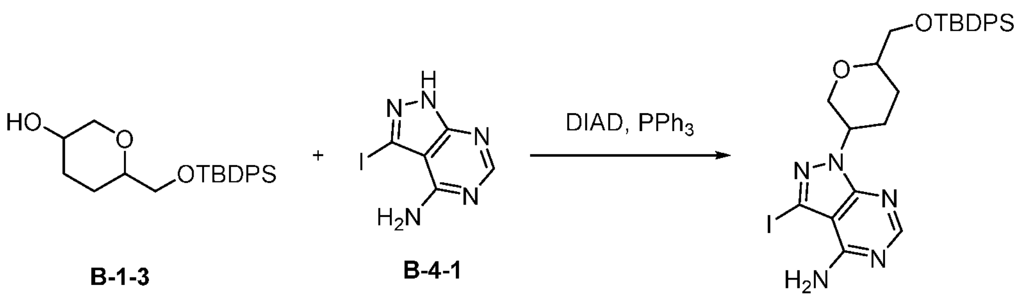


B-1-3 B-4-1 B-4-A B-4-B

To a reaction flask, compound B-4-1 (100 mg, 0.383 mmol), B-1-3 (170 mg, 0.460 mmol), triphenylphosphine (251 mg, 0.958 mmol) and THF (2 mL) were added. The reaction solution was subjected to nitrogen replacement, heated

to 60°C, added dropwise with DIAD (194 mg, 0.958 mmol), and kept at a constant temperature for reaction overnight. The reaction solution was cooled, concentrated to dryness under reduced pressure, and purified by silica gel column chromatography to obtain compound B-4-A (52 mg, spot with low polarity) and B-4-B (140 mg, spot with high polarity, containing impurity triphenoxyphosphine) with an overall yield of 82%.

**Preparation of Intermediate B-5:**

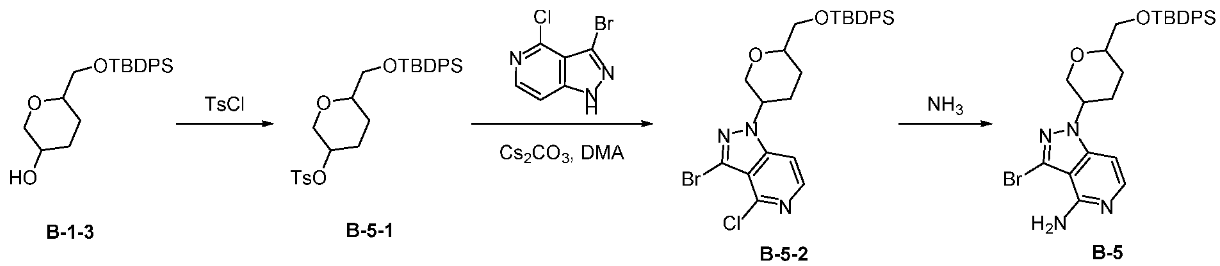


B-1-3 B-5-1 B-5-2 B-5

To a reaction flask, compound B-1-3 (500 mg, 1.35 mmol), TEA (273 mg, 2.70 mmol), DCM (5 mL) and p-toluenesulfonyl chloride (309 mg, 1.62 mmol) were added. The reaction solution was stirred at room temperature for 2 h, as TLC showed almost no reaction. The reaction solution was added with DMAP (198 mg, 1.62 mmol), to perform a reaction overnight. The reaction solution was added into water, adjusted with dilute hydrochloric acid, and extracted twice with ethyl acetate. The organic phases were combined, washed with water followed by saturated NaCl solution, concentrated under reduced pressure to dryness, and purified by silica gel column chromatography to obtain compound B-5-1 (550 mg, 78%).

To a reaction flask, compound B-5-1 (200 mg, 0.381 mmol), 3-bromo-4-chloro-1H-pyrazolo[4,3-C]pyridine (89 mg, 0.381 mmol), cesium carbonate (149 mg, 0.457 mmol) and DMA (2 mL) were added. The reaction solution was heated to 100°C and stirred overnight. The reaction solution was cooled, added into water, and extracted twice with ethyl acetate. The organic phases were combined, washed with water followed by saturated NaCl solution, concentrated under reduced pressure to dryness, and purified by a preparative silica gel plate to obtain compound B-5-2 (60 mg, 27%).

To a sealed tube, compound B-5-2 (60 mg, 0.103 mmol), ammonia water (2 mL) and n-butanol (1 mL) were added. the reaction system was heated to 100°C, and stirred overnight. The reaction solution was cooled, spin-dried in vacuo, and purified by a preparative silica gel plate to obtain compound B-5 (38 mg, 66%).

**Preparation of Intermediate B-6:**

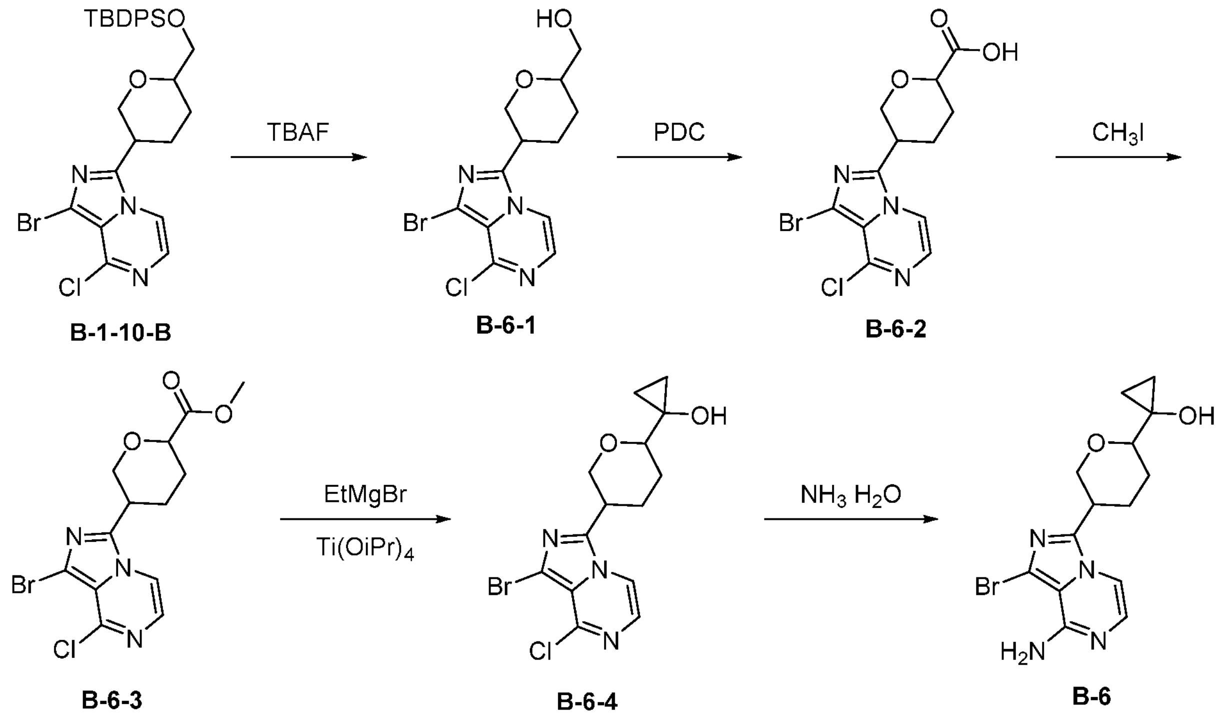


B-1-10-B B-6-1 B-6-2

B-6-3 B-6-4 B-6

To a reaction flask, compound B-1-10-B (200 mg, 0.342 mmol), THF (3 mL) and TBAF (1 M, 0.7 mL, 0.7 mmol) were added. The reaction solution was stirred at room temperature for 4 h, and directly purified by a preparative silica gel plate to obtain compound B-6-1 (108 mg, 91%).

Compound B-6-1 was oxidized with PDC (see the synthesis of B-2-14 from B-2-13) to prepare B-6-2.

To a reaction flask, compound B-6-2 (90 mg, 0.25 mmol), potassium carbonate (69 mg, 0.50 mmol), DMF (1 mL) and methyl iodide (53 mg, 0.374 mmol) were added. The reaction solution was stirred overnight at room temperature, added to water, and extracted twice with ethyl acetate. The organic phases were combined, washed with water followed by saturated NaCl solution, dried over anhydrous sodium sulfate, filtered under vacuum, concentrated under reduced pressure to dryness, and purified by a preparative silica gel plate to obtain compound B-6-3 (80 mg, 85%).

Compound B-6-3 (80 mg, 0.21 mmol) and THF (2 mL) were added to a reaction flask, subjected to nitrogen replacement, and cooled by an ice water bath. To the reaction solution, tetraisopropyl titanate (28 mg, 0.10 mmol) was added, and then ethylmagnesium bromide (0.6 mL, 0.6 mmol, 1 M) was slowly added dropwise. After the addition, the reaction solution was warmed to room temperature and stirred overnight. The reaction solution was poured into aqueous ammonium chloride solution to quench the reaction, and extracted twice with ethyl acetate. The organic phases were combined, washed with saturated brine, concentrated to dryness under reduced pressure, purified by a preparative silica gel plate to obtain 22 mg of compound B-6-4 with a yield of 28%.

B-6 was prepared from B-6-4 with reference to the method for preparing B-2 from B-2-17.

**Preparation of Intermediate B-7:**

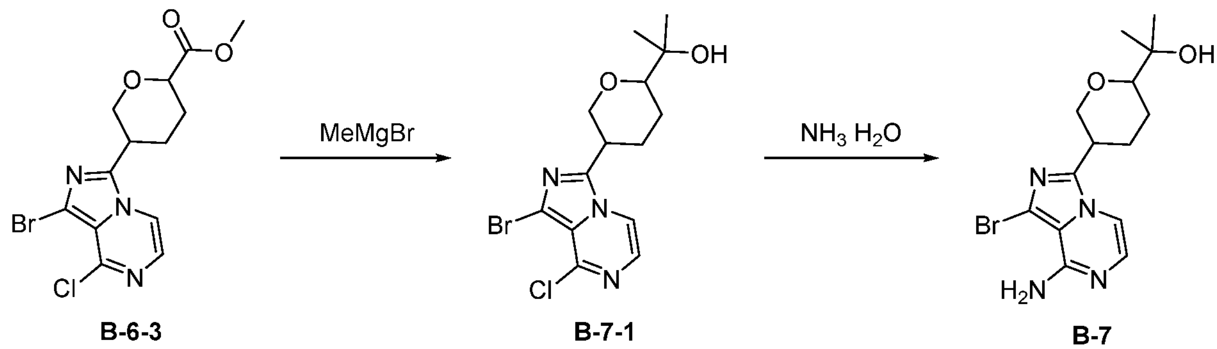


B-6-3 B-7-1 B-7

Compound B-6-3 (100 mg, 0.267 mmol) and THF (2 mL) were added to a reaction flask, subjected to nitrogen replacement, and cooled by an ice water bath. Methylmagnesium bromide (0.8 mL, 0.8 mmol, 1 M) was added dropwise to the reaction solution. After the addition, the reaction solution was warmed to room temperature and stirred overnight. The reaction solution was poured into aqueous ammonium chloride solution to quench the reaction, and extracted twice with ethyl acetate. The organic phases were combined, washed with saturated brine, concentrated to dryness under reduced pressure, and purified by a preparative silica gel plate to obtain 65 mg of compound B-7-1 with a yield of 65%.
B-7 was prepared from B-7-1 with reference to the method for preparing B-2 from B-2-17.

**Preparation of Intermediate B-8:**

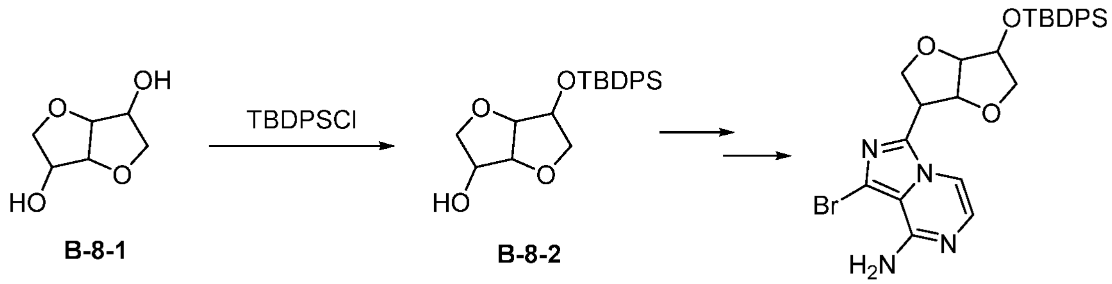


B-8-1 B-8-2 B-8

To a reaction flask, compound B-8-1 (CAS: 652-67-5, 1.00 g, 6.84 mmol), imidazole (559 mg, 8.21 mmol) and DMF (1 5 mL) were added, and then TBDPSCl (1.88 g, 6.84 mmol) was added. The mixture was allowed to react overnight at room temperature. The reaction solution was poured into water, and extracted twice with ethyl acetate. The organic phases were combined, washed with water followed by saturated NaCl solution, concentrated under reduced pressure and purified by silica gel column chromatography to obtain compound B-8-2 (1.63 g, 62%).
B-8 was prepared from B-8-2 with reference to the method for preparing B-1-A (B) from B-1-3.

**Preparation of Intermediate B-9:**

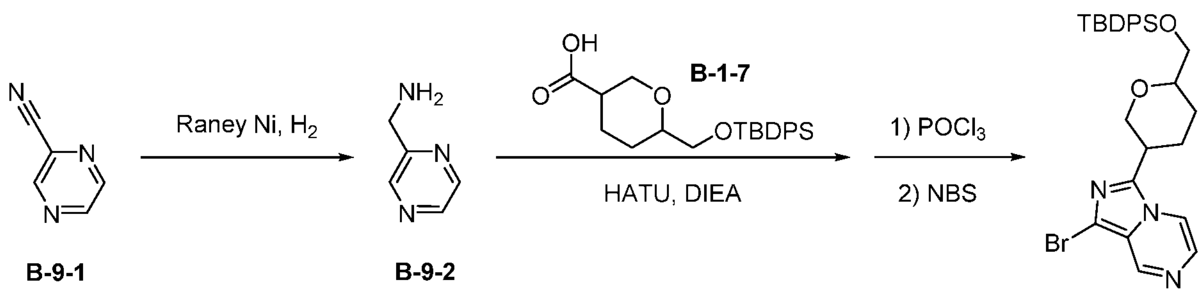


B-9-1 B-9-2 B-9

To a reaction flask, compound B-9-1 (8.0 g, 76.1 mmol), dioxane (120 mL) and RaneyNi (about 1 g) were added. The reaction solution was subjected to nitrogen replacement, heated to 90°C under the pressure of a hydrogen bag and stirred for reaction overnight. TLC showed that the raw materials basically reacted completely. The reaction solution was cooled and suction-filtered, and the filtrate was spin-dried under reduced pressure to obtain 8.3 g of product B-9-2 with a yield of 100%. The product was used directly in the next step without purification.
B-9-2 was condensed with B-1-7, ring-closed, and brominated to obtain B-9. For the specific method, please refer to the method for preparing B-1-10-A (B) from B-1-7.

**Preparation of Intermediate B-10:**

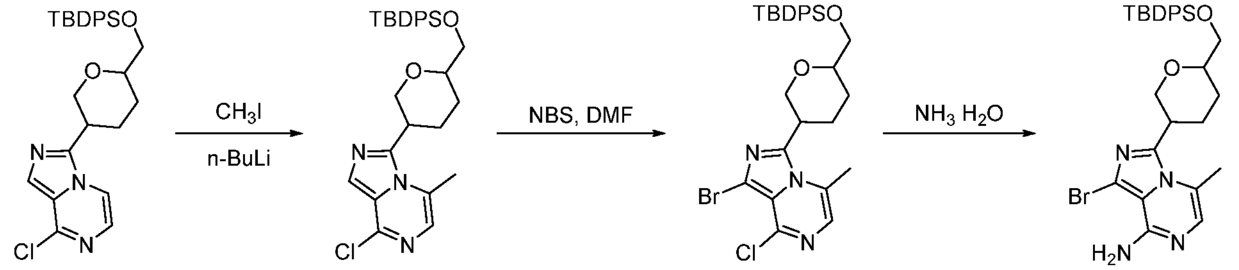


B-1-9 B-10-1 B-10-2 B-10

To a reaction flask, compound B-1-9 (200 mg, 0.395 mmol) and tetrahydrofuran (3 mL) were added. The reaction solution was subjected to nitrogen replacement, cooled in a dry ice/ethanol bath to -70°C, and added dropwise with n-butyllithium (2.5 M, 0.19 mL, 0.474 mmol). After the addition, the reaction was carried out for 30 min at a constant temperature. Methyl iodide (112 mg, 0.790 mmol) was added dropwise to the reaction solution. After the addition, the reaction solution was slowly warmed to room temperature, added with aqueous ammonium chloride solution to quench the reaction, and extracted twice with ethyl acetate. The organic phases were combined, concentrated under reduced pressure to dryness, and purified by column chromatography to obtain B-10-1 (160 mg, 78%).

B-10-1 was brominated with NBS, and then ammonolyzed to prepare B-10. For the specific method, please refer to the method for preparing B-1-A (B) from B-1-9.

Example 1: Preparation of Compound 1

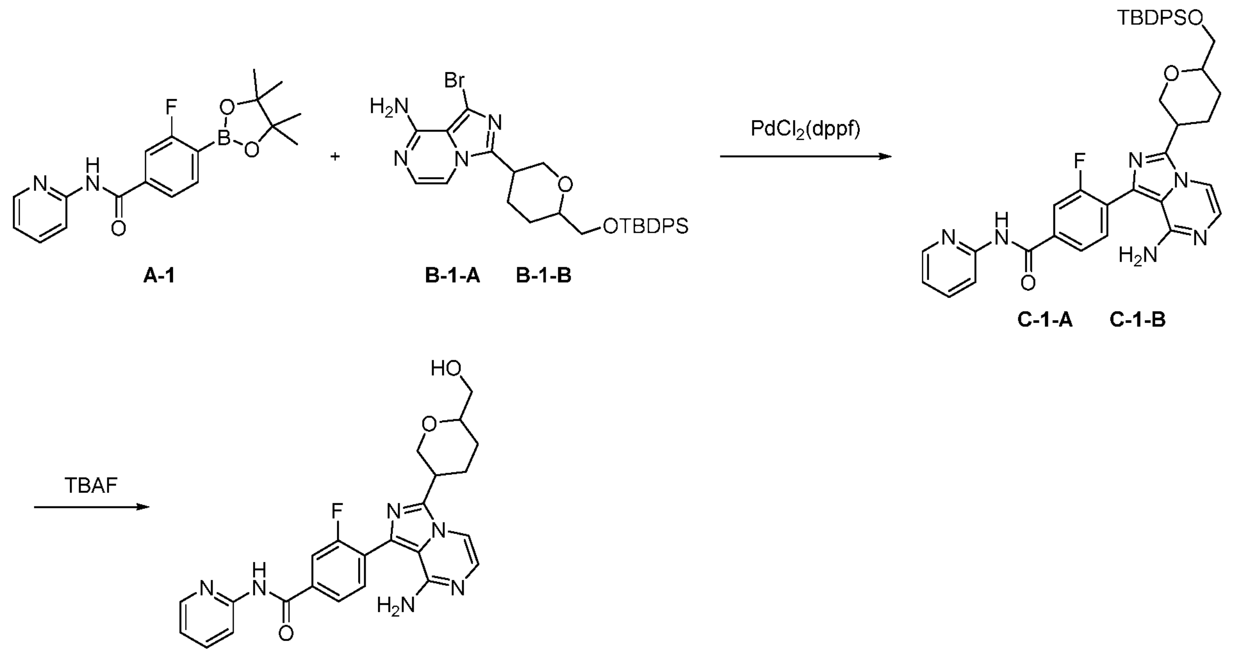


A-1 B-1-A B-1-B C-1-A C-1-B

1-A 1-B

Compound B-1-B (205 mg, 0.362 mmol), A-1 (161 mg, 0.471 mmol), $Na_2CO_3$ (77 mg, 0.724 mmol), $PdCl_2$ (dppf) (20 mg), dioxane (6 mL) and water (2 mL) were added to a reaction flask, subjected to nitrogen replacement, and warmed to 95°C to react for 2.5 h, as TLC showed that the reaction was complete. The reaction solution was diluted with ethyl acetate, directly mixed with silica gel, and then purified by silica gel column chromatography to obtain 182 mg of product C-1-B with a yield of 72%.

To a reaction flask, compound C-1-B (182 mg, 0.260 mmol) and tetrahydrofuran (4 mL) were added. To the reaction solution, TBAF (1 M, 0.39 mL) was added, and stirred at room temperature for 1.5 h of reaction. TLC showed the reaction was complete. The reaction solution was directly purified by a preparative silica gel plate (DCM/MeOH=15/1) to obtain 70 mg of product 1-B with a yield of 58%.

The structure of the product was characterized by NMR and mass spectrometry, and the results are as follows:

$^1H$ NMR (400 MHz, d6-DMSO) δ 1.56-1.60 (1H, m), 1.94-2.07 (2H, m), 2.20-2.25 (1H, m), 3.28-3.31 (1H, m), 3.38-3.42 (2H, m), 3.47-3.51 (1H, m), 3.77 (1H, dd, J = 11.8 Hz, 3.2 Hz), 4.11 (1H, dd, J = 11.8 Hz, 1.8 Hz), 4.59 (1H, t, J = 5.8 Hz), 6.03 (2H, brs), 7.07 (1H, d, J = 5.0 Hz), 7.20 (1H, ddd, J = 7.4 Hz, 4.9 Hz, 1.0 Hz), 7.63 (2H, dd, J = 10.4 Hz, 5.4 Hz), 7.85-7.90 (1H, m), 7.98-8.02 (2H, m), 8.21 (1H, d, J = 8.4 Hz), 8.41-8.43 (1H, m), 10.97 (1H, s).

MS(ESI) m/z $(M+H)^+$: 463.0.

1-A was prepared using A-1 and B-1-A in the same way as 1-B was synthesized.

The structure of the product was characterized by NMR and mass spectrometry, and the results are as follows:

$^1H$ NMR (400 MHz, d6-DMSO) δ 1.38-1.50 (1H, m), 1.73-1.75 (1H, m), 1.80-1.92 (1H, m), 2.12-2.15 (1H, m), 3.36-3.47 (3H, m), 3.62 (1H, t, J = 11.0 Hz), 4.07-4.10 (1H, m ), 4.69 (1H, t, J = 5.5 Hz), 6.02 (2H, s), 7.07 (1H, d, J = 5.0 Hz), 7.20 (1H, dd, J = 6.9 Hz, 5.2 Hz), 7.61 (1H, t, J = 7.9 Hz), 7.76 (1H, d, J = 5.0 Hz), 7.83-7.91 (1H, m), 7.95-8.04 (2H, m), 8.21 (1H, d, J = 8.4 Hz), 8.42 (1H, d, J = 3.8 Hz ),10.97 (1H, s).

MS(ESI) m/z $(M+H)^+$: 463.1.

**Example 2: Preparation of Compound 2**

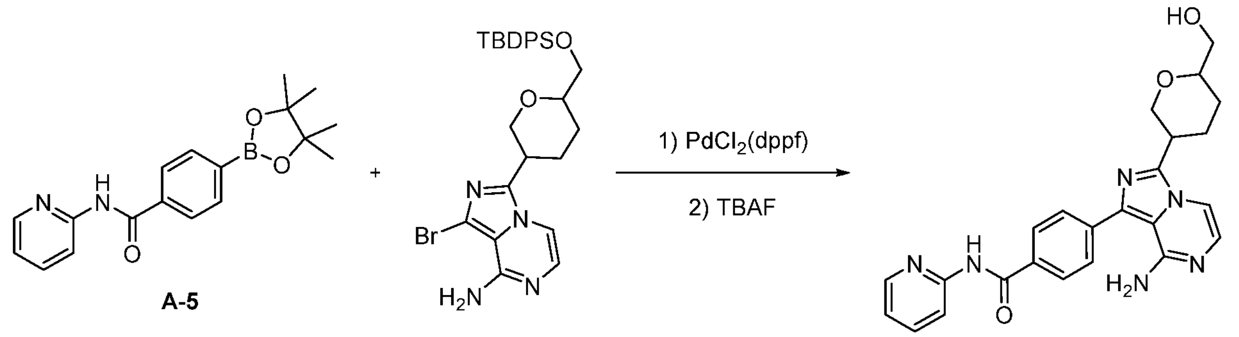


A-5

B-1-A B-1-B

2-A (2-A-P1 2-A-P2)
2-B

2-A was prepared using A-5 and B-1-A in the same way as 1-B was synthesized.

The structure of the product was characterized by NMR and mass spectrometry, and the results are as follows:

$^1H$ NMR (400 MHz, d6-DMSO) 6 1.41-1.51 (1H, m), 1.50-1.78 (1H, m), 1.85-1.97 (1H, m), 2.13-2.15 (1H, m), 3.35-3.49 (4H, m), 3.65 (1H, t, J = 11.0 Hz), 4.10 (1H, ddd, J = 11.0 Hz, 3.6 Hz, 1.6 Hz), 4.69 (1H, t, J = 5.6 Hz), 6.13 (2H, brs), 7.09 (1H, d, J = 4.9 Hz), 7.19 (1H, dd, J = 6.9 Hz, 5.2 Hz), 7.76 (3H, dd, J = 9.5 Hz, 6.7 Hz), 7.83-7.90 (1H, m), 8.16 (2H, d, J = 8.4 Hz), 8.23 (1H, d, J = 8.4 Hz), 8.41 (1H, dd, J = 4.8 Hz, 1.0 Hz), 10.84 (1H, s).

MS(ESI) m/z $(M+H)^+$: 445.2.

Compound 2-A was separated by SFC to obtain 2-A-P1 (peak first) and 2-A-P2 (peak last).

Conditions for preparative SFC:

Instrument: SFC-80 (Thar, Waters)

Column: CHIRALCEL OJ(30×250mm 5μm) (Daicel)

Column temperature: 35°C

Mobile phase: A=$CO_2$ Co-Solvent B= ETOH

Cycle Time:12.5 min Run Time:21 min

| $CO_2$ Flow Rate | Co-Solvent Flow Rate | Co-Solvent B% | Total Flow Rate | Back Pressure | Wavelength | Inj. Vol. |
|---|---|---|---|---|---|---|
| 20.25ml/min | 24.75 ml/min | 55 | 45 ml/min | 80 bar | 215 nm | 4.8 ML |

2-B was prepared using A-5 and B-1-B in the same way as 1-B was synthesized.

The structure of the product was characterized by NMR and mass spectrometry, and the results are as follows: $^1$H NMR (400 MHz, d6-DMSO) δ 1.58-1.63 (1H, m), 1.95-2.02 (1H, m), 2.08-2.17 (1H, m), 2.24-2.28 (1H, m), 3.39-3.51 (4H, m), 3.78 (1H, dd, J = 11.7, 3.2 Hz), 4.10 (1H, d, J = 10.1 Hz), 4.60 (1H, t, J = 5.2 Hz), 6.14 (2H, brs), 7.09 (1H, d, J = 4.9 Hz), 7.18 (1H, dd, J = 6.9 Hz, 5.3 Hz), 7.63 (1H, d, J = 5.0 Hz), 7.76 (2H, d, J = 8.3 Hz), 7.84-7.88 (1H, m), 8.16 (2H, d, J = 8.3 Hz), 8.23 (1H, d, J = 8.3 Hz), 8.41 (1H, dd, J = 4.8 Hz, 1.0 Hz), 10.84 (1H, s).

MS(ESI) m/z $(M+H)^+$: 445.2.

Example 3: Preparation of Compound 3

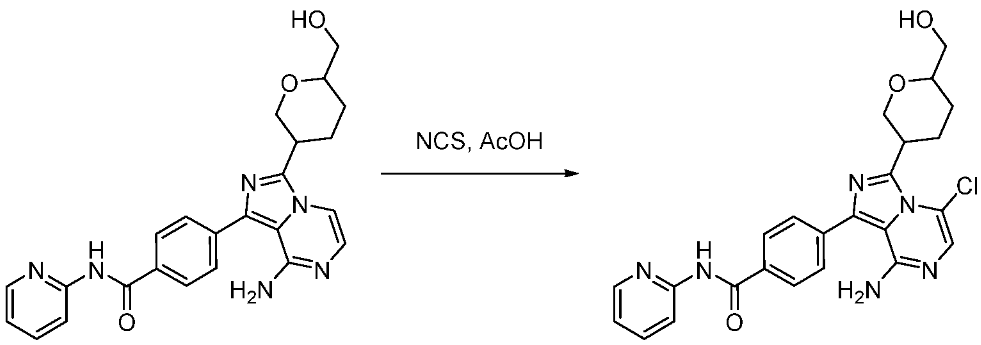


**2-B** **3**

To a reaction flask, compound 2-B (50 mg, 0.113 mmol), NCS (16.5 mg, 0.124 mmol) and glacial acetic acid (1 mL) were added. The reaction solution was heated to 80°C for 2 h of reaction. The reaction solution was concentrated to dryness under reduced pressure, added with aqueous sodium bicarbonate solution, and extracted twice with ethyl acetate. The organic phases were combined, washed with saturated aqueous sodium chloride solution, dried over anhydrous sodium sulfate, filtered under vacuum, concentrated to dryness under reduced pressure, and purified by a preparative silica gel plate to obtain 28 mg of product 3 with a yield of 52%.

MS(ESI) m/z $(M+H)^+$: 479.2.

**Examples 4-119: Preparation of Compounds 4-119**

Compounds 4-119 were prepared using different intermediates according to the method for preparing compound 1-B or 3, and the numbers of the used intermediates, structural formulas, MS and $^1$H-NMR data used are shown in Table 13.

Table 13: Structure, MS and $^1$H-NMR data of Examples 4-119

| Example | Structural formula | Intermediate No. | MS (ESI) m/z $(M+H)^+$ | $^1$H NMR (400 MHz, d6-DMSO) |
|---|---|---|---|---|

| | | | | |
|---|---|---|---|---|
| 4 | 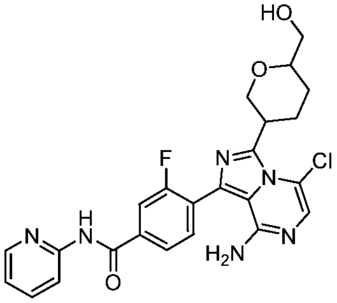 | 1-B | 497.1 | |
| 5 | 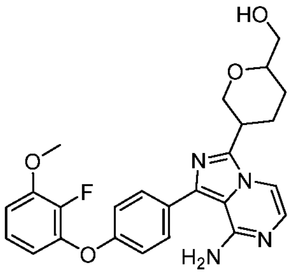 | A-2<br>B-1-B | 465.2 | δ 1.57-1.61 (1H, m), 1.94-2.01 (1H, m), 2.05-2.13 (1H, m), 2.21-2.26 (1H, m), 3.35-3.51 (4H, m), 3.76 (1H, dd, J = 11.6 Hz, 3.2 Hz), 3.90 (3H, s), 4.08 (1H, dd, J = 11.7 Hz, 2.8 Hz), 4.57 (1H, t, J = 5.7 Hz), 6.06 (2H, brs), 6.83 (1H, t, J = 7.6 Hz), 7.02-7.08 (2H, m), 7.11 (2H, d, J = 8.6 Hz), 7.18 (1H, td, J = 8.3 Hz, 1.8 Hz), 7.57-7.61 (3H, m). |
| 6 | 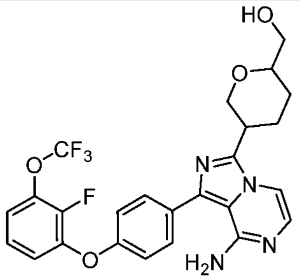 | A-13<br>B-1-B | 518.2 | |
| 7 | 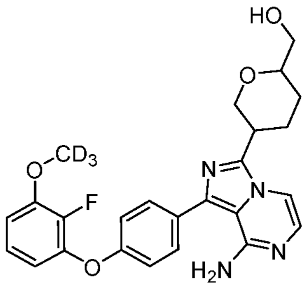 | A-14<br>B-1-B | 468.2 | |
| 8 | 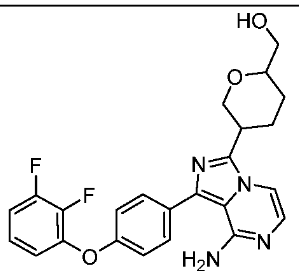 | A-6<br>B-1-B | 453.2 | |

| | | | | |
|---|---|---|---|---|
| 9 | 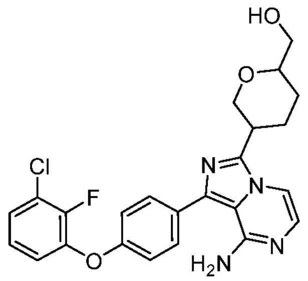 | A-7<br>B-1-B | 468.1 | δ 1.56-1.60 (1H, m), 1.93-1.99 (1H, m), 2.05-2.11 (1H, m), 2.20-2.25 (1H, m), 3.29-3.50 (4H, m), 3.76 (1H, dd, J = 11.8 Hz, 3.2 Hz), 4.08 (1H, dd, J = 11.5 Hz, 1.7 Hz), 4.58 (1H, t, J = 5.8 Hz), 6.05 (2H, brs), 7.03 (1H, d, J = 5.0 Hz), 7.18 (2H, d, J = 8.7 Hz), 7.22-7.29 (2H, m), 7.42-7.46 (1H, m), 7.57 (1H, d, J = 5.0 Hz), 7.63 (2H, d, J = 8.7 Hz). |
| 10 | 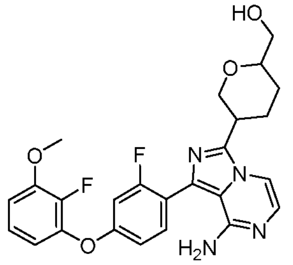 | A-8<br>B-1-B | 483.2 | |
| 11 | 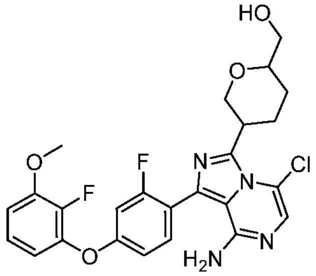 | 10 | 516.0 | |
| 12 | 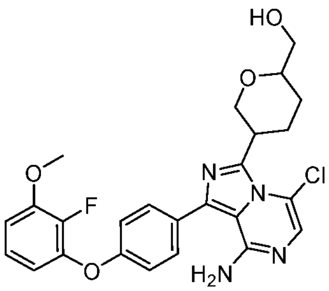 | 5 | 498.0 | δ 1.56-1.61 (1H, m), 1.97-2.08 (2H, m), 2.22-2.27 (1H, m), 3.31-3.38 (1H, m), 3.43-3.50 (2H, m), 3.84 (1H, dd, J = 11.6 Hz, 3.5 Hz), 3.89 (3H, s), 3.94-3.99 (1H, m), 4.08 (1H, dd, J = 11.5 Hz, 3.7 Hz), 4.59 (1H, t, J = 5.7 Hz), 6.15 (2H, brs), 6.81-6.85 (1H, m), 7.04-7.08 (2H, m), 7.11 (2H, d, J = 8.7 Hz), 7.18 (1H, td, J = 8.4 Hz, 1.9 Hz), 7.58 (2H, d, J = 8.7 Hz). |
| 13 | 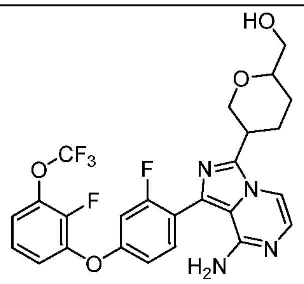 | A-25<br>B-1-B | 536.1 | |

| | | | | |
|---|---|---|---|---|
| 14 | 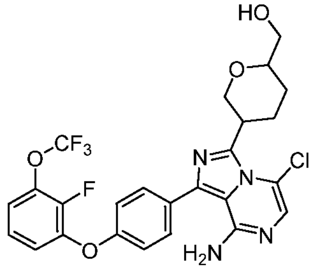 | 6 | 552.1 | |
| 15 | 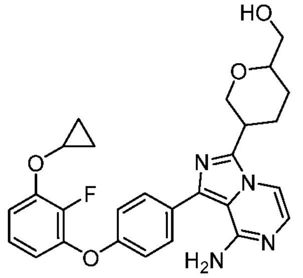 | A-15<br>B-1-B | 491.2 | |
| 16 | 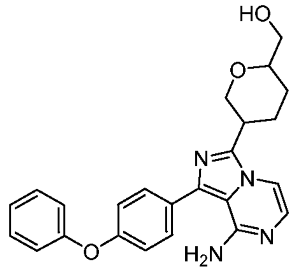 | A-9<br>B-1-B | 416.2 | |
| 17 | 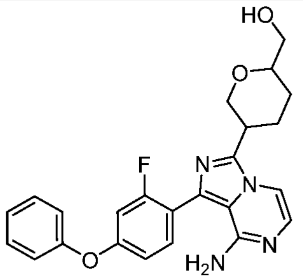 | A-3<br>B-1-B | 435.2 | |
| 18 | 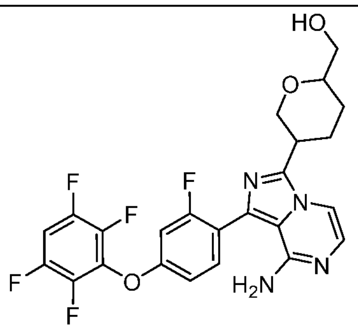 | A-4<br>B-1-B | 507.0 | |

| | | | | |
|---|---|---|---|---|
| 19 | 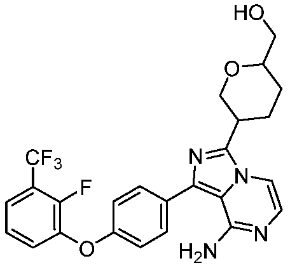 | A-10<br>B-1-B | 503.2 | |
| 20 | 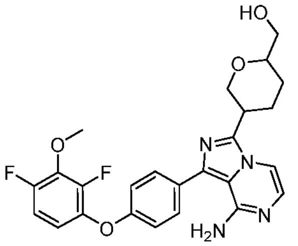 | A-16<br>B-1-B | 482.2 | |
| 21 | 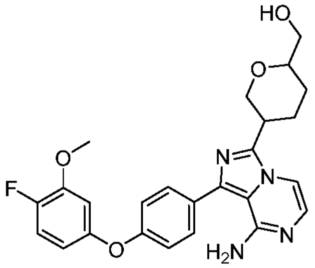 | A-17<br>B-1-A | 465.2 | |
| 22 | 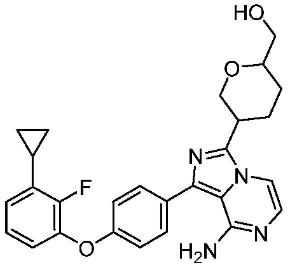 | A-18<br>B-1-B | 475.2 | |
| 23 | 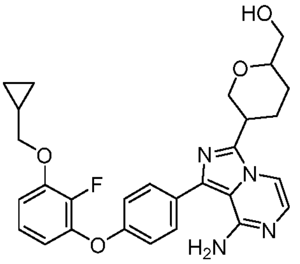 | A-19<br>B-1-B | 505.2 | |
| 24 | 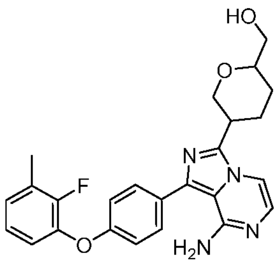 | A-11<br>B-1-B | 449.2 | δ 1.53-1.62 (1H, m), 1.91-2.01 (1H, m), 2.05-2.14 (1H, m), 2.19-2.26 (1H, m), 2.30 (3H, d, J = 2.0 Hz ), 3.35-3.53 (3H, m), 3.75 (1H, dd, J = 11.7 Hz, 3.3 Hz ), 4.07 (1H, d, J = 11.8 Hz ), 4.58 (1H, t, J = 5.7 Hz), 6.02 (2H, s), 7.02 (1H, d, J = 5.0 Hz ), 7.05-7.198 (5H, m), 7.51-7.69 (3H, m). |

| | | | | |
|---|---|---|---|---|
| 25 | 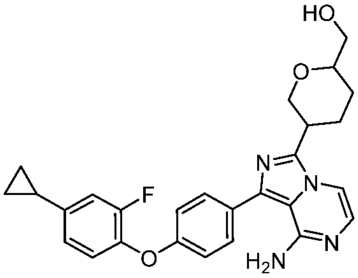 | A-20<br>B-1-B | 475.2 | |
| 26 | 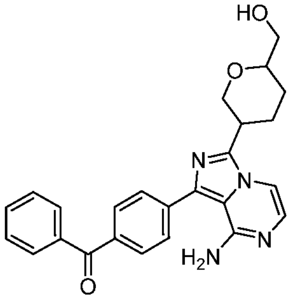 | A-12<br>B-1-B | 429.2 | |
| 27 | 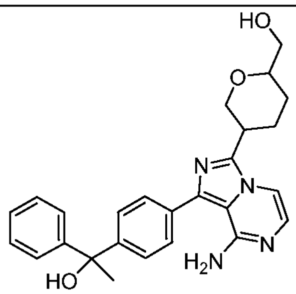 | A-21<br>B-1-B | 445.2 | |
| 28 | 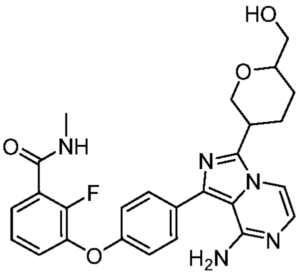 | A-74<br>B-1-B | 492.2 | |
| 29 | 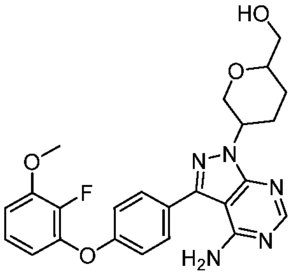 | A-2<br>B-4-A | 466.2 | |

| | | | | |
|---|---|---|---|---|
| 30 | 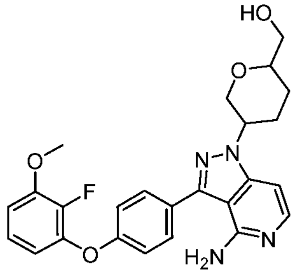 | A-2<br>B-5 | 465.2 | |
| 31 | 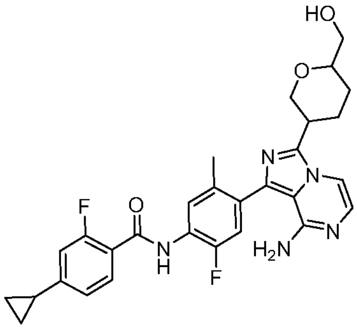 | A-22<br>B-1-B | 534.2 | δ 0.77-0.81 (2H, m), 1.03-1.08 (2H, m), 1.52-1.56 (1H, m), 1.94-2.06 (3H, m), 2.11 (3H, s), 2.20-2.25 (1H, m), 3.27-3.37 (3H, m), 3.44-3.49 (1H, m), 3.75 (1H, dd, J = 11.7 Hz, 3.0 Hz), 4.12 (1H, d, J = 11.2 Hz), 4.58 (1H, t, J = 5.8 Hz), 5.85 (2H, brs), 7.02 (1H, d, J = 5.0 Hz), 7.05-7.09 (3H, m), 7.57-7.60 (2H, m), 7.66 (1H, t, J = 8.0 Hz), 9.85 (1H, d, J = 2.4 Hz). |
| 32 | 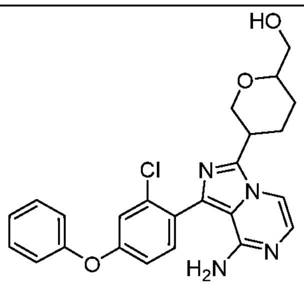 | A-23<br>B-1-B | 451.1 | |
| 33 | 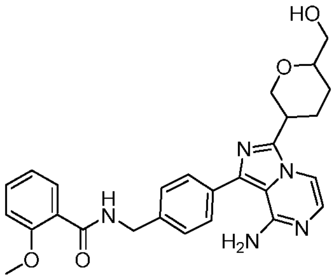 | A-24<br>B-1-B | 488.2 | |
| 34 | 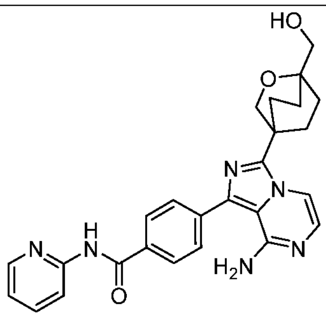 | A-5<br>B-2 | 471.2 | δ 1.71-1.90 (4H, m), 1.95-2.07 (2H, m), 2.34-2.45 (2H, m), 3.26 (2H, d, J = 5.9 Hz), 4.23 (2H, s), 4.66 (1H, t, J = 5.9 Hz), 6.12 (2H, brs), 7.05 (1H, d, J = 5.0 Hz), 7.19 (1H, dd, J = 6.7 Hz, 5.1 Hz), 7.72 (2H, d, J = 8.3 Hz), 7.82-7.90 (1H, m), 7.94 (1H, d, J = 5.1 Hz), 8.15 (2H, d, J = 8.3 Hz), 8.23 (1H, d, J = 8.4 Hz), 8.41 (1H, d, J = 3.8 Hz), 10.85 (1H, s). |

| | | | | |
|---|---|---|---|---|
| 35 | 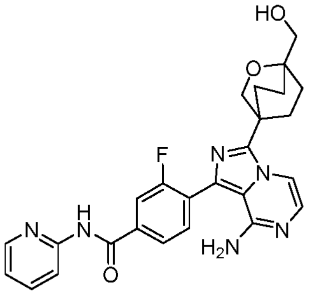 | A-5<br>B-2 | 489.2 | |
| 36 | 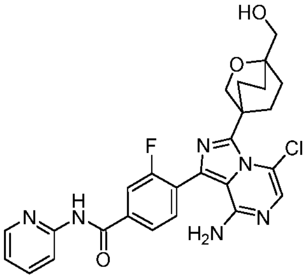 | 35 | 523.2 | |
| 37 | 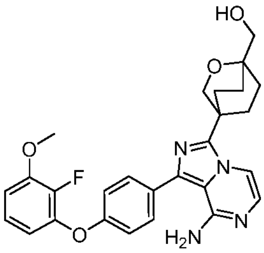 | A-2<br>B-2 | 491.2 | |
| 38 | 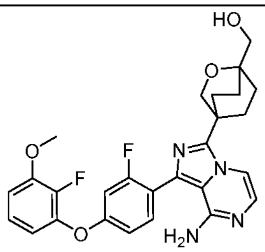 | A-8<br>B-2 | 509.1 | |
| 39 | 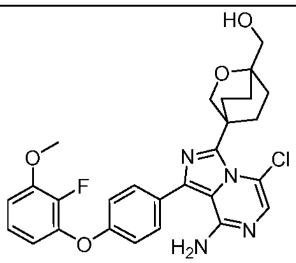 | 37 | 525.2 | |
| 40 | 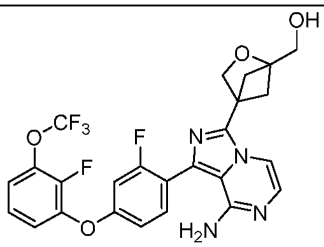 | A-25<br>B-3 | 535.1 | |

| | | | | |
|---|---|---|---|---|
| 41 | 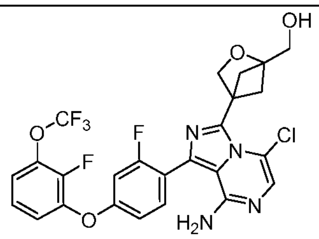 | 40 | 569.1 | |
| 42 | 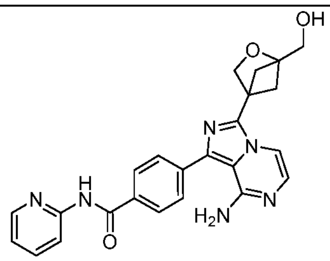 | A-5<br>B-3 | 443.2 | |
| 43 | 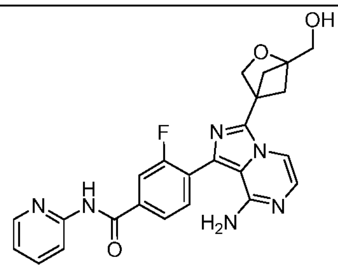 | A-1<br>B-3 | 461.2 | |
| 44 | 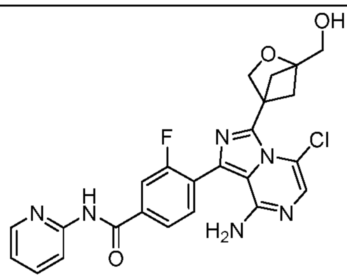 | 43 | 495.0 | |
| 45 | 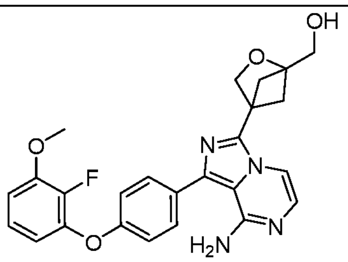 | A-2<br>B-3 | 463.2 | |
| 46 | 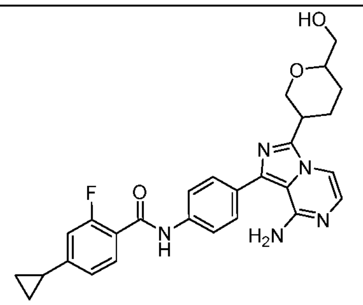 | A-26<br>B-1-B | 502.2 | |

| | | | | |
|---|---|---|---|---|
| 47 | 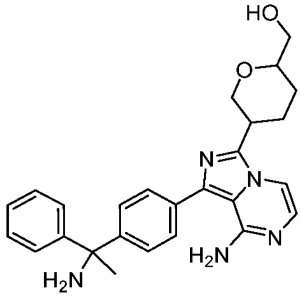 | A-27<br>B-1-B | 444.2 | |
| 48 | 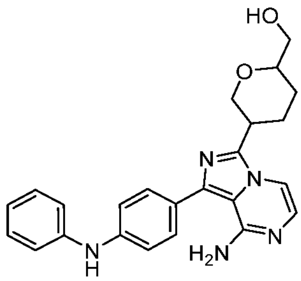 | A-28<br>B-1-B | 416.2 | δ 1.57-1.61 (1H, m), 1.92-2.0 (1H, m), 2.05-2.14 (1H, m), 2.21-2.25 (1H, m), 3.38-3.55 (2H, m), 3.75 (1H, dd, J = 11.8 Hz, 3.3 Hz ), 4.08 (1H, dd, J = 11.4 Hz, 2.2 Hz ), 4.59 (1H, t, J = 5.8 Hz), 6.02 ( 2H, s), 6.87 (1H, t, J = 7.3 Hz), 6.99 (1H, d, J = 5.0 Hz), 7.17 (4H, dd, J = 13.1 Hz, 8.1 Hz ), 7.27 (2H, t, J = 7.9 Hz), 7.45 (2H, d, J = 8.5 Hz), 7.54 (1H, d, J = 5.1 Hz), 8.39 (1H, s). |
| 49 | 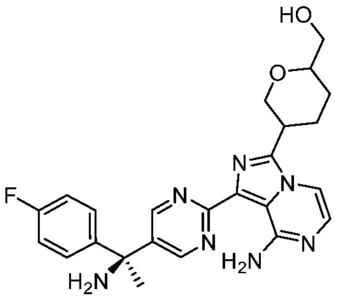 | A-29<br>B-1-B | 464.2 | |
| 50 | 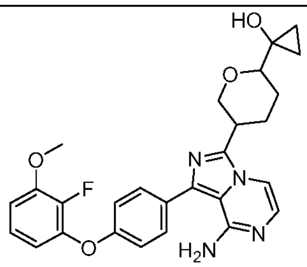 | A-2<br>B-6 | 491.2 | |
| 51 | 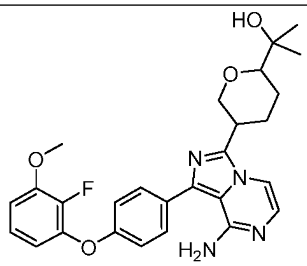 | A-2<br>B-7 | 493.2 | |

| | | | | |
|---|---|---|---|---|
| 52 | 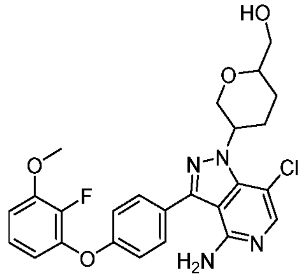 | 30 | 499.1 | |
| 53 | 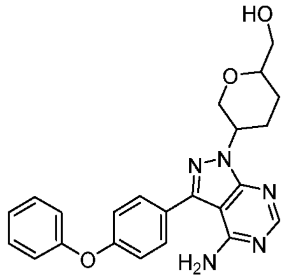 | A-9<br>B-4 | 418.2 | |
| 54 | 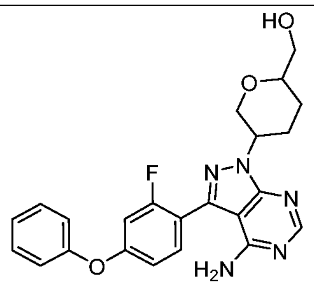 | A-3<br>B-4 | 436.2 | |
| 55 | 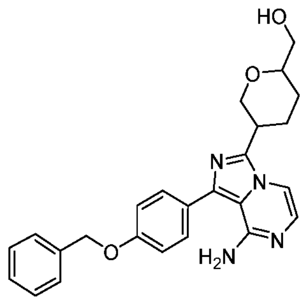 | A-30<br>B-1-B | 431.2 | δ 1.53-1.61 (1H, m), 1.92-1.99 (1H, m), 2.03-2.14 (1H, m), 2.18-2.27 (1H, m), 3.38-3.49 (3H, m), 3.75 (1H, dd, J = 11.7 Hz, 3.3 Hz), 4.07 (1H, dd, J = 11.5 Hz, 1.7 Hz), 4.58 (1H, d, J = 5.8 Hz), 5.17 (2H, s), 5.98 (2H, s), 7.00 (1H, d, J = 5.0 Hz), 7.15 (2H, d, J = 8.7 Hz), 7.31-7.38 (1H, m), 7.37-7.45 (2H, m), 7.46-7.58 (5H, m). |
| 56 | 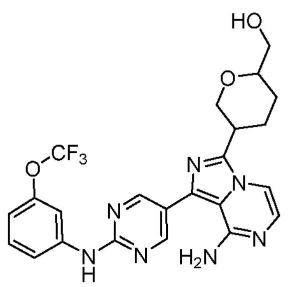 | A-31<br>B-1-B | 502.2 | δ 1.52-1.66 (1H, m), 1.90-2.05 (2H, m), 2.07-2.17 (1H, m), 2.20-2.30 (1H, m), 3.39-3.62 (3H, m), 3.77 (1H, dd, J = 11.7 Hz, 3.2 Hz ), 4.09 (1H, d, J = 10.22 Hz ), 4.59 (1H, t, J = 5.7 Hz), 6.29 ( 2H, s), 6.93 (1H, d, J = 8.2 Hz), 7.06 (1H, d, J = 5.0 Hz), 7.42 (1H, t, J = 8.2 Hz ), 7.61 (1H, d, J = 5.0 Hz), 7.79 (1H, d, J = 8.2 Hz), 8.03 (1H, s), 8.70 (2H, s), 10.14 (1H, s). |

| | | | | |
|---|---|---|---|---|
| 57 | 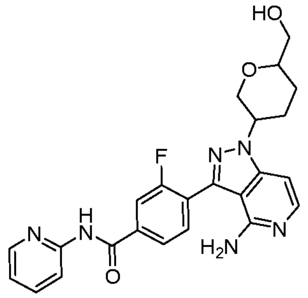 | A-1<br>B-5 | 463.1 | |
| 58 | 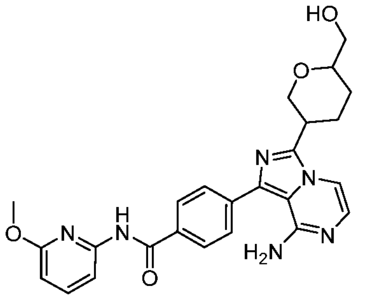 | A-32<br>B-1-B | 475.2 | |
| 59 | 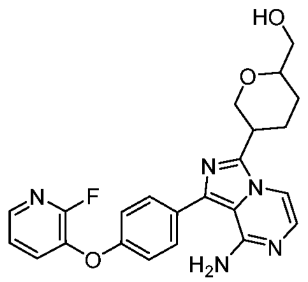 | A-33<br>B-1-B | 436.1 | δ 1.53-1.63 (1H, m), 1.89-2.02 (1H, m), 2.03-2.15 (1H, m), 2.20-2.26 (1H, m), 3.36-3.52 (3H, m), 3.76 (1H, dd, J = 11.7 Hz, 3.2 Hz ), 4.08 (1H, d, J = 9.9 Hz ), 4.58 (1H, t, J = 5.8 Hz), 6.06 (2H, s), 7.03 (1H, d, J = 5.0 Hz ), 7.20 (2H, d, J = 8.7 Hz ), 7.41 (1H, dd, J = 7.8 Hz, 4.8 Hz ), 7.58 (1H, d, J = 5.0 Hz ), 7.64 (2H, d, J = 8.7 Hz ), 7.75-7.85 (1H, m), 8.05 (1H, d, J = 4.8 Hz ). |
| 60 | 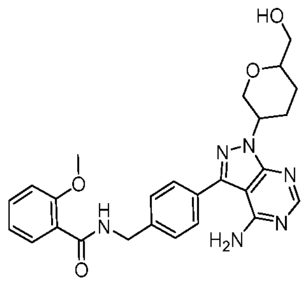 | A-24<br>B-4-A(B) | 489.2 | 60-A: δ 1.84-1.88 (1H, m), 1.95-2.02 (1H, m), 2.11-2.15 (1H, m), 2.24-2.31 (1H, m), 3.11-3.21 (3H, m), 3.36-3.48 (3H, m), 3.73 (1H, t, J = 10.8 Hz), 3.91 (3H, s), 3.99-4.02 (1H, m), 4.58 (2H, t, J = 6.1 Hz), 4.68-4.81 (2H, m), 7.05 (1H, d, J = 7.5 Hz), 7.16 (1H, d, J = 8.2 Hz), 7.44-7.54 (3H, m), 7.63 (2H, d, J = 8.1 Hz), 7.76 (1H, dd, J = 7.7 Hz, 1.8 Hz), 8.25 (1H, s), 8.78 (1H, t, J = 6.0 Hz ). |
| 61 | 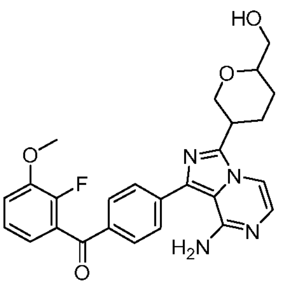 | A-34<br>B-1-B | 477.2 | δ 1.54-1.64 (1H, m), 1.94-2.02 (1H, m), 2.07-2.16 (1H, m), 2.19-2.29 (1H, m), 3.37-3.54 (3H, m), 3.77 (1H, dd, J = 11.7 Hz, 3.3 Hz), 3.92 (3H, s), 4.09 (1H, d, J = 9.8 Hz), 4.59 (1H, t, J = 5.8 Hz), 6.18 (2H, s), 7.08-7.11 (2H, m), 7.32 (1H, t, J = 7.9 Hz), 7.43 (1H, d, J = 7.6 Hz), 7.64 (1H, d, J = 5.0 Hz), 7.82 (2H, d, J = 8.5 Hz), 7.88 (2H, d, J = 8.2 Hz). |

| | | | | |
|---|---|---|---|---|
| 62 | 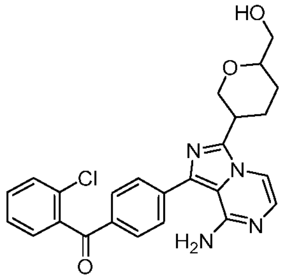 | A-35 B-1-B | 463.1 | |
| 63 | 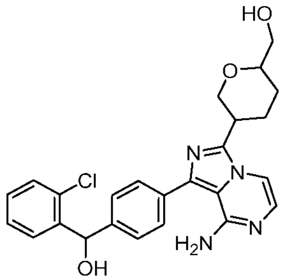 | A-36 B-1-B | 465.1 | δ 1.52-1.60 (1H, m), 1.91-1.99 (1H, m), 2.04-2.13 (1H, m), 2.17-2.25 (1H, m), 3.35-3.52 (3H, m), 3.74 (1H, dd, J = 11.8 Hz, 3.3 Hz), 4.06 (1H, dd, J = 11.5 Hz, 1.7 Hz), 4.57 (1H, d, J = 5.7 Hz), 5.98 (2H, s), 6.07 (1H, d, J = 4.3 Hz), 6.14 (1H, d, J = 4.4 Hz), 7.02 (1H, d, J = 5.0 Hz), 7.25-7.34 (1H, m), 7.40-7.47 (4H, m), 7.51-7.60 (3H, m), 7.74 (1H, dd, J = 8.0 Hz, 1.76 Hz). |
| 64 | 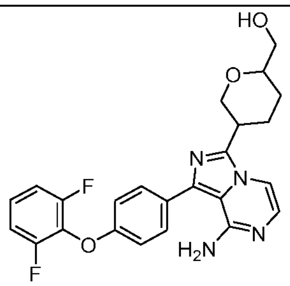 | A-37 B-1-B | 453.2 | |
| 65 | 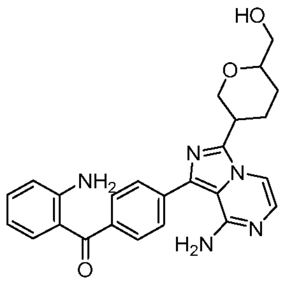 | A-38 B-1-A | 444.2 | δ 1.41-1.50 (1H, m), 1.75-1.78 (1H, m), 1.85-1.96 (1H, m), 2.10-2.18 (1H, m), 3.37-3.52 (4H, m), 3.65 (1H, t, J = 11.0 Hz), 4.05-4.14 (1H, m), 4.69 (1H, t, J = 5.4 Hz), 6.17 (2H, brs), 6.54 (1H, d, J = 7.4 Hz), 6.88 (1H, d, J = 8.3 Hz), 7.09 (3H, d, J = 5.4 Hz), 7.31 (1H, t, J = 7.5 Hz), 7.42 (1H, d, J = 8.1 Hz), 7.67 (2H, d, J = 8.1 Hz), 7.73-7.77 (3H, m). |
| 66 | 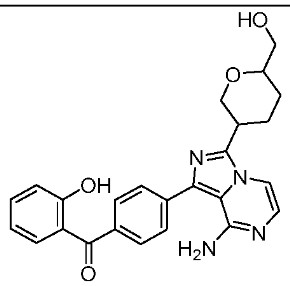 | A-39 B-1-A | 445.1 | |

| | | | | |
|---|---|---|---|---|
| 67 | 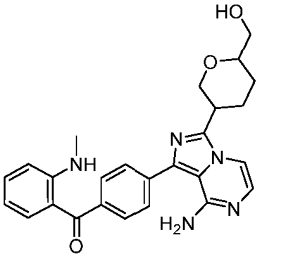 | A-40<br>B-1-A | 458.2 | |
| 68 | 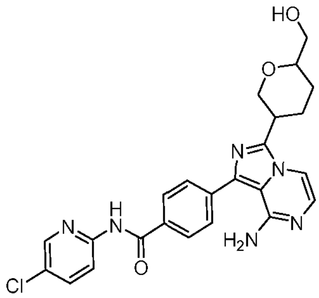 | A-41<br>B-1-B | 479.1 | δ 1.57-1.66 (1H, m), 1.94-2.04 (1H, m), 2.08-2.17 (1H, m), 2.22-2.31 (1H, m), 3.36-3.55 (3H, m), 3.79 (1H, dd, J = 11.8 Hz, 3.2 Hz), 4.11 (1H, dd, J = 11.2 Hz, 2.0 Hz), 4.60 (1H, d, J = 5.7 Hz), 5.76 (1H, s), 6.14 (2H, s), 7.09 (1H, d, J = 4.9 Hz), 7.63 (1H, d, J = 5.0 Hz), 7.77 (2H, d, J = 8.4 Hz), 7.99 (1H, dd, J = 8.9 Hz, 2.7 Hz), 8.16 (2H, d, J = 8.4 Hz), 8.28 (1H, d, J = 8.9 Hz), 8.47 (1H, d, J = 2.6 Hz), 11.05 (1H, s). |
| 69 | 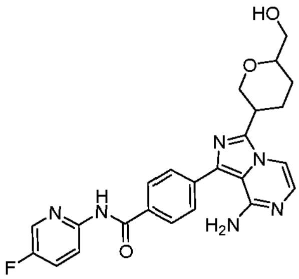 | A-42<br>B-1-B | 463.1 | δ 1.56-1.67 (1H, m), 1.94-2.05 (1H, m), 2.06-2.19 (1H, m), 2.21-2.31 (1H, m), 3.36-3.55 (3H, m), 3.79 (1H, dd, J = 11.8 Hz, 3.3 Hz), 4.11 (1H, dd, J = 11.6 Hz, 1.8 Hz), 4.60 (1H, d, J = 5.7 Hz), 6.14 (2H, s), 7.09 (1H, d, J = 4.9 Hz), 7.63 (1H, d, J = 5.0 Hz), 7.73-7.88 (3H, m), 8.16 (2H, d, J = 8.4 Hz), 8.27 (1H, dd, J = 9.1 Hz, 4.1 Hz), 8.43 (1H, d, J = 3.1 Hz), 10.97 (1H, s). |
| 70 | 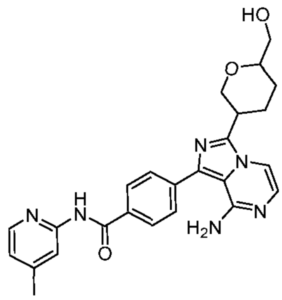 | A-43<br>B-1-B | 459.2 | δ 1.56-1.66 (1H, m), 1.94-2.05 (1H, m), 2.06-2.19 (1H, m), 2.21-2.31 (1H, m), 2.38 (3H, s), 3.38-3.55 (4H, m), 3.79 (1H, dd, J = 11.7 Hz, 3.4 Hz), 4.11 (1H, dd, J = 11.6 Hz, 3.1 Hz), 4.60 (1H, d, J = 5.8 Hz), 6.14 (2H, brs), 7.03 (1H, d, J = 5.1 Hz), 7.09 (1H, d, J = 4.9 Hz), 7.63 (1H, d, J = 5.0 Hz), 7.76 (2H, d, J = 8.3 Hz), 8.10 (1H, s), 8.16 (2H, d, J = 8.3 Hz), 8.26 (1H, d, J = 5.0 Hz), 10.74 (1H, s). |
| 71 | 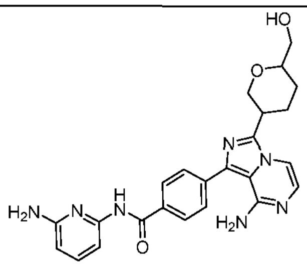 | A-44<br>B-1-B | 460.1 | |

| | | | | |
|---|---|---|---|---|
| 72 | 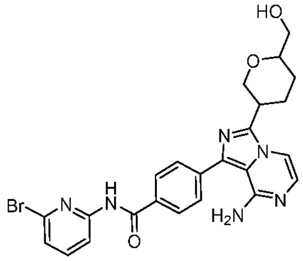 | A-45<br>B-1-B | 523.0 | |
| 73 | 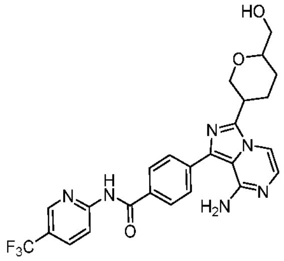 | A-46<br>B-1-B | 513.2 | |
| 74 | 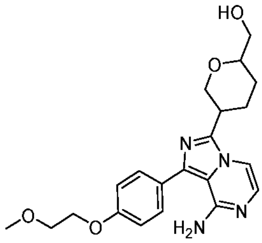 | A-47<br>B-1-B | 399.2 | |
| 75 | 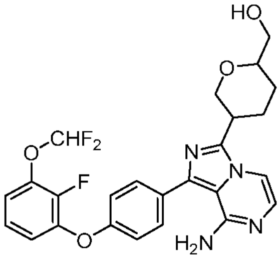 | A-48<br>B-1-B | 501.1 | |
| 76 | 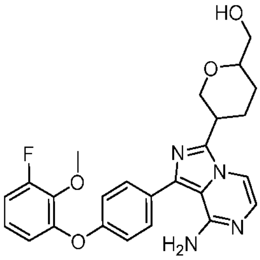 | A-49<br>B-1-B | 465.2 | |
| 77 | 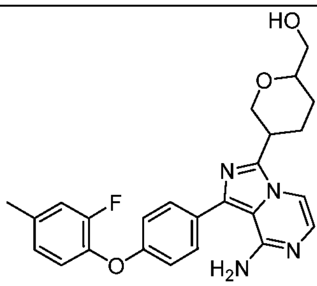 | A-50<br>B-1-B | 449.2 | |

| | | | | |
|---|---|---|---|---|
| 78 | 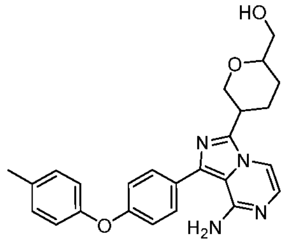 | A-51<br>B-1-B | 431.2 | |
| 79 | 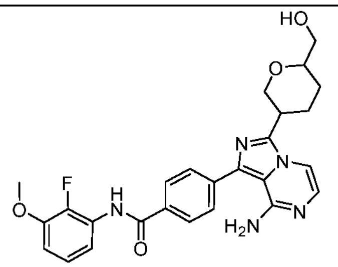 | A-52<br>B-1-B | 492.2 | |
| 81 | 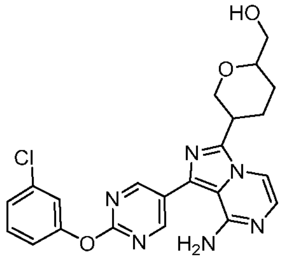 | A-54<br>B-1-B | 453.1 | |
| 82 | 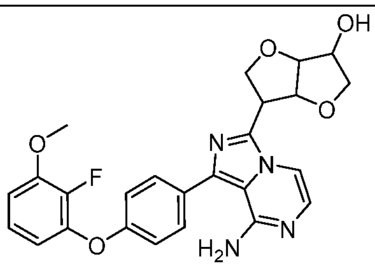 | A-2<br>B-8 | 479.2 | |
| 83 | 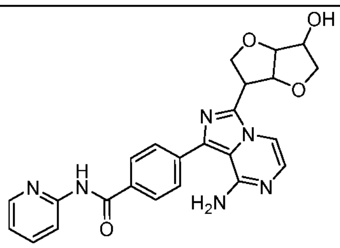 | A-5<br>B-8 | 459.2 | |
| 84 | 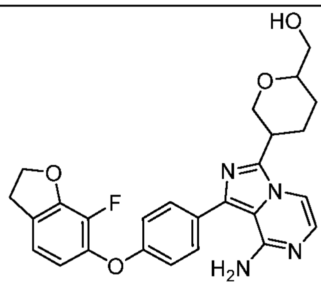 | A-55<br>B-1-B | 477.1 | |

| | | | | |
|---|---|---|---|---|
| 85 | 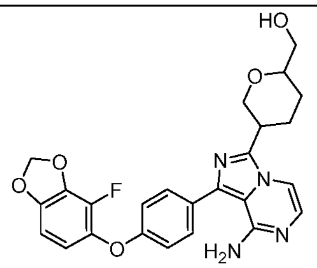 | A-56<br>B-1-B | 479.1 | |
| 86 | 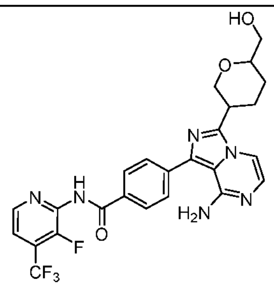 | A-57<br>B-1-B | 531.2 | |
| 87 | 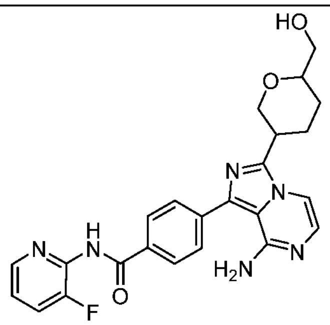 | A-58<br>B-1-B | 463.0 | |
| 88 | 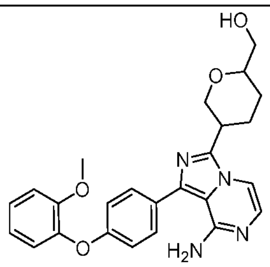 | A-59<br>B-1-B | 447.2 | |
| 89 | 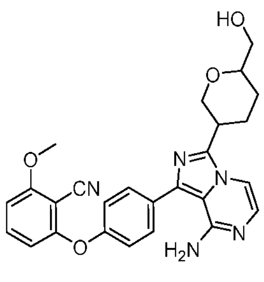 | A-60<br>B-1-A | 472.2 | δ 1.39-1.51 (1H, m), 1.74-1.77 (1H, m), 1.83-1.95 (1H, m), 2.12-2.15 (1H, m), 3.36-3.48 (4H, m), 3.64 (1H, t, J = 11.0 Hz), 3.96 (3H, s), 4.05-4.12 (1H, m), 4.68 (1H, t, J = 5.6 Hz), 6.09 (2H, brs), 6.65 (1H, d, J = 8.4 Hz), 7.00 (1H, d, J = 8.5 Hz), 7.05 (1H, d, J = 4.9 Hz), 7.26 (2H, d, J = 8.6 Hz), 7.56-7.69 (3H, m), 7.72 (1H, d, J = 5.0 Hz). |

Compound 89 was separated by SFC to obtain 89-P1 (peak first) and 89-P2 (peak last).

Conditions for preparative SFC:

Instrument: SFC-80 (Thar, Waters)

Column: CHIRALPAK AD(30×250mm 5μm) (Daicel)

Column temperature: 35°C

Mobile phase: A=CO2 Co-Solvent B= IPA(0.2% 7M NH3 MEOH)

Sample solution: Crude 330mg solid in 50mL IPA+ACN

Cycle Time:6.5min Run Time:20min

| $CO_2$ Flow Rate | Co-Solvent Flow Rate | Co-Solvent B% | Total Flow Rate | Back Pressure | Wavelength | Inj. Vol. |
|---|---|---|---|---|---|---|
| 22.5 ml/min | 22.5 ml/min | 50 | 45 ml/min | 80 bar | 215 nm | 3ML |

| | | | | |
|---|---|---|---|---|
| 90 | 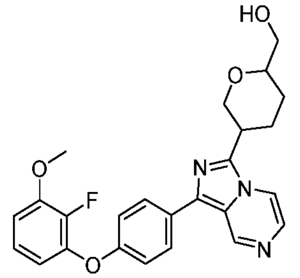 | A-2<br>B-9 | 450.1 | |
| 92 | 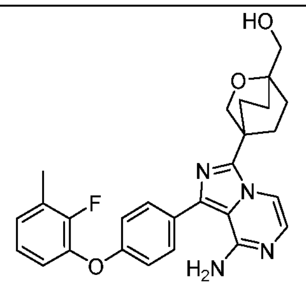 | A-11<br>B-2 | 475.2 | |
| 93 | 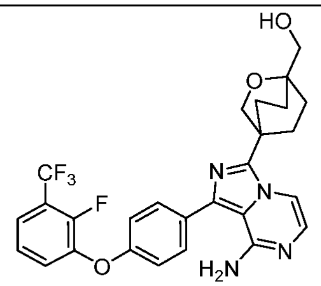 | A-10<br>B-2 | 529.1 | |
| 94 | 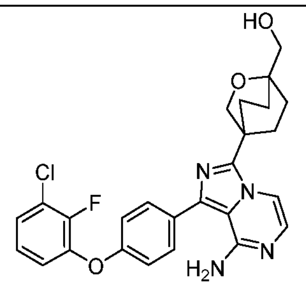 | A-7<br>B-2 | 495.0 | |

| | | | | |
|---|---|---|---|---|
| 95 | 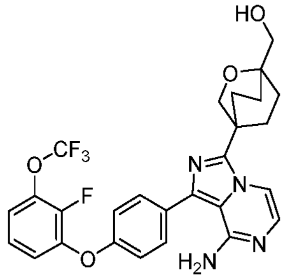 | A-13<br>B-2 | 545.1 | |
| 96 | 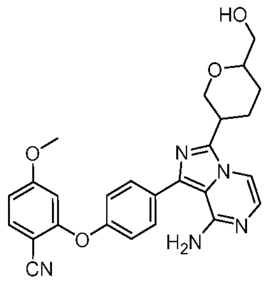 | A-61<br>B-1-A | 472.2 | δ 1.39-1.49 (1H, m), 1.73-1.75 (1H, m), 1.83-1.94 (1H, m), 2.10-2.14 (1H, m), 3.36-3.47 (4H, m), 3.63 (1H, t, J = 11.0 Hz), 3.79 (3H, s), 4.04-4.11 (1H, m), 4.69 (1H, t, J = 5.6 Hz), 6.10 (2H, brs), 6.64 (1H, d, J = 2.3 Hz), 6.91 (1H, dd, J = 8.8 Hz, 2.4 Hz), 7.04 (1H, d, J = 5.0 Hz), 7.25 (2H, d, J = 8.7 Hz), 7.65 (2H, d, J = 8.7 Hz), 7.72 (1H, d, J = 5.1 Hz), 7.85 (1H, d, J = 8.7 Hz). |
| 97 | 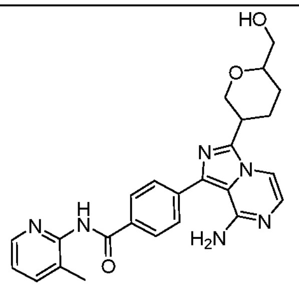 | A-62<br>B-1-B | 459.2 | |
| 98 | 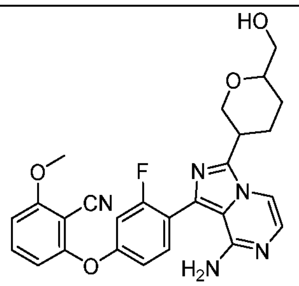 | A-63<br>B-1-A | 490.1 | |
| 99 | 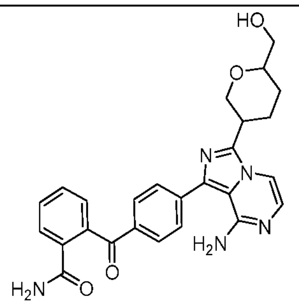 | A-64<br>B-1-A | 472.2 | |

| | | | | |
|---|---|---|---|---|
| 100 | 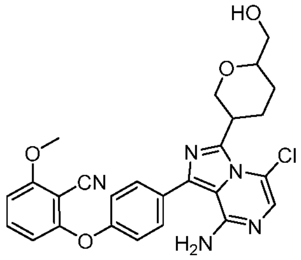 | 89 | 506.2 | |
| 101 | 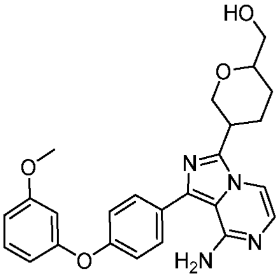 | A-65<br>B-1-A | 447.2 | δ 1.39-1.50 (1H, m), 1.74-1.77 (1H, m), 1.83-1.94 (1H, m), 2.10-2.14 (1H, m), 3.35-3.48 (4H, m), 3.63 (1H, t, J = 11.0 Hz), 3.76 (3H, s), 4.08 (1H, ddd, J = 11.2 Hz, 4.1 Hz, 1.8 Hz), 4.68 (1H, t, J = 5.6 Hz), 6.05 (2H, brs), 6.61-6.70 (2H, m), 6.76 (1H, dd, J = 8.0 Hz, 2.1 Hz), 7.03 (1H, d, J = 5.0 Hz), 7.13 (2H, d, J = 8.7 Hz), 7.32 (1H, t, J = 8.2 Hz), 7.59 (2H, d, J = 8.7 Hz), 7.71 (1H, d, J = 5.1 Hz). |
| 102 | 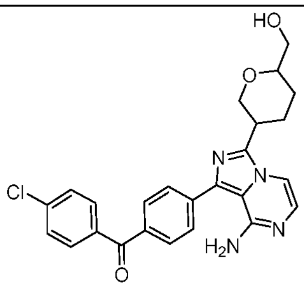 | A-66<br>B-1-A | 463.2 | |
| 103 | 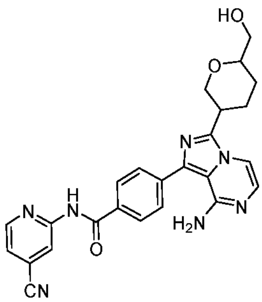 | A-67<br>B-1-A | 470.2 | δ 1.40-1.50 (1H, m), 1.74-1.77 (1H, m), 1.84-1.97 (1H, m), 2.09-2.18 (1H, m), 3.37-3.48 (4H, m), 3.64 (1H, t, J = 11.0 Hz), 4.03-4.13 (1H, m), 4.69 (1H, t, J = 5.6 Hz), 6.14 (2H, brs), 7.09 (1H, d, J = 5.0 Hz), 7.64 (1H, dd, J = 5.0 Hz, 1.4 Hz), 7.71-7.82 (3H, m), 8.16 (2H, d, J = 8.5 Hz), 8.53-8.56 (1H, m), 8.67 (1H, dd, J = 5.0 Hz, 0.8 Hz), 11.34 (1H, s). |
| 104 | 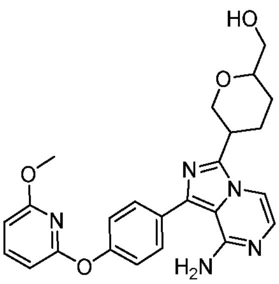 | A-68<br>B-1-A | 448.2 | δ 1.40-1.49 (1H, m), 1.74-1.78 (1H, m), 1.85-1.95 (1H, m), 2.12-2.19 (1H, m), 3.35-3.47 (4H, m), 3.64 (1H, t, J = 11.0 Hz), 3.72 (3H, s), 4.09 (1H, ddd, J = 11.3 Hz, 4.0 Hz, 1.8 Hz), 4.68 (1H, t, J = 5.5 Hz), 6.04 (2H, brs), 6.57 (2H, dd, J = 11.7 Hz, 7.9 Hz), 7.04 (1H, d, J = 5.0 Hz), 7.29 (2H, dd, J = 8.6 Hz), 7.63 (2H, d, J = 8.6 Hz), 7.69-7.81 (2H, m). |

| | | | | |
|---|---|---|---|---|
| 105 | 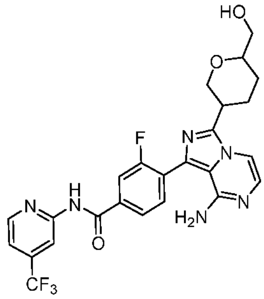 | A-69<br>B-1-A | 531.2 | |
| 106 | 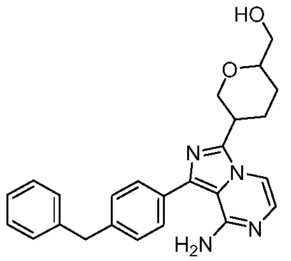 | A-70<br>B-1-A | 415.2 | δ 1.38-1.47 (1H, m), 1.72-1.75 (1H, m), 1.81-1.90 (1H, m), 2.09-2.12 (1H, m), 3.34-3.47 (4H, m), 3.60 (1H, t, J = 11.0 Hz), 4.01 (2H, s), 4.07 (1H, ddd, J = 11.4 Hz, 3.9 Hz, 1.8 Hz), 4.67 (1H, t, J = 5.6 Hz), 5.97 (2H, brs), 7.01 (1H, d, J = 5.0 Hz), 7.17-7.23 (1H, m), 7.26-7.37 (6H, m), 7.50 (2H, d, J = 8.1 Hz), 7.70 (1H, d, J = 5.1 Hz). |
| 107 | 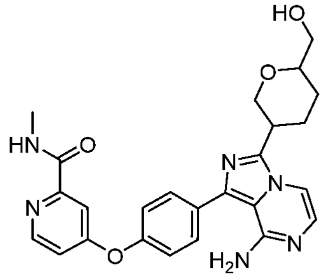 | A-71<br>B-1-A | 475.2 | |
| 108 | 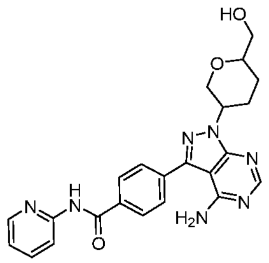 | A-5<br>B-4-A | 446.1 | δ 1.43-1.56 (1H, m), 1.85-1.89 (1H, m), 2.14-2.17 (1H, m), 2.27-2.35 (1H, m), 3.35-3.52 (4H, m), 3.76 (1H, t, J = 11.7 Hz), 3.98-4.08 (1H, m), 4.68-4.64 (2H, m), 7.17-7.21 (1H, m), 7.79 (2H, d, J = 8.4 Hz), 7.84-7.90 (1H, m), 8.21 (3H, t, J = 8.5 Hz), 8.29 (1H, s), 8.41-8.42 (1H, m), 10.89 (1H, s). |
| 109 | 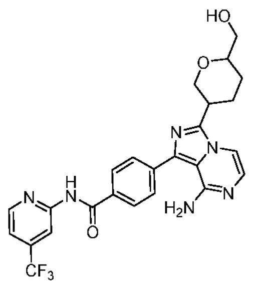 | A-72<br>B-1-A | 513.2 | δ 1.40-1.49 (1H, m), 1.74-1.78 (1H, m), 1.85-1.96 (1H, m), 2.12-2.15 (1H, m), 3.36-3.48 (4H, m), 3.64 (1H, t, J = 11.0 Hz), 4.04-4.13 (1H, m), 4.69 (1H, t, J = 5.7 Hz), 6.14 (2H, brs), 7.09 (1H, d, J = 5.0 Hz ), 7.56 (1H, dd, J = 5.1 Hz, 1.0 Hz), 7.75-7.78 (3H, m), 8.17 (2H, d, J = 8.5 Hz), 8.58 (1H, s), 8.70 (1H, d, J = 5.1 Hz), 11.35 (1H, s). |
| Compound 109 was separated by SFC to obtain 109-P1 (peak first) and 109-P2 (peak last).<br>Conditions for preparative SFC: | | | | |

Instrument: SFC-80 (Thar, Waters)

Column: CHIRALCEL OX (30×250mm 10μm) (Daicel)

Column temperature: 35°C

Mobile phase: A=$CO_2$ Co-Solvent B= ETOH (0.2% 7M NH3 MEOH)

Sample solution: Crude 320mg solid in 50mL ETOH

Cycle Time: 9min Run Time: 18min

| $CO_2$ Flow Rate | Co-Solvent Flow Rate | Co-Solvent B% | Total Flow Rate | Back Pressure | Wavelength | Inj. Vol. |
|---|---|---|---|---|---|---|
| 20.25ml/min | 24.75 ml/min | 55 | 45 ml/min | 100 bar | 215 nm | 4.5 ML |

| | | | | |
|---|---|---|---|---|
| 110 | 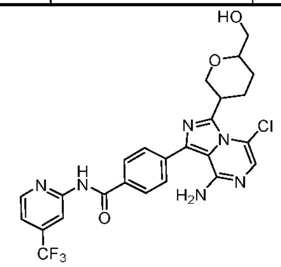 | 109 | 547.1 | |
| 111 | 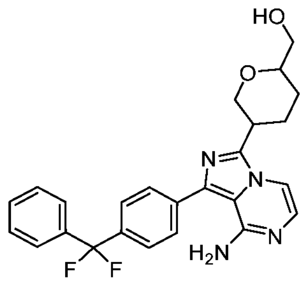 | A-73<br>B-1-A | 451.2 | δ 1.42-1.48 (1H, m), 1.72-1.75 (1H, m), 1.83-1.91 (1H, m), 2.07-2.16 (1H, m), 3.35-3.47 (4H, m), 3.61 (1H, t, J = 11.0 Hz), 4.07 (1H, ddd, J = 11.0 Hz, 4.1 Hz, 1.9 Hz), 4.68 (1H, t, J = 5.6 Hz), 6.09 (2H, brs), 7.06 (1H, d, J = 4.9 Hz), 7.50-7.65 (7H, m), 7.70-7.76 (3H, m). |

Compound 111 was separated by SFC to obtain 111-P1 (peak first) and 111-P2 (peak last).

Conditions for preparative SFC:

Instrument: SFC-80 (Thar, Waters)

Column: CHIRALCEL OJ (30×250mm 5μm) (Daicel)

Column temperature: 35°C

Mobile phase: A=$CO_2$ Co-Solvent B= MEOH/ACN=1/1

Sample solution: Crude 480 mg solid in 100 mL MEOH+ACN

Cycle Time: 5.2 min Run Time: 12 min

| $CO_2$ Flow Rate | Co-Solvent Flow Rate | Co-Solvent B% | Total Flow Rate | Back Pressure | Wavelength | Inj. Vol. |
|---|---|---|---|---|---|---|
| 27 ml/min | 18 ml/min | 40 | 45 ml/min | 100 bar | 215 nm | 4.7ML |

| | | | | |
|---|---|---|---|---|
| 112 | 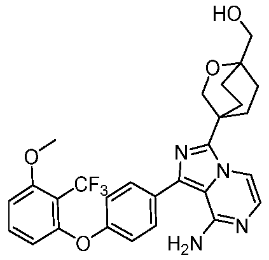 | A-76<br>B-2 | 541.2 | |
| 113 | 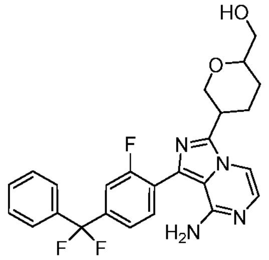 | A-75<br>B-1-A | 469.2 | |
| 114 | 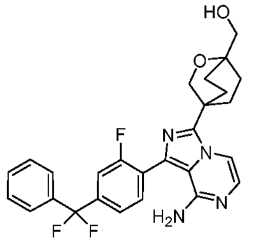 | A-75<br>B-2 | 495.1 | |
| 115 | 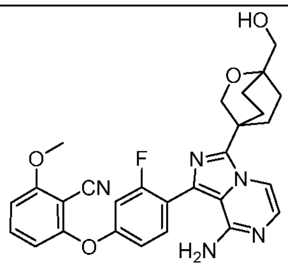 | A-63<br>B-2 | 516.2 | |
| 116 | 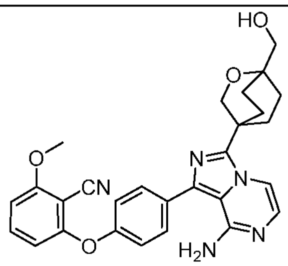 | A-60<br>B-2 | 498.2 | δ 1.71-1.89 (4H, m), 1.93-2.06 (2H, m), 2.32-2.44 (2H, m), 3.26 (2H, d, J = 6.0 Hz), 3.96 (3H, s), 4.21 (2H, brs), 4.65 (1H, t, J = 6.0 Hz), 6.09 (2H, brs), 6.66 (1H, d, J = 8.4 Hz), 6.93-7.07 (2H, m), 7.26 (2H, d, J = 8.6 Hz), 7.56-7.69 (3H, m), 7.90 (1H, d, J = 5.2 Hz). |
| 117 | 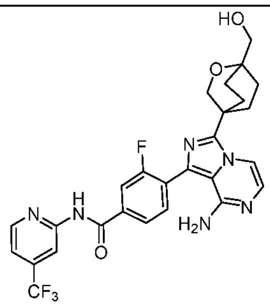 | A-69<br>B-2 | 557.2 | |

| | | | | |
|---|---|---|---|---|
| 118 | 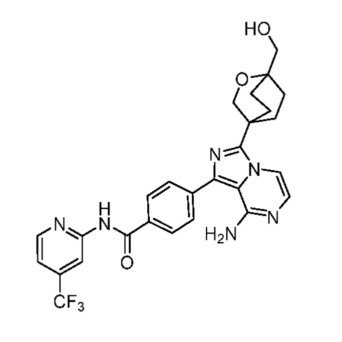 | A-72<br>B-2 | 539.2 | δ 1.70-1.89 (4H, m), 1.94-2.06 (2H, m), 2.34-2.44 (2H, m), 3.25 (2H, d, J = 5.9 Hz), 4.22 (2H, s), 4.66 (1H, t, J = 6.0 Hz), 6.12 (2H, brs), 7.04 (1H, d, J = 5.1 Hz), 7.55 (1H, d, J = 4.2 Hz), 7.74 (2H, d, J = 8.4 Hz), 7.94 (1H, d, J = 5.1 Hz), 8.16 (2H, d, J = 8.4 Hz), 8.57 (1H, s), 8.69 (1H, d, J = 5.1 Hz), 11.35 (1H, s). |
| 119 | 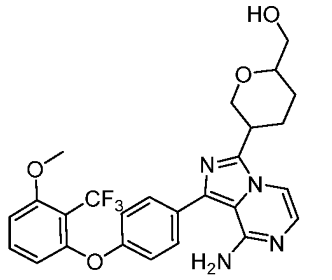 | A-76<br>B-1-A | 515.2 | |

**Example 120: Preparation of Compound 120**

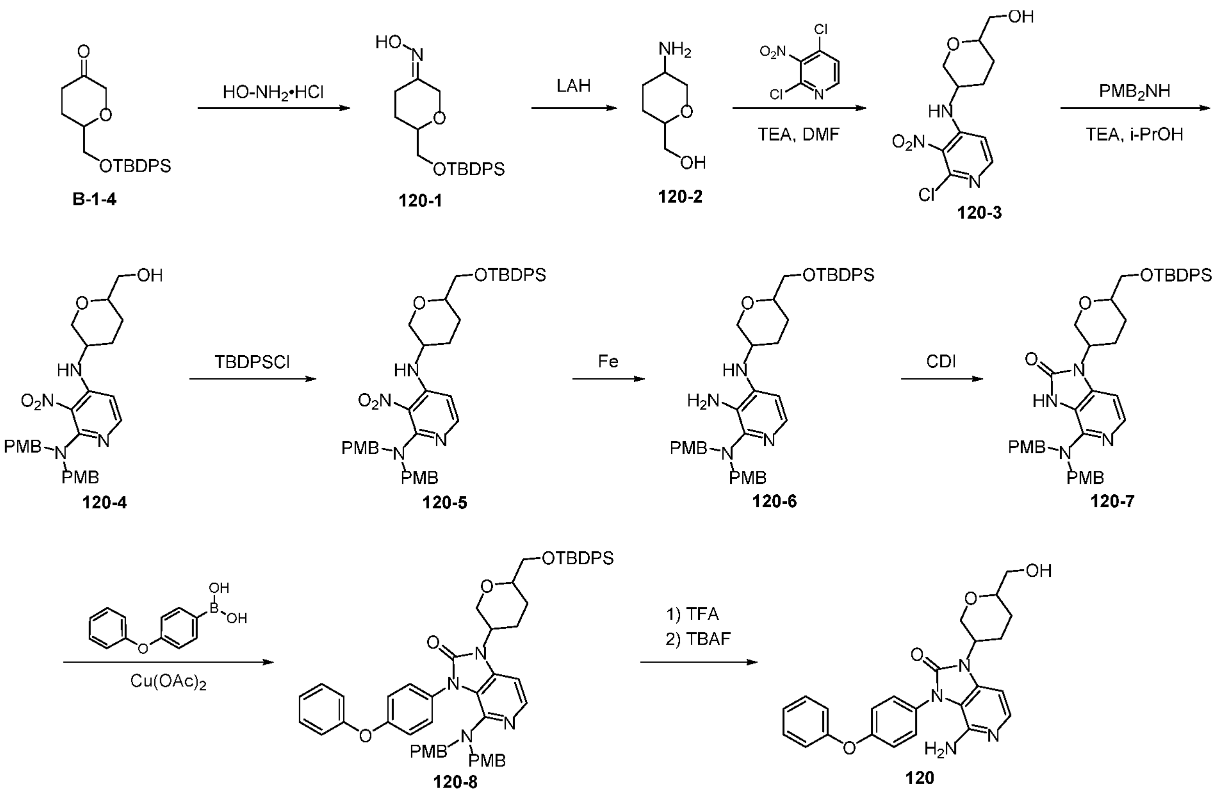


To a reaction flask, compound B-1-4 (3.10 g, 8.41 mmol), methanol (31 mL), hydroxylamine hydrochloride (1.17 g, 16.8 mmol) and sodium acetate (2.07 g, 25.2 mmol) were added. The reaction solution was stirred overnight at room temperature, poured into water, and extracted twice with ethyl acetate. The organic phases were combined, washed with saturated brine, concentrated under reduced pressure to dryness, and then purified by silica gel column chromatography to obtain 1.90 g of product 120-1 with a yield of 59%.

Compound 120-1 (1.90 g, 4.95 mmol) and tetrahydrofuran (20 mL) were added to a reaction flask, and cooled by an ice bath. Lithium aluminum hydride (376 mg, 9.91 mmol) was added to the reaction solution in portions. The reaction solution was warmed to room temperature for 3 h of reaction. To the reaction solution having been re-cooled by an ice bath, water (380 mg), 15% aqueous NaOH solution (380 mg), and water (1.14 g) were slowly added dropwise in sequence to quench the reaction. The resulting suspension was filtered under vacuum and washed with DCM/MeOH (10/1), and the filtrate was concentrated to dryness under reduced pressure, and then purified by silica gel column chromatography to obtain 200 mg of product 120-2 with a yield of 31%.

To a reaction flask, compound 2,4-dichloro-3-nitropyridine (294 mg, 1.52 mmol), DMF (2 mL), 120-2 (200 mg, 1.52 mmol) and triethylamine (231 mg, 2.29 mmol) were added. The reaction solution was stirred at room temperature for 4 h, poured into water, and extracted three times with ethyl acetate. The organic phases were combined, washed with saturated brine, dried over anhydrous sodium sulfate, filtered, and concentrated to dryness under reduced pressure to obtain 464 mg of product 120-3 with a yield of 100%. The product was not further purified.

To a reaction flask, compound 120-3 (464 mg, 1.61 mmol), isopropanol (5 mL), bis-(4-methoxybenzyl)-amine (415 mg, 1.61 mmol) and triethylamine (212 mg, 2.10 mmol) were added. The reaction solution was heated to 95°C, stirred for 4h, cooled, concentrated to dryness under reduced pressure, and then purified by silica gel column chromatography to obtain 540 mg of product 120-4 with a yield of 66%.

To a reaction flask, compound 120-4 (388 mg, 0.76 mmol), DMF (4 mL), imidazole (78 mg, 1.14 mmol), DMAP (10 mg, 0.076 mmol) and tert-butyldiphenylchlorosilane (210 mg, 0.76 mmol) were added. The reaction solution was heated to 60°C and stirred overnight. TLC showed a lot of raw material remaining. The reaction solution was supplemented with imidazole (150 mg), DMAP (40 mg) and tert-butyldiphenylchlorosilane (100 mg), heated to 80°C for 2 h of reaction, and supplemented with tert-butyldiphenylchlorosilane (200 mg) again. After 2 h, TLC showed that the reaction was complete. The reaction solution was cooled, poured into water, and extracted 3 times with ethyl acetate. The organic phases were combined, washed with saturated brine, dried over anhydrous sodium sulfate, filtered, concentrated to dryness under reduced pressure, and then purified by silica gel column chromatography to obtain 650 mg of product 120-5 with a yield of 100%.

To a reaction flask, compound 120-5 (650 mg, 0.87 mmol), methanol/glacial acetic acid (5 mL/5 mL) and iron powder (486 mg, 8.7 mmol) were added. The reaction solution was stirred at room temperature for 4 h, slowly poured into aqueous $NaHCO_3$ solution, and extracted twice with ethyl acetate. The organic phases were combined, washed with saturated brine, dried over anhydrous sodium sulfate, filtered, and concentrated to dryness under reduced pressure to obtain 612 mg of product 120-6 with a yield of 98%. The product was not further purified.

To a reaction flask, compound 120-6 (612 mg, 0.85 mmol), acetonitrile (6 mL) and N,N'-carbonyldiimidazole (280 mg, 1.71 mmol) were added. The reaction solution was heated to 80°C and stirred overnight. The reaction solution was cooled, concentrated to dryness under reduced pressure, and then purified by silica gel column chromatography to obtain 470 mg of product 120-7 with a yield of 74%.

To a reaction flask, compound 120-7 (470 mg, 0.63 mmol), dichloromethane (15 mL), 4-phenoxyphenylboronic acid (271 mg, 1.27 mmol), ketone acetate (115 mg, 0.63 mmol), 4A molecular sieve (500 mg) and triethylamine (192 mg, 1.90 mmol) were added. The reaction solution was stirred at room temperature for 36 h, filtered under vacuum with diatomaceous earth, and washed with ethyl acetate. The filtrate was concentrated to dryness under reduced pressure, and then purified by silica gel column chromatography to obtain 240 mg of product 120-8 with a yield of 41%.

To a reaction flask, compound 120-8 (260 mg, 0.29 mmol), dichloromethane (4 mL) and trifluoroacetic acid (4 mL) were added. The reaction solution was heated to 50°C and stirred for 3 h. The reaction solution was cooled, concentrated to dryness under reduced pressure, added with aqueous $NaHCO_3$ solution, and extracted twice with ethyl acetate. The organic phases were combined, washed with saturated brine, dried over anhydrous sodium sulfate, filtered, and concentrated under reduced pressure. The residue was dissolved in tetrahydrofuran (2 mL), added with TBAF (1 M, 0.2 mL), and stirred at room temperature for 1 h. The reaction solution was directly purified by a preparative silica gel plate to obtain 40 mg of product 120 with a yield of 30%.

The structure of the product was characterized by NMR and mass spectrometry, and the results are as follows:

$^1$H NMR (400 MHz, d6-DMSO) $\delta$ 1.37-1.53 (1H, m), 1.56-1.71 (1H, m), 1.81-1.95 (1H, m), 2.12-2.29 (1H, m), 2.34-2.44 (4H, m), 3.40-3.54 (1.5H, m), 3.59-3.70 (1H, m), 3.77-4.02 (1H, m), 4.17-4.39 (1H, m), 4.71 (0.5H, t, J = 5.7 Hz ), 4.76-4.83 (2H, m), 6.94 (0.5H, d, J = 5.6 Hz), 7.13 (4H, t, J = 8.5 Hz), 7.18-7.25 (1.5H, m), 7.40-7.48 (4H, m), 7.74 (1H, t, J = 5.6 Hz).

MS(ESI) m/z $(M+H)^+$: 433.2.

**Examples 121-138: Preparation of Compounds 121-138**

Compounds 121-138 were prepared using different intermediates according to the method for preparing compound 1-B or 3, and the numbers of the use intermediates, structural formulas, MS and $^1$H-NMR data are shown in Table

14.

Table 14: Structure, MS and $^1$H-NMR data of Examples 121-138

| Example | Structural formula | Intermediate No. | MS (ESI) m/z $(M+H)^+$ | $^1$H NMR (400 MHz, d6-DMSO) |
|---|---|---|---|---|
| 121 | 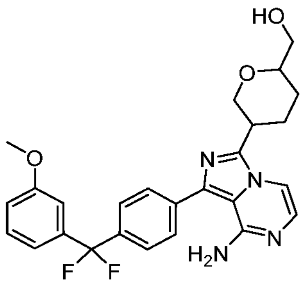 | A-77<br>B-1-A | 481.2 | δ 1.39-1.47 (1H, m), 1.72-1.75 (1H, m), 1.80-1.93 (1H, m), 2.10-2.13 (1H, m), 3.35-3.48 (4H, m), 3.61 (1H, t, J = 11.0 Hz), 3.80 (3H, s), 4.07 (1H, dd, J = 11.0 Hz, 2.0 Hz ), 4.69 (1H, t, J = 5.3 Hz), 6.09 (2H, brs), 7.00-7.18 (4H, m), 7.44 (1H, t, J = 8.3 Hz), 7.63 (2H, d, J = 8.3 Hz), 7.68-7.78 (3H, m). |
| 122 | 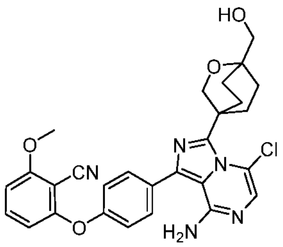 | 116 | 532.2 | |
| 123 | 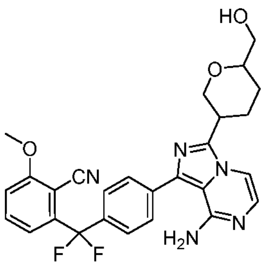 | A-13<br>B-1-A | 506.2 | |
| 124 | 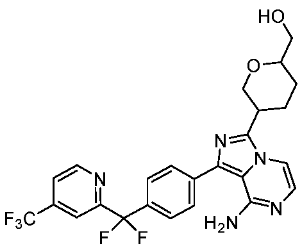 | A-79<br>B-1-A | 520.2 | δ 1.37-1.51 (1H, m), 1.72-1.75 (1H, m), 1.79-1.92 (1H, m), 2.09-2.13 (1H, m), 3.35-3.48 (4H, m), 3.61 (1H, t, J = 11.0 Hz), 4.02-4.11 (1H, m), 4.69 (1H, t, J = 5.5 Hz), 6.09 (2H, brs), 7.06 (1H, d, J = 4.9 Hz), 7.74 (5H, m), 7.99 (1H, d, J = 5.0 Hz), 8.22 (1H, s), 8.98 (1H, d, J = 5.0 Hz). |
| 125 | 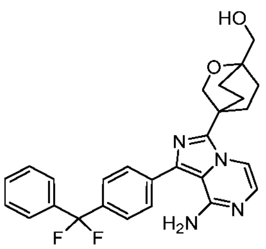 | A-73<br>B-2 | 477.2 | δ 1.69-1.87 (4H, m), 1.92-2.02 (2H, m), 2.34-2.42 (2H, m), 3.24 (2H, d, J = 5.8 Hz), 4.19 (2H, s), 4.64 (1H, t, J = 5.9 Hz), 6.15 (2H, brs), 7.02 (1H, d, J = 5.1 Hz), 7.49-7.54 (3H, m), 7.56-7.65 (4H, m), 7.71 (2H, d, J = 8.2 Hz), 7.92 (1H, d, J = 5.2 Hz). |

(continued)

| Example | Structural formula | Intermediate No. | MS (ESI) m/z $(M+H)^+$ | $^1$H NMR (400 MHz, d6-DMSO) |
|---|---|---|---|---|
| 126 | 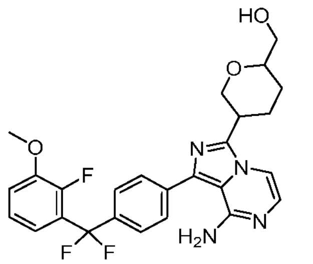 | A-80<br>B-1-A | 499.2 | |
| 127 | 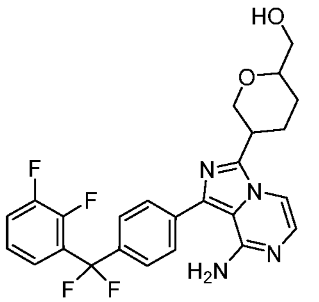 | A-81<br>B-1-A | 487.1 | |
| 128 | 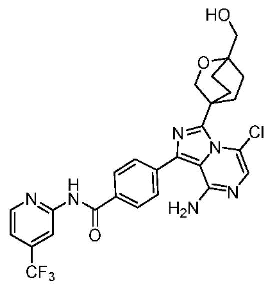 | 118 | 573.1 | |
| 129 | 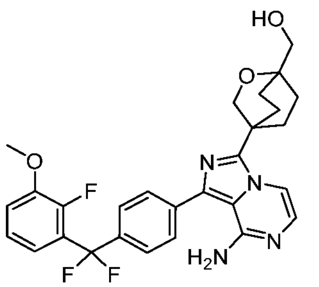 | A-80<br>B-2 | 525.2 | |
| 130 | 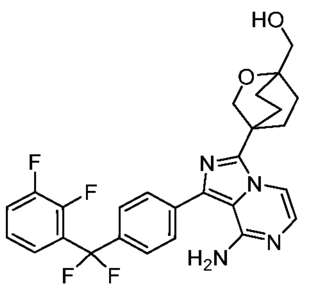 | A-81<br>B-2 | 513.2 | δ 1.71-1.87 (4H, m), 1.94-2.01 (2H, m), 2.34-2.41 (2H, m), 3.24 (2H, d, J = 5.7 Hz), 4.20 (2H, s), 4.64 (1H, t, J = 5.8 Hz), 6.07 (2H, brs), 7.02 (1H, d, J = 5.0 Hz), 7.38-7.45 (H, m), 7.52 (1H, t, J = 6.6 Hz), 7.62-7.74 (4H, m), 7.91 (1H, d, J = 5.1 Hz). |

(continued)

| Example | Structural formula | Intermediate No. | MS (ESI) m/z $(M+H)^+$ | $^1H$ NMR (400 MHz, d6-DMSO) |
|---|---|---|---|---|
| 131 | 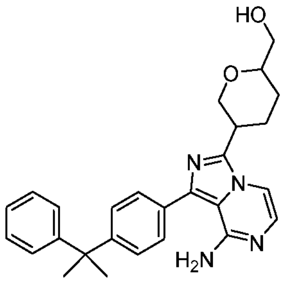 | A-82 B-1-A | 443.2 | |
| 132 | 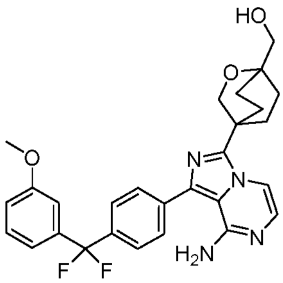 | A-77 B-2 | 507.2 | |
| 133 | 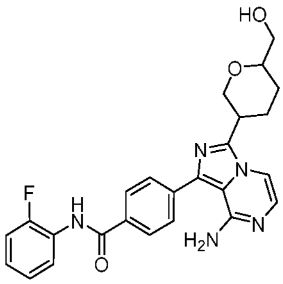 | A-53 B-1-B | 462.2 | |
| 134 | 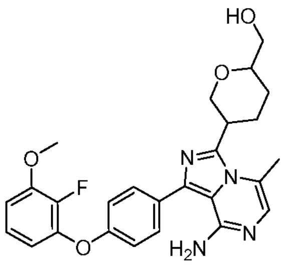 | A-2 B-10 | 479.2 | |
| 135 | 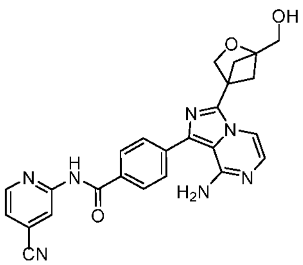 | A-67 B-3 | 468.1 | |

(continued)

| Example | Structural formula | Intermediate No. | MS (ESI) m/z (M+H)+ | 1H NMR (400 MHz, d6-DMSO) |
|---|---|---|---|---|
| 136 | 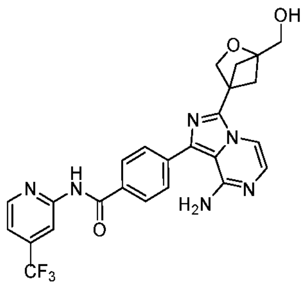 | A-72 B-3 | 511.2 | |
| 137 | 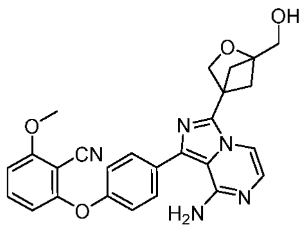 | A-60 B-3 | 470.2 | |
| 138 | 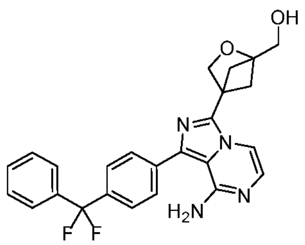 | A-73 B-3 | 449.2 | |
| 139 | 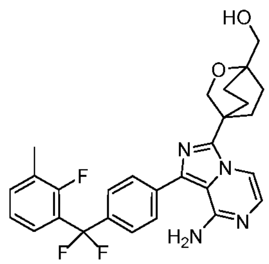 | A-83 B-2 | 509.2 | |
| 140 | 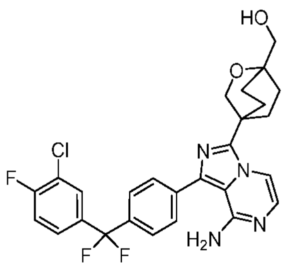 | A-84 B-2 | 529.1 | |

(continued)

| Example | Structural formula | Intermediate No. | MS (ESI) m/z (M+H)$^+$ | $^1$H NMR (400 MHz, d6-DMSO) |
|---|---|---|---|---|
| 141 | 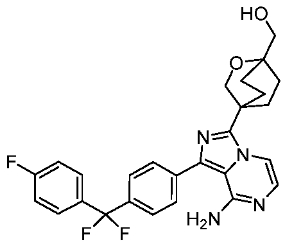 | A-85<br>B-2 | 495.2 | |
| 142 | 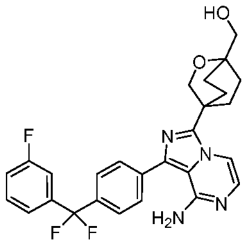 | A-86<br>B-2 | 495.1 | |
| 143 | 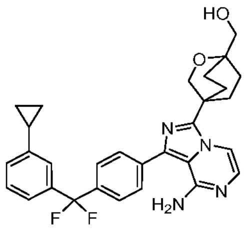 | A-87<br>B-2 | 517.1 | |
| 144 | 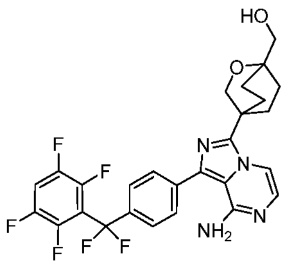 | A-88<br>B-2 | 549.1 | δ 1.71-1.87 (4H, m), 1.94-2.01 (2H, m), 2.35-2.41 (2H, m), 3.24 (2H, d, J = 5.5 Hz), 4.19 (2H, s), 4.64 (1H, t, J = 5.8 Hz), 6.21 (2H, brs), 7.03 (1H, d, J = 5.1 Hz), 7.73 (4H, dd, J = 20.4, 8.4 Hz), 7.93 (1H, d, J = 5.2 Hz), 8.16-8.25 (1H, m). |
| 145 | 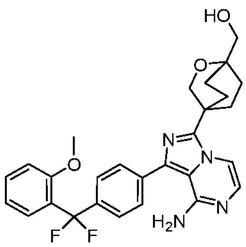 | A-89<br>B-2 | 495.0 | δ 1.71-1.86 (4H, m), 1.94-2.01 (2H, m), 2.34-2.41 (2H, m), 3.24 (2H, d, J = 6.0 Hz), 4.19 (2H, s), 4.64 (1H, t, J = 6.0 Hz), 6.07 (2H, brs), 7.02 (1H, d, J = 5.1 Hz), 7.33-7.41 (2H, m), 7.59-7.73 (6H, m), 7.91 (1H, d, J = 5.1 Hz). |
| 146 | 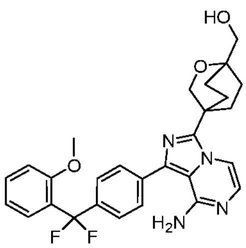 | A-90<br>B-2 | 529.0 | δ 1.71-1.86 (4H, m), 1.94-2.01 (2H, m), 2.34-2.41 (2H, m), 3.24 (2H, d, J = 6.0 Hz), 4.20 (2H, s), 4.64 (1H, t, J = 6.0 Hz), 6.06 (2H, brs), 7.02 (1H, d, J = 5.1 Hz), 7.57-7.74 (7H, m), 7.91 (1H, d, J = 5.1 Hz). |

(continued)

| Example | Structural formula | Intermediate No. | MS (ESI) m/z (M+H)+ | 1H NMR (400 MHz, d6-DMSO) |
|---|---|---|---|---|
| 147 | 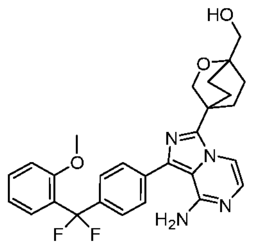 | A-91 B-2 | 509.1 | |
| 148 | 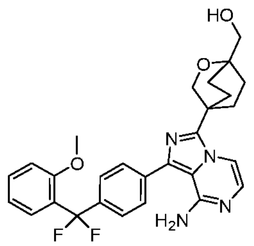 | A-92 B-2 | 507.1 | |
| 149 | 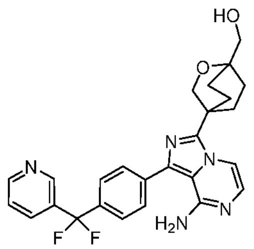 | A-93 B-2 | 478.1 | |
| 150 | 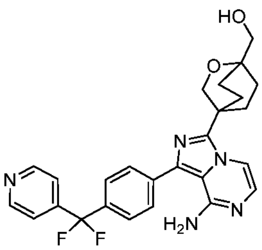 | A-94 B-2 | 478.1 | |
| 151 | 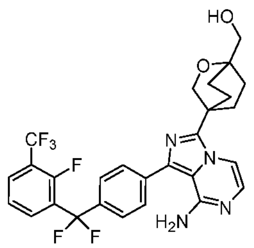 | A-95 B-2 | 563.0 | |
| 152 | 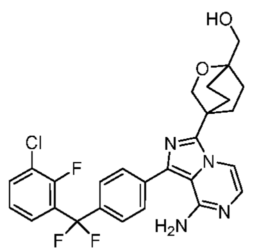 | A-96 B-2 | 529.0 | δ 1.71-1.86 (4H, m), 1.94-2.01 (2H, m), 2.34-2.41 (2H, m), 3.24 (2H, d, J = 5.9 Hz), 4.20 (2H, s), 4.64 (1H, t, J = 6.0 Hz), 6.10 (2H, brs), 7.02 (1H, d, J = 5.0 Hz), 7.43 (1H, t, J = 8.0 Hz), 7.63 (2H, d, J = 8.3 Hz), 7.68-7.75 (3H, m), 7.83 (1H, t, J = 6.9 Hz), 7.91 (1H, d, J = 5.1 Hz). |

(continued)

| Example | Structural formula | Intermediate No. | MS (ESI) m/z $(M+H)^+$ | $^1$H NMR (400 MHz, d6-DMSO) |
|---|---|---|---|---|
| 153 | 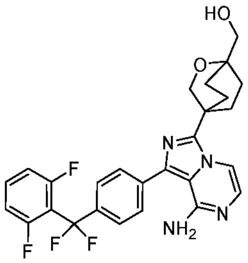 | A-97<br>B-2 | 513.0 | |
| 154 | 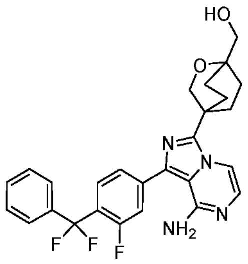 | A-98<br>B-2 | 495.1 | δ 1.70-1.86 (4H, m), 1.94-2.00 (2H, m), 2.34-2.41 (2H, m), 3.24 (2H, d, J = 6.0 Hz), 4.19 (2H, s), 4.64 (1H, t, J = 6.0 Hz), 6.04 (2H, brs), 7.02 (1H, d, J = 5.0 Hz), 7.54-7.61 (5H, m), 7.70 (2H, d, J = 8.2 Hz), 7.87 (1H, d, J = 6.7 Hz), 7.91 (1H, d, J = 5.1 Hz). |
| 155 | 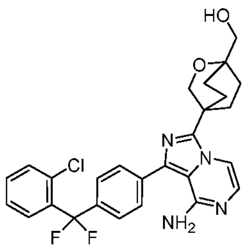 | A-99<br>B-2 | 511.0 | |
| 156 | 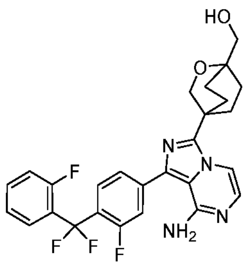 | A-100<br>B-2 | 513.0 | |
| 157 | 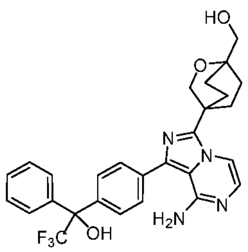 | A-101<br>B-2 | 525.1 | |

(continued)

| Example | Structural formula | Intermediate No. | MS (ESI) m/z (M+H)+ | $^1$H NMR (400 MHz, d6-DMSO) |
|---|---|---|---|---|
| 158 | 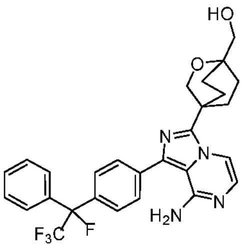 | A-102<br>B-2 | 527.1 | |
| 159 | 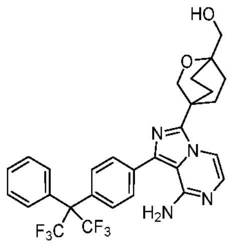 | A-103<br>B-2 | 577.0 | |

**Drug efficacy test**

**Test Example 1: BTK kinase activity inhibition test in vitro**

1: Compound preparation
Compound powders were dissolved in 100% DMSO to make 10 mM stock solutions, which were stored at -20°C in the dark.

2: Kinase reaction process

(1) Preparation of 1 × Kinase buffer.
(2) Preparation of compound with gradient concentrations: The test compound was tested at a concentration of 1 μM, diluted to a 100-fold final concentration of 100% DMSO solution in a 384 source plate, and 3-fold diluted to 10 concentrations. 250 nL of the compound with 100-fold final concentration was transferred to a destination plate OptiPlate-384F by using a liquid handler Echo 550.
(3) A kinase solution with 2.5-fold final concentration was prepared with 1 × Kinase buffer.
(4) 10 μL of the kinase solution with 2.5-fold final concentration was added to compound wells and positive control wells; and 10 μL of 1×Kinase buffer was added to negative control wells.
(5) After centrifugation at 1000 rpm for 30 seconds, the reaction plate was shaken to mix well and incubated at room temperature for 10 min.
(6) A 5/3-fold final concentration of a mixed solution of ATP and Kinase substrate 2 was prepared with 1 × Kinase buffer.
(7) 15 μL of the mixed solution of ATP and substrate with 5/3-fold final concentration was added to start the reaction.
(8) The 384-well plate was centrifuged at 1000 rpm for 30 seconds, shaken to mix well, and incubated at room temperature for 10 min.
(9) 30 μL of a stop solution was added to stop the kinase reaction, and after centrifugation at 1000 rpm for 30 seconds, the plate was shaken to mix well;
(10) The conversion rate was read with Caliper EZ Reader.

3: Data analysis

Calculation formula:

$$\% \text{ Inhibition} = \frac{\text{Conversion\%_max} - \text{Conversion\%_sample}}{\text{Conversion\%_max} - \text{Conversion\%_min}} \times 100$$;

where Conversion%_sample indicates the conversion rate reading of the sample; Conversion%_min is the average reading of the negative control wells, representing the conversion rate reading of the wells without enzyme activity; Conversion%_max is the average reading of the ratio of the positive control wells, representing the conversion rate reading of the wells without compound inhibition.

Dose-response curve fitting

With the log value of the concentration as the X-axis, and the percentage inhibition rate as the Y-axis, the dose-effect curve was fitted using the log(inhibitor) vs. response-Variable slope of the analysis software GraphPad Prism 5, so as to determine the IC50 value of each compound on the enzyme activity.

The calculation formula was:

$$Y=Bottom+(Top-Bottom)/(1+10^{\wedge}((LogIC50-X)\times HillSlope)).$$

The inhibitory activities of the compounds of the present disclosure against BTK wild-type and BTK mutation-type C481S kinases are shown in Table 15.

IC50: A≤5 nM; 5 nM<B≤20 nM; 20 nM<C≤100 nM; 100 nM<D≤1000 nM; E>1000 nM.

Table 15: Inhibitory activity of compounds of the present disclosure against BTK and BTK-C481S kinases

| Example | BTK IC50 | BTK-C481S | Example | BTK IC50 | BTK-C481S |
|---|---|---|---|---|---|
| 1-A | B | \ | 69 | C | \ |
| 1-B | C | B | 70 | B | \ |
| 2-A-P1 | A | A | 71 | C | \ |
| 2-A-P2 | C | C | 72 | D | \ |
| 2-B | C | \ | 73 | D | \ |
| 3 | C | \ | 74 | E | \ |
| 4 | C | \ | 75 | B | \ |
| 5 | A | A | 76 | D | \ |
| 6 | A | A | 77 | C | \ |
| 7 | A | A | 78 | C | \ |
| 8 | A | A | 79 | E | \ |
| 9 | A | \ | 81 | E | \ |
| 10 | A | \ | 82 | C | \ |
| 11 | A | \ | 83 | D | \ |
| 12 | A | A | 84 | C | \ |
| 13 | A | \ | 85 | D | \ |
| 14 | A | \ | 86 | D | \ |
| 15 | B | \ | 87 | D | \ |
| 16 | B | A | 88 | C | \ |

(continued)

| Example | BTK IC50 | BTK-C481S | Example | BTK IC50 | BTK-C481S |
|---|---|---|---|---|---|
| 17 | B | B | 89-P1 | A | A |
| 18 | C | \ | 89-P2 | A | \ |
| 19 | B | \ | 90 | C | \ |
| 20 | B | \ | 92 | A | \ |
| 21 | C | \ | 93 | B | \ |
| 22 | C | \ | 94 | B | \ |
| 23 | D | \ | 95 | B | \ |
| 24 | A | \ | 96 | C | \ |
| 25 | C | \ | 97 | D | \ |
| 26 | B | B | 98 | A | \ |
| 27 | C | \ | 99 | E | \ |
| 28 | C | \ | 100 | A | \ |
| 29 | B | \ | 101 | A | \ |
| 30 | B | \ | 102 | C | \ |
| 31 | E | \ | 103 | A | A |
| 32 | B | \ | 104 | B | \ |
| 33 | C | \ | 105 | A | \ |
| 34 | B | B | 106 | A | A |
| 35 | B | \ | 107 | D | \ |
| 36 | B | \ | 108 | B | \ |
| 37 | A | \ | 109-P1 | A | A |
| 38 | B | \ | 109-P2 | A | \ |
| 39 | A | \ | 110 | A | \ |
| 40 | B | \ | 111 | A | A |
| 41 | B | \ | 112 | B | \ |
| 42 | C | \ | 113 | B | \ |
| 43 | C | \ | 114 | B | \ |
| 44 | C | \ | 115 | A | \ |
| 45 | B | \ | 116 | A | \ |
| 46 | D | \ | 117 | A | \ |
| 47 | D | \ | 118 | A | A |
| 48 | C | \ | 119 | A | \ |
| 49 | E | \ | 120 | C | B |
| 50 | C | \ | 121 | B | B |
| 51 | C | \ | 122 | A | \ |
| 52 | B | \ | 123 | C | \ |
| 53 | C | \ | 124 | D | D |
| 54 | C | \ | 125 | B | A |

(continued)

| Example | BTK IC50 | BTK-C481S | Example | BTK IC50 | BTK-C481S |
|---|---|---|---|---|---|
| 55 | C | \ | 126 | B | \ |
| 56 | E | \ | 127 | B | \ |
| 57 | C | \ | 128 | A | \ |
| 58 | E | \ | 129 | B | \ |
| 59 | E | \ | 130 | B | \ |
| 60-A | B | \ | 131 | E | \ |
| 60-B | C | \ | 132 | C | \ |
| 61 | B | \ | 133 | D | \ |
| 62 | C | \ | 134 | B | \ |
| 63 | D | \ | 135 | B | \ |
| 64 | B | \ | 136 | B | \ |
| 65 | B | \ | 137 | B | \ |
| 66 | C | \ | 138 | C | \ |
| 67 | D | \ | ARQ-531 | B | A |
| 68 | C | \ | Ibrutinib | A | B |
| 111-P1 | A | A | 111-P2 | C | \ |
| 140 | D | \ | 141 | D | \ |
| 142 | D | \ | 143 | C | \ |
| 145 | C | \ | 152 | D | \ |
| 155 | D | \ | Tirabrutinib | C | D |
| "\" means that this test was not done. | | | | | |

**Test Example 2: Liver microsome metabolic stability test**

1: To the sample wells of T0, T5, T10, T20, T30, T60 and NCF60, 10 μL of the test or reference working solution and 80 μL of microsome working solution (hepatic microsome with a protein concentration of 0.5 mg/mL) were added, and to Blank60 well, only the microsome working solution was added. Then samples of the Blank60, T5, T10, T20, T30 and T60 other than T0 and NCF60 were placed in a 37°C water bath and pre-incubated for about 10 min.

2: To the T0 sample well, 300 μL of stop solution (containing 200 ng/mL tolbutamide and 200 ng/mL labetalol in acetonitrile solution) was added first, followed by 10 μL of NADPH regeneration system working solution.

3: After the pre-incubation of Blank60, T5, T10, T20, T30 and T60 incubation plates, 10 μL of NADPH regeneration system working solution was added to each sample well to start the reaction, and 10 μL of 100 mM potassium phosphate buffer was added to the NCF60 sample well.

4: After incubation for an appropriate time (such as 5, 10, 20, 30 and 60 min), to each test sample well and control well of the Blank60, T5, T10, T20, T30, T60 and NCF60 plates, 300 μL of stop solution was added to terminate the reaction.

5: All sample plates were shaken to mix well and centrifuged at 4000 rpm for 20 min, and 100 μL of the supernatant of the test sample or control sample was taken and diluted with 300 μL of pure water for LC-MS/MS analysis.

6: Data analysis: $T_{1/2}$ and $CL_{int(mic)}$ (μL/min/mg) values were calculated according to the first-order elimination kinetics. The first-order elimination kinetics equation was:

$$C_t = C_0 \bullet e^{-k_e \bullet t}$$

*when* $C_t = \frac{1}{2} C_0$,

$$T_{1/2} = \frac{Ln2}{k_e} = \frac{0.693}{k_e}$$

$$CL_{int(mic)} = \frac{0.693}{\text{(in vitro) } T_{1/2}} \bullet \frac{1}{mg/mL \text{ (Protein concentration of liver microsome in the reaction system)}}$$

$$CL_{int(liver)} = CL_{int(mic)} \bullet \frac{mg \text{ (Mass of liver microsome)}}{g \text{ (Mass of liver)}} \bullet \frac{g \text{ (Mass of liver)}}{kg \text{ (Body weight)}}$$

The test results of human and rat liver microsome metabolic stability are shown in Table 16:

Table 16: Test results of liver microsome metabolic stability of compounds of the present disclosure

| Example / Species | $T_{1/2}$ (min) | | $CL_{int}$(μL/min/mg) | | %Remaining (60min) | |
|---|---|---|---|---|---|---|
| | Human | Rat | Human | Rat | Human | Rat |
| 1-B | >145 | 76.1 | <9.6 | 18.2 | 99.9 | 63.7 |
| 2-A-P1 | >145 | >145 | <9.6 | <9.6 | 122.5 | 95.5 |
| 5 | 35.7 | 11.4 | 38.8 | 121.8 | 31.7 | 12.7 |
| 8 | 40.9 | 20.8 | 33.9 | 66.5 | 37.4 | 13.6 |
| 9 | 28.6 | 12.2 | 48.4 | 113.5 | 24.5 | 3.2 |
| 12 | 11.1 | 11.8 | 124.3 | 117.5 | 2.6 | 3.0 |
| 16 | 37.5 | 21.7 | 36.9 | 63.9 | 31.8 | 14.6 |
| 19 | 29.4 | 16.7 | 47.1 | 83.1 | 23.5 | 7.9 |
| 24 | 18.5 | 5.4 | 74.9 | 255.0 | 9.7 | 0.1 |
| 26 | 44.5 | 30.1 | 31.1 | 46.1 | 39.1 | 24.5 |
| 34 | >145 | >145 | <9.6 | <9.6 | 112.0 | 90.3 |
| 60-A | >145 | 143.2 | <9.6 | 9.7 | 109.9 | 69 |
| 64 | 37.7 | 17.0 | 36.8 | 81.6 | 31.7 | 8.5 |

| | | | | | | |
|---|---|---|---|---|---|---|
| 70 | 105.2 | 45.7 | 13.2 | 30.3 | 77.7 | 37.3 |
| 89-P1 | >145 | 88.4 | <9.6 | 15.7 | 100.4 | 61.0 |
| 101 | >145 | 46.5 | <9.6 | 29.8 | 81.1 | 39.5 |
| 103 | >145 | >145 | <9.6 | <9.6 | 100.3 | 85.7 |
| 106 | 105.5 | 46.9 | 13.1 | 29.5 | 66.3 | 39.1 |
| 109-P1 | >145 | 113.7 | <9.6 | 12.2 | 85.4 | 72.7 |
| 111 | 127.3 | 53.2 | 10.9 | 26.1 | 73.5 | 46 |
| 118 | >145 | 101.2 | <9.6 | 13.7 | 77.4 | 64.1 |
| 125 | 95.0 | 63.8 | 14.6 | 21.7 | 61.2 | 49 |
| Ibrutinib | 2.8 | 1.4 | 512 | 1904 | 0.3 | 0.2 |
| ARQ-531 | >145 | >145 | <9.6 | <9.6 | 94.2 | 81.1 |
| 145 | 61.2 | 60.8 | 22.7 | 22.8 | 46.2 | 49.6 |
| 152 | 30.2 | 13.5 | 45.9 | 102.5 | 25.4 | 25.8 |
| 155 | 33.3 | 22.3 | 41.7 | 62.2 | 25.8 | 14.2 |
| Tucatinib | 31.8 | 39.8 | 43.7 | 34.9 | 28.8 | 38.2 |
| Tirabrutinib | 106.1 | 42.1 | 13.1 | 32.9 | 66.3 | 37.0 |

**Test Example 3: Pharmacokinetic Test**

Each test compound was administered orally (10 mg/kg, 3 rats in each group) to SD rats for pharmacokinetic study. The test compound was dissolved in 5% DMSO+10% solutol+85% saline, vortexed for 1-2 min, and ultrasonicated for 5-10 min to prepare into a colorless, transparent and clear administration solution. Animals were fasted overnight before oral administration, and fed again after 4 h of administration. After SD rats were administered orally, pharmacokinetic samples were collected through orbital blood collection at the collection time points of 0.25 h, 0.5 h, 1 h, 2 h, 2.5 h, 3 h, 4 h, 6 h, 8 h and 10 h after administration. At each collection time point, 3 whole blood samples were collected at a the collection volume of about 0.2-0.3 mL. The blood samples were placed on ice once collected, and centrifuged to separate the plasma within 15 min (centrifugation conditions: 8000 rpm, 1 min, room temperature). The collected plasma was stored at -20°C before analysis. 20 μL of plasma sample was added into a 1.6 mL 96-well deep-well plate, added with 200 μL of working internal standard solution (the same volume of vehicle was added to the blank instead of internal standard), vortexed for 1 min, and centrifuged at 5800 rpm for 10 min. 100 μL of the supernatant was added to a 96-well injection plate for LC-MS/MS analysis.

The pharmacokinetic test results of some compounds of the present disclosure are shown in Table 17 below:

Table 17 Pharmacokinetic test results of some compounds of the present disclosure

| **Example** | T1/2 (h) | Cmax (ng/ml) | AUC(0-∞) (ng/ml×h) | Cl (ml/hr/kg) |
|---|---|---|---|---|
| 2-A-P1 | 2.21 | 25188 | 77004 | 131 |
| 5 | 1.17 | 463 | 2519 | 4148 |
| 34 | 1.70 | 11071 | 20631 | 486 |
| 60-B | 2.89 | 946 | 889 | 11329 |
| 89-P1 | 1.16 | 2975 | 8119 | 1232 |

(continued)

| Example | T1/2 (h) | Cmax (ng/ml) | AUC(0-∞) (ng/ml×h) | Cl (ml/hr/kg) |
|---|---|---|---|---|
| 101 | 1.25 | 1770 | 3673 | 2925 |
| 103 | 1.73 | 4729 | 10391 | 1000 |
| 106 | 1.87 | 1221 | 2576 | 3885 |
| 109-P1 | 2.83 | 16705 | 103882 | 97 |
| 111 | 3.15 | 1779 | 9628 | 1070 |
| 116 | 1.35 | 858 | 2010 | 5084 |
| 118 | 3.35 | 25721 | 211031 | 48 |
| 119 | 0.64 | 663 | 2250 | 4555 |
| 125 | 4.19 | 1181 | 8865 | 1045 |
| Ibrutinib | 1.28 | 222 | 362 | 2210 |
| ARQ-531 | 3.98 | 2414 | 16185 | 619 |
| 145 | 2.78 | 1552 | 17199 | 1262 |
| 152 | 2.82 | 199.5 | 1326 | 16367 |
| Tucatinib | 1.72 | 444.6 | 2270 | 9678 |
| Tirabrutinib | 0.33 | 920 | 965 | 11395 |

**Test Example 4: In vitro cell proliferation inhibitory activity test**

1: Cell Culture

Cells were cultured in 1640 medium, added with 10% inactivated FBS and 1% double antibiotics, and cultured at 37°C and 5% $CO_2$.

2: Cell Plating

(1) Cells were routinely cultured until the cell density was 80%-90%, and the cells were harvested when their number reached the requirement.

(2) Cells were resuspended with corresponding culture medium, counted, and prepared into cell suspension at appropriate density.

(3) The cell suspension was added to a 96-well plate, 100 μL per well.

(4) Cells were cultured overnight in a 37°C, 5% $CO_2$ incubator.

3: Compound Preparation

(1) The compounds to be tested were diluted with DMSO to make a stock solution with a final concentration of 20 mM for later use.

(2) The stock solution was diluted 10 times from 20 mM to 2 mM with DMSO, and then diluted 3 times from 2 mM to 9 concentrations.

(3) The blank control wells were cells plus 0.5% DMSO, as high-reading control wells.

(4) Wells with no cells and only medium were used as low-reading control wells.

4: Cell treatment with compound

(1) 24 h after the cells were plated, the compounds acted alone, and 99 μL of growth medium was added to each well, followed by 1 μL of the compound prepared in step 3. The plate was shaken gently to mix uniformly, and then placed in a 37°C, 5% $CO_2$ incubator.

(2) The cell plate was placed in an incubator for 72 h.

5: Detection by CTG method

(1) The cell plate to be tested was placed at room temperature for 30 min, and 100 μL of medium was discarded from each well.

(2) 100 μL of CTG reagent (CelltiterGlo kit) was added to each well, placed on a fast shaker for 2 min, and stood at room temperature in the dark for 30 min.

(3) The chemiluminescent signal value was read with the Envision instrument.

6: Data Analysis

$IC_{50}$ was calculated by using GraphPad Prism 8 software. The $IC_{50}$ (half inhibitory concentration) of the compound was derived using the following nonlinear fitting formula, and the results are shown in the following table:

$$Y=Bottom + (Top-Bottom)/(1+10^{\wedge}((LogIC_{50}-X)\times HillSlope))$$

X: log value of compound concentration, Y: inhibition rate (% inhibition)

Inhibition rate (% inhibition) = (reading of high-reading control well- reading of compound well) / (reading of high-reading control well- reading of low-reading control well) × 100

Table 18: Inhibitory activity of some compounds of the present disclosure against TMD8 cell proliferation

| Example | TMD8 IC50 (nm) | Example | TMD8 IC50 (nm) |
|---|---|---|---|
| 2-A-P1 | 61.9 | 111-P2 | 275 |
| 34 | 56.9 | 115 | 6.1 |
| 89-P1 | 4.3 | 118 | 23.8 |
| 103 | 25.1 | 125 | 92.4 |
| 106 | 49.5 | ARQ-531 | 74.6 |
| 109-P1 | 12.2 | Tirabrutinib | 158 |
| 111-P1 | 71.9 | LOXO-305 | 184 |

Table 19: Inhibitory activity of some compounds of the present disclosure against DOHH2 cell proliferation

| Example | DOHH2 IC50 (nm) | Example | DOHH2 IC50 (nm) |
|---|---|---|---|
| 111-P1 | 116 | ARQ-531 | 174 |
| 118 | 250 | Tirabrutinib | 8086 |
| 125 | 114 | LOXO-305 | 4990 |

Table 20: Inhibitory activity of some compounds of the present disclosure against BT474 cell proliferation

| Example | BT474 IC50 (nm) | Example | BT474 IC50 (nm) |
|---|---|---|---|
| 111-P1 | 1546 | 144 | 191 |
| 111-P2 | 2182 | 145 | 40.9 |
| 114 | 173 | 146 | 86.9 |
| 125 | 340 | 147 | 377 |
| 129 | 208 | 152 | 65.5 |
| 130 | 89.9 | 153 | 121 |
| 139 | 209 | 155 | 46.4 |
| 140 | 504 | 156 | 269 |
| 142 | 153 | Tucatinib | 30.2 |
| 143 | 467 | Lapatinib | 55.2 |
| 157 | 2269 | 159 | >1000 |
| 158 | 4947 | | |

Table 21: Inhibitory activity of some compounds of the present disclosure against NCI-N87 cell proliferation

| Example | NCI-N87 IC50 (nm) |
|---|---|
| 130 | 100.3 |
| 142 | 106.2 |
| 145 | 37.5 |
| 146 | 82.0 |
| 152 | 43.4 |
| Tucatinib | 26.0 |

**Test example 5: HER2 kinase activity test**

1. Her2 Kinase test steps

1) 1× kinase reaction buffer was prepared from 1 volume of 5× kinase reaction buffer and 4 volumes of water, 1 mM dithiothreitol, 5 mM magnesium chloride, 1 mM manganese chloride and 12.5 mM SEB.

2) 100 nl of the diluted compound working solution was transferred into each well of a reaction plate (784075, Greiner) by an Echo 550 liquid hander. The reaction plate was sealed with a sealing film and centrifuged at 1000 g for 1 min.

3) 1 ng/μL Her2 kinase solution was prepared with 1 × kinase reaction buffer.

4) 5 μL of the kinase solution prepared above was added to each well of the reaction plate. The plate was sealed with a sealing film, centrifuged at 1000 g for 1 min, and placed at room temperature for 10 min.

5) A mixture of 2 × kinase substrate and ATP was prepared by using 1 × kinase reaction buffer, and the 2 × Her2 kinase substrate was 2 μM TK-substrate-biotin and 4 μM ATP

6) 5 μL of the mixture of 2 × TK-substrate-biotin and ATP was added to the reaction plate, centrifuged at 1000g for 30 seconds to start the reaction.

7) Her2 kinase test was performed at room temperature for 50 min of reaction.

8) A mixture of Sa-XL 665 (125 nM) and TK-antibody-Cryptate was prepared by using HTRF detection buffer.

9) 10 μL of the mixture of Sa-XI, 665 and TK-antibody-Cryptate was added to each well, centrifuged at 1000 g for 30 seconds, and reacted at room temperature for 1 h.

10) The fluorescence signals at 615 nm (Cryptate) and 665 nm (XI,665) were read by Envision 2104.

2. Data Analysis

1) The percentage inhibition was calculated as follows:

$$\%\text{inhibition} = 100 - \left[\frac{\text{Ratio}_{\text{compound}} - \overline{\text{Ratio}}_{\text{positive control}}}{\overline{\text{Ratio}}_{\text{negative control}} - \overline{\text{Ratio}}_{\text{positive control}}}\right] \times 100$$

$\overline{\text{Ratio}}_{\text{positive control}}$: average value of Ratio 665/615 nm of all positive control wells in the plate.

$\overline{\text{Ratio}}_{\text{negative control}}$: average value of Ratio 665/615 nm of all negative control wells in the plate.

2) Calculation of IC50 and fitting of dose-effect curve of compound:

The $IC_{50}$ of the compound was derived using the following nonlinear fitting formula with GraphPad 6.0.

$$Y=Bottom + (Top-Bottom)/(1+10^{\wedge}((LogIC50-X)\times HillSlope))$$

X: log value of compound concentration; Y: percentage inhibition of compound

Table 22: Inhibitory activity of some compounds of the present disclosure on HER2 kinase

| Example | HER2 IC50 (nm) |
|---|---|
| 130 | 4.35 |
| 145 | 1.69 |
| 146 | 3.92 |
| 152 | 4.19 |
| 155 | 9.44 |
| Tucatinib | 3.00 |

**Test Example 6: Blood-brain barrier permeability test**

Each test compound was administered orally at a single dose of 10 mg/kg to SD rats for pharmacokinetic study. Each group included 9 rats. The test compound was dissolved in 5% DMSO+10% solutol+85% saline, vortexed for 1-2 min, and ultrasonicated for 5-10 min to prepare into a colorless, transparent and clear administration solution. Animals were fasted overnight before administration. 1 h, 2 h, and 4 h after administration, three SD rats were selected from each group to take about 0.2-0.3 mL of blood through their orbit. The blood sample was placed on ice once collected, and centrifuged to separate the plasma within 15 min (centrifugation conditions: 8000 rpm, 1 min, room temperature). The collected plasma was stored at -20°C before analysis. Immediately after blood collection, the cerebrospinal fluid and brain tissue were collected. The cerebrospinal fluid was draw out by dural puncture with a micro-sampler syringe under direct vision. Namely, about 100 μl of cerebrospinal fluid was collected with a 100 μl micro sample syringe from the rat that was anesthetized by chloral hydrate, with the head-fixed, the back hair-cut off, a transverse incision (2 cm) made at the line connecting the roots of the two ears, and the muscle layer of the neck and skull base bluntly scraped

to expose the foramen magnum. The cerebrospinal fluid was stored at -20°C before analysis. The rat then was sacrificed immediately, with its head cut off. The dissected brain tissue, with the surface capillaries peeled off, was weighed, added with 3 times the amount of cold saline, homogenized by a homogenizer for 1 min, and stored at -20°C before analysis. 20 μL of plasma sample and brain homogenate sample was respectively added into 200 μL of working internal standard solution (the same volume of vehicle was added to the blank instead of internal standard), vortexed for 1 min, and centrifuged at 13500 rpm for 10 min. 100 μL of the supernatant was taken and analyzed by LC-MS/MS. 20 μL of the cerebrospinal fluid was added into 60 μL of working internal standard solution (the same volume of vehicle, instead of internal standard, was added to the blank), vortexed for 1 min, and centrifuged at 13500 rpm for 10 min. 50 μL of the supernatant was taken and analyzed by LC-MS/MS.

Table 23: Test results of blood-brain barrier permeability of some compounds of the present disclosure

| **Example** | Time point | Plasma drug concentration (ng/ml) | Drug concentratio n in brain tissue (ng/g) | Brain tissue/ plasm a (%) | Drug concentratio n in cerebrospinal fluid (ng/ml) | Cerebrospin al fluid/plasma (%) |
|---|---|---|---|---|---|---|
| 111-P1 | 1h | 2013 | 1251 | 62 | 66.2 | 3.3 |
| | 2h | 1584 | 1066 | 67 | 52.3 | 3.3 |
| | 4h | 1343 | 787 | 59 | 41.9 | 3.1 |
| 125 | 1h | 1468 | 1339 | 91 | 34.1 | 2.3 |
| | 2h | 1132 | 1168 | 103 | 30.4 | 2.7 |
| | 4h | 1256 | 1056 | 84 | 29.6 | 2.4 |
| 145 | 1h | 1652 | 643 | 39 | 59.7 | 3.6 |
| | 4h | 1706 | 910 | 53 | 74.3 | 4.4 |
| 146 | 1h | 380 | 296 | 78 | 27.4 | 7.2 |
| | 4h | 373 | 410 | 110 | 34.3 | 9.2 |
| 152 | 1h | 1061 | 288 | 27 | 187 | 18 |
| | 2h | 961 | 378 | 39 | 227 | 24 |
| | 4h | 715 | 232 | 32 | 181 | 25 |
| 155 | 1h | 1252 | 1515 | 121 | 292 | 23 |
| | 2h | 1430 | 1855 | 130 | 374 | 26 |
| | 4h | 1061 | 1260 | 119 | 220 | 21 |
| Tirabrutinib | 1h | 713 | 84.0 | 11.8 | \ | \ |
| | 2h | 515 | 31.1 | 6.0 | \ | \ |
| | 4h | 226 | 13.3 | 5.4 | \ | \ |
| Tucatinib | 1h | 2027 | 26.5 | 1.3 | 19.2 | 0.9 |
| | 2h | 1945 | 29.5 | 1.5 | 10.4 | 0.5 |
| | 4h | 1900 | 24.5 | 1.3 | 14.9 | 0.8 |

**Test Example 7: TMD8 Pharmacodynamic model test**

Human diffuse large B-cell lymphoma TMD8 cells were monolayer-cultured in vitro in RPMI1640 medium with 10% fetal bovine serum, 100 U/mL penicillin and 100 μg/mL streptomycin, in a 37°C, 5% $CO_2$ incubator. Routine digestion with trypsin-EDTA was performed twice a week for passaging. When the cell density was 80%-90% and the number reached the requirement, cells were harvested, counted and inoculated. 0.2 ml ($1\times10^7$ cells) of TMD8 cells (added with matrigel at a volume ratio of 1:1) were subcutaneously inoculated on the right back of each mouse. The mice were administered in groups when the average tumor volume reached about 137 $mm^3$. Tumor diameters were measured twice a week with vernier calipers. The tumor volume was calculated by the formula $V = 0.5\ a \times b^2$, where a and b

represent the long and short diameters of the tumor, respectively.

The results are shown in FIGs. 1 and 2. According to FIG. 1, for the drug efficacy model based on the TMD8 mouse subcutaneous xenograft tumor, the two compounds of Example 118 and Example 89-P1 had a significantly better inhibitory effect against the tumor than the clinical phase II drug ARQ-531 and the marketed drug ibrutinib at the same dose of 10 mg/kg. According to FIG. 2, for the drug efficacy model based on TMD8 mouse subcutaneous xenograft tumor, the two compounds of Example 111-P1 and Example 125 had a significantly better inhibitory effect against the tumor than Tirabrutinib at the same dose of 20 mg/kg. The compound of Example 111-P1, particularly, had a TGI of 93% in terms of tumor inhibition rate, which was nearly 2 times that of Tirabrutinib, almost completely controlling the growth of the tumor, with a considerably advantage of drug efficacy.

Test Example 8: DOHH-2-Luc intracerebral tumor drug efficacy model test

1. Cell Culture

DOHH-2-luc tumor cells were cultured in vitro with RPMI 1640 medium containing 10% fetal bovine serum and 500 ng/mL puromycin in a 37°C, 5% $CO_2$ incubator. Medium was supplemented or replaced every 2 to 3 days, and the number of passages did not exceed 4-5 times. Tumor cells in logarithmic growth phase were used for inoculation of tumors in vivo.

2. Inoculation of tumor cell and grouping

After the animal was anesthetized by intramuscular injection of Zoletil, it was fixed on the operating table in a prone position. The skin on the top of the head was disinfected with iodine and 75% alcohol respectively, and the skin was cut about 0.5 cm along the midline of the head to expose the coronal and sagittal lines. Being located about 0.5-1.0 mm above the coronal line and about 2 mm to the right of the sagittal line by using a brain locator, a hole was drilled with a 1 mL syringe needle. The micro-injector was inserted vertically to a depth of 3 mm at the location, slowly (about 1 min) injected with $3 \times 10^5$ DOHH-2-luc tumor cells /2 $\mu$L suspension and kept for 1 min. After pulling out the needle, the needle hole was quickly sealed with bone wax, and the wound was sutured with a stapler. About the 7th day after tumor inoculation, the animals were randomly divided into 5 groups according to the body weight of the animals and the optical signal intensity of the tumor site, with 5 animals in each group.

3. Image Analysis

The mice were imaged 1-2 times a week according to their state using the small animal in vivo imaging system IVIS Lumina III (Perkin Elmer). The bioluminescence imaging (BLI, unit: photons/s) signal intensity at the tumor cell inoculation site of mouse was monitored as the main indicator for evaluating tumor growth and drug efficacy. The specific operation is as follows:

The mice were intraperitoneally injected with D-luciferin (15 mg/mL, 5 $\mu$L/g according to the body weight of the experimental animal), and then inhaled anesthetized with 1%-2% isoflurane. 10 min after the injection of D-luciferin, the animals were imaged with IVIS Lumina III. The data were analyzed and processed using Living Image software (Perkin Elmer), and the optical signal intensity in ROI (regions of interest) of each animal was calculated.

The results are shown in FIGs. 3 and 4. According to FIG. 3, in the study of the DOHH2 tumor model in the mouse brain, the compounds of Example 111-P1 and Example 125 had a significantly better inhibitory effect against the tumor than Tirabrutinib at the same dose of 30 mg/kg (BID), indicating a considerable advantage of drug efficacy. In addition, no side effects had been found after 21 days of administration.

FIG. 4 shows the fluorescence image of all the tested animals after being imaged, indicating the tumor size in the brain by color and area size, and the redder the color, the larger the tumor. It can be seen from the picture that under the same dose, the compounds of Example 111-P1 and Example 125 had a very good inhibitory effect against the tumor, with almost no red area, indicating very small brain tumors of these two groups of animals. While all the animals in the model group and Tirabrutinib group had large red areas, indicating that the tumors were large.

It can be seen from the above examples that the compounds of the present disclosure as a BTK protein kinase inhibitor have a structure represented by formula I, preferably a structure represented by formula II; and that the compounds have a strong inhibitory effect against both wild-type BTK and mutant BTK (C 481S), with good pharmacokinetic properties, and thus can be used to prepare medicines for treating diseases caused by overexpression of BTK kinase. Some of these compounds are significantly better than the marketed BTK inhibitors Ibrutinib, Tirabrutinib and the clinical phase II drug ARQ-531 in TMD8 subcutaneous tumor efficacy model experiments.

Some of the compounds of the present disclosure are significantly superior over the marketed drugs Tirabrutinib and Tucatinib in terms of blood-brain barrier permeability, liver microsome metabolic stability, pharmacokinetics and the

like. In the DOHH-2-Luc intracerebral drug efficacy model, some compounds have very good drug efficacy, which also verified by brain-permeable data. Therefore, the compounds of the present disclosure can be used to prepare medicines for treating diseases caused by overexpression of BTK or HER2 kinase, especially brain diseases.

The above-mentioned compound or the stereoisomer, solvate, hydrate, pharmaceutically acceptable salt or cocrystal thereof can be used to prepare medicines for the treatment of a disease selected from the group consisting of an autoimmune disease, inflammatory disease, thromboembolic disease, hypersensitivity, infectious disease, proliferative disorder, a cancer and a combination thereof, and is expected to provide new good treatment options.

The description herein relates to only preferred embodiments of the present disclosure, and it should be noted that for those skilled in the art, various modifications to these embodiments without departing from the technical principle of the present disclosure are possible and should also fall into the protection scope of the present invention.

## Claims

1. A compound as a BTK inhibitor, having a structure represented by formula I:

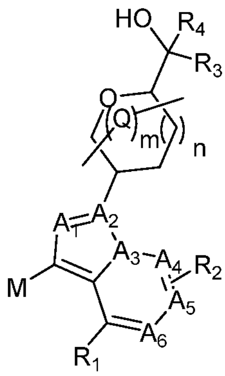

Formula I

wherein $A_1$, $A_2$, $A_3$, $A_4$, $A_5$ and $A_6$ are each independently selected from the group consisting of C-$R_5$ and N, and at least one of $A_1$, $A_2$, $A_3$, $A_4$, $A_5$ and $A_6$ is N;
M is selected from the group consisting of substituted or unsubstituted saturated hydrocarbyl or heterosaturated hydrocarbyl, substituted or unsubstituted unsaturated cyclyl or heterocyclyl, and substituted or unsubstituted monocyclic, bicyclic or tricyclic aryl or heteroaryl; wherein the substituent is each independently selected from the group consisting of aryl or heteroaryl, alkyl or heteroalkyl, cycloalkyl or heterocycloalkyl, unsaturated cyclyl or heterocyclyl, phenoxy, halogen, hydroxyl, cyano, amino, an ester group, nitro, mercapto, amido, sulfonyl, phosphoryl, alkyl phosphoryl, alkyl sulfone and alkyl sulfoxide that are substituted by any group; further, the substituent is aryl or heteroaryl substituted by any group, more preferably phenyl substituted by any group;
Q is selected from the group consisting of C-$R_{10}R_{11}$, N-$R_{12}$, O, S, S(O) and $S(O)_2$;
$R_1$, $R_2$, $R_3$, $R_4$, $R_5$, $R_{10}$, $R_{11}$ and $R_{12}$ are each independently selected from the group consisting of hydrogen, deuterium, halogen, substituted or unsubstituted alkyl or heteroalkyl, substituted or unsubstituted cycloalkyl or heterocycloalkyl, substituted or unsubstituted unsaturated cyclyl or heterocyclyl, substituted or unsubstituted aryl or heteroaryl, hydroxyl, cyano, amino, an ester group, nitro, mercapto, amido, sulfonyl, phosphoryl, alkyl phosphoryl, alkyl sulfone and alkyl sulfoxide; or $R_3$, $R_4$ and the carbon atom connecting therewith together form substituted or unsubstituted C3-C10 cycloalkyl or heterocycloalkyl; wherein the substituent is selected from the group consisting of halogen, hydroxyl, cyano, amino, mercapto, nitro, carboxyl, hydroxylamino, alkyl, cycloalkyl, heteroalkyl, heterocycloalkyl, aryl, heteroaryl, an ester group, acyl, amido, sulfonyl and phosphoryl;
m is an integer selected from 0 to 6; and
n is an integer selected from 0 to 3;
or a tautomer, a mesomer, a racemate, an enantiomer, a diastereomer or a mixture thereof, or a pharmaceutically acceptable hydrate, solvate or salt thereof.

2. The compound according to claim 1, having a structure represented by formula II,

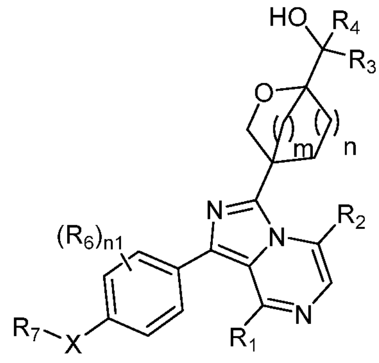

Formula II

wherein $R_1$ is selected from the group consisting of hydrogen, halogen, hydroxyl, cyano, amino, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; further, $R_1$ is selected from the group consisting of hydrogen, amino, methyl, ethyl, methoxy, cyano, trifluoromethyl, isopropyl and cyclopropyl; further, $R_1$ is selected from the group consisting of hydrogen, amino and methyl;

$R_2$ is selected from the group consisting of hydrogen, halogen, hydroxyl, cyano, amino, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; further, $R_2$ is selected from the group consisting of hydrogen, fluorine, chlorine, bromine, methyl, ethyl, methoxy, cyano, trifluoromethyl, isopropyl and cyclopropyl; further, $R_2$ is selected from the group consisting of hydrogen, chlorine and methyl;

$R_3$ and $R_4$ are selected from the group consisting of hydrogen, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; or $R_3$, $R_4$ and the carbon atom connecting therewith together form substituted or unsubstituted C3-C6 cycloalkyl or heterocycloalkyl containing N or O atom; further, $R_3$ and $R_4$ are selected from the group consisting of hydrogen, methyl, ethyl, isopropyl and cyclopropyl, or $R_3$, $R_4$ and the carbon atom connecting therewith together form cyclopropyl, azetidinyl, azacyclopentyl, azacyclohexyl, oxetanyl, oxacyclopentyl, or oxacyclohexyl;

$R_6$ is selected from the group consisting of hydrogen, halogen, hydroxyl, cyano, amino, substituted or unsubstituted C1-C6 alkyl, substituted or unsubstituted C3-C6 cycloalkyl, substituted or unsubstituted C1-C6 heteroalkyl, and substituted or unsubstituted C3-C6 heterocycloalkyl; further, $R_6$ is selected from the group consisting of hydrogen, halogen, cyano, substituted or unsubstituted C1-C3 alkyl, and substituted or unsubstituted C1-C3 alkoxy; further, $R_6$ is selected from the group consisting of hydrogen, fluorine, chlorine, bromine, trifluoromethyl, methyl, methoxy, trifluoromethoxy and difluoromethoxy; further, $R_6$ is hydrogen or fluorine;

m is selected from 0, 1, 2 or 3;

n is selected from 0, 1 or 2;

n1 is selected from 0, 1, 2, 3 or 4;

$R_7$ is selected from the group consisting of substituted or unsubstituted aryl, or substituted or unsubstituted pyridyl, wherein the substituent is independently selected from halogen, hydroxyl, amino, cyano, alkyl, heteroalkyl, cycloalkyl and heterocycloalkyl; further, the substituent is independently selected from the group consisting of fluorine, chlorine, bromine, cyano, amino, C1-C3 alkyl, C1-C3 alkoxy, C3-C6 cycloalkyl and C3-C6 heterocycloalkyl; further, the substituent is independently selected from the group consisting of fluorine, chlorine, bromine, cyano, trifluoromethyl, trifluoromethoxy, difluoromethoxy, methoxy, deuterated methoxy, cyclopropyl, cyclopropylmethoxy, ethyl, isopropyl and isobutyl; wherein the number of the substituent is an integer between 0 and 5;

X is selected from the group consisting of

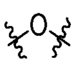, 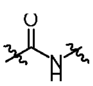, 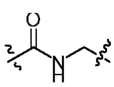, 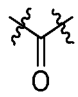, 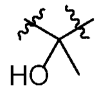,

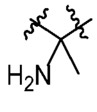, 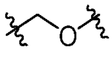, 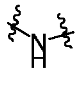, 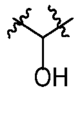, 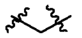 and 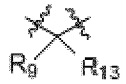,

wherein $R_9$ and $R_{13}$ are independently selected from the group consisting of hydrogen, halogen other than F, hydroxyl, amino, cyano, C1-C3 alkyl, C1-C3 alkoxyl, C3-C6 cycloalkyl and C3-C6 heterocycloalkyl, or $R_9$, $R_{13}$ and the carbon atom connecting therewith together form substituted or unsubstituted C3-C6 cycloalkyl, or substituted or unsubstituted C3-C6 heterocycloalkyl containing N or O; further, $R_9$ and $R_{13}$ are independently selected from the group consisting of hydrogen, chlorine, cyano, methyl, ethyl, isopropyl, cyclopropyl and iso-butyl, or $R_9$, $R_{13}$ and the carbon atom connecting therewith together form cyclopropyl; further, $R_9$ and $R_{13}$ are selected from the group consisting of hydrogen, deuterium, chlorine, methyl, hydroxyl and amino;
or a tautomer, a mesomer, a racemate, an enantiomer, a diastereomer or a mixture thereof, or a pharmaceutically acceptable hydrate, solvate or salt thereof.

**3.** The compound according to claim 2, having a structure represented by formula III or formula IV

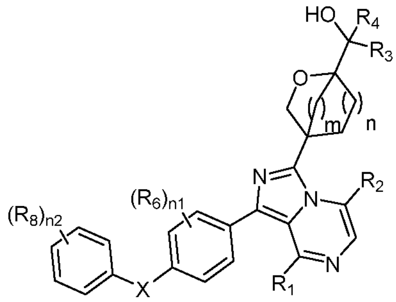
Formula III

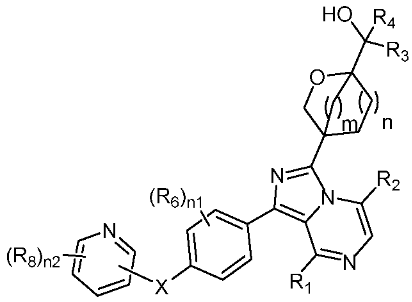
Formula IV

wherein n2 is selected from 0, 1, 2, 3 or 4;
$R_8$ is independently selected from the group consisting of hydrogen, halogen, hydroxyl, amino, cyano, alkyl, heteroalkyl, cycloalkyl and heterocycloalkyl; further, $R_8$ is independently selected from the group consisting of hydrogen, fluorine, chlorine, bromine, cyano, amino, C1-C3 alkyl, C1-C3 alkoxy, C3-C6 cycloalkyl and C3-C6 heterocycloalkyl; further, $R_8$ is independently selected from the group consisting of hydrogen, fluorine, chlorine, bromine, cyano, trifluoromethyl, trifluoromethoxy, difluoromethoxy, methoxy, deuterated methoxy, cyclopropyl, cyclopropylmethoxy, ethyl, isopropyl and isobutyl; wherein the number of the substituent is an integer between 0 and 5,
or a tautomer, a mesomer, a racemate, an enantiomer, a diastereomer or a mixture thereof, or a pharmaceutically acceptable hydrate, solvate or salt thereof.

**4.** The compound according to claim 1, wherein the compound has a structure selected from the group consisting of:

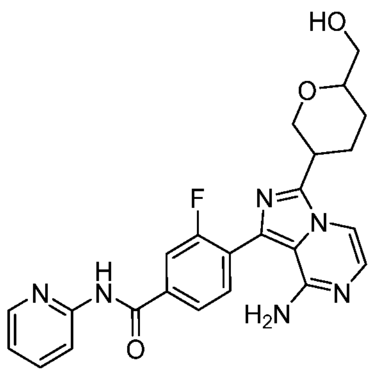
1,

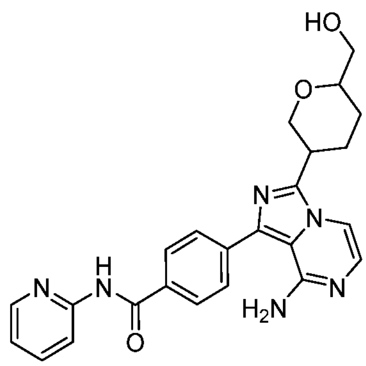
2,

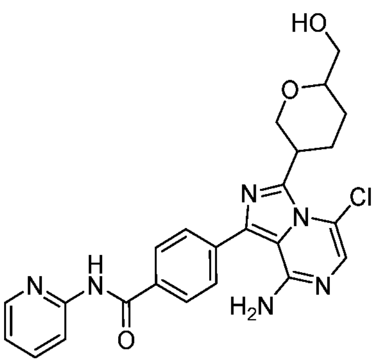
3,

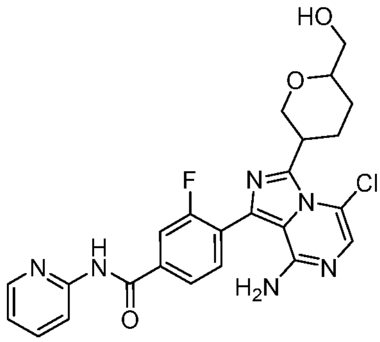 4, 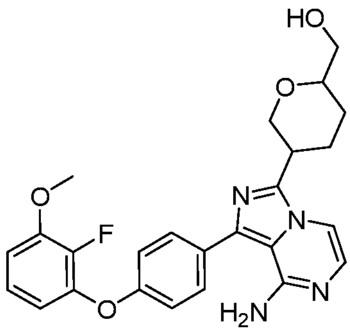 5, 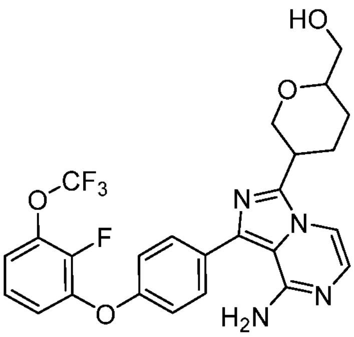 6,

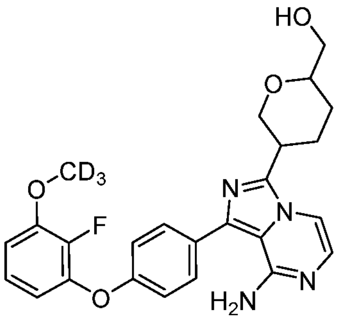 7, 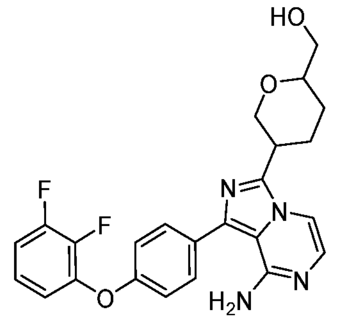 8, 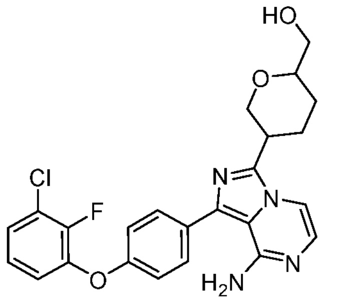 9,

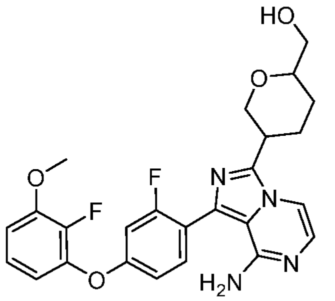 10, 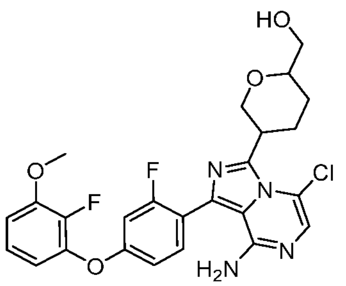 11, 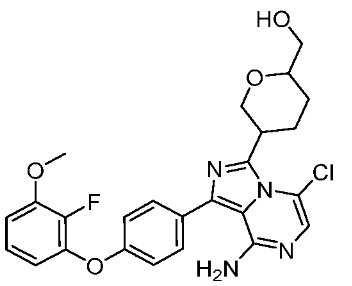 12,

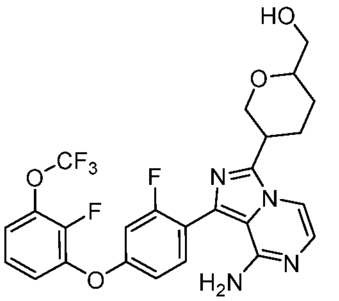 13, 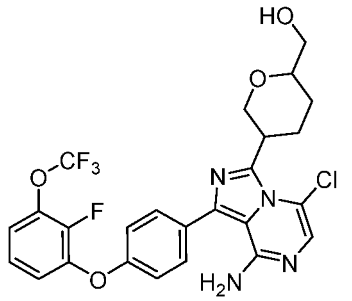 14, 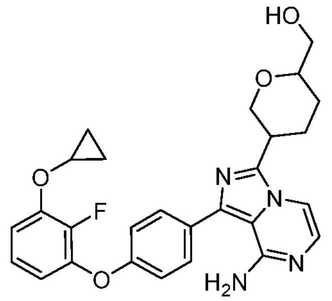 15,

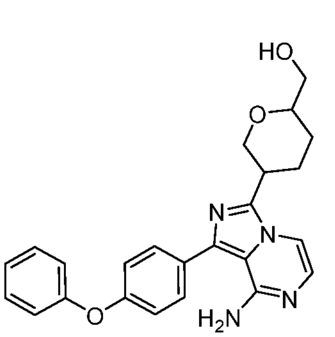 16, 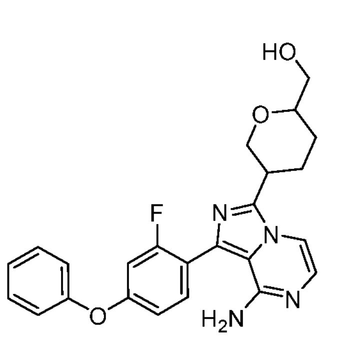 17, 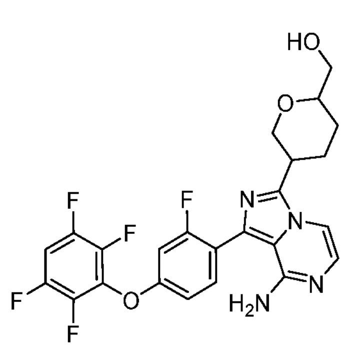 18,

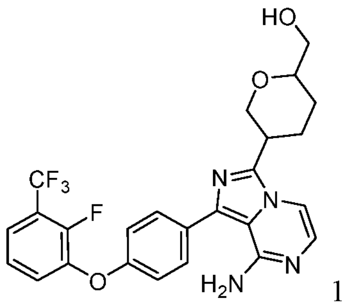 19, 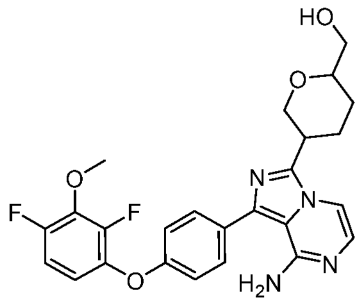 20, 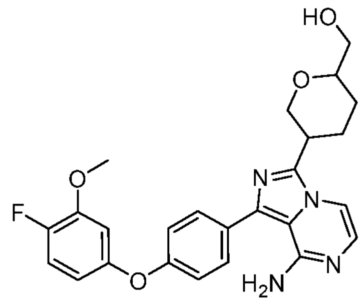 21,

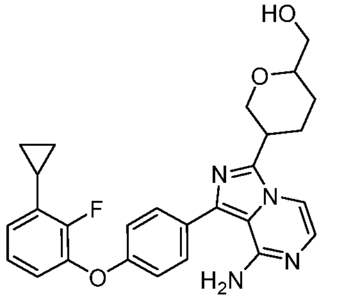 22, 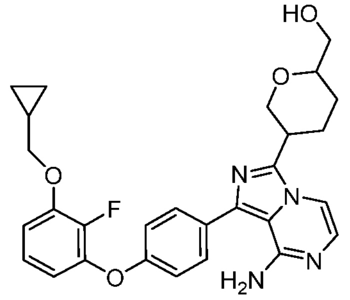 23, 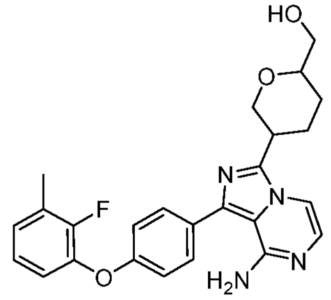 24,

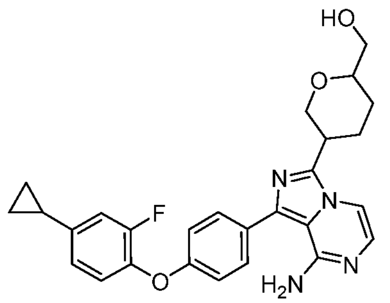 25, 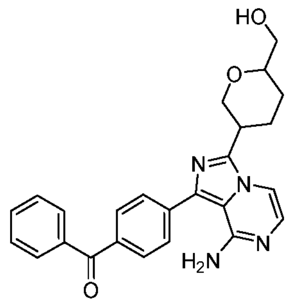 26, 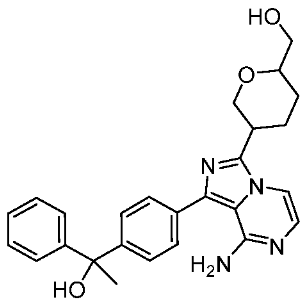 27,

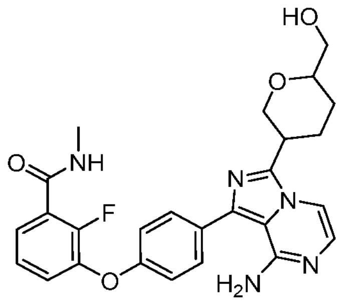 28, 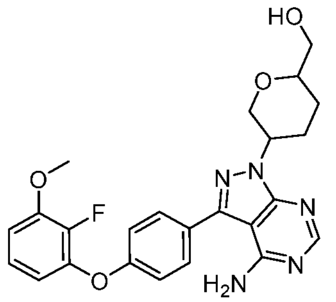 29, 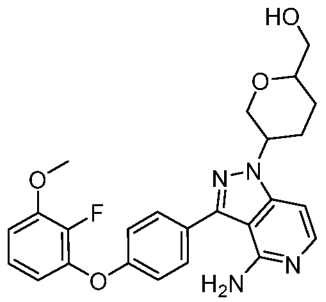 30,

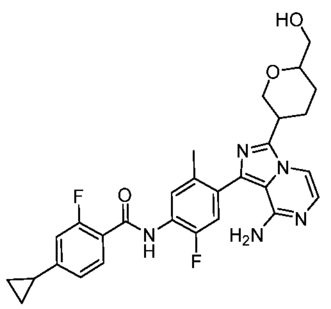 31, 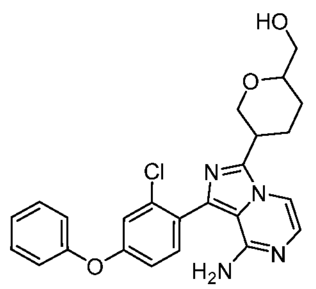 32, 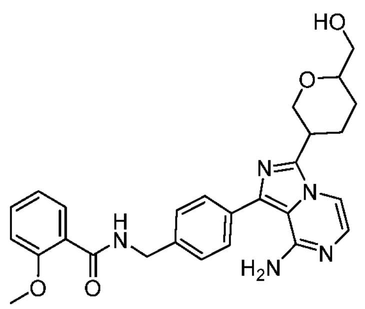 33,

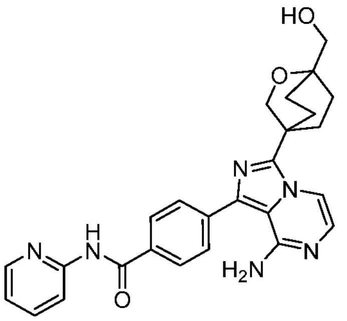 34,

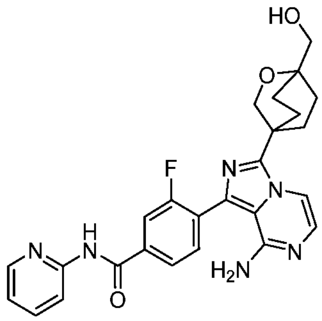 35,

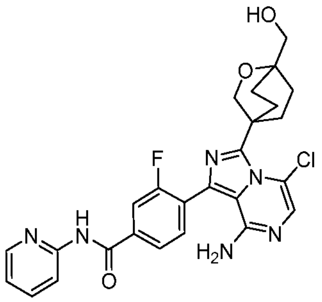 36,

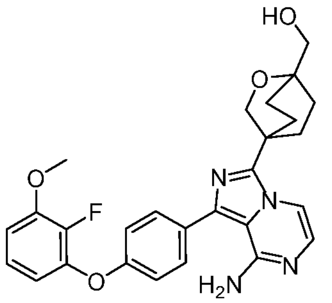 37,

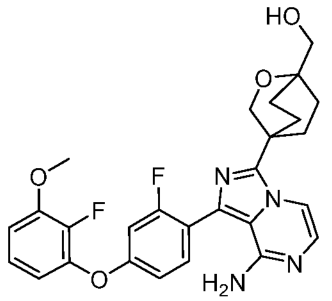 38,

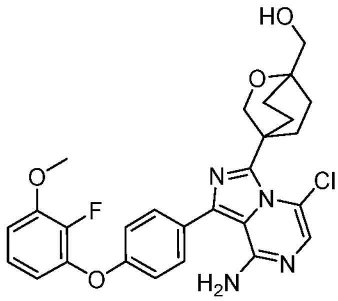 39,

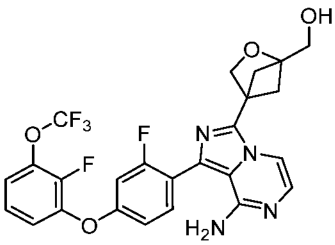 40,

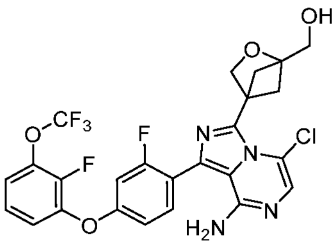 41,

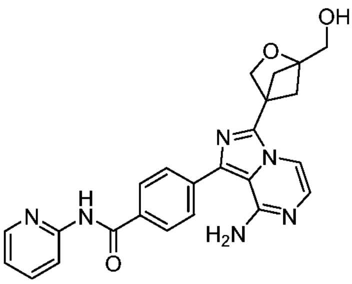 42,

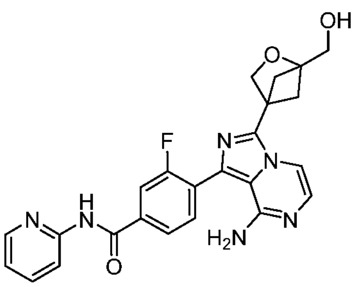 43,

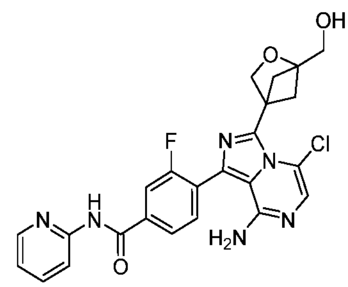 44,

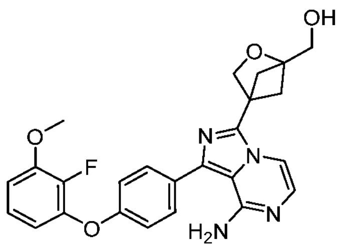 45,

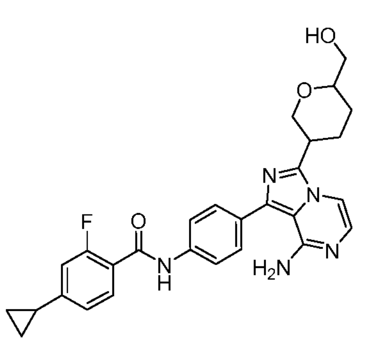 46,

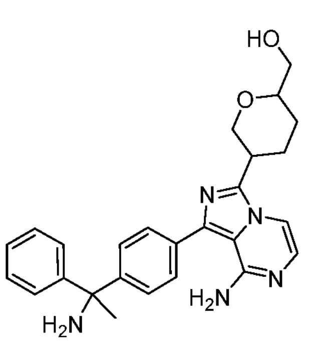 47,

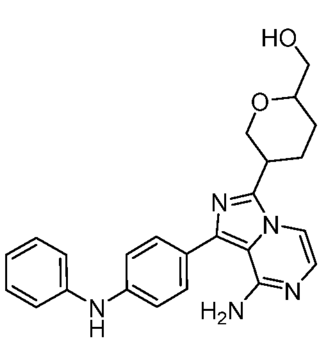 48,

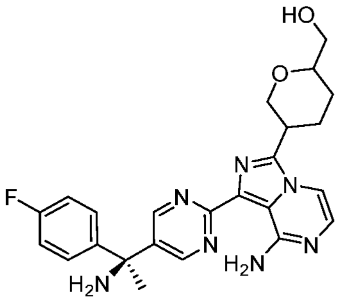 49, 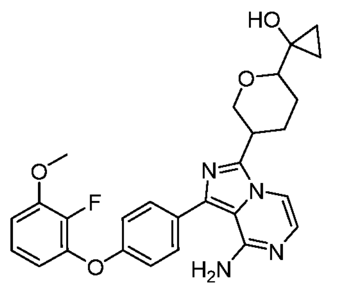 50, 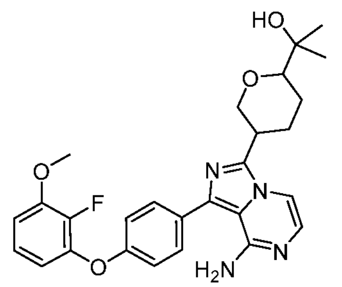 51,

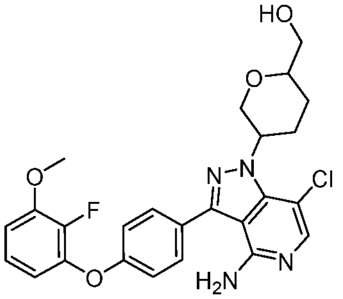 52, 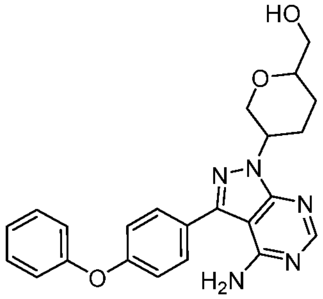 53, 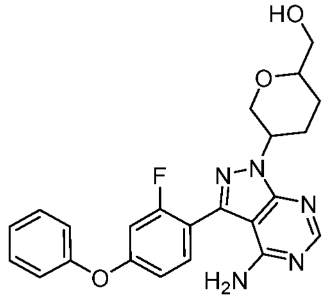 54,

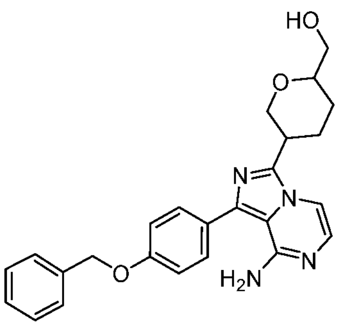 55, 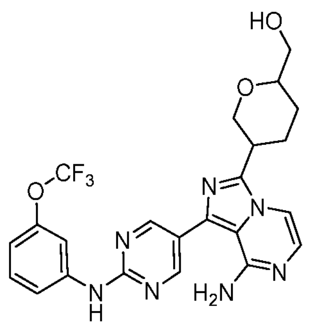 56, 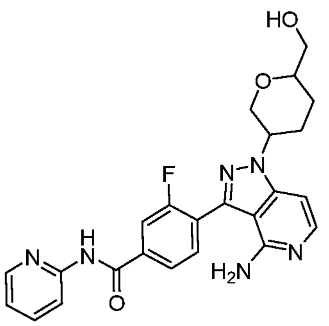 57,

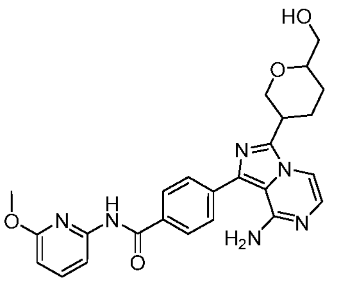 58, 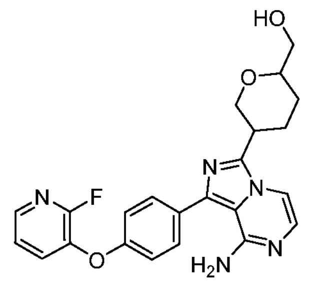 59, 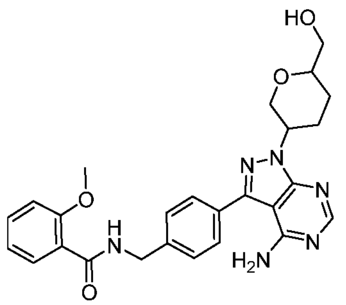 60,

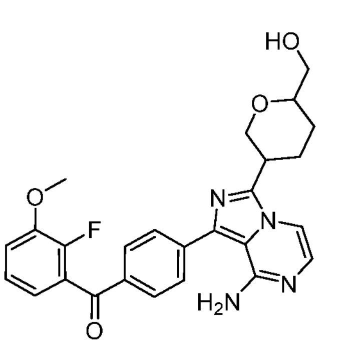 61, 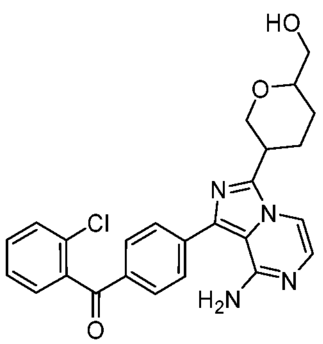 62, 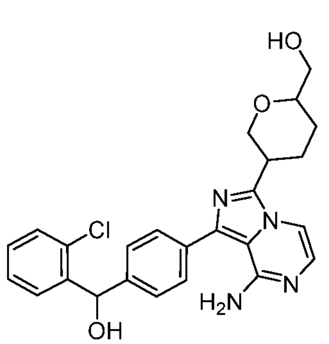 63,

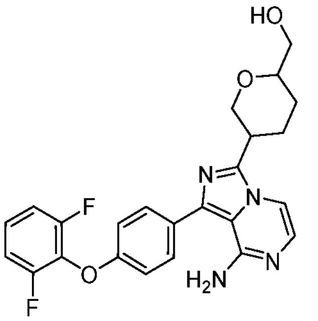 64, 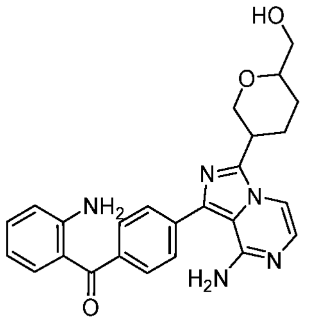 65, 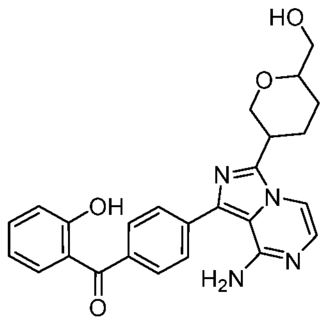 66,

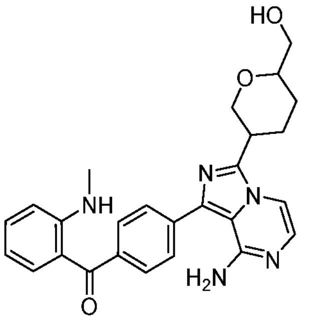 67, 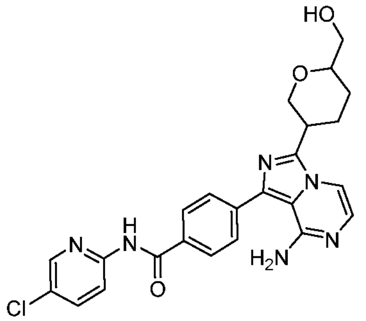 68, 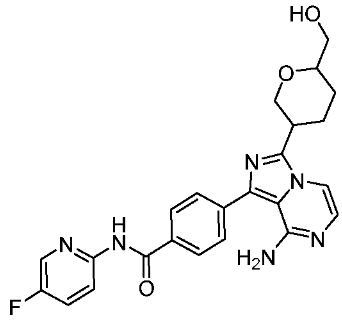 69,

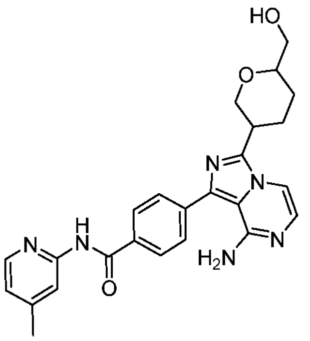 70, 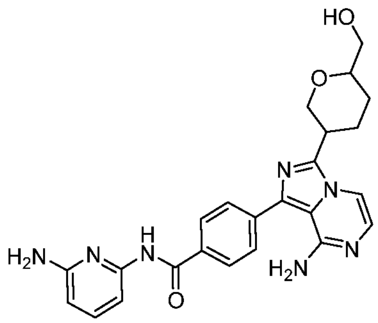 71, 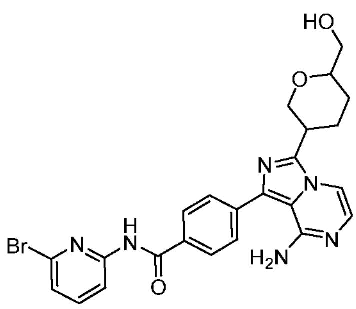 72,

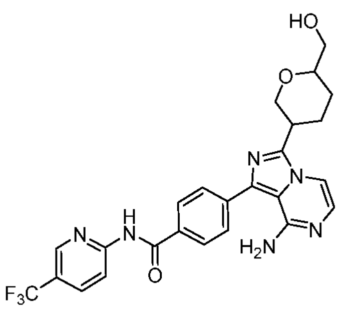 73, 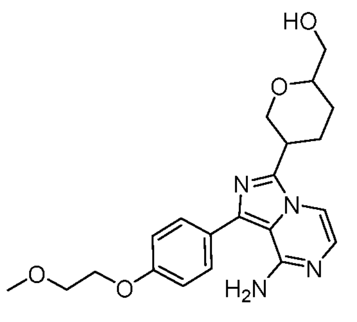 74, 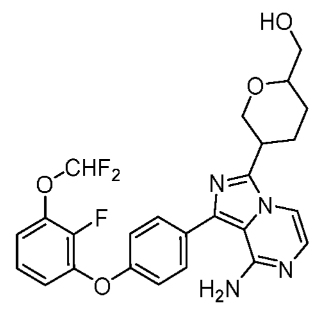 75,

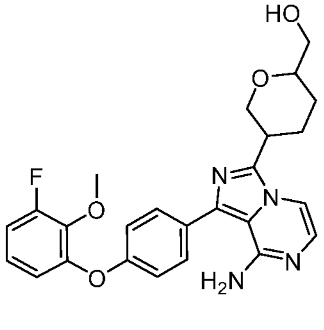 76, 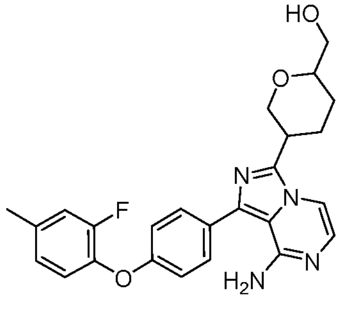 77, 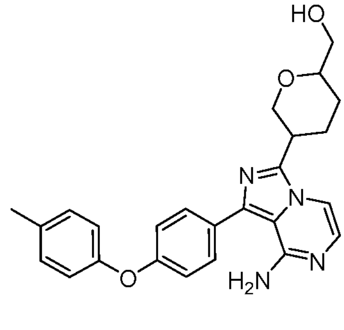 78,

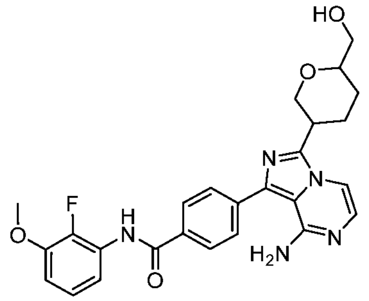 79, 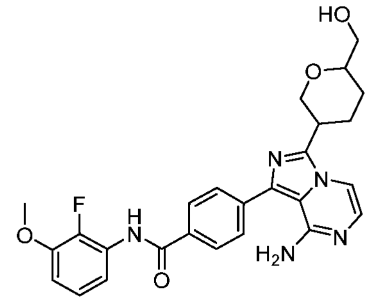 80, 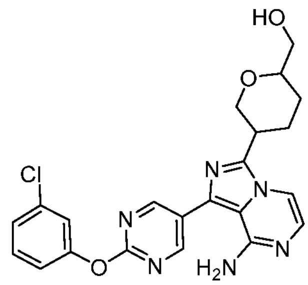 81,

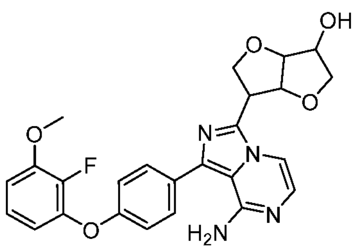 82, 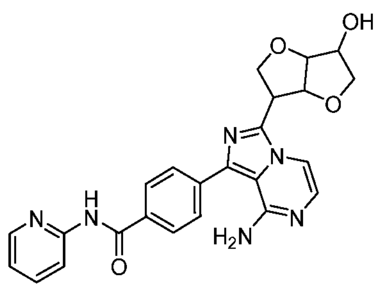 83, 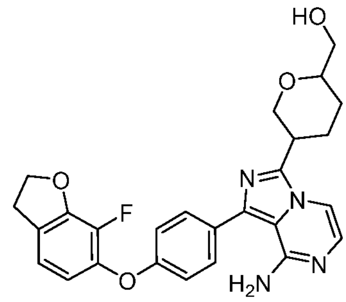

84, 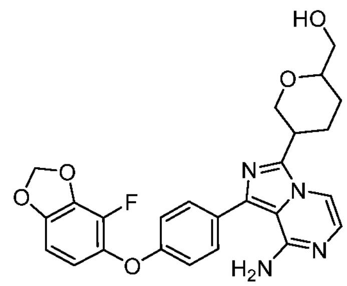 85, 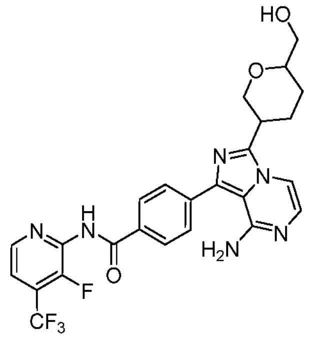 86, 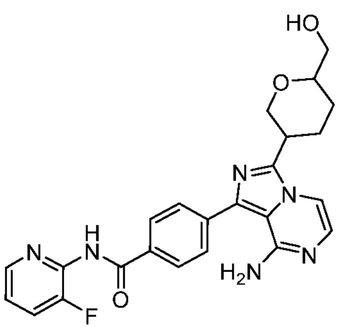 87,

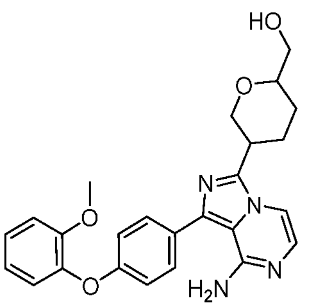 88, 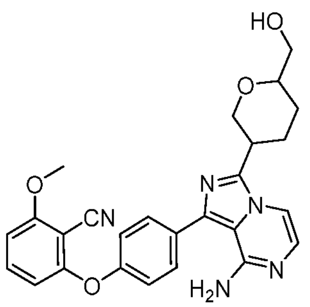 89, 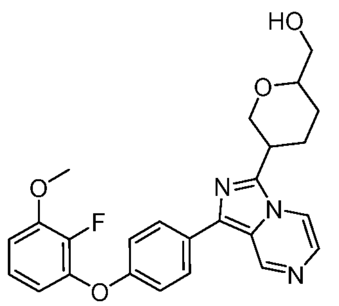 90,

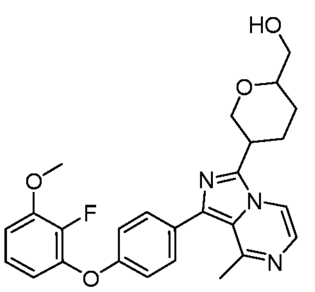 91, 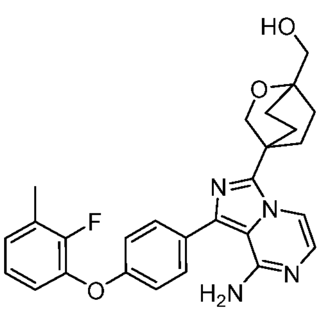 92, 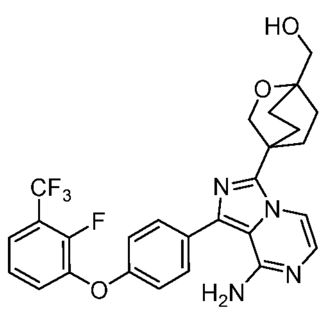 93,

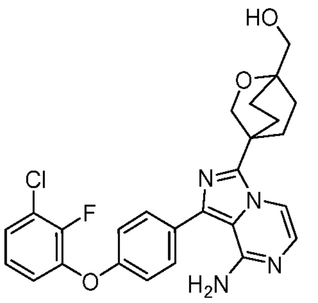 94, 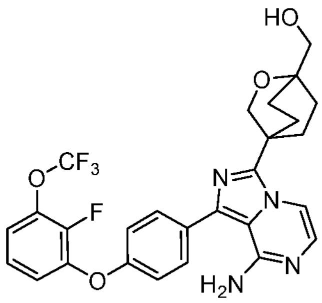 95, 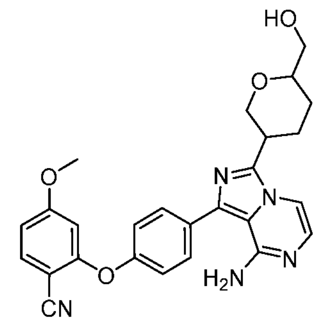 96,

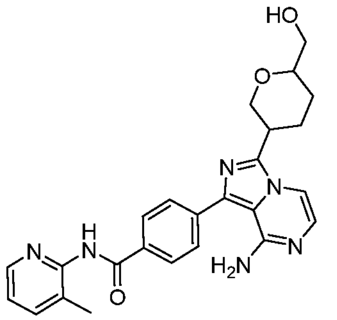 97, 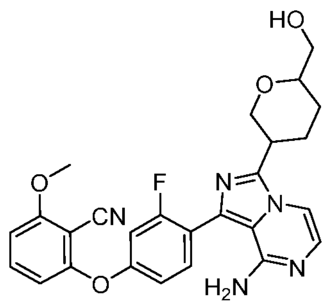 98, 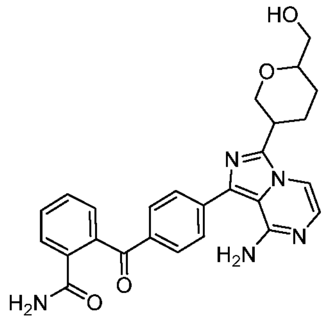 99,

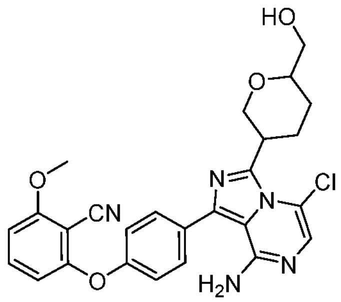 100, 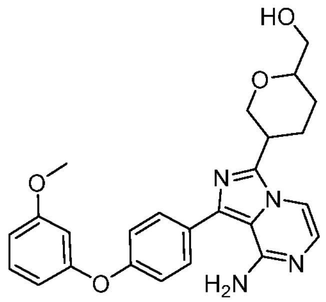 101, 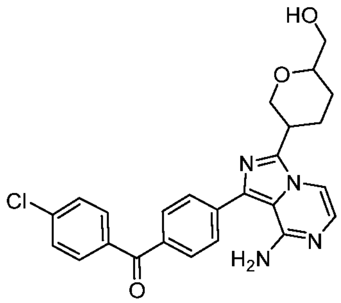 102,

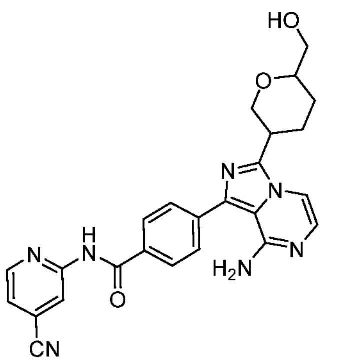 103, 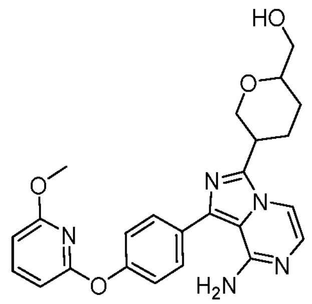 104, 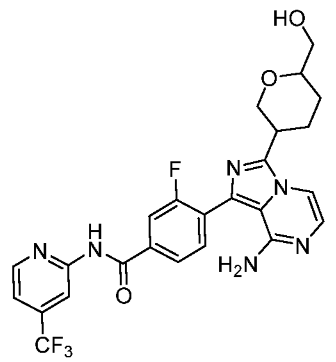 105,

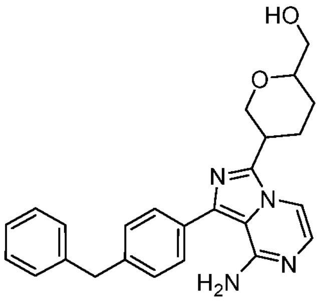 106, 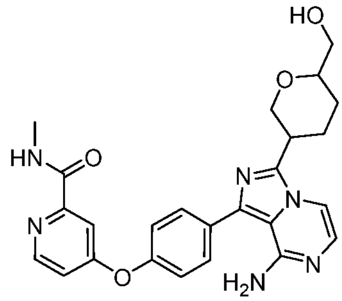 107, 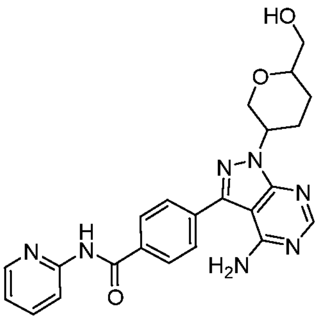 108,

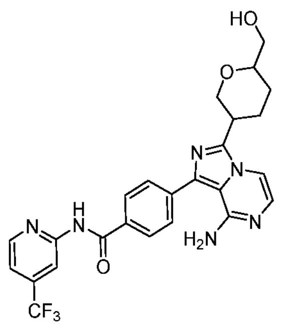 109, 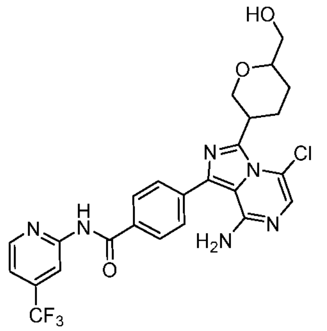 110, 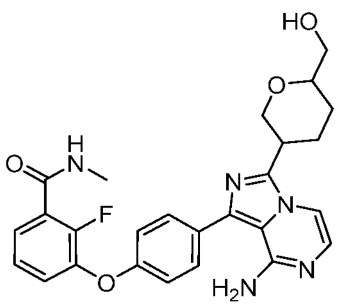 112,

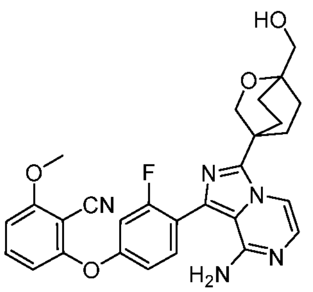 115, 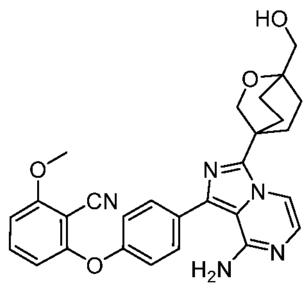 116, 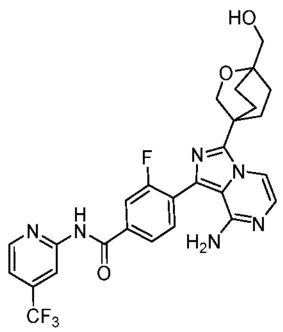 117,

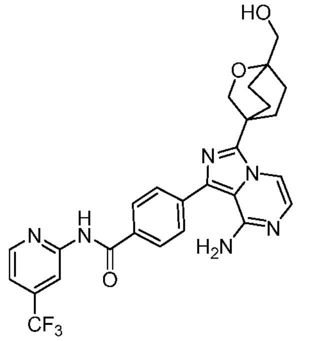 118, 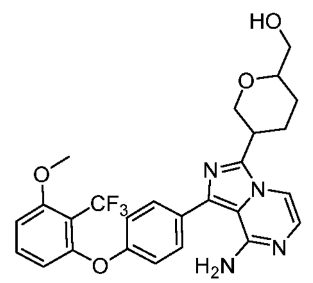 119, 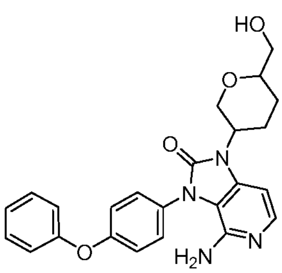 120,

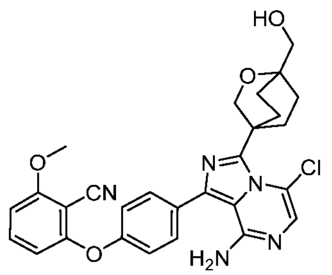 122, 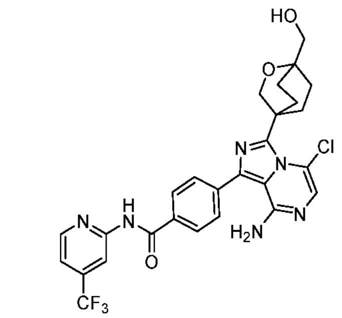 128, 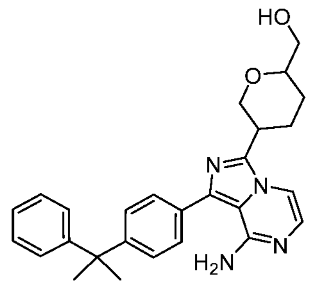 131,

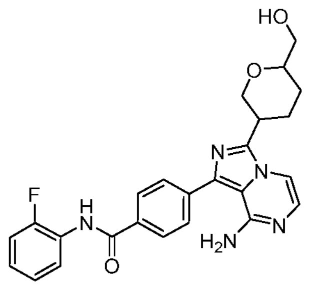 133, 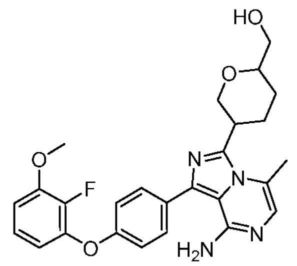 134, 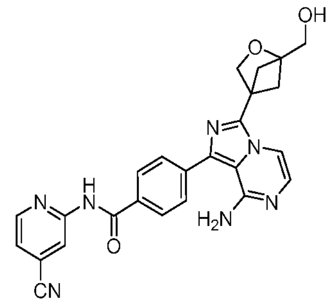 135,

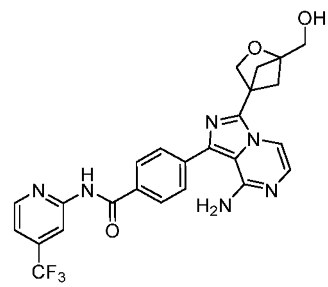 136, and 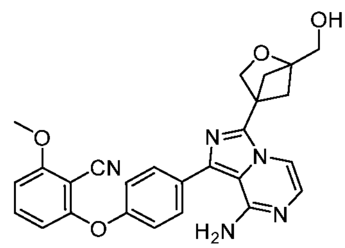 137.

**5.** A method for preparing the compound according to claim 2, comprising steps of:

reacting boronic acid or a borate compound represented by formula A with a bromide represented by formula B in a manner of a Suzuki reaction, to obtain an intermediate represented by formula C;
performing deprotection of the intermediate represented by formula C to obtain the compound represented by formula II;

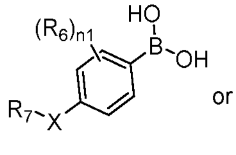 or 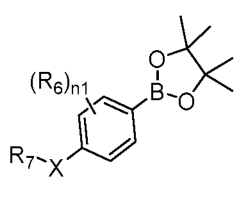

**A** ,

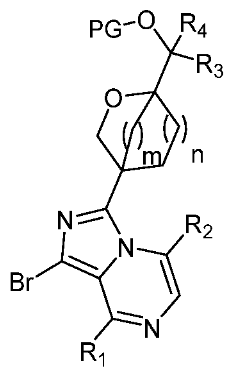

**B** ,

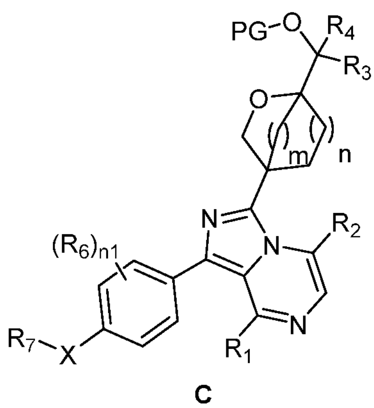

**C** .

**6.** A pharmaceutical composition, comprising an active ingredient selected from the group consisting of the compound or the stereoisomer, solvate, hydrate, pharmaceutically acceptable salt or cocrystal thereof according to any one of

claims 1-4, and a combination thereof.

**7.** Use of the compound or the stereoisomer, solvate, hydrate, pharmaceutically acceptable salt or cocrystal thereof according to any one of claims 1-4 in the manufacture of a protein kinase inhibitor; further, the kinase inhibitor is a BTK inhibitor.

**8.** Use of the compound or the stereoisomer, solvate, hydrate, pharmaceutically acceptable salt or cocrystal thereof according to any one of claims 1-4 in the manufacture of a medicament for the treatment of a diseases caused by overexpression of BTK kinase.

**9.** Use of the compound or the stereoisomer, solvate, hydrate, pharmaceutically acceptable salt or cocrystal thereof according to any one of claims 1-4 in the manufacture of a medicament for the treatment of a disease selected from the group consisting of an autoimmune disease, inflammatory disease, thromboembolic disease, hypersensitivity, infectious disease, proliferative disorder, a cancer and a combination thereof.

**10.** The use according to claim 9, wherein the disease is selected from the group consisting of arthritis, rheumatoid arthritis, urticaria, vitiligo, organ transplant rejection, ulcerative colitis, Crohn's disease, dermatitis, asthma, Sjögren's syndrome, systemic lupus erythematosus, multiple sclerosis, idiopathic thrombocytopenic purpura, rash, antineutrophil cytoplasmic antibody-associated vasculitis, pemphigus, pemphigus vulgaris, chronic obstructive pulmonary disease, psoriasis, breast cancer, mantle cell lymphoma, ovarian cancer, esophageal cancer, laryngeal cancer, glioblastoma, neuroblastoma, gastric cancer, hepatocellular carcinoma, gastric cancer, glioma, endometrial carcinoma, melanoma, kidney cancer, bladder cancer, melanoma, bladder cancer, biliary tract cancer, renal carcinoma, pancreatic cancer, lymphoma, hairy cell leukemia, nasopharyngeal cancer, pharyngeal cancer, colorectal cancer, rectal cancer, cancer of brain and central nervous system, cervical cancer, prostate cancer, testicular cancer, genitourinary tract cancer, lung cancer, non-small cell lung cancer, small cell cancer, lung adenocarcinoma, bone cancer, colon cancer, adenoma, pancreatic cancer, adenocarcinoma, thyroid cancer, follicular carcinoma, Hodgkin's leukemia, bronchial carcinoma, thyroid carcinoma, corpus carcinoma, cervical carcinoma, multiple myeloma, acute myeloid leukemia, chronic myeloid leukemia, lymphocytic leukemia, chronic lymphoblastic leukemia, myelogenous leukemia, non-Hodgkin's lymphoma and primary macroglobulinemia.

**11.** An intermediate for preparing the compound as the BTK inhibitor according to claim 2, having a structure represented by:

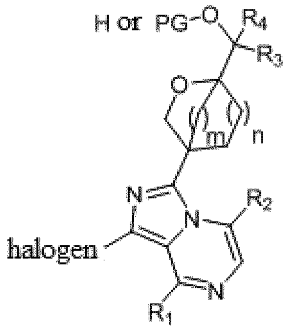

Formula B';

for example:

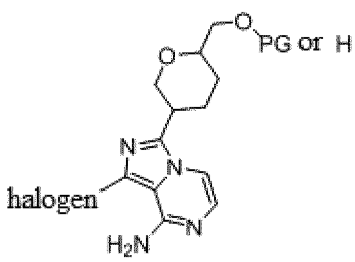

or

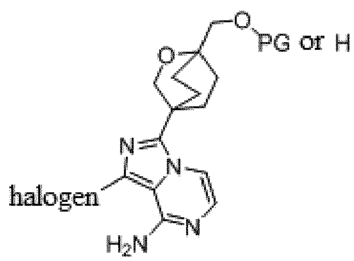

.

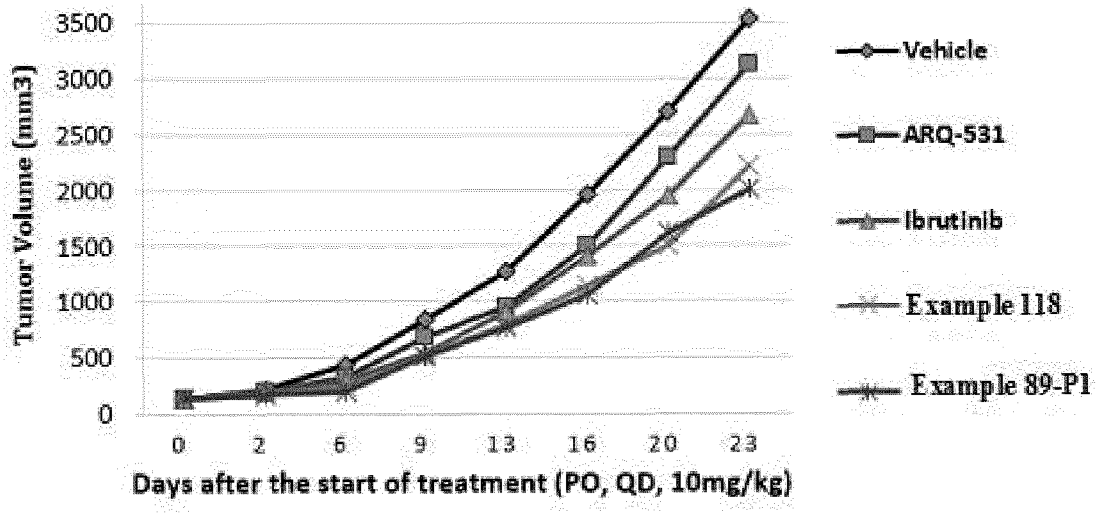

Tumor Volume (mm3)
3500
3000
2500
2000
1500
1000
500
0
0
2
6
9
13
16
20
23
Days after the start of treatment (PO, QD, 10mg/kg)
Vehicle
ARQ-531
Ibrutinib
Example 118
Example 89-P1


FIG. 1

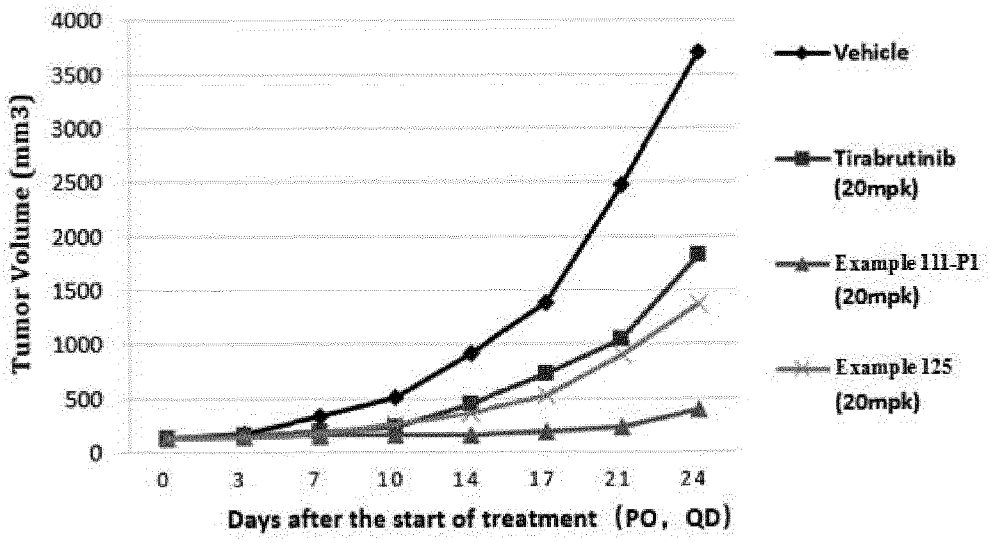

Tumor Volume (mm3)
4000
3500
3000
2500
2000
1500
1000
500
0
0
3
7
10
14
17
21
24
Days after the start of treatment (PO, QD)
Vehicle
Tirabrutinib (20mpk)
Example 111-P1 (20mpk)
Example 125 (20mpk)


FIG. 2

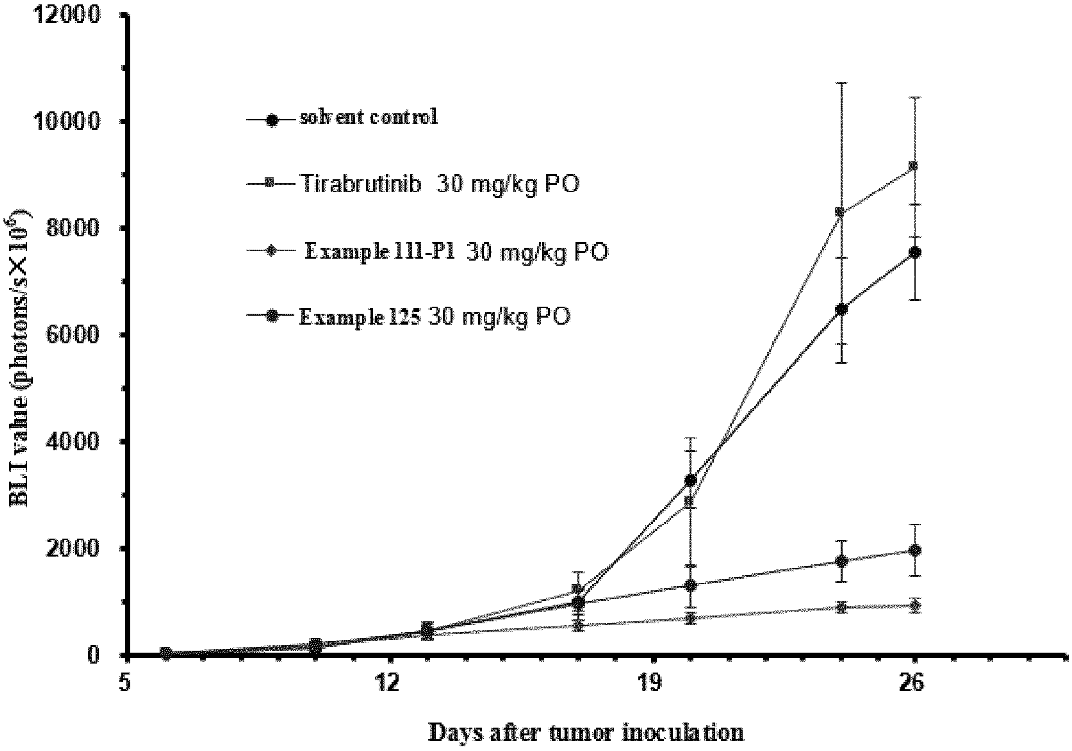
12000
10000
8000
6000
4000
2000
0
5
12
19
26
BLI value (photons/s×10⁶)
Days after tumor inoculation
solvent control
Tirabrutinib 30 mg/kg PO
Example 111-P1 30 mg/kg PO
Example 125 30 mg/kg PO

FIG. 3

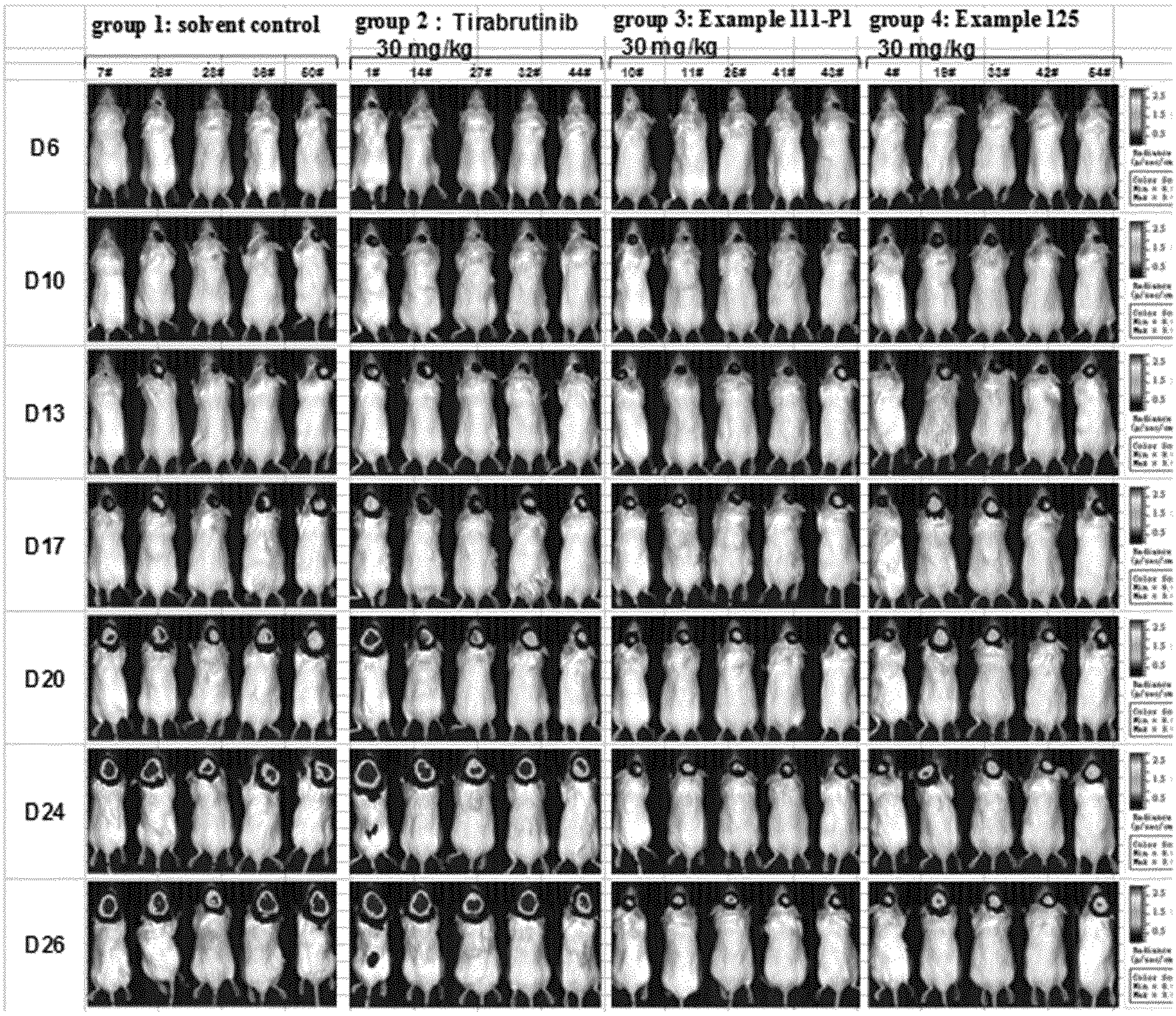
group 1: solvent control
group 2 : Tirabrutinib 30 mg/kg
group 3: Example 111-P1 30 mg/kg
group 4: Example 125 30 mg/kg
7# 26# 28# 36# 50#
1# 14# 27# 32# 44#
10# 11# 25# 41# 43#
4# 19# 33# 42# 54#
D6
D10
D13
D17
D20
D24
D26

FIG. 4

**INTERNATIONAL SEARCH REPORT**

International application No.
**PCT/CN2021/105961**

**A. CLASSIFICATION OF SUBJECT MATTER**

C07D 487/04(2006.01)i; C07D 519/00(2006.01)i; C07D 471/04(2006.01)i; A61K 31/4985(2006.01)i; A61K 31/437(2006.01)i; A61K 31/519(2006.01)i; A61P 19/02(2006.01)i; A61P 37/08(2006.01)i; A61P 37/06(2006.01)i; A61P 1/00(2006.01)i; A61P 1/04(2006.01)i; A61P 11/06(2006.01)i; A61P 17/00(2006.01)i; A61P 7/04(2006.01)i; A61P 25/28(2006.01)i; A61P 9/00(2006.01)i; A61P 11/00(2006.01)i; A61P 17/06(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D487/-, C07D519/-, C07D471/-, A61K31/-, A61P19/-, A61P37/-, A61P1/-, A61P11/-, A61P17/-, A61P7/-, A61P25/-, A61P9/-, A61P35/-

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, STN: 成都海博为药业有限公司, BTK, 布鲁顿酪氨酸, 蛋白激酶, 抑制剂, 四氢呋喃, 吡喃, 自身免疫, 炎性, 血栓栓塞, 过敏症, 感染, 增生, 癌症, bruton, protein kinase, inhibitor, tetrahydrofuran, pyran, autoimmune, inflammation, thromboembolism, allergy, infection, proliferation, cancer

**C. DOCUMENTS CONSIDERED TO BE RELEVANT**

| Category* | Citation of document, with indication, where appropriate, of the relevant passages | Relevant to claim No. |
|---|---|---|
| X | "CAS RN 344891-61-8" *STN Registry*, 08 July 2001 (2001-07-08), | 1 |
| PX | WO 2021136219 A1 (IZAL JIANGSU PHARMACEUTICAL CO., LTD.) 08 July 2021 (2021-07-08) description, page 36, method A | 11 |
| PX | WO 2020147798 A1 (MINGHUI PHARMACEUTICAL (SHANGHAI) LIMITED et al.) 23 July 2020 (2020-07-23) abstract, claim 1 and embodiment 30 | 2-11 |
| PX | WO 2020150681 A1 (LIAO XIBIN) 23 July 2020 (2020-07-23) abstract, claim 1 and compound 28 | 2-11 |
| X | WO 2016106652 A1 (MERCK SHARP & DOHME CORP. et al.) 07 July 2016 (2016-07-07) abstract, claims 1, 6-12, and description page 37 reaction formula, page 49 intermediate 8, pages 71-72 compounds 26-27 | 1-11 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 September 2021** | **12 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/105961**

**C. DOCUMENTS CONSIDERED TO BE RELEVANT**

| Category* | Citation of document, with indication, where appropriate, of the relevant passages | Relevant to claim No. |
|---|---|---|
| X | CN 102656173 A (PHARMACYCLICS, INC.) 05 September 2012 (2012-09-05)<br>abstract, claims 1 and 86 | 1-11 |
| X | CN 105814057 A (MERCK PATENT GMBH) 27 July 2016 (2016-07-27)<br>abstract, claim 1 and description paragraph [0148] compounds 2-6 | 1-11 |
| A | CN 109153680 A (DAEWOONG PHARMACEUTICAL CO., LTD.) 04 January 2019 (2019-01-04)<br>abstract, and claim 1 | 1-11 |
| A | CN 105017256 A (ZHEJIANG DAOMING PHARMACEUTICAL CO., LTD.) 04 November 2015 (2015-11-04)<br>abstract, and claim 1 | 1-11 |

Form PCT/ISA/210 (second sheet) (January 2015)

**INTERNATIONAL SEARCH REPORT**
**Information on patent family members**

International application No.
**PCT/CN2021/105961**

| Patent document cited in search report | Publication date (day/month/year) | Patent family member(s) | Publication date (day/month/year) |
|---|---|---|---|
| WO 2021136219 A1 | 08 July 2021 | None | |
| WO 2020147798 A1 | 23 July 2020 | CN 111454268 A | 28 July 2020 |
| WO 2020150681 A1 | 23 July 2020 | None | |
| WO 2016106652 A1 | 07 July 2016 | EP 3240545 A1 | 08 November 2017 |
| | | EP 3240545 A4 | 06 June 2018 |
| | | WO 2016109219 A1 | 07 July 2016 |
| | | US 2017362243 A1 | 21 December 2017 |
| CN 102656173 A | 05 September 2012 | IN 3012DEN2012 A | 31 July 2015 |
| | | BR 112012008624 A2 | 13 June 2017 |
| | | JP 5717109 B2 | 13 May 2015 |
| | | AU 2010306921 B2 | 13 August 2015 |
| | | ZA 201202552 B | 27 November 2013 |
| | | EP 2393816 A2 | 14 December 2011 |
| | | IL 219076 D0 | 28 June 2012 |
| | | EA 021715 B1 | 31 August 2015 |
| | | EP 2650294 B1 | 20 July 2016 |
| | | MX 2012004258 A | 29 May 2012 |
| | | EP 2393816 A4 | 14 December 2011 |
| | | ES 2590905 T8 | 09 December 2020 |
| | | US 2014080844 A1 | 20 March 2014 |
| | | WO 2011046964 A3 | 25 August 2011 |
| | | WO 2011046964 A2 | 21 April 2011 |
| | | CL 2012000917 A1 | 13 July 2012 |
| | | US 9012463 B2 | 21 April 2015 |
| | | CR 20120244 A | 03 January 2013 |
| | | JP 2013507448 A | 04 March 2013 |
| | | US 2011086866 A1 | 14 April 2011 |
| | | SG 10201506776 R A | 29 October 2015 |
| | | AU 2010306921 C1 | 17 March 2016 |
| | | CA 2776543 A1 | 21 April 2011 |
| | | JP 2015134802 A | 27 July 2015 |
| | | KR 20120097376 A | 03 September 2012 |
| | | IL 219076 A | 30 June 2016 |
| | | ES 2590905 T3 | 24 November 2016 |
| | | US 7741330 B1 | 22 June 2010 |
| | | AU 2010306921 A1 | 10 May 2012 |
| | | US 7718662 B1 | 18 May 2010 |
| | | CN 102656173 B | 16 March 2016 |
| | | EP 2650294 A1 | 16 October 2013 |
| | | NZ 599396 A | 28 March 2014 |
| | | CO 6531429 A2 | 28 September 2012 |
| | | EA 201270553 A1 | 28 September 2012 |
| CN 105814057 A | 27 July 2016 | EP 3049417 B1 | 17 October 2018 |
| | | CN 105814057 B | 03 May 2019 |
| | | CA 2918242 A1 | 05 February 2015 |
| | | BR 112016001600 A2 | 25 July 2017 |
| | | IL 243844 A | 31 October 2019 |
| | | JP 2016531119 A | 06 October 2016 |
| | | IL 243844 D0 | 21 April 2016 |

Form PCT/ISA/210 (patent family annex) (January 2015)

**INTERNATIONAL SEARCH REPORT**
**Information on patent family members**

International application No.
**PCT/CN2021/105961**

| Patent document cited in search report | Publication date (day/month/year) | Patent family member(s) | Publication date (day/month/year) |
|---|---|---|---|
| | | SG CN 10201900025U A | 27 February 2019 |
| | | RU 2712220 C2 | 27 January 2020 |
| | | SI 3049417 T1 | 29 March 2019 |
| | | RS 58361 B1 | 29 March 2019 |
| | | US 9738648 B2 | 22 August 2017 |
| | | AU 2014296261 B2 | 15 November 2018 |
| | | MX 2016001146 A | 29 April 2016 |
| | | RU 2016106760 A | 04 September 2017 |
| | | US 2016159804 A1 | 09 June 2016 |
| | | KR 20160036053 A | 01 April 2016 |
| | | JP 6612751 B2 | 27 November 2019 |
| | | DK 3049417 T3 | 14 January 2019 |
| | | PT 3049417 T | 24 January 2019 |
| | | LT 3049417 T | 11 February 2019 |
| | | ZA 201600564 B | 24 April 2019 |
| | | ES 2706150 T3 | 27 March 2019 |
| | | SG 11201600159 T A | 26 February 2016 |
| | | EP 3049417 A1 | 03 August 2016 |
| | | HU E042111 T2 | 28 June 2019 |
| | | BR 112016001600 A8 | 21 January 2020 |
| | | NZ 715838 A | 29 May 2020 |
| | | PL 3049417 T3 | 29 March 2019 |
| | | AU 2014296261 A1 | 11 February 2016 |
| | | HR P20190054 T1 | 22 February 2019 |
| | | MX 367085 B | 05 August 2019 |
| | | US 2017226113 A1 | 10 August 2017 |
| | | WO 2015017502 A1 | 05 February 2015 |
| | | US 10005784 B2 | 26 June 2018 |
| CN 109153680 A | 04 January 2019 | US 2019161488 A1 | 30 May 2019 |
| | | EP 3481831 A4 | 11 March 2020 |
| | | KR 20180006334 A | 17 January 2018 |
| | | US 10399988 B2 | 03 September 2019 |
| | | JP 2019524676 A | 05 September 2019 |
| | | JP 6726773 B2 | 22 July 2020 |
| | | CN 109153680 B | 23 April 2021 |
| | | EP 3481831 A1 | 15 May 2019 |
| | | WO 2018009017 A1 | 11 January 2018 |
| CN 105017256 A | 04 November 2015 | IL 248546 D0 | 29 December 2016 |
| | | AU 2015252654 A1 | 03 November 2016 |
| | | KR 20160144378 A | 16 December 2016 |
| | | EP 3138842 A1 | 08 March 2017 |
| | | BR 112016025132 A2 | 15 August 2017 |
| | | AU 2015252654 C1 | 23 January 2020 |
| | | EA 037227 B1 | 24 February 2021 |
| | | US 2018207161 A1 | 26 July 2018 |
| | | JP 2017518276 A | 06 July 2017 |
| | | EA 201892740 A2 | 30 April 2019 |
| | | JP 2020040989 A | 19 March 2020 |
| | | EA 201892740 A3 | 30 August 2019 |

Form PCT/ISA/210 (patent family annex) (January 2015)

**INTERNATIONAL SEARCH REPORT**
**Information on patent family members**

International application No.
**PCT/CN2021/105961**

| Patent document cited in search report | Publication date (day/month/year) | Patent family member(s) | | | Publication date (day/month/year) |
|---|---|---|---|---|---|
| | | EA | 201692176 | A1 | 31 March 2017 |
| | | HK | 1256857 | A1 | 04 October 2019 |
| | | EP | 3138842 | B1 | 09 December 2020 |
| | | WO | 2015165279 | A1 | 05 November 2015 |
| | | US | 2017065594 | A1 | 09 March 2017 |
| | | US | 9861636 | B2 | 09 January 2018 |
| | | MX | 2016014248 | A | 30 May 2017 |
| | | AU | 2015252654 | B2 | 03 October 2019 |
| | | CN | 106061976 | A | 26 October 2016 |
| | | CA | 2947338 | A1 | 05 November 2015 |
| | | CN | 108295073 | A | 20 July 2018 |
| | | CN | 106061976 | B | 15 May 2018 |
| | | JP | 6670756 | B2 | 25 March 2020 |
| | | EP | 3138842 | A4 | 27 September 2017 |
| | | CN | 108295073 | B | 16 June 2020 |
| | | US | 10300066 | B2 | 28 May 2019 |
| | | US | 9532990 | B2 | 03 January 2017 |
| | | EA | 032100 | B1 | 30 April 2019 |
| | | US | 2016200730 | A1 | 14 July 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010679776
- CN 202011337022


### Non-patent literature cited in the description


- A-22 was synthesized with reference to the literature. *Journal of Medicinal Chemistry,* 2020, vol. 63 (10), 5102-5118
- *Angew. Chem. Int. Ed.,* 2020, vol. 59, 7161-7167
- *CHEMICAL ABSTRACTS,* 652-67-5